(19) Europäisches Patentamt / European Patent Office / Office européen des brevets

(11) EP 3 251 698 A1

(12) **EUROPEAN PATENT APPLICATION**
published in accordance with Art. 153(4) EPC

(43) Date of publication:
**06.12.2017 Bulletin 2017/49**

(21) Application number: **16748591.1**

(22) Date of filing: **26.01.2016**

(51) Int Cl.:
***A61K 47/69*** *(2017.01)* ***C07K 16/30*** *(2006.01)*
***A61P 35/00*** *(2006.01)*

(86) International application number:
**PCT/CN2016/072129**

(87) International publication number:
**WO 2016/127790 (18.08.2016 Gazette 2016/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.02.2015 CN 201510083765**

(71) Applicants:
- **Jiangsu Hengrui Medicine Co. Ltd.**
  **Jiangsu 222047 (CN)**
- **Shanghai Hengrui Pharmaceutical Co. Ltd.**
  **Minhang District**
  **Shanghai 200245 (CN)**

(72) Inventors:
- **XU, Jianyan**
  **Shanghai 200245 (CN)**
- **ZHANG, Ying**
  **Shanghai 200245 (CN)**
- **QU, Bolei**
  **Shanghai 200245 (CN)**
- **ZHANG, Fuyao**
  **Shanghai 200245 (CN)**
- **YU, Xiuzhao**
  **Shanghai 200245 (CN)**
- **LIANG, Jindong**
  **Shanghai 200245 (CN)**
- **JIANG, Guiyang**
  **Shanghai 200245 (CN)**
- **ZHANG, Lianshan**
  **Shanghai 200245 (CN)**
- **LI, Ang**
  **Shanghai 200245 (CN)**
- **WANG, Yali**
  **Shanghai 200245 (CN)**

(74) Representative: **Regimbeau**
**20, rue de Chazelles**
**75847 Paris Cedex 17 (FR)**

# (54) LIGAND-CYTOTOXICITY DRUG CONJUGATE, PREPARING METHOD THEREFOR, AND APPLICATION THEREOF

(57) Provide are a ligand-cytotoxicity drug conjugate with a general formula of PC-L-Dr, a preparing method thereof, and applications of the ligand-cytotoxicity drug conjugate and pharmaceutical compositions comprising the same in preparing drugs for treating cancers by means of receptor regulation.

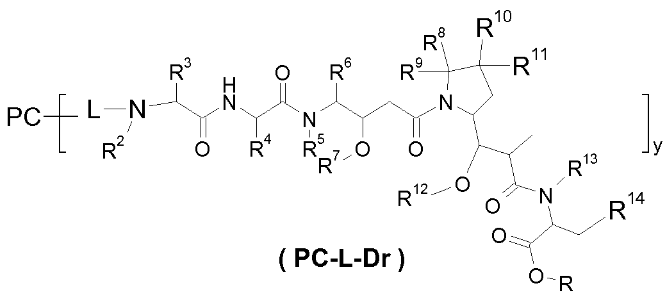

**( PC-L-Dr )**

EP 3 251 698 A1

## Description

### FIELD OF THE INVENTION

The present disclosure relates to a new kind of ligand-cytotoxic drug conjugate, specifically relates to a ligand-cytotoxic drug conjugate, a preparing method thereof, a pharmaceutical composition comprising the same and the use of the ligand-cytotoxic drug conjugate or the pharmaceutical composition .

### BACKGROUND OF THE INVENTION

Chemotherapy remains one of the most important anti-cancer means including surgery, radiotherapy, and targeted therapy. Although there are many types of highly efficient cytotoxins , the poor discriminability between tumor and healthy cells by these agents limits their broader application in clinical due to the toxic side effects. Therapeutic antibodies have emerged as an important class of biological anticancer agents because of their ability in specific binding to tumor-associated antigens. While this important class of biologics can be used as single agents for the treatment of cancer, their therapeutical efficacy is often limited.

Antibody drug conjugates (ADCs) combines the monoclonal antibody or antibody fragment with a biologically active cytotoxin through a chemical stable linker, taking full advantage of the specificity of the antibody binding to the surface antigens of normal cell and tumor cell and the high efficiency of the cytotoxin, while avoiding low efficacy of the antibody and the toxic side effects of the cytotoxin. Therefore, compared with the traditional chemotherapy drugs, antibody drug conjugates could accurately recognize tumor cells and reduce the damage on normal cells.

Earlier ADCs mainly use mouse monoclonal antibodies, and so some of the drugs were difficult to reach the target due to the human immune response. Secondly, the effector molecules on the early application such as doxorubicin exhibit low biological activity, limiting the efficiency of the first generation antibody drug conjugates. In addition, the source of the antibody, the way and the number of the linker connections were not optimized.

Kadcyla® (ado-trastuzumab emtansine, T-DM1) was approved by U.S. FDA in February 2013 for the treatment of HER2-positive advanced or metastatic breast cancer patients resistant to trastuzumab (Trastuzumab, trade name: Herceptin®) and paclitaxel. Mylotarg® and Adcetris® are used in target therapy aganist blood tumors, whose tissue structure is relatively simple compared to the solid tumors. Kadcyla® is the first ADC drug approved by FDA for the treatment of solid tumors.

Kadcyla® links the highly active mitotic inhibitor DM1 to Roche's Trastuzumab with a stable thioether linker using ImmunoGen's technology, with an average drug antibody ratio of about 3.5. Trastuzumab specifically binds to breast cancer cells in the patient. After endocytosis, the DM1 is released, and the concentration of DM1 in the cells is sufficient to kill the cells due to the mitotic catastrophe. T-DM1 retains the antibody-dependent cell proliferation inhibition effect of Herceptin®, while increasing the potential effect of cytotoxic drugs. Because its toxin is released inside the tumor cells, so it's side effect does not increase as the curative effect increases.

Pertuzumab (also known as 2C4, trade name Perjeta) is a recombinant humanized monoclonal antibody, which is the first monoclonal antibody known as the "HER dimerization inhibitor". Pertuzumab blocks the dimerization of HER2 with other HER receptors by binding to HER2. Pertuzumab has been shown to inhibit the tumor growth in the prostate cancer with HER2 overexpression or low expression.

Unlike Trastuzumab (trade name Herceptin), which inhibits the downstream signaling pathway by binding to the sites located in the HER2 extracellular domain of the proximal region IV sub-domain, Pertuzumab can inhibit HER2 heterodimerization effectively by binding to the II domain (dimerization Domain). Thus, Trastuzumab can only have a certain effect on patients with HER2 overexpressing cancer, especially on patients with breast cancer, while Pertuzumab, having the same target and endocytosis as Trastuzumab, for their different action mechanism, may has a wider range of applications than those drugs blocking HER2 signaling pathway because Pertuzumab can cut off the signal pathway mediated by the ErbB family receptors after inhibiting the dimerization.

At present, there are mainly two ADC drug coupling methods: one method used in T-DM1 is the random conjugation between cytotoxic drugs and the free amino groups of antibodies; and the other method used in Adcetris® is the conjugation between cytotoxic drugs and the free thiols resulted from the reduction of the antibody hinge area. Both coupling methods result in a mixture with the inconsistent drug loading. For instance, although the average drug loading of T-DM1 is 3.5, but the distribution of drug loading ranges from 0 to 8. A low drug loading can affect the efficacy of the ADC, while a high drug loading might cause the ADC drug being recognized and destroyed by the tissue macrophage system. This not only reduces the half-life of the ADC, but also increases the toxic side effects due to accumulation of the drug in the non-target tissue. In Adcetris®, a reducing agent was used to reduce the disulfide bond in the antibody hinge region, which might affect the stability of the antibody itself. Related ADCs are disclosed in WO2007008603, WO2013173393, WO2005081711, WO2013173391, WO2013173392, WO2013173393 and WO2012010287. The present invention provides a novel ADC compound which has a novel coupling way and a novel combination of toxin

and antibody, and thus has more beneficial effects.

## SUMMARY OF THE INVENTION

In order to improve the ligand, especially the coupling effect between antibody and drug, the present invention provides a compound of formula (PC-L-Dr) comprising an improved linker or a pharmaceutically acceptable salt or solvate thereof:

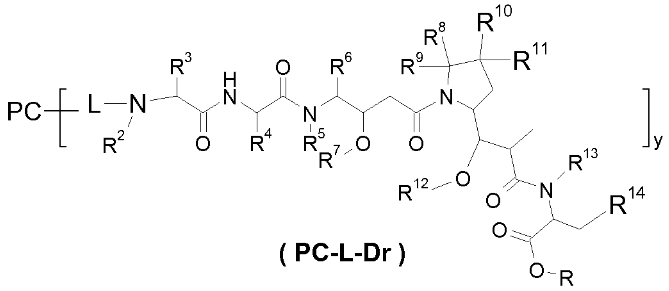

**( PC-L-Dr )**

wherein:

- R, $R^2$-$R^7$ are each selected from the group consisting of hydrogen, halogen, hydroxyl, cyano, alkyl, alkoxy and cycloalkyl ;
- at least one of $R^8$-$R^{11}$ is selected from the group consisting of halogen, alkenyl, alkyl and cycloalkyl, and the rest of $R^8$-$R^{11}$ are hydrogen;
- or any two of $R^8$-$R^{11}$ are attached to form a cycloalkyl, the rest two are each selected from the group consisting of hydrogen, alkyl and cycloalkyl;
- $R^{12}$-$R^{13}$ are each selected from the group consisting of hydrogen, alkyl and halogen;
- $R^{14}$ is selected from aryl or heteroaryl,wherein the aryl or heteroaryl is optionally substituted with one or more groups selected from the group consisting of hydrogen, halogen, hydroxy, alkyl, alkoxy and cycloalkyl;
- y is 1-8, preferably 2-5; y is a positive real number which can be an integral number or a decimal number;
- PC is a ligand; L is a linker.

In a preferred embodiment of the present invention, a compound of formula (PC-L-Dr) or a pharmaceutically acceptable salt or solvate thereof, is a compound of formula (PC-L-D) or a pharmaceutically acceptable salt or solvate thereof:

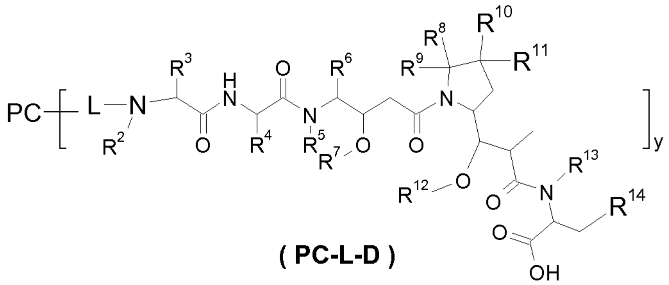

**( PC-L-D )**

wherein, $R^2$-$R^{14}$ are as defined as formula (PC-L-Dr).

In another preferred embodiment of the present invention, a compound of formula (PC-L-Dr) or a pharmaceutically acceptable salt or solvate thereof, is a compound of formula (PC-L'-Dr) or a pharmaceutically acceptable salt or solvate thereof:

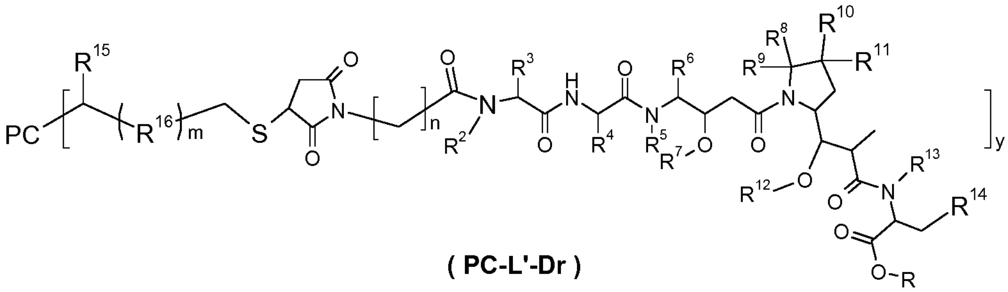

**( PC-L'-Dr )**

wherein:

$R^{15}$ is selected from the group consisting of hydrogen, halogen, hydroxyl, cyano, alkyl, alkoxy and cycloalkyl;
$R^{16}$ is selected from the group consisting of alkyl, cycloalkyl, alkoxy and heterocyclyl;
n is 2-6, preferably2-5;
m is 0-5, preferably 1-3;
PC, y, n, R, $R^2$-$R^{14}$ are as defined in formula(PC-L-Dr).

In another preferred embodiment of the present invention, a compound of formula (PC-L-Dr) or a pharmaceutically acceptable salt or solvate thereof, is a compound of formula (PC-L'-D) or a pharmaceutically acceptable salt or solvate thereof:

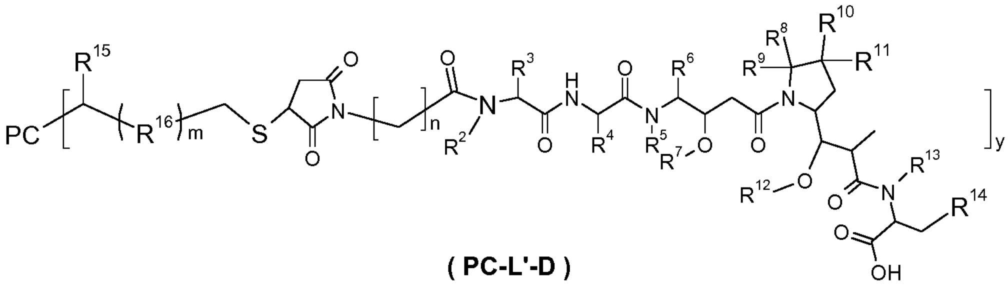

**( PC-L'-D )**

wherein:

$R^{15}$, $R^{16}$, m are as defined in formula (PC-L'-Dr);
PC, y, n, $R^2$-$R^{14}$ are as defined in formula (PC-L-Dr).

In another preferred embodiment of the present invention, a compound of formula (PC-L'-D) or a pharmaceutically acceptable salt or solvate thereof, is a compound of formula (PC-L'-D1) or a pharmaceutically acceptable salt or solvate thereof:

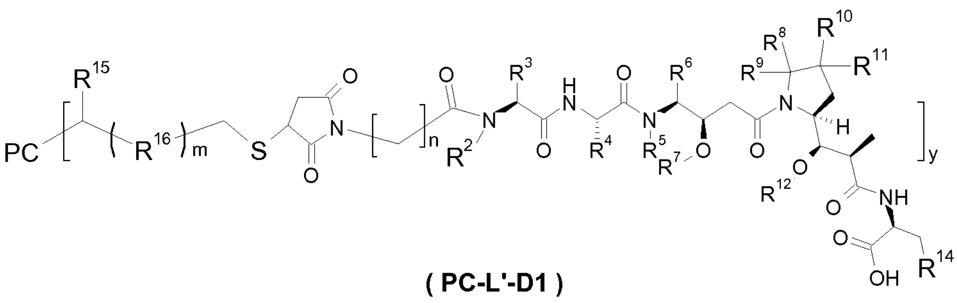

**( PC-L'-D1 )**

;

wherein PC, y, n, m, $R^2$-$R^{16}$ are as defined in formula (PC-L'-D).

The ligand-cytotoxic drug conjugates of the present invention include, but are not limited to:

| Structure | compound (D) / (L1-D) example |
|---|---|
| 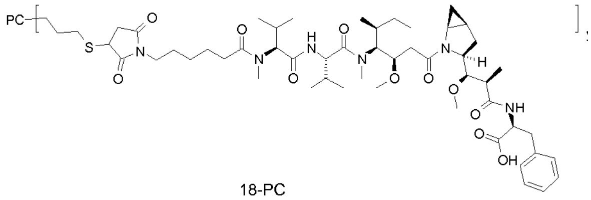 18-PC | 1/4 |
| 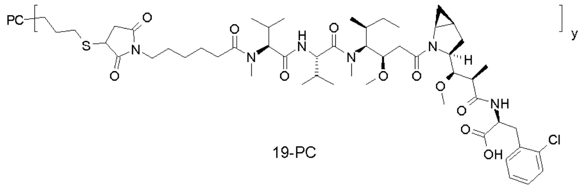 19-PC | 2/5 |
| 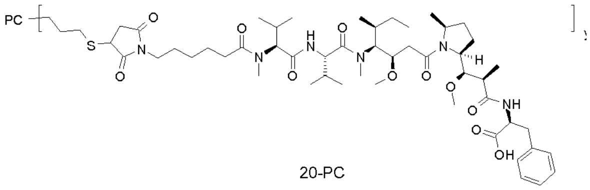 20-PC | 3/6 |
| 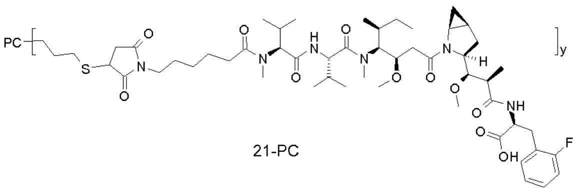 21-PC | 7/8 |
| 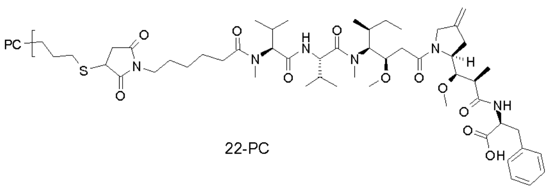 22-PC | 9/10 |
| 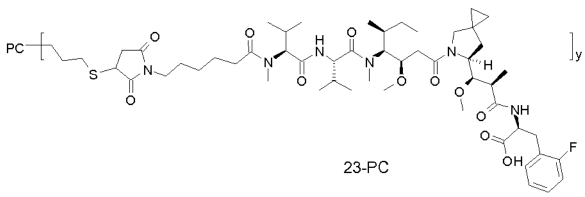 23-PC | 11/12 |
| 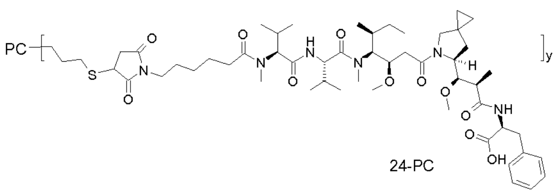 24-PC | 13/14 |
| 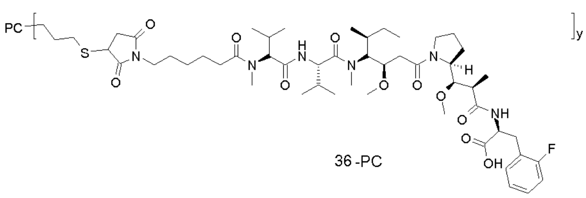 36 -PC | 25/26 |

(continued)

| Structure | compound (D) / (L1-D) example |
|---|---|
| 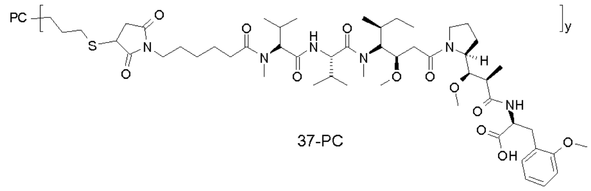 37-PC | 27/28 |
| 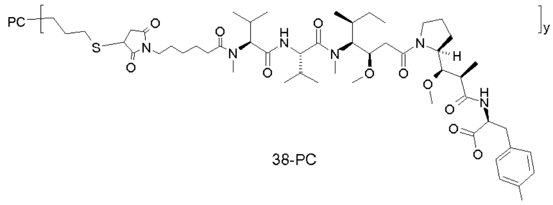 38-PC | 29/30 |
| 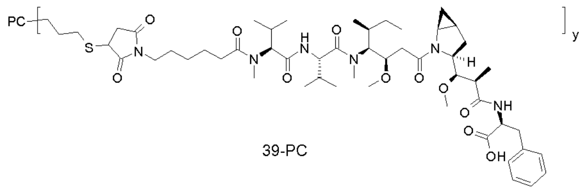 39-PC | 1/4 |
| 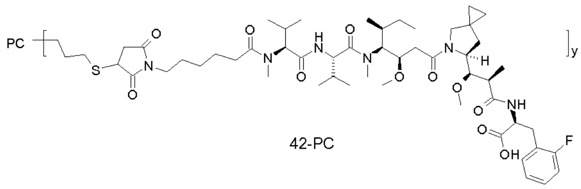 42-PC | 11/12 |
| 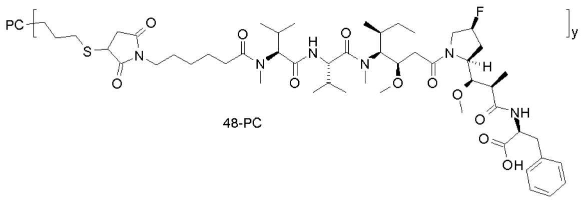 48-PC | 44/45 |
| 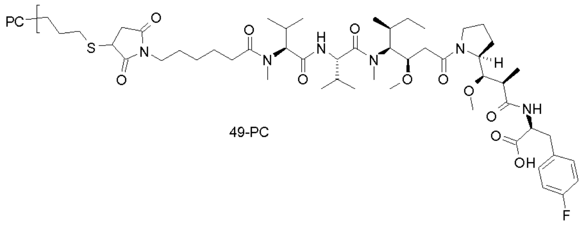 49-PC | 46/47 |

or a pharmaceutically acceptable salt or solvate thereof.

In another preferred embodiment of the present invention, a compound of formula (PC-L-Dr), wherein PC is antibody, preferably selected from Pertuzumab, Nimotuzumab and Trastuzumab.

The typical ligand-cytotoxic drug conjugates of the present invention include, but are not limited to:

| Example | Structure | compound (D)/(L1-D) Example |
|---|---|---|
| 18 | 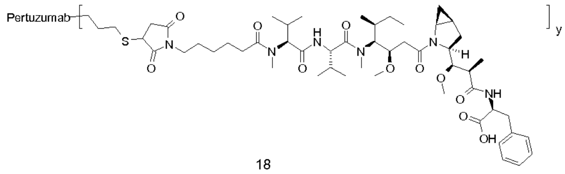 18 | 1/4 |
| 19 | 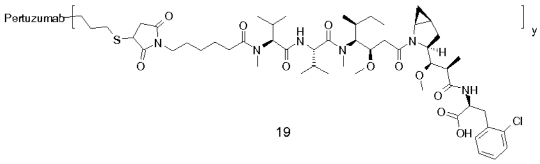 19 | 2/5 |
| 20 | 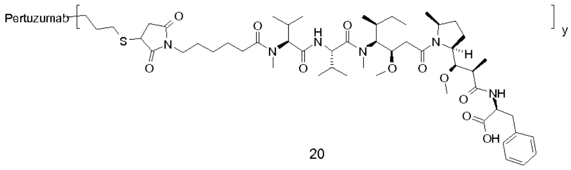 20 | 3/6 |
| 21 | 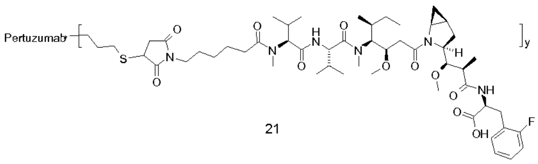 21 | 7/8 |
| 22 | 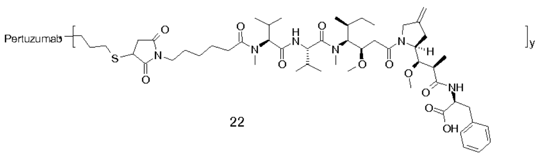 22 | 9/10 |
| 23 | 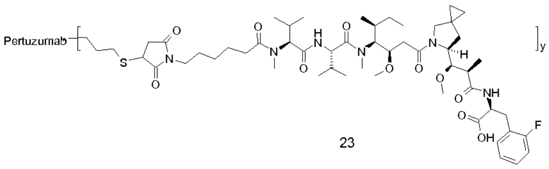 23 | 11/12 |
| 24 | 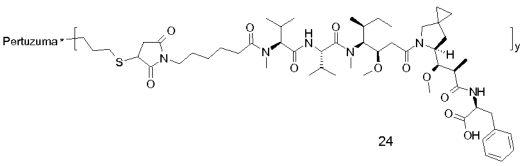 24 | 13/14 |
| 36 | 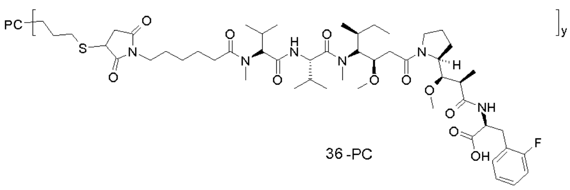 36 -PC | 25/26 |
| 37 | 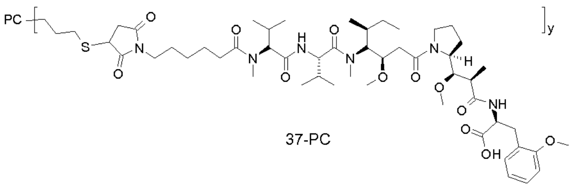 37-PC | 27/28 |

(continued)

| Example | Structure | compound (D)/(L1-D) Example |
|---|---|---|
| 38 | 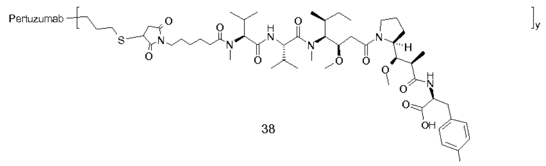 38 | 29/30 |
| 39 | 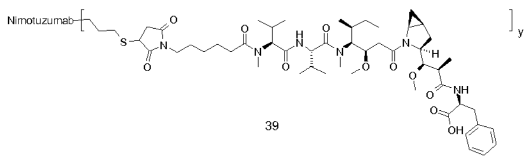 39 | 1/4 |
| 40 | 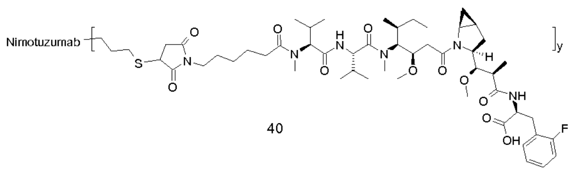 40 | 7/8 |
| 41 | 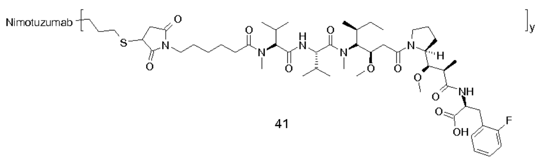 41 | 25/26 |
| 42 | 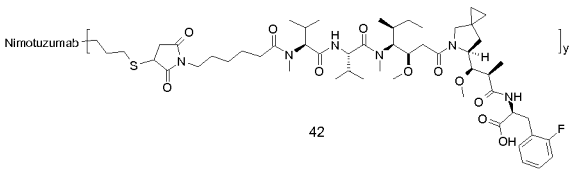 42 | 11/12 |
| 43 | 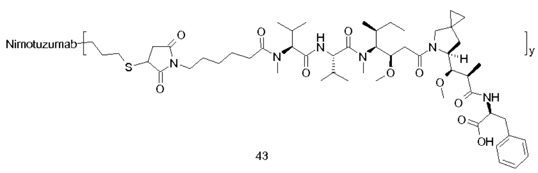 43 | 13/14 |
| 48 | 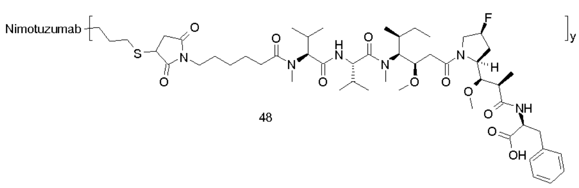 48 | 44/45 |
| 49 | 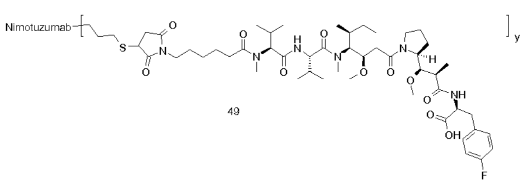 49 | 46/47 |

(continued)

| Example | Structure | compound (D)/(L1-D) Example |
|---|---|---|
| 50 | 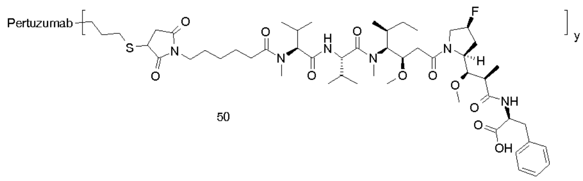 50 | 44/45 |
| 51 | 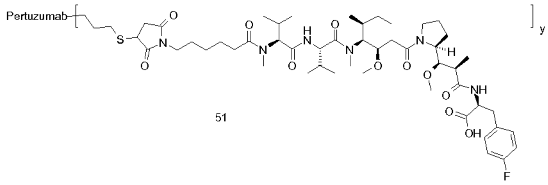 51 | 46/47 |
| 52 | 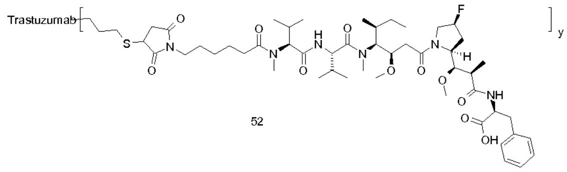 52 | 44/45 |
| 53 | 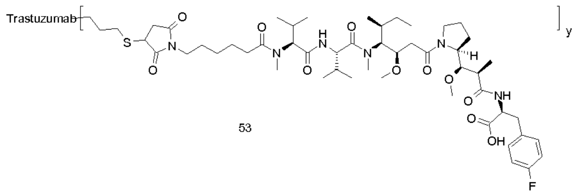 53 | 46/47 |

or a pharmaceutically acceptable salt or solvate thereof.

Another aspect of this invention is directed to a compound of formula (Dr):

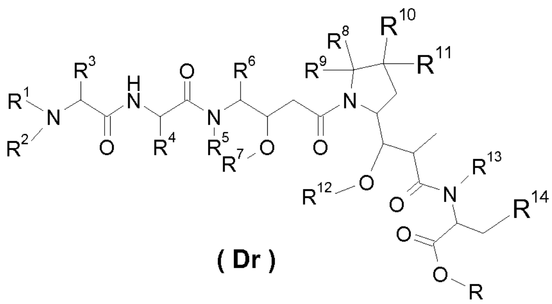

( Dr )

or a tautomer, mesomer, racemate, enantiomer, diastereomer, or mixtures thereof, or pharmaceutically acceptable salts thereof, which can be used as the intermidate in the preparation of the compound of formula(PC-L-Dr):

wherein:

R, $R^1$-$R^7$ is each selected from the group consisting of hydrogen, halogen, hydroxyl,cyano, alkyl, alkoxy and cycloalkyl;

at least one of $R^8$-$R^{11}$ is selected from the group consisting of halogen, alkenyl, alkyl and cycloalkyl, and the rest of $R^8$-$R^{11}$ are hydrogen;

or any two of $R^8$-$R^{11}$ are attached to form a cycloalkyl, the rest two are each selected from the group consisting of hydrogen, alkyl and cycloalkyl;

$R^{12}$-$R^{13}$ are each selected from the group consisting of hydrogen, alkyl and halogen,

$R^{14}$ is selected from aryl and heteroaryl,wherein the aryl and heteroaryl are optionally further substituted with

one or more groups selected from the group consisting of hydrogen, halogen, hydroxy, alkyl, alkoxy and cycloalkyl.

In another preferred embodiment of the present invention, a compound of formula (Dr), is a compound of formula (D) or a pharmaceutically acceptable salt or solvate thereof:

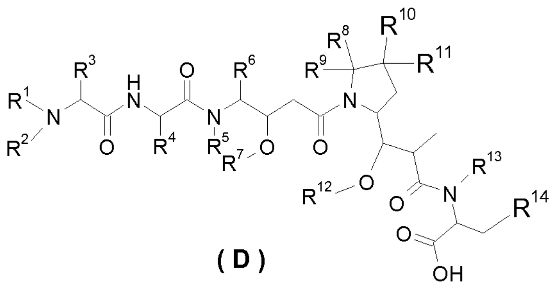

**( D )**

or a tautomer, mesomer, racemate, enantiomer, diastereomer, or mixtures thereof, or pharmaceutically acceptable salts thereof, wherein $R^1$-$R^{14}$ are as defined in formula(Dr).

In another preferred embodiment of the present invention, a compound of formula (Dr) is a compound of formula (D1):

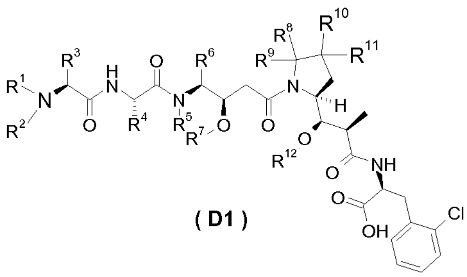

**( D1 )**

or a tautomer, mesomer, racemate, enantiomer, diastereomer, or mixtures thereof, or pharmaceutically acceptable salts thereof, wherein $R^1$-$R^{14}$ are as defined in formula(D).

The typical compounds for the preparation of ligand-cytotoxic drug conjugate of the present invention include, but are not limited to:

| Example | Structure and Name |
|---|---|
| 1 | 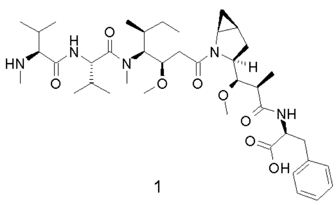 1 |
| | (*S*)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino) butanamido)butanamido)-3-methoxy-5-methyl heptanoyl)-2-azabicyclo [3.1.0]hexan-3-yl)-3-methoxy-2-methylpropanamido)-3-phenylpropanoi c acid |
| 2 | 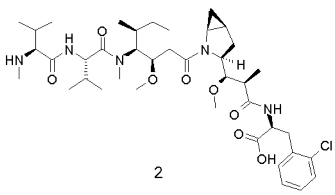 2 |

(continued)

| Example | Structure and Name |
|---|---|
| | (*S*)-3-(2-chlorophenyl)-2-((2*R*,3*R*)-3-((1*S*,3*S*,5*S*)-2-((3*R*,4*S*,5*S*)-4-((*S*)-*N*, 3-dimethyl-2-((*S*)-3-methyl-2-(methylamino)butanamido)butanamido)-3 -methoxy-5-methylheptanoyl)-2-azabicyclo[3.1.0]hexan-3-yl)-3-methox y-2-methylpropanamido)propanoic acid |
| 3 | 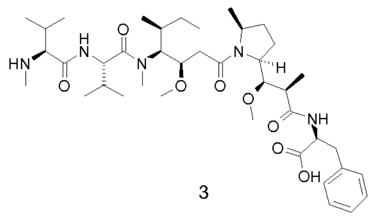 3 |
| | (*S*)-2-((2*R*,3*R*)-3-((2*S*,5*S*)-1-((3*R*,4*S*,5*S*)-4-((*S*)-*N*,3-dimethyl-2-((*S*)-3-m ethyl-2-(methylamino) butanamido)butanamido)-3-methoxy-5-methylhe ptanoyl)-5-methylpyrrolidin-2-yl)-3-methoxy-2-methylpropanamido)-3-phenylpropanoic acid |
| 7 | 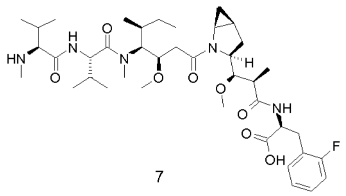 7 |
| | (*S*)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino) butanamido)butanamido)-3-methoxy-5-methyl heptanoyl)-2-azabicyclo[3.1.0]hexan-3-yl)-3-methoxy-2-methylpropana mido)-3-(2-fluorophenyl)propanoic acid |
| 9 | 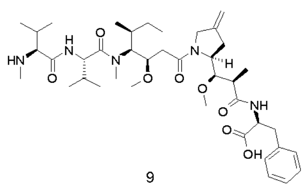 9 |
| | (*S*)-2-((2*R*,3*R*)-3-((*S*)-1-((3*R*,4*S*,5*S*)-4-((*S*)-*N*,3-dimethyl-2-((*S*)-3-methyl -2-(methylamino)butanamido) butanamido)-3-methoxy-5-methylheptano yl)-4-methylenepyrrolidin-2-yl)-3-methoxy-2-methylpropanamido)-3-ph enylpropanoic acid |
| 11 | 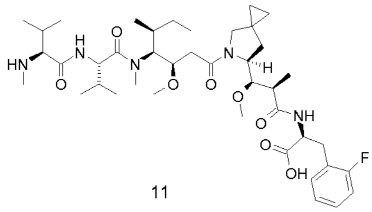 11 |
| | (*S*)-2-((2*R*,3*R*)-3-((*S*)-5-((3*R*,4*S*,5*S*)-4-((*S*)-*N*,3-dimethyl-2-((*S*)-3-methyl -2-(methylamino)butanamido) butanamido)-3-methoxy-5-methylheptano yl)-5-azaspiro[2.4]heptan-6-yl)-3-methoxy-2-methylpropanamido)-3-(2-fluorophenyl)propanoic acid |

(continued)

| Example | Structure and Name |
|---|---|
| 13 | 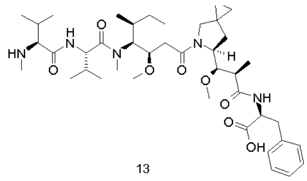 13 |
| | (*S*)-2-((2*R*,3*R*)-3-((*S*)-5-((3*R*,4*S*,5*S*)-4-((*S*)-*N*,3-dimethyl-2-((S)-3-methy 1-2-(methylamino) butanamido)butanamido)-3-methoxy-5-methylheptan oyl)-5-azaspiro[2.4]heptan-6-yl)-3-methoxy-2-methylpropanamido)-3-p henylpropanoic acid |
| 15 | 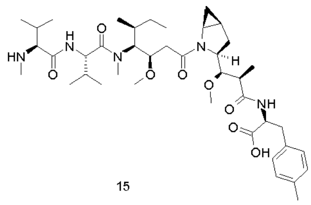 15 |
| | (*S*)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino) butanamido)butanamido)-3-methoxy-5-methyl heptanoyl)-2-azabicyclo[3.1.0]hexan-3-yl)-3-methoxy-2-methylpropana mido)-3-(p-tolyl)propanoic acid |
| 16 | 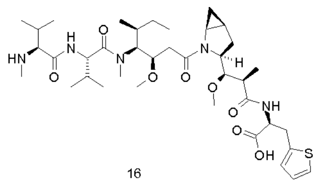 16 |
| | (*S*)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino) butanamido)butanamido)-3-methoxy-5-methyl heptanoyl)-2-azabicyclo[3.1.0]hexan-3-yl)-3-methoxy-2-methylpropana mido)-3-(thiophen-2-yl)propanoic acid |
| 17 | 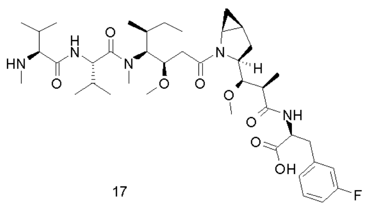 17 |
| | (*S*)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino) butanamido)butanamido)-3-methoxy-5-methyl heptanoyl)-2-azabicyclo[3.1.0]hexan-3-yl)-3-methoxy-2-methylpropana mido)-3-(3-fluorophenyl)propanoic acid |
| 25 | 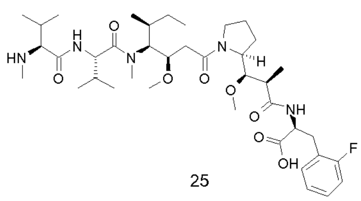 25 |
| | (*S*)-2-((2*R*,3*R*)-3-((*S*)-1-((3*R*,4*S*,5*S*)-4-((*S*)-*N*,3-dimethyl-2-((*S*)-3-methyl -2-(methylamino)butanamido) butanamido)-3-methoxy-5-methylheptano |
| | yl)pyrrolidin-2-yl)-3-methoxy-2-methylpropanamido)-3-(2-fluorophenyl )propanoic acid |

(continued)

| Example | Structure and Name |
|---|---|
| 27 | 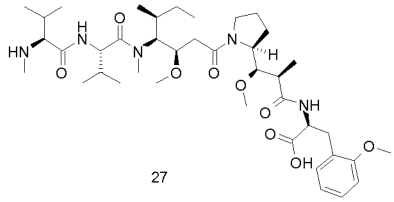 27 |
| | (*S*)-2-((2*R*,3*R*)-3-((*S*)-1-((3*R*,4*S*,5*S*)-4-((*S*)-*N*,3-dimethyl-2-((*S*)-3-methyl -2-(methylamino)butanamido) butanamido)-3-methoxy-5-methylheptano yl)pyrrolidin-2-yl)-3-methoxy-2-methylpropanamido)-3-(2-methoxyphe nyl)propanoic acid |
| 29 | 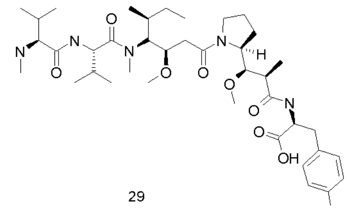 29 |
| | (*S*)-2-((2*R*,3*R*)-3-((*S*)-1-((3*R*,4*R*,5*S*)-4-((*S*)-*N*,3-dimethyl-2-((*S*)-3-methy 1-2-(methylamino) butanamido)butanamido)-3-methoxy-5-methylheptan oyl)pyrrolidin-2-yl)-3-methoxy-2-methylpropanamido)-3-(p-tolyl)propa noic acid |
| 31 | 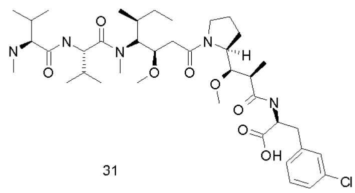 31 |
| | (*S*)-3-(3-chlorophenyl)-2-((2*R*,3*R*)-3-((*S*)-1-((3*R*,4*R*,5*S*)-4-((*S*)-*N*,3-dime thyl-2-((*S*)-3-methyl-2-(methylamino)butanamido)butanamido)-3-metho xy-5-methylheptanoyl)pyrrolidin-2-yl)-3-methoxy-2-methylpropanamid o)propanoic acid |
| 32 | 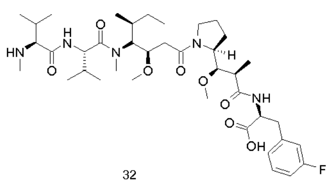 32 |
| | (*S*)-2-((2*R*,3*R*)-3-((*S*)-1-((3*R*,4*R*,5*S*)-4-((*S*)-*N*,3-dimethyl-2-((*S*)-3-methy 1-2-(methylamino) butanamido)butanamido)-3-methoxy-5-methylheptan oyl)pyrrolidin-2-yl)-3-methoxy-2-methylpropanamido)-3-(3-fluorophen yl)propanoic acid |
| 33 | 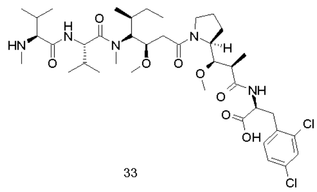 33 |
| | (*S*)-3-(2,4-dichlorophenyl)-2-((2*R*,3*R*)-3-((*S*)-1-((3*R*,4*R*,5*S*)-4-((*S*)-*N*,3-d imethyl-2-((*S*)-3-methyl-2-(methylamino)butanamido)butanamido)-3-m ethoxy-5-methylheptanoyl)pyrrolidin-2-yl)-3-methoxy-2-methylpropana mido)propanoic acid |

(continued)

| Example | Structure and Name |
|---|---|
| 34 | 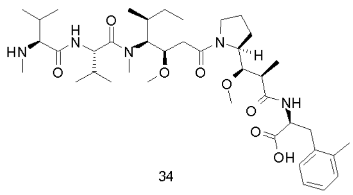 34 |
| | (*S*)-2-((2*R*,3*R*)-3-((*S*)-1-((3*R*,4*S*,5*S*)-4-((*S*)-*N*,3-dimethyl-2-((*S*)-3-methyl -2-(methylamino)butanamido) butanamido)-3-methoxy-5-methylheptano yl)pyrrolidin-2-yl)-3-methoxy-2-methylpropanamido)-3-(o-tolyl)propan oic acid |
| 35 | 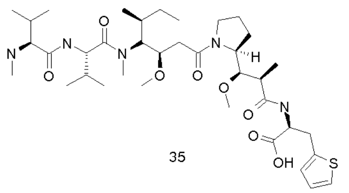 35 |
| | (*S*)-2-((2*R*,3*R*)-3-((*S*)-1-((3*R*,4*R*,5*S*)-4-((*S*)-*N*,3-dimethyl-2-((*S*)-3-methy 1-2-(methylamino) butanamido)butanamido)-3-methoxy-5-methylheptan oyl)pyrrolidin-2-yl)-3-methoxy-2-methylpropanamido)-3-(thiophen-2-yl )propanoic acid |
| 44 | 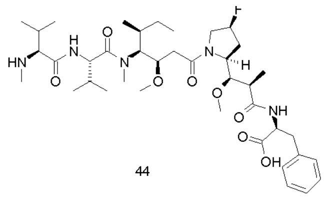 44 |
| | (*S*)-2-((2*R*,3*R*)-3-((2*S*,4*S*)-1-((3*R*,4*S*,5*S*)-4-((*S*)-*N*,3-dimethyl-2-((*S*)-3-m ethyl-2-(methylamino) butanamido)butanamido)-3-methoxy-5-methylhe ptanoyl)-4-fluoropyrrolidin-2-yl)-3-methoxy-2-methylpropanamido)-3-p henylpropanoic acid |
| 46 | 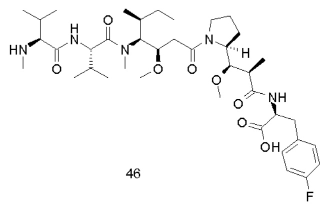 46 |
| | (*S*)-2-((2*R*,3*R*)-3-((*S*)-1-((3*R*,4*S*,5*S*)-4-((*S*)-*N*,3-dimethyl-2-((*S*)-3-methyl -2-(methylamino)butanamido) butanamido)-3-methoxy-5-methylheptano yl)pyrrolidin-2-yl)-3-methoxy-2-methylpropanamido)-3-(4-fluorophenyl )propanoic acid |

Another aspect of the present invention is directed to a compound of formula (L1-Dr):

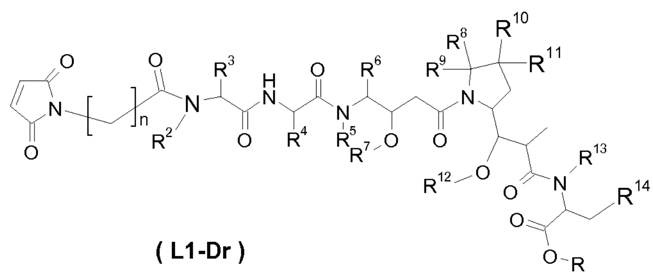

**( L1-Dr )**

which can be used as the intermediatein the preparation of the compound of formula (PC-L'-D):

wherein:

n is 2-6, preferably 2-5;
R, $R^1$-$R^7$ is each selected from the group consisting of hydrogen, halogen, hydroxyl, cyano, alkyl, alkoxy and cycloalkyl;
at least one of $R^8$-$R^{11}$ is selected from the group consisting of halogen, alkenyl, alkyl and cycloalkyl, and the rest of $R^8$-$R^{11}$ are hydrogen;
or any two of $R^8$-$R^{11}$ are attached to form a cycloalkyl, the rest two are each selected from the group consisting of hydrogen, alkyl and cycloalkyl;
$R^{12}$-$R^{13}$ are each selected from the group consisting of hydrogen, alkyl and halogen,
$R^{14}$ is selected from aryl and heteroaryl, wherein the aryl or heteroaryl are optionally further substituted with one or more groups selected from the group consisting of hydrogen, halogen, hydroxy, alkyl, alkoxy and cycloalkyl.

In another preferred embodiment of the present invention, a compound of formula ($L_1$-Dr) is a compound of formula ($L_1$-D):

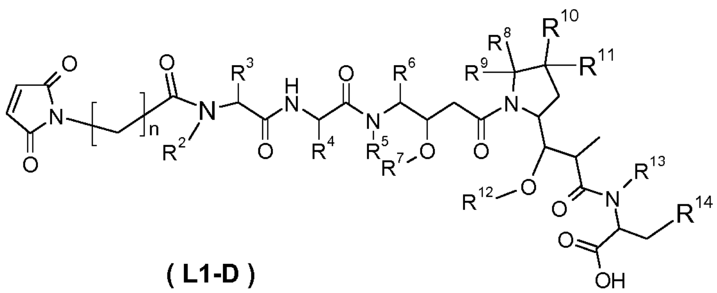

( L1-D )

wherein n, $R^2$-$R^{14}$ are as defined in formula ($L_1$-Dr).
In another preferred embodiment of the present invention, a compound of formula ($L_1$-D), is a compound of formula ($L_1$-D1):

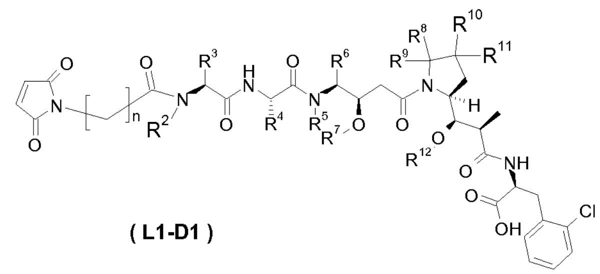

( L1-D1 )

wherein n, $R^1$-$R^{12}$ are as defined in formula($L_1$-D).
The typical pro-drugs (with the linker) and the intermediates for the preparation of ligand-cytotoxic drug conjugate of the present invention include, but are not limited to:

| Example | Structure and Name |
|---|---|
| 4 | 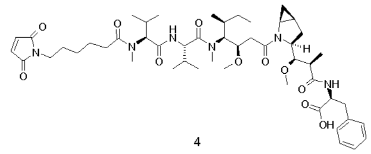<br>4<br>(*S*)-2-((2*R*,3*R*)-3-((1*S*,3*S*,5*S*)-2-((3*R*,4*S*,5S)-4-((*S*)-2-((*S*)-2-(6-(2,5-dioxo-2,5 -dihydro-1*H*-pyrrol-1-yl)-*N*-methylhexanamido)-3-methylbutanamido)-*N*,3-dimethylbutanamido)-3-methoxy-5-methylheptanoyl)-2-azabicyclo[3.1.0]he xan-3-yl)-3-methoxy-2-methylpropanamido)-3-phenylpropanoic acid |
| 5 | 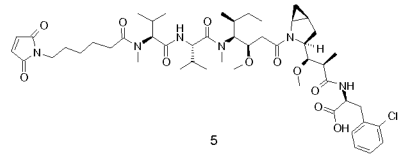<br>5<br>(*S*)-3-(2-chlorophenyl)-2-((2*R*,3*R*)-3-((1*S*,3*S*,5*S*)-2-((3*R*,4S,5*S*)-4-((*S*)-2-((*S*) -2-(6-(2,*S*-dioxo-2,*S*-dihydro-1*H*-pyrrol-1-yl)-*N*-methylhexanamido)-3-meth ylbutanamido)-*N*,3-dimethylbutanamido)-3-methoxy-5-methylheptanoyl)-2-azabicyclo[3.1.0]hexan-3-yl)-3-methoxy-2-methylpropanamido) propanoic acid |
| 6 | 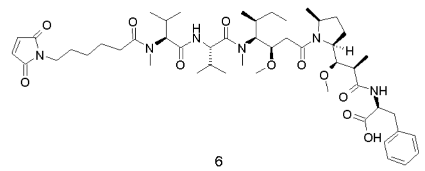<br>6<br>(*S*)-2-((2*R*,3*R*)-3-((2*S*,5*S*)-1-((3*R*,4*S*,5*S*)-4-((*S*)-2-((*S*)-2-(6-(2,5-dioxo-2,5-di hydro-1*H*-pyrrol-1-yl)-*N*-methylhexanamido)-3-methylbutanamido)-*N*,3-di methylbutanamido)-3-methoxy-5-methylheptanoyl)-5-methylpyrrolidin-2-yl )-3-methoxy-2-methylpropanamido)-3-phenylpropanoic acid |
| 8 | 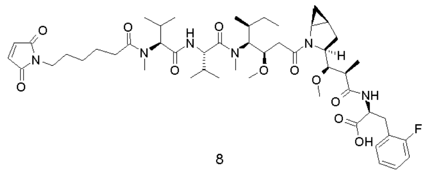<br>8<br>(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5 -dihydro-1*H*-pyrrol-1-yl)-*N*-methylhexanamido)-3-methylbutanamido)-*N*,3-dimethylbutanamido)-3-methoxy-5-methylheptanoyl)-2-azabicyclo[3 .1.0]he xan-3-yl)-3-methoxy-2-methylpropanamido)-3-(2-fluorophenyl)propanoic acid |
| 10 | 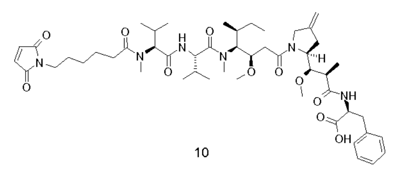<br>10<br>(*S*)-2-((2*R*,3*R*)-3-((*S*)-1-((3*R*,4*S*,5*S*)-4-((*S*)-2-((*S*)-2-(6-(2,5-dioxo-2,5-dihydr o-1*H*-pyrrol-1-yl)-*N*-methylhexanamido)-3-methylbutanamido)-*N*,3-dimethy lbutanamido)-3-methoxy-5-methylheptanoyl)-4-methylenepyrrolidin-2-yl)-3 -methoxy-2-methylpropanamido)-3-phenylpropanoic acid |

(continued)

| Example | Structure and Name |
|---|---|
| 12 | 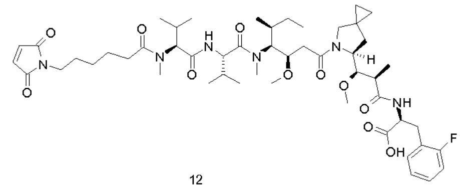 12 |
| | (*S*)-2-((2*R*,3*R*)-3-((*S*)-5-((3*R*,4*S*,5*S*)-4-((*S*)-2-((*S*)-2-(6-(2,5-dioxo-2,5-dihydr o-1*H*-pyrrol-1-yl)-*N*-methylhexanamido)-3-methylbutanamido)-*N*,3-dimethy lbutanamido)-3-methoxy-5-methylheptanoyl)-5-azaspiro[2.4]heptan-6-yl)-3-methoxy-2-methylpropanamido)-3 -(2-fluorophenyl) propanoic acid |
| 14 | 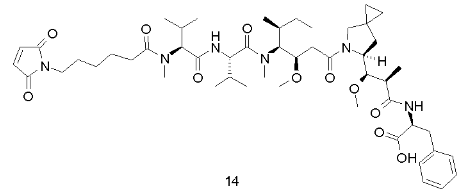 14 |
| | (*S*)-2-((2*R*,3*R*)-3-((*S*)-5-((3*R*,4*S*,5*S*)-4-((*S*)-2-((*S*)-2-(6-(2,5-dioxo-2,5-dihydr o-1*H*-pyrrol-1-yl)-*N*-methylhexanamido)-3-methylbutanamido)-*N*,3-dimethy lbutanamido)-3-methoxy-5-methylheptanoyl)-5-azaspiro[2.4]heptan-6-yl)-3-methoxy-2-methylpropanamido)-3 -phenylpropanoic acid |
| 45 | 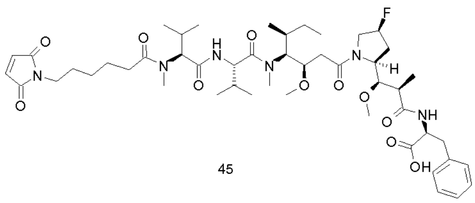 45 |
| | (*S*)-2-((2*R*,3*R*)-3-((2*S*,4*S*)-1-((3*R*,4*S*,5*S*)-4-((*S*)-2-((*S*)-2-(6-(2,5-dioxo-2,5-di hydro-1*H*-pyrrol-1-yl)-*N*-methylhexanamido)-3-methylbutanamido)-*N*,3-di methylbutanamido)-3-methoxy-5-methylheptanoyl)-4-fluoropyrrolidin-2-yl) -3-methoxy-2-methylpropanamido)-3-phenylpropanoic acid |
| 26 | 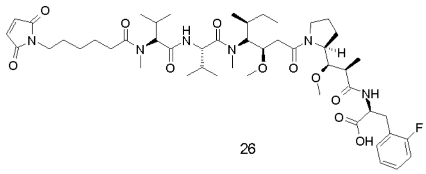 26 |
| | (*S*)-2-((2*R*,3R)-3-((*S*)-1-((3*R*,4*R*,5*S*)-4-((*S*)-2-((*R*)-2-(6-(2,5-dioxo-2,5-dihyd ro-1*H*-pyrrol-1-yl)-*N*-methylhexanamido)-3-methylbutanamido)-*N*,3-dimeth |
| | ylbutanamido)-3-methoxy-5-methylheptanoyl)pyrrolidin-2-yl)-3-methoxy-2-methylpropanamido)-3 -(2-fluorophenyl)propanoic acid |

(continued)

| Example | Structure and Name |
|---|---|
| 28 | 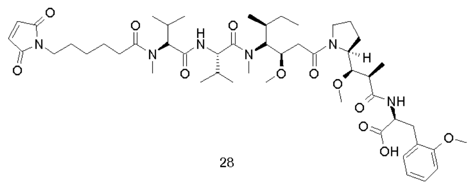 28 |
| | (*S*)-2-((2*R*,3*R*)-3-((*S*)-1-((3*R*,4*R*,5*S*)-4-((*S*)-2-((*R*)-2-(6-(2,5-dioxo-2,5-dihyd ro-1*H*-pyrrol-1-yl)-*N*-methylhexanamido)-3-methylbutanamido)-*N*,3-dimeth ylbutanamido)-3-methoxy-5-methylheptanoyl)pyrrolidin-2-yl)-3-methoxy-2-methylpropanamido)-3 -(2-methoxyphenyl)propanoic acid |
| 30 | 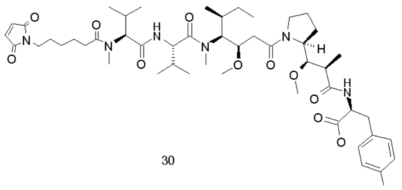 30 |
| | (*S*)-2-((2*R*,3*R*)-3-((*S*)-1-((3*R*,4*S*,5*S*)-4-((*S*)-2-((*S*)-2-(6-(2,5-dioxo-2,5-dihydr o-1*H*-pyrrol-1-yl)-*N*-methylhexanamido)-3-methylbutanamido)-*N*,3-dimethy lbutanamido)-3-methoxy-5-methylheptanoyl)pyrrolidin-2-yl)-3-methoxy-2-methylpropanamido)-3-(p-tolyl)propanoic acid |
| 47 | 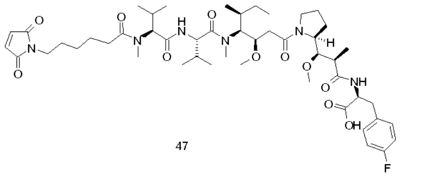 47 |
| | (*S*)-2-((2*R*,3*R*)-3-((*S*)-1-((3*R*,4*S*,5*S*)-4-((*S*)-2-((*S*)-2-(6-(2,5-dioxo-2,5-dihydr o-1*H*-pyrrol-1-yl)-*N*-methylhexanamido)-3-methylbutanamido)-*N*,3-dimethy lbutanamido)-3-methoxy-5-methylheptanoyl)pyrrolidin-2-yl)-3-methoxy-2-methylpropanamido)-3 -(4-fluorophenyl)propanoic acid |

Another aspect of this invention is directed to a compound of formula (PC-$L_2$):

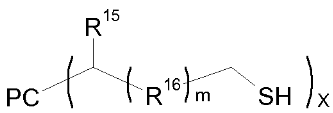

**(PC-L2)**

wherein

PC is ligand, preferably antibody, more preferably selected from Pertuzumab, Nimotuzumab and Trastuzumab; $R^{15}$ is selected from the group consisting of hydrogen, halogen, hydroxyl, cyano, alkyl, alkoxy and cycloalkyl; $R^{16}$ is selected from the group consisting of alkyl, cycloalkyl, alkoxy and heterocyclyl; m is 0-5, preferably 1-3; X is 0-5, preferably 1-3; X is a positive real number, including decimal and integers.

In another preferred embodiment of the present invention, a compound of formula (PC-$L_2$) is selected the group consisting of:

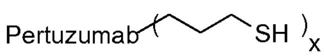
18c

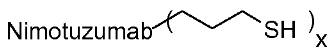
39c

and

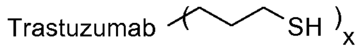
52c

.

The present invention further relates to a process of preparing a compound of formula ($PC-L_2$) comprising the steps of:

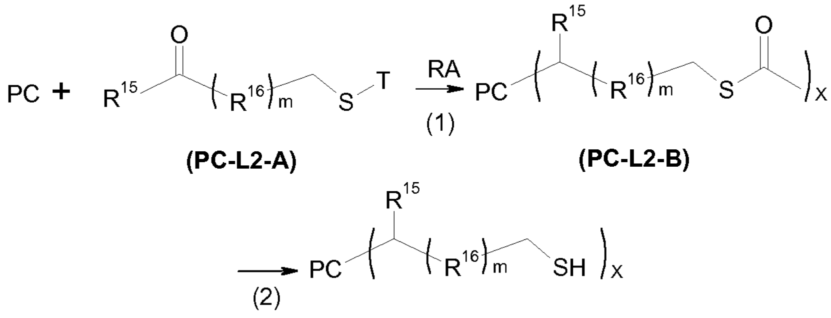

(PC-L2-A) (PC-L2-B) (PC-L2)

1) PC and a compound of formula (PC-L2-A) are reacted under a reducing agent RA to give a compound of formula (PC-L2-B); wherein RA is preferably sodium cyanoborohydride or sodium triacetoxyborohydride, more preferably sodium cyanoborohydride;
2) a compound of formula (PC-L2-B) is added with a deprotecting agent to remove the protecting group T of the thiol group to give a compound of formula (PC-L2),
wherein:

T is selected from the group consisting of H, t-butyl, acetyl, n-propionyl, isopropionyl, triphenylmethyl, methoxymethyl and 2-(trimethylsilyl) ethoxymethyl, preferably H or acetyl;
the compound of formula (PC-L2-A) is preferably S-(3-carbonylpropyl) thioacetate; PC, $R^{15}$, $R^{16}$, m and x are as defined in formula (PC-L2).

The present invention further relates to a process of preparing a compound of formula (PC-L'-D) comprising the steps of:

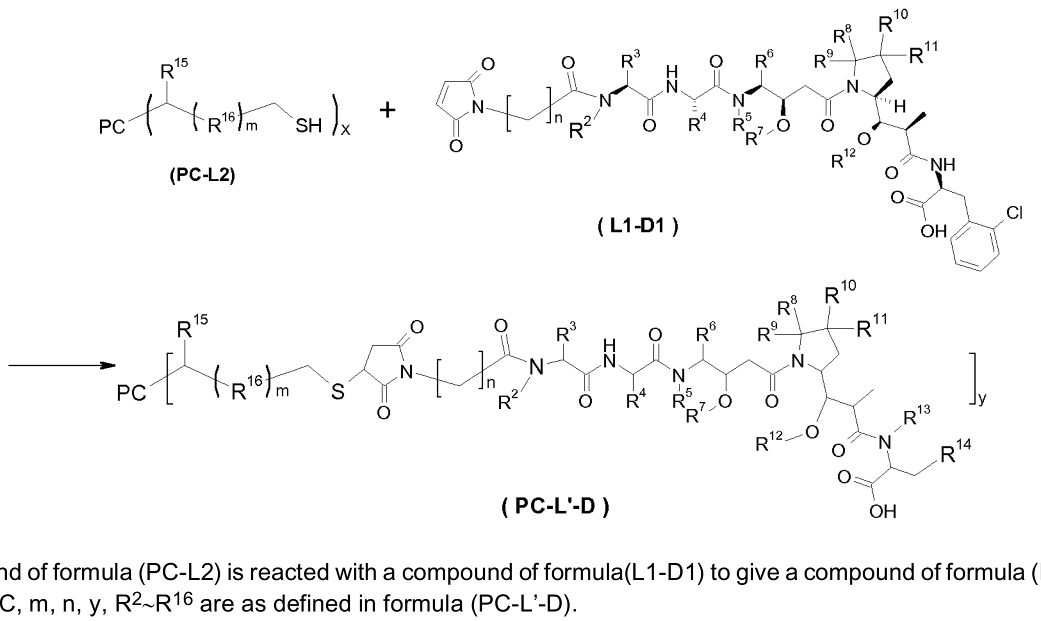
(PC-L2) (L1-D1) (PC-L'-D)

a compound of formula (PC-L2) is reacted with a compound of formula(L1-D1) to give a compound of formula (PC-L'-D); wherein PC, m, n, y, $R^2$~$R^{16}$ are as defined in formula (PC-L'-D).

The present invention further relates to a compound of formula (D-A a):

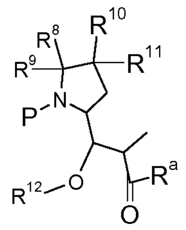

**( D-Aa)**

or a tautomer, mesomer, racemate, enantiomer, diastereomer, or mixtures thereof, or pharmaceutically acceptable salts thereof, which can be used as an intermediate for the synthesis of typical intermediate compounds of the ligand drug conjugate ADC of the present invention,
wherein:

any two of $R^8$-$R^{11}$ are attached to form a cycloalkyl, the rest two are each optionally selected from the group consisting of hydrogen, alkyl and cycloalkyl;
$R^{12}$ is selected from the group consisting of hydrogen, alkyl and halogen;
P is a hydrogen atom or a protecting group, and the protecting group is preferably Boc, Bn, Cbz, most preferably Boc;
$R^a$ is selected from the group consisting of hydroxy, amino, alkoxy, cycloalkoxy and alkylamino.

The present invention further relates to a compound of formula(D-Aa), which is a compound of formula (D-A):

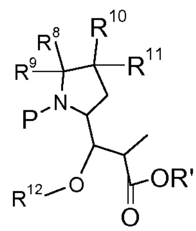

**( D-A )**

or a tautomer, mesomer, racemate, enantiomer, diastereomer, or mixtures thereof, or pharmaceutically acceptable salts thereof, which can be used as an intermediate for the synthesis of typical prodrug compounds of the ligand drug conjugate ADC of the present invention,
wherein:

any two of $R^8$-$R^{12}$ and P are attached to form a cycloalkyl, the rest two are each optionally selected from the group consisting of hydrogen, alkyl and cycloalkyl;
R' is selected from the group consisting of hydrogen, alkyl and cycloalkyl.

The intermediates (D-Aa) or (D-A) of the typical prodrug compound of the ligand drug conjugate ADC of the present invention include, but are not limited to,

| Example | Structure and name |
|---|---|
| 1e | 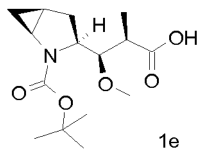 1e |
| | (2*R*,3*R*)-3-((1*S*,3*S*,5*S*)-2-(*tert*-butoxycarbonyl)-2-azabicyclo[3.1.0]hexan-3-y l)-3-methoxy-2-methylpropanoic acid |

(continued)

| Example | Structure and name |
|---|---|
| 3d | 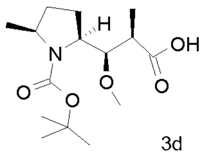 3d |
| | (2*R*,3*R*)-3-((2*S*,5*S*)-1-(tert-butoxycarbonyl)-5-methylpyrrolidin-2-yl)-3-meth oxy-2-methylpropanoic acid |
| 9e | 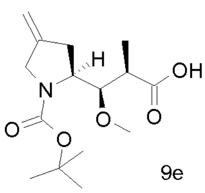 9e |
| | (2*R*,3*R*)-3-((*S*)-1-(tert-butoxycarbonyl)-4-methylenepyrrolidin-2-yl)-3-meth oxy-2-methylpropanoic acid |
| 11e | 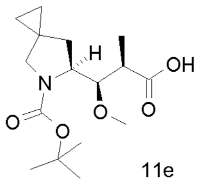 11e |
| | (2*R*,3*R*)-3-((*S*)-5-(tert-butoxycarbonyl)-5-azaspiro[2.4]heptan-6-yl)-3-methoxy-2-methylpropanoic acid |
| 44e | 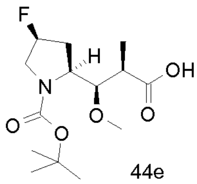 44e |
| | (2*R*,3*R*)-3-((2*S*,4*S*)-1-(tert-butoxycarbonyl)-4-fluoropyrrolidin-2-yl)-3-meth oxy-2-methylpropanoic acid |

The present invention further relates to a pharmaceutical composition comprising a therapeutically effective amount of the ligand-cytotoxic drug conjugate of formula (PC-L-Dr), formula (PC-L'-D), or a compound of formula (Dr), formula (L1-Dr), formula (D), formula (L1-D), or the pharmaceutically acceptable salt or solvate thereof as described above, and pharmaceutically acceptable carriers, diluents or excipients.

The present invention further relates to the use of the ligand-cytotoxic drug conjugate of formula (PC-L-Dr), formula (PC-L'-D), or a compound of formula (Dr), formula (L1-Dr), formula(D), formula(L1-D), or the pharmaceutically acceptable salt or solvate thereof as described above, or a pharmaceutical composition comprising the same, in the preparation of a medicament for the treatment of cancer, wherein the cancer is associated with HER2, HER3 or EGFR expression.

The present invention further relates to a method for treating cancer in mammals comprising administering the mammal a therapeutically effective amount of the ligand-cytotoxic drug conjugate of formula (PC-L-Dr), formula(PC-L'-D) as , or the pharmaceutically acceptable salt or solvate thereof, or a compound of formula (Dr), formula (L1-Dr), formula (D), formula (L1-D) or the pharmaceutically acceptable salt or solvate thereof as described above, or a pharmaceutical composition comprising the same; wherein said cancer is associated with HER2, HER3, EGFR expression.

The present invention further relates to the use of the ligand-cytotoxic drug conjugate of formula (PC-L-Dr), formula (PC-L'-D), or a compound of formula (Dr), formula (L1-Dr), formula(D), formula(L1-D), or the pharmaceutically acceptable salt or solvate thereof as described above, or a pharmaceutical composition comprising the same, in the preparation of a medicament for the treatment of cancer in mammals, wherein the mammal is human, the cancer is selected from the group consisting of breast cancer, ovarian cancer, stomach cancer, endometrial cancer, salivary gland cancer, lung cancer, colon cancer, renal cancer, colorectal cancer, thyroid cancer, pancreatic cancer, prostate cancer, bladder cancer, acute lymphocytic leukemia, acute myeloid leukemia, acute promyelocytic leukemia, chronic myelogenous leukemia, chronic lymphocytic leukemia, Hodgkin's lymphoma, non-Hodgkin's lymphoma and relapsed anaplastic

large cell lymphoma, preferably breast cancer, Hodgkin's lymphoma or relapsed anaplastic large cell lymphoma; more preferably breast cancer associated with HER2 expression.

The present invention further relates to a method for treating cancer in mammals, the method comprises administering the mammal a therapeutically effective amount of the ligand-cytotoxic drug conjugate of formula (PC-L-Dr), formula (PC-L'-D), or a compound of formula (Dr), formula (L1-Dr), formula(D), formula(L1-D), or the pharmaceutically acceptable salt or solvate thereof as described above, or a pharmaceutical composition comprising the same; wherein the mammal is human, the cancer is selected from the group consisting of breast cancer, ovarian cancer, stomach cancer, endometrial cancer, salivary gland cancer, lung cancer, colon cancer, renal cancer, colorectal cancer, thyroid cancer, pancreatic cancer, prostate cancer, bladder cancer, acute lymphocytic leukemia, acute myeloid leukemia, acute promyelocytic leukemia, chronic myelogenous leukemia, chronic lymphocytic leukemia, Hodgkin's lymphoma, non-Hodgkin's lymphoma and relapsed anaplastic large cell lymphoma, preferably breast cancer, Hodgkin's lymphoma or relapsed anaplastic large cell lymphoma; more preferably HER2 over-expressing breast cancer of 2+ level or higher level, most preferably breast cancer associated with HER2 expression.

In the present invention, it dosen't need to reduce the antibody hinge region.The unit L containing free mercapto groups link to the amino groups of the N-terminal amino groups and/or the lysine residue of the antibody. Therefore reducing the impact on the antibody structure is reduced, and the carbon-nitrogen bond structure is stable, difficult to break down in body circulation. The drug loading can be distributed in the range of 0~5 by further controlling the reaction conditions.

## DETAILED DESCRIPTION OF THE INVENTION

Unless otherwise defined, all of the technical and scientific terms used herein are in accordance with the usual understanding of one of ordinary skill in the art to which this invention pertains. Although the present invention may be practiced or tested using any of the methods and materials similar or equivalent to those described herein, preferred methods and materials are described herein. The following terms are used to describe and protect the present invention in accordance with the following definitions.

When the trade name is used in the present invention, the applicant is intended to include a preparation of the product of the trade name, a generic drug and an active drug part of the product.

Unless otherwise stated, the terms used in the specification and claims have the meanings described below.

"Alkyl" refers to a saturated aliphatic hydrocarbon group including $C_1$-$C_{20}$ straight chain and branched chain groups. Preferably, an alkyl group is an alkyl having 1 to 12 carbon atoms, more preferably 1 to 10 carbon atoms, and most preferably an alkyl having 1 to 6 carbon atoms. Representative examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and isomers of branched chains thereof. More preferably, an alkyl group is a lower alkyl having 1 to 6 carbon atoms. Representative examples include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl group can be substituted or unsubstituted. When substituted, the substituent group(s) can be substituted at any available connection point, and preferably the substituent group(s) is one or more groups independently selected from the group consisting of alkyl, alkenyl, alkynyl, alkyloxyl, alkylsulfo, alkylamino, halogen, thiol, hydroxy, nitro, cyano, cycloalkyl, heterocyclyl, aryl, heteroaryl, cycloalkoxy, heterocyclic alkoxy, cycloalkylthio, heterocyclic alkylthio, oxo group.

"Cycloalkyl" refers to a saturated and/or partially unsaturated monocyclic or polycyclic hydrocarbon group having 3 to 20 carbon atoms, preferably 3 to 12 carbon atoms, more preferably 3 to 10 carbon atoms, and most preferably 3 to 8 carbon atoms. Unlimited examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes a cycloalkyl having a spiro ring, fused ring or bridged ring.

"Heterocyclyl" refers to a 3 to 20 membered saturated and/or partially unsaturated monocyclic or polycyclic hydrocarbon group having one or more heteroatoms selected from the group consisting of N, O, and $S(O)_m$ (wherein m is an integer selected from 0 to 2) as ring atoms, but excluding -O-O-, -O-S- or -S-S- in the ring, and the remaining ring atoms being carbon atoms. Preferably, heterocyclyl has 3 to 12 atoms with 1 to 4 heteroatoms, more preferably 3 to 10

atoms. Unlimited examples of monocyclic heterocyclyl include, but are not limited to, pyrrolidinyl, piperidyl, piperazinyl, morpholinyl, thiomorpholinyl, homopiperazinyl and the like. Polycyclic heterocyclyl includes a heterocyclyl having a spiro ring, fused ring or bridged ring.

Said heterocyclyl can be fused to aryl, heteroaryl or cycloalkyl, wherein the ring bound to the parent structure is heterocyclyl. Unlimited examples include, but are not limited to:

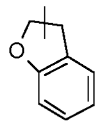 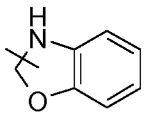 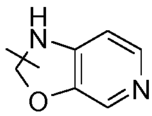 and 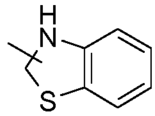

and on the like.

The heterocyclyl may be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocylylthio, oxo.

"Aryl" refers to a 6 to 14 membered full-carbon monocyclic ring or polycyclic fused ring (i.e. each ring in the system shares an adjacent pair of carbon atoms with another ring in the system) group having a completely conjugated pi-electron system; preferably 6 to 10 membered aryl, more preferably phenyl and naphthyl, and most preferably phenyl. The aryl can be fused to heteroaryl, heterocyclyl or cycloalkyl, wherein the ring bound to parent structure is aryl. Unlimited examples include, but are not limited to:

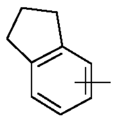 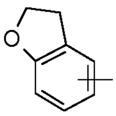 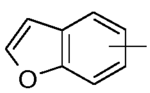 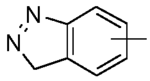 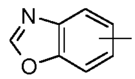 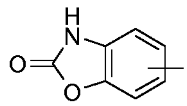 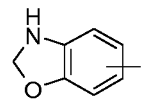

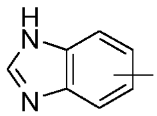 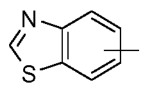 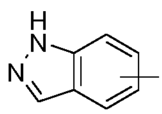 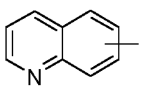 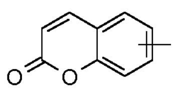 and 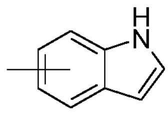 .

The aryl may be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclylthio.

"Heteroaryl" refers to an aryl system having 1 to 4 heteroatoms selected from the group consisting of O, S and N, and having 5 to 14 ring atoms. Preferably, a heteroaryl is 5- to 10- membered, more preferably 5- or 6- membered, for example furyl, thienyl, pyridyl, pyrrolyl, N-alkyl pyrrolyl, pyrimidinyl, pyrazinyl, imidazolyl, tetrazolyl, etc. The heteroaryl can be fused with the ring of an aryl, heterocyclyl or cycloalkyl, wherein the ring bound to the parent structure is heteroaryl. Representative examples include, but are not limited to:

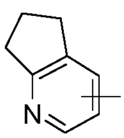 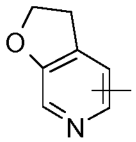 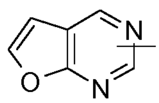 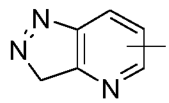 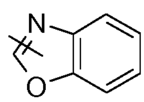 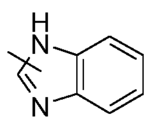

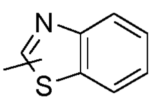 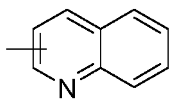 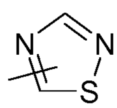 and 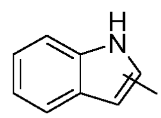 .

The heteroaryl group can be substituted or unsubstituted. When substituted, the substituent group(s) is preferably one or more groups independently selected from the group consisting of alkyl, alkenyl, alkynyl, alkoxy, alkylsulfo,

alkylamino, halogen, thiol, hydroxy, nitro, cyano, cycloalkyl, heterocyclyl, aryl, heteroaryl, cycloalkoxy, heterocyclic alkoxy, cycloalkylthio, heterocyclic alkylthio.

"Alkoxy" refers to an -O-(alkyl) or an -O-(unsubstituted cycloalkyl) group, wherein the alkyl or cycloalkyl is as defined above. Unlimited examples include methoxy, ethoxy, propoxy, butoxy, cyclopropyloxy, cyclobutyloxy, cyclopentyloxy, cyclohexyloxy, and the like. The alkoxy may be optionally substituted or unsubstituted. When substituted, the substituent is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocylic alkoxy, cycloalkylthio, heterocyclylthio.

"Alkylamino" refers to -N- (alkyl) and -N- (unsubstituted cycloalkyl), wherein the alkyl or cycloalkyl is as defined above. Unlimited examples of alkylamino groups include methylamino, ethylamino, propylamino, butylamino, cyclopropylamino, cyclobutylamino, cyclopentylamino, cyclohexylamino. The alkylamino group may be optionally substituted or unsubstituted, and when substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio.

The term "bond" refers to covalent bond represented by "-".

"Hydroxy" refers to an -OH group.

"Halogen" refers to fluorine, chlorine, bromine or iodine.

"Alkoxycarbonyl" refers to a -C(O)O(alkyl) or (cycloalkyl) group, wherein the alkyl and cycloalkyl are defined as above.

"Optional" or "optionally" means that the event or circumstance described subsequently can, but need not, occur, and such description includes the situation in which the event or circumstance may or may not occur. For example, "the heterocyclic group optionally substituted with an alkyl" means that an alkyl group can be, but need not be, present, and such description includes the situation of the heterocyclic group being substituted with an alkyl and the heterocyclic group being not substituted with an alkyl.

"Substituted" refers to one or more hydrogen atoms in a group, preferably up to 5, more preferably 1 to 3 hydrogen atoms, independently substituted with a corresponding numbers of substituents. It goes without saying that the substituents only exist in their possible chemical position. The person skilled in the art is able to determine whether the substitution is possible or impossible by experiments or theory without paying excessive efforts. For example, when amino or hydroxy having free hydrogen is bound to a carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof and other chemical components such as physiologically/pharmaceutically acceptable carriers and excipients. The purpose of a pharmaceutical composition is to facilitate administration of a compound to an organism and the absorption of the active ingredient and thus displaying biological activity.

"Pharmaceutically acceptable salts" refers to salts of ligand-cytotoxic drug conjugates of the invention that are safe and effective in mammals and have the desired biological activity. The antibody-antibody drug conjugated compound of the present invention contains at least one amino group and thus can form a salt with an acid. Unlimited examples of the pharmaceutically acceptable salts include hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, mesylate, ethanesulfonate, benzenesulfonate, p-toluenesulfonate.

"Solvate" refers to a pharmaceutically acceptable solvate resulted from a ligand-drug coupling compound of the present invention with one or more solvent molecules. Unlimited examples of solvent molecules include water, ethanol, acetonitrile, isopropanol, DMSO, ethyl acetate.

"Ligand" refers to a macromolecule compound capable of recognizing and binding to an antigen or receptor associated with a target cell. The role of the ligand is to deliver the drug to the target cell population that binds to the ligand. Such ligands include, but are not limited to, protein hormones, lectins, growth factors, antibodies, or other molecules that bind to cells. In an embodiment of the present invention, the ligand is expressed as PC, preferably an antibody, and the ligand may form a bond with the linker by a heteroatom on the ligand.

"Ligand drug conjugate" refers to a ligand linked to a biologically active cytotoxin by a chemically stable linker compound. In an embodiment of the present invention, the ligand is preferably an antibody, and the "ligand drug conjugate" is preferably an antibody drug conjugate (ADC), which refers to connect a monoclonal antibody or antibody fragment to a biologically active cytotoxin by a chemically stable linker compound.

"Antigen or receptor" may be identified by ligand and bind to a target cell. The preferred ligands in the present invention are those cell surface antigens or receptors expressed on target cells and/or tissues of proliferative diseases, such as cancer. Unlimited examples of cell surface receptor are selected from the cell surface receptors of HER2, HER3, HER4, CD20, CD22, CD30, CD33, CD44, Lewis Y, CD56, CD105, VEGFR or GPNMB; most preferably selected from

cell surface receptors of HER2 or EGFR. A specific preferred unlimited example is Trastuzumab, which specifically binds to the HER2 target; or Pertuzumab, which specifically binds to the HER2 target; or Nimotuzumab, which specifically binds to the EGFR target.

"Antibody" refers to any form of antibody that exhibits the desired biological activity. Thus, it is used in its broadest sense, in particular, including but not limited to full length antibodies, antibody binding fragments or derivatives. Sources of antibodies include, but are not limited to, monoclonal antibodies, polyclonal antibodies, genetically engineered antibodies (e.g., bispecific antibodies).

"Full length antibody" refers to an immunoglobulin molecule (e.g., IgM) comprising four polypeptide chains, i.e., two heavy chains and two light chains, which are cross-linked by disulfide bonds to form a polymer. Each heavy chain contains a heavy chain variable region (VH) and a heavy chain constant region, and the heavy chain constant region comprises three domains: CH1, CH2 and CH3. Each light chain comprises a light chain variable region (VL) and a light chain constant region, and the light chain constant region comprises one domain (CL1). The VH and VL regions can be further divided into hypervariable regions, the term of which is complementarity determining regions (CDRs), and the more conserved domains interspersed between the complementary regions, which is called the framework region (FR).

"Antibody binding fragment or derivative" includes any polypeptide or glycoprotein that can specifically bind to an antigen to form a complex, which is naturally occurred or obtained enzymatically, synthetically, or by genetic engineering. It usually comprises at least part of the antigen-binding region or variable region (e.g., one or more CDRs) of the parent antibody and retains at least some of the binding specificity of the parent antibody. "Antibody binding fragments or derivatives " may be derived from antibodies, such as derived from the transformation of the whole length of the antibody by appropriate standard techniques including proteolytic or recombinant genetically engineered techniques (including manipulation and expression of DNA expressing antibody variable regions and partially constant regions). "Antibody binding fragment or derivative" include but not limited to: (i) Fab fragments; (ii) $F(ab')_2$ fragments; (iii) Fd fragments; (iv) Fv fragments; (v) single chain Fv (scFv); (vi) dAb fragments; and (vii) the minimum recognition unit (e.g., an isolated complementarity determining region (CDR)) that mimics the amino acid residue of the hypervariable region of the antibody. Other engineering molecules such as bivalent antibodies, trivalent antibodies, tetravalent antibodies and micro-antibodies are also within the scope of "antibody-binding fragments or derivatives".

"Fab fragment" consists of a complete VH and CH1 functional regions of light and heavy chain. The heavy chain of the Fab molecule can not form a disulfide bond with another heavy chain molecule.

"Fc" region contains two heavy chain fragments containing the CH1 and CH2 domains of the antibody. Two heavy chain fragments are held together by two or more disulfide bonds and hydrophobic effects through the CH3 domain.

"Fab' fragment " contains a light chain and the VH and CH1 functional regions of the heavy chain, and also contains regions between the CH1 and CH2 domains, so that interchain disulfide bond can be formed between the two heavy chains of the two Fab'fragments to form F(ab')2 molecules.

" F(ab')2 fragment" contains two light chains and two heavy chains containing a partial constant region between the CH1 and CH2 domains, so that interchain disulfide bond can be formed between the two heavy chains. Thus, F(ab')2 fragment is formed with two Fab' fragment through the interchain disulfide bond between the two heavy chains.

" Fv fragment" includes the variable region VH functional region of the light chain and/or the heavy chain.

"Fc region" corresponds to the CH2 and CH3 functional regions of IgG, and has no antigen-binding activity, but is the interreaction site between antibody molecule and the effector molecule and cell.

"Hinge region" is used to link the Fab and Fc fragments of the antibody. In the present invention, it is used to link the bispecific fusion proteins to the Fc fragments.

The antibody of the present invention is preferably a specific antibody against a cell surface antigen on a target cell. Unlimited examples are as follows. Trastuzumab, a humanized anti-HER2 antibody for the treatment of breast cancer, is suitable for the treatment of metastatic breast cancer with HER2 overexpression. Pertuzumab, also known as 2C4, trade name of Perjeta, is a recombinant humanized monoclonal antibody, and is the first monoclonal antibody known as the "HER dimerization inhibitor" that reduce the growth of the tumor by binding HER2 to block the dimerization of HER2 with other HER receptors. Pertuzumab has been shown to inhibit tumor growth in prostate cancer patient with HER2 overexpression and low expression. Pertuzumab has been approved by the US FDA for the treatment of HER2-positive metastatic breast cancer. Nimotuzumab is a monoclonal antibody that targets the epidermal growth factor receptor (EGFR) and is a humanized monoclonal antibody that can be used to treat malignant tumors. EGFR is overexpressed in a variety of solid tumors, such as head and neck cancer, lung cancer, colorectal cancer.

"Cytotoxic drug" refers to a chemical molecule that has a strong ability to destroy its normal growth in tumor cells. Principally cytotoxic drugs all can kill tumor cells at a sufficiently high concentration, but because of lack of specificity, it can cause normal cell apoptosis and cause serious side effects while killing tumor cells. In an embodiment of the present invention, the cytotoxic drug is expressed as D/D1.

"Linker" in the present invention is expressed as L. It is a chemical structural fragment or bond which is covalently linked to a ligand at one end and linked to a cytotoxic drug at another end. The structure of L in the present invention is as following:

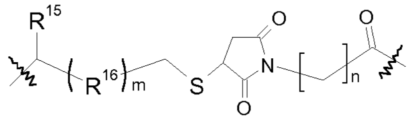

**( PC-L'-D )** ;

wherein R15, R16, m, n are defined as fomula (PC-L'-D).

"Drug loading" refers to the average number of cytotoxic drug loaded on each ligand in formula (I), and may also be expressed as the ratio of the number of drug to the number of antibody. The drug loading can range from 1 to 8 cytotoxic drugs (D) per ligand (Pc). In an embodiment of the present invention, the drug loading is expressed as y, and the number of drug products per ADC molecule after coupling reaction can be determined by conventional methods such as UV / visible spectroscopy, mass spectrometry, ELISA test and HPLC characterization.

In the present invention, y may be limited by the number of connection sites. In an embodiment of the present invention, the cytotoxic drug is coupled to the N-terminal amino and/or the ε-amino of lysine residues of the ligand via a linker. Typically the number of drug molecules conjugated to the antibody in a coupling reaction will be less than the theoretical maximum.

The following unlimited methods can be used to control the loading of the ligand-cytotoxic drug conjugates:

(1) to control the molar ratio of the linking reagent to the monoclonal antibody,
(2) to control the reaction time and temperature,
(3) to select a different reaction reagent.

The preparation of conventional pharmaceutical compositions can be found in Chinese Pharmacopoeia.

"Carrier" used in the medicament of the present invention refers to a system that can change the way of the drug entering human body and the distribution, the drug release rate, and deliver the drug to the targeted organ. Drug carrier release and targeting systems can reduce drug degradation and loss, reduce side effects and improve bioavailability. For example, the polymer surfactants which can be used as carriers can be self-assembled to form various forms of aggregates due to their unique amphiphilic structure. Preferred examples are such as micelles, microemulsions, gels, liquid crystals, vesicles, and the like. These aggregates have the ability to encapsulate drug molecules, while has good permeability to the membrane, and can be used as an excellent drug carrier.

"Excipient" is an adjunct to a pharmaceutical formulation other than a main drug and may also be referred to as an adjuvant, such as adhesives, fillers, disintegrants or lubricants of tablets; ointments of semi-solid preparations; matrix parts of the cream; preservatives, antioxidants, flavoring agents, fragrances, co-solvents, emulsifiers, solubilizers, osmotic pressure regulators or colorants of liquid preparations, and the like.

"Diluent", also known as a filler, is primarily intended to increase the weight and volume of the tablet. The addition of the diluent not only ensures a certain volume size, but also reduces the dose deviation of the main components, improves the compression profile of the drug. When the tablet contains oily component, the absorbent is added to the oily substance to keep the "dry" state to facilitate the tablet formation, such as starch, lactose, inorganic salts of calcium, microcrystalline cellulose and the like.

The pharmaceutical composition can be in the form of a sterile injectable aqueous solution. The acceptable medium and solvents that can be employed are water, Ringer's solution and isotonic sodium chloride solution. The sterile injectable preparation can also be a sterile injectable oil-in-water microemulsion in which the active ingredient is dissolved in the oil phase. For example, the active ingredient can be firstly dissolved in a mixture of soybean oil and lecithin, the oil solution is then introduced into a mixture of water and glycerol to form a microemulsion. The injectable solution or microemulsion can be infused into an individual's bloodstream by local mass injection. Alternatively, it may be advantageous to administer the injectable solution or microemulsion in such a way of maintaining a constant circulating concentration of the present compound. In order to maintain such a constant concentration, a continuous intravenous delivery device can be utilized. An example of such device is Deltec CADD-PLUS ™ 5400 intravenous injection pump.

The pharmaceutical composition can be in the form of a sterile injectable aqueous or oily suspension for intramuscular and subcutaneous administration. Such a suspension can be formulated with suitable dispersants or wetting agents and suspending agents as described above according to known techniques. The sterile injectable preparation can also be a sterile injectable solution or suspension prepared in a nontoxic parenterally acceptable diluent or solvent, for example, a solution prepared in 1,3-butanediol. Moreover, sterile fixed oils can be used as a solvent or suspending medium. For this purpose, any blending fixed oils including synthetic mono- or di-glyceride can be employed. Moreover, fatty acids, such as oleic acid, can also be employed in the preparation of an injection.

"Reducing agent" is a substance that losts electrons in the redox reactionor has electronic deviation. The reducing agent, in a broad sense, is an antioxidant, which has a reducing property and can be oxidized, and its product

is called an oxidation product. In an embodiment of the present invention, the reducing agent is expressed as RA, and unlimited examples include $H_2$, C, CO, Fe, Zn, alkali metal (commonly used with Li, Na, K), other active metals (such as Mg, Al, Ca, La, etc.), $SnCl_2$, oxalic acid, $KBH_4$, $NaBH_4$, $NaCNBH_3$), $(CH3COO)_3BHNa$, $LiAlH_4$, hypophosphorous acid, sodium hypophosphite, $Na_2S_2O_3$. The preferred reducing agent of the present invention is $NaCNBH_3$ or $(CH3COO)_3BHNa$.

"Mercapto protecting group" refers to a group which protects the mercapto group and can be removed at the end of the reaction, so that the reaction only occurs at an intended position while the mercapto group is not involved when a chemical molecule containing both mercapto groups and other groups is involved. In an embodiment of the present invention, the mercapto protecting group is expressed as T, and its unlimited examples include -tert-butyl, -acetyl, n-propionyl, -isopropanoyl, -triphenylmethyl, -methoxymethyl, -2-(trimethylsilyl)ethoxymethyl. The preferred mercapto protecting group of the present invention is acetyl.

SYNTHESIS METHOD OF THE PRESENT INVENTION

In order to accomplish the purpose of the synthesis of the present invention, the following synthetic scheme is adopted.

Scheme 1

The process for preparing a compound of formula (PC-L-DR) according to the present invention comprises:

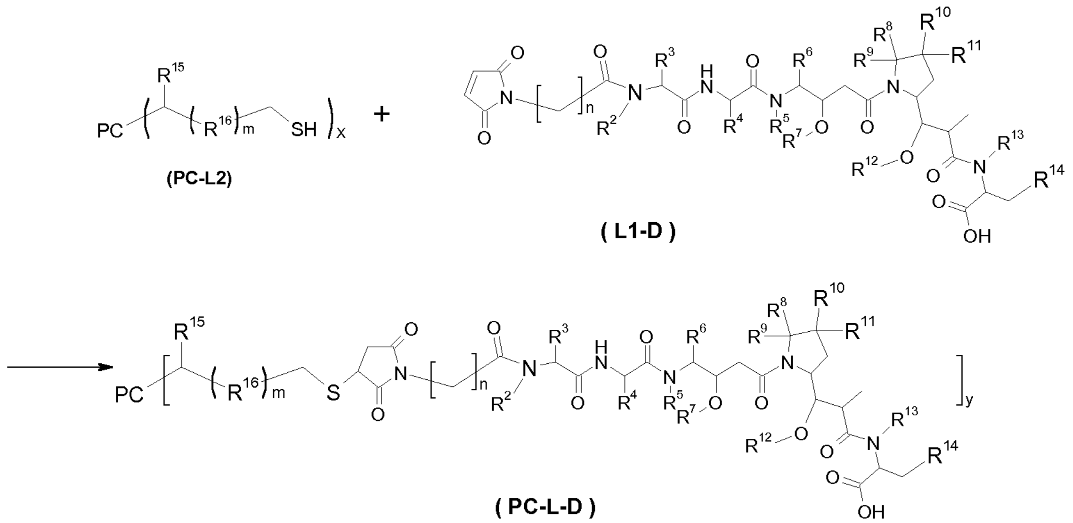

(PC-L2)

( L1-D )

( PC-L-D )

Scheme 2

The process for preparing a compound of formula (PC-L-DR1) according to the present invention comprises:

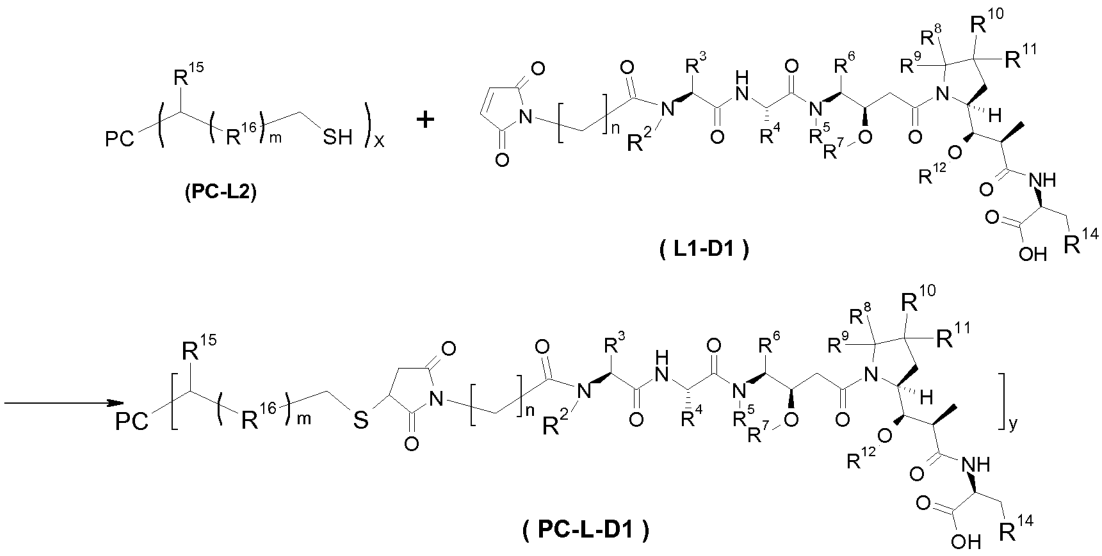

(PC-L2)

( L1-D1 )

( PC-L-D1 )

The compound of the formula (PC-L2) is reacted with a compound of formula (L1-D1) in an acetonitrile solution. A compound of formula (PC-L-DR) is obtained after desalting purification by the Sephadex G25 gel column; Wherein, PC, m, n, y, $R^2$~$R^{16}$ are as defined in formula (PC-L-DR).

**DETAILED DESCRIPTION OF THE INVENTION**

The present invention will be further described with the following examples, but the examples should not be considered as limiting the scope of the invention.

Conditions that are not specified in the examples are the common conditions in the art or the recommended conditions of the raw materials by the product manufacturer. For the reagents which are not indicated, it is the commercially available conventional reagent.

Examples

The structure of the compound is identified by nuclear magnetic resonance (NMR) and/or mass spectrometry (MS). NMR chemical shifts (δ) are given in $10^{-6}$ (ppm). NMR is determined by Bruker AVANCE-400. The solvents are deuterated-dimethyl sulfoxide (DMSO-$d_6$), deuterated-chloroform ($CDCl_3$) and deuterated-methanol ($CD_3OD$) with tetramethylsilane (TMS) as an internal standard.

MS is determined by a FINNIGAN LCQAd (ESI) mass spectrometer (manufacturer: Thermo, type: Finnigan LCQ advantage MAX).

High performance liquid chromatography (HPLC) is determined on an Agilent 1200DAD high pressure liquid chromatography spectrometer (Sunfire C18 150×4.6 mm chromatographic column) and a Waters 2695-2996 high pressure liquid chromatography spectrometer (Gimini C18 150×4.6 mm chromatographic column).

The average inhibition rate of kinase and $IC_{50}$ values are determined by a NovoStar ELISA (BMG Co., Germany).

Yantai Huanghai HSGF254 or Qingdao GF254 silica gel plate is used for thin-layer silica gel chromatography (TLC). The dimension of the silica gel plate used in TLC is 0.15 mm to 0.2 mm, and the dimension of the silica gel plate used in product purification is 0.4 mm to 0.5 mm.

Yantai Huanghai 200 to 300 mesh silica gel is used as carrier for column chromatography.

The known raw materials of the present invention are prepared by conventional synthesis methods in the art, or can be purchased from ABCR GmbH & Co. KG, Acros Organnics, Aldrich Chemical Company, Accela ChemBio Inc., or Dari chemical Company, etc.

Unless otherwise stated, all of the reactions can be carried out under nitrogen atmosphere or argon atmosphere.

The term "nitrogen atmosphere" or "argon atmosphere" means that a reaction flask was equipped with a 1 L nitrogen or argon balloon.

Unless otherwise stated, the solution used in the reactions refers to an aqueous solution.

Unless otherwise stated, the reaction temperature in the reactions refers to room temperature. Room temperature is the optimum reaction temperature which is in the range of 20°C to 30°C.

Preparation of PBS buffer with pH = 6.5 in the reaction: $KH_2PO_4$ 8.5g, $K_2HPO_4.3H_2O$ 8.56g, NaCl 5.85g, EDTA 1.5g were set to 2L in the bottle, and then subject to ultrasonic to give the buffer.

Preparation of acetic acid/sodium acetate buffer with pH = 4.5 in the reaction: 9 g of anhydrous sodium acetate were placed in the bottle, added with purified water, and set to 2L, then sodium acetate 4.9 mL was added with stirring to give the buffer.

Preparation of phosphate buffer with pH = 7.0 in the reaction: 39mL of 0.2*M* $NaH_2PO_4$ was added to 61mL of 0.2*M* $Na_2HPO_4$ with stirring to give a 0.2*M* buffer with pH=7.

The reaction process is monitored by thin layer chromatography (TLC), and the elution systems included: A: dichloromethane and methanol, B: *n*-hexane and ethyl acetate, C: petroleum ether and ethyl acetate, D: acetone. The ratio of the volume of the solvent is adjusted according to the polarity of the compounds.

The elution systems for purification of the compounds by column chromatography and thin layer chromatography included: A: dichloromethane and methanol, B: *n*-hexane and ethyl acetate, C: dichloromethane and acetone, D: ethyl acetate and dichloromethane, E: ethyl acetate and dichloromethane and *n*-hexane, F: ethyl acetate and dichloromethane and acetone. The ratio of the volume of the solvent is adjusted according to the polarity of the compounds, and sometimes a little alkaline reagent such as triethylamine or acidic reagent was added.

Some of the structures of the compounds of the present invention are determined by Q-TOF LC/MS. For Q-TOF LC/MS, Agilent 6530 Accurate-Mass Quadrupole - Time of Flight Mass Spectrometer and Agilent 1290-Infinity UHPLC (Agilent Poroshell 300SB-C8 5μm, 2.1 X 75mm Column) are used.

Known starting materials of the present invention are synthesized by adopting or using the methods known in the art, and the experimental methods in the following examples for which the specific conditions are not indicated are carried out according to conventional conditions or the conditions recommended by the product manufacturers. The experimental reagents for which the specific sources are not indicated are the conventional reagents generally purchased from market.

1, Preparation of antibodies as intermediates

The following antibodies were prepared according to conventional methods: for instance, vector construction, HEK293 cell transfection (Life Technologies Cat. No. 11625019), purification and expression.

Antibody sequences

(1) Pertuzumab, capable of specifically binding to target HER2:

Sequence of light chain:

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO.1

Sequence of heavy chain:

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO.2

(2) Nimotuzumab, capable of specifically binding to target EGFR:

Sequence of light chain:

DIQMTQSPSSLSASVGDRVTITCRSSQNIVHSNGNTYLDWYQQTPGKAPKLLIY
KVSNRFSGVPSRFSGSGSGTDFTFTISSLQPEDIATYYCFQYSHVPWTFGQGTKL
QITRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO：3

Sequence of heavy chain:

QVQLQQSGAEVKKPGSSVKVSCKASGYTFTNYYIYWVRQAPGQGLEWIGGINP
TSGGSNFNEKFKTRVTITADESSTTAYMELSSLRSEDTAFYFCTRQGLWFDSDGR
GFDFWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO：4

(3) Trastuzumab, capable of specifically binding to target HER2:

Sequence of light chain:

DIQMTQSPSSLSASVGDRVTITCRASQDVNTAVAWYQQKPGKAPKLLIYSASFL
YSGVPSRFSGSRSGTDFTLTISSLQPEDFATYYCQQHYTTPPTFGQGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

SEQ ID NO：5

Sequence of heavy chain:

EVQLVESGGGLVQPGGSLRLSCAASGFNIKDTYIHWVRQAPGKGLEWVARIYP
TNGYTRYADSVKGRFTISADTSKNTAYLQMNSLRAEDTAVYYCSRWGGDGFY
AMDYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNV
FSCSVMHEALHNHYTQKSLSLSPGK

SEQ ID NO：6

2, Preparation of drug, drug linker, ligand drug conjugate(ADC)

Example 1

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo [3.1.0]hexan-3-yl)-3-methoxy-2-methylpropanamido)-3-phenylpropanoic acid

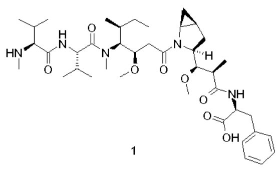

1

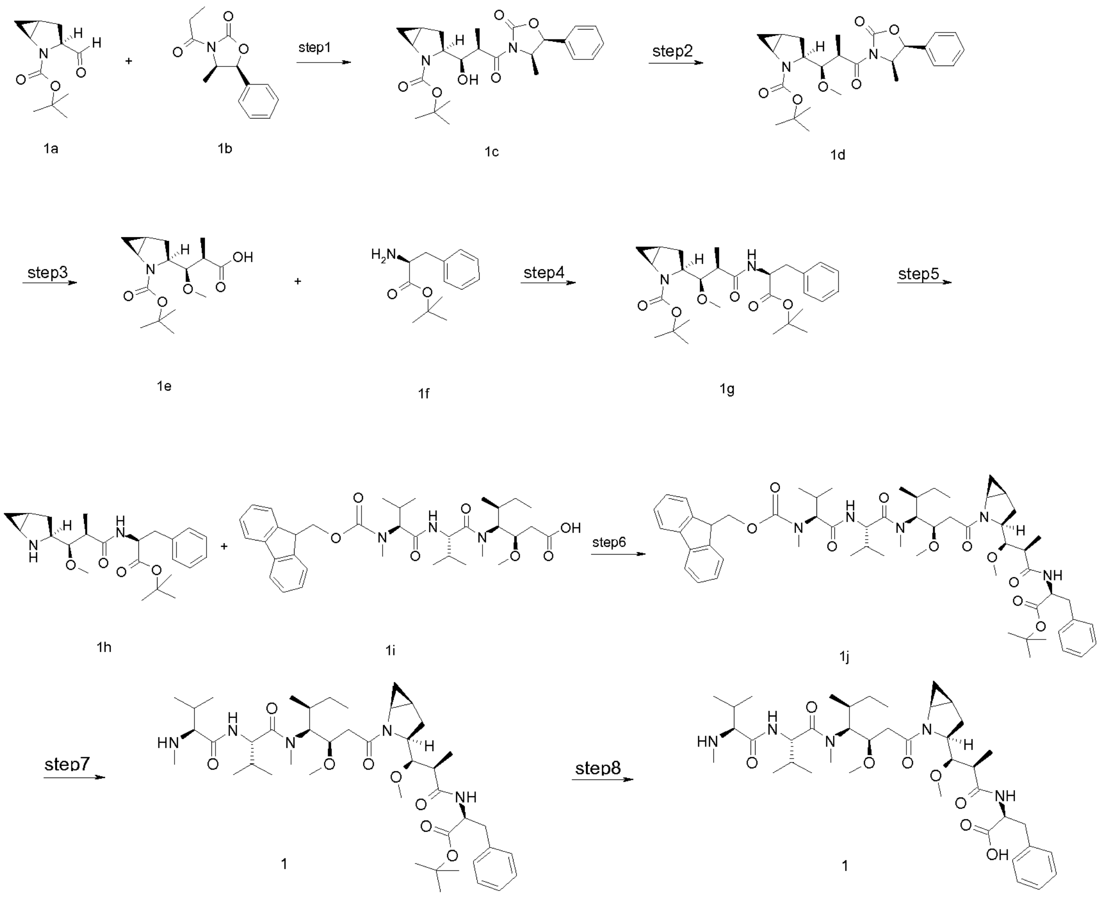


1a 1b 1c 1d

1e 1f 1g

1h 1i 1j

1 1

Step 1

(1*S*,3*S*,5*S*)-tert-butyl 3-((1*R*,2*R*)-1-hydroxy-2-methyl-3-((4*R*,5*S*)-4-methyl-2-oxo-5-phenyloxazolidin-3-yl)-3-oxopropyl)-2-azabicyclo[3.1.0]hexane-2-carboxylate

(4*R*,5*S*)-4-methyl-5-phenyl-3-propionyloxazolidin-2-one **1b** (1.96 g, 9.26 mmol, prepared according to the known method "Journal of the American Chemical Society, 2003, 125(50), 15512-15520") was dissolved in 25 mL of dichloromethane under argon atmosphere, and cooled to 0 °C. The above reaction solution was added with trimethylamine (1.49 mL, 10.93 mmol) and dibutylboron trifluoromethanesulfonate (9.7 mL, 9.72 mmol) dropwise, and then stirred for 50 min at 0°C. The resulting mixture was cooled to -75 °C in an dry ice acetone bath, then added with a 7 mL solution of (1*S*,3*S*,5*S*)-tert-butyl 3-formyl-2-azabicyclo[3.1.0]hexane-2-carboxylate **1a** (2.16 g, 9.26 mmol, prepared according to the known method "US20100249190") in dichloromethane, and stirred for 1.5 hours at -75°C, then for 2 hours at 0°C, and then for 1 hour at room temperature. The reaction mixture was added with 36 mLof a mixture of phosphate buffer (pH=7.0) and methanol (V/V=1:3), then added with a 36 mL mixture solution of methanol and hydrogen peroxide (30%) (V/V=2:1) at 0°C, and stirred for 1 hour at room temperature. The reaction mixture was concentrated under reduced pressure to remove the orgnic phase. The residues were added with a little water and extracted with ether (50 mL x 3), washed sequentially with 5% sodium bicarbonate solution and 150 mL of saturated sodium chloride solution, dried over anhydrous sodium sulfate, filtered, and the filtrate was concentrated under reduced pressure. The residue was purified by silica gel column chromatography with eluent system B to give the title product of (1*S*,3*S*,5*S*)-tert-butyl 3-((1*R*,2*R*)-1-hydroxy-2-methyl-3-((4*R*,5*S*)-4-methyl-2-oxo-5-phenyloxazolidin-3-yl)-3-oxopropyl)-2-azabicyclo[3.1.0]hexane-2-carboxylate **1c** (2.4 g, white foam solid), yield 58.5%.
MS m/z (ESI): 345.1 [M-100+1]

Step2

(1*S*,3*S*,5*S*)-*tert*-butyl 3-((1*R*,2*R*)-1-methoxy-2-methyl-3-((4*R*,5*S*)-4-methyl-2-oxo-5-phenyloxazolidin-3-yl)-3 -oxopropyl)-2-azabicyclo[3.1.0]hexane-2-carboxylate

(1*S*,3*S*,5*S*)-tert-butyl3-((1*R*,2*R*)-1-hydroxy-2-methyl-3-((4*R*,5*S*)-4-methyl-2-oxo-5-phe nyloxazolidin-3-yl)-3-oxopropyl)-2-azabicyclo[3.1.0]hexane-2-carboxylate **1c** (1.4 g, 3.15 mmol) was dissolved in 20 mL dichloromethane, and added with 1.4 g crushed molecular sieves. The mixture was added with 1,8-bisdimethylaminonaphthalene (1.75 g, 8.19 mmol) and trimethyloxonium tetrafluoroboron (1.16 g, 7.87 mmol) at 0°C, under argon atmosphere. The reaction mixture was kept dark and stirred at room temperature for 40 hours. After the reaction was completed, the reaction mixture was filited and the filter cake was washed with methylene chloride. The combined filtrate was washed with saturated ammonium chloride solution (50 mL x 4) to remove the excess 1,8-bisdimethylaminonaphthalene and washed with saturated sodium chloride solution (120 mL), dried over anhydrous sodium sulfate, filtered, and the filtrate was concentrated under reduced pressure. The residues were purified by flash column chromatography using eluent system B to give the title product of (1*S,*3*S,*5*S*)-*tert*-butyl 3-((1*R*,2*R*)-1-methoxy-2-methyl-3-((4*R*,5*S*)-4-methyl-2-oxo-5-phenyloxazolidin-3-yl)-3 -oxopropyl)-2-azabicyclo[3.1.0]hexane-2-carboxylate **1d** (400 mg, white solid), yield 27.8%.
MS m/z (ESI): 459.4 [M+1]

Step3

(2*R*,3*R*)-3-((1*S*,3*S*,5*S*)-2-(*tert*-butoxycarbonyl)-2-azabicyclo[3.1.0]hexan-3-yl)-3-metho xy-2-methylpropanoic acid

(1*S*,3*S*,5*S*)-*tert*-butyl 3-((1*R*,2*R*)-1-methoxy-2-methyl-3-((4*R*,5*S*)-4-methyl-2-oxo-5-phenyloxazolidin-3-yl)-3 -oxopropyl)-2-azabicyclo[3.1.0]hexane-2-carboxylate **1d** (400 mg, 0.87 mmol) was dissolved in 24 mL of tetrahydrofuran and cooled to 0°C under argon atmosphere. 30% hydrogen peroxide (0.34 mL/0.38 g, 3.31 mmol) was added dropwise slowly, and then lithium hydroxide monohydrate (62 mg, 1.48 mmol) was added. The reaction mixture was allowed to react at room temperature for 20 hours. The reaction solution was added with sodium sulfite solid (440 mg, 3.48 mmol), and stirred at room temperature for 1 hour. Then 10 mL of water was added and the organic phase was concentrated under reduced pressure. The residues were extracted with dichloromethane (40 mL x 2). The separated aqueous phase was added with 2N hydrochloric acid dropwise in an ice bath until the solution arrived at pH of 3 to 4. Then the aqueous phase was extracted with ethyl acetate (25 mL x 3), and the combined ethyl acetate phase was washed successively with water (50 mL) and saturated sodium chloride solution (50 mL), dried over anhydrous sodium sulfate, filtered and the filtrate was concentrated under reduced pressure to give the title product of (2*R*,3*R*)-3-((1*S*,3*S*,5*S*)-2-(*tert*-butoxycarbonyl)-2-azabicyclo[3.1.0]hexan-3-yl)-3-metho xy-2-methylpropanoic acid **1e** (230 mg, colorless liquid), yield 88.0%.
MS m/z (ESI): 200.1 [M-100+1]

Step4

(1*S*,3*S*,5*S*)-tert-butyl 3-((1*R*,2*R*)-3-((*S*)-1-tert-butoxy-1-oxo-3-phenylpropan-2-ylamino)-1-methoxy-2-methyl -3-oxopropyl)-2-azabicyclo[3.1.0]hexane-2-carboxylate

(2*R*,3*R*)-3-((1*S*,3*S*,5*S*)-2-(*tert*-butoxycarbonyl)-2-azabicyclo[3.1.0]hexan-3-yl)-3-metho xy-2-methylpropanoic acid **1e** (100 mg, 0.334 mmol) was dissolved in 6 mL of mixed solvent of dichloromethane and dimethylformamide (V/V = 5: 1), and added with (*S*)-tert-butyl 2-amino-3-phenylpropanoate **1f** (73.9 mg, 0.334 mmol, prepared according to the known method "Tetrahedron: Asymmetry, 2006, 17(4), 603-606") and then *N,N*-diisopropylethylamine (0.29 mL, 67 mmol) and 2- (7-azobenzotriazole) -*N,N,N',N'*-tetramethyluronium hexafluophosphate (152.3 mg, 0.40 mmol). The reaction mixture was stirred under an argon atmosphere at room temperature for 1 hour, and then added with 10 mL of water under stirring. The dichloromethane phase was washed with saturated sodium chloride solution (10 mL), dried over anhydrous sodium sulfate, filtered, and the filtrate was concentrated under reduced pressure. The residues were purified by silica gel column chromatography using eluent system B to give the title product of (1*S*,3*S*,5*S*)-tert-butyl 3-((1*R*,2*R*)-3-((*S*)-1-tert-butoxy-1-oxo-3-phenylpropan-2-ylamino)-1-methoxy-2-methyl -3-oxopropyl)-2-azabicyclo[3.1.0]hexane-2-carboxylate **lg** (140 mg, colorless viscous liquid), yield 83.7%.
MS m/z (ESI): 503.3 [M+1]

Step5

(*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3 -phenylpropanoate

(1*S*,3*S*,5*S*)-tert-butyl 3-((1*R*,2*R*)-3-((*S*)-1-tert-butoxy-1-oxo-3-phenylpropan-2-ylamino)-1-methoxy-2-methyl -3-oxopropyl)-2-azabicyclo[3.1.0]hexane-2-carboxylate **Ig** (140 mg, 0.28 mmol) was dissolved in 2 mL of dioxane, and then added with a 5.6 M solution of hydrogen chloride in dioxane (0.15 mL, 0.835 mmol). The reaction system was sealed and then stirred for 8 hours at room temperature and placed in a refrigerator for 12 hours at 0 ° C. After the reaction, 3 mL of dichloromethane, 3 mL of water, 3 mL of saturated sodium bicarbonate solution were added successively and stirred for 10 minutes. The dichloromethane phase was washed with saturated sodium chloride solution (20 mL × 2), dried over anhydrous sodium sulfate, filtered and the filtrate was concentrated under reduced pressure to abtain the title product of (*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-phenylpropanoate **Ih** (86 mg, yellow solid), yield76.7%.
MS m/z (ESI): 403.4 [M+1]

Step6

(*S*)-tert-butyl 2-((2*R*,3*R*)-3-((1*S*,3*S*,5*S*)-2-((5*S*,8*S*,11*S*,12R*)*-11-((*S*)-sec-butyl)-1-(9iH-fluoren-9-yl)-5,8 -diisopropyl-12-methoxy-4,10-dimethyl-3,6,9-trioxo-2-oxa-4,7,10-triazatetradecan-14-o yl)-2-azabicyclo[3.1.0]hexan-3-yl)-3-methoxy-2-methylpropanamido)-3-phenylpropano ate

(*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-phenylpropanoate **Ih** (86 mg, 0.213 mmol), (5*S*,8*S,*11*S,*12*R*)-11-((*S*)-sec-butyl)-1-(9*H*-fluoren-9-yl)-5,8-diisopropyl-12-methoxy-4,1 0-dimethyl-3,6,9-trioxo-2-oxa-4,7,10-triazatetradecan-14-oic acid **1i** (136 mg, 0.213 mmol, prepared according to the known method "WO 2013072813") was dissolved in 6 mL of mixed solvent of dichloromethane and dimethylformamide (V/V = 5: 1), and then added with *N,N*-diisopropylethylamine (0.19 mL, 1.065 mmol) and 2-(7-azobenzotriazole)-*N,N,N',N'*-tetramethyluronium hexafluophosphatehexafluorophosphate (97.4 mg, 0.256 mmol). The reaction mixture was stirred under an argon atmosphere at room temperature for 1 hour and then added with 20 mL of water. The dichloromethane layer was washed with saturated sodium chloride solution (20 mL), dried over anhydrous sodium sulfate, filtered and the filtrate was concentrated under reduced pressure. The residues were purified by silica gel column chromatography using eluent system B to give the title product of (*S*)-tert-butyl 2-((2*R*,3*R*)-3-((1*S*,3*S*,5*S*)-2-((5*S*,8*S*,11*S*,12*R*)-11-((*S*)-sec-butyl)-1-(9*H*-fluoren-9-yl)-5,8 -diisopropyl-12-methoxy-4,10-dimethyl-3,6,9-trioxo-2-oxa-4,7,10-triazatetradecan-14-o yl)-2-azabicyclo[3.1.0]hexan-3-yl)-3-methoxy-2-methylpropanamido)-3-phenylpropano ate **1j** (120 mg, white foam solid), yield 54.9%.
MS m/z (ESI): 1023.1 [M+1]

Step7

(*S*)-tert-butyl 2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyla mino)butanamido)butanamido)-3-methoxy-5-methylheptanoyl)-2-azabicyclo[3.1.0]hexa n-3-yl)-3-methoxy-2-methylpropanamido)-3-phenylpropanoate

(*S*)-tert-butyl 2-((2*R*,3*R*)-3-((1*S*,3*S*,5*S*)-2-((5*S*,8*S*,11*S*,12R*)*-11-((*S*)-sec-butyl)-1-(9*H*-fluoren-9-yl)-5,8 -diisopropyl-12-methoxy-4,10-dimethyl-3,6,9-trioxo-2-oxa-4,7,10-triazatetradecan-14-o yl)-2-azabicyclo [3.1.0]hexan-3-yl)-3-methoxy-2-methylpropanamido)-3-phenylpropano ate **1j** (120 mg, 0.117 mmol) was dissolved in 2 mL of dichloromethane and added with 2 mL of diethylamine. The reaction was stirred under an argon atmosphere at room temperature for 2 hours. The reaction solution was concentrated under reduced pressure to give a crude title product of (*S*)-tert-butyl 2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyla mino)butanamido)butanamido)-3-methoxy-5-methylheptanoyl)-2-azabicyclo[3.1.0]hexa n-3-yl)-3-methoxy-2-methylpropanamido)-3-phenylpropanoate **Ik** (124 mg, yellow liquid). The product is used in the next step without further purification.
MS m/z (ESI): 801.5 [M+1].

Step8

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-phenylpropanoic acid

The crude product of (*S*)-tert-butyl 2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyla mino)butanamido)butanamido)-3-methoxy-5-methylheptanoyl)-2-azabicyclo[3.1.0]hexa n-3-yl)-3-methoxy-2-methylpropanamido)-3-phenylpropanoate **1k** (90 mg, 0.112 mmol) was dissolved in 1 mL dioxane, and then added with 5.6 M solution of hydrogen chloride in dioxane (3 mL). The reaction system was sealed and stirred at room temperature for 12 hours. The reaction mixture was concentrated under reduced pressure and the residues were purified by high performance liquid chromatography to obtain a title product of (*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-phenylpropanoic acid 1 (19 mg, white solid), yield 22.7%.
MS m/z (ESI): 744.6 [M+1]
$^1$H NMR (400 MHz, $CD_3OD$): δ 7.34-7.21(m, 5H), 4.76-4.70(m, 2H), 4.26-4.19 (m, 1H), 4.14-4.06(m, 1H), 3.91-3.86(m, 1H), 3.85-3.77(m, 1H), 3.75-3.56(m, 2H), 3.44-3.10(m, 9H), 2.98-2.83(m, 1H), 2.71-2.57(m, 4H), 2.26-1.99(m, 4H), 1.92-1.77 (m, 1H), 1.75-1.58(m, 2H), 1.49-1.27(m,4H),1.21-0.95(m, 18H), 0.93-0.79(m, 4H), 0.76-0.61(m, 1H).

Example 2

(*S*)-3-(2-chlorophenyl)-2-((2*R*,3*R*)-3-((1*S*,3*S*,5*S*)-2-((3*R*,4*S*,5*S*)-4-((*S*)-*N*,3-dimethyl-2-(( *S*)-3-methyl-2-(methylamino)butanamido)butanamido)-3-methoxy-5-methylheptanoyl)-2-azabicyclo[3.1.0]hexan-3-yl)-3-methoxy-2-methylpropanamido)propanoic acid

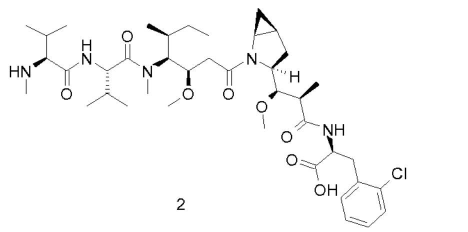

2

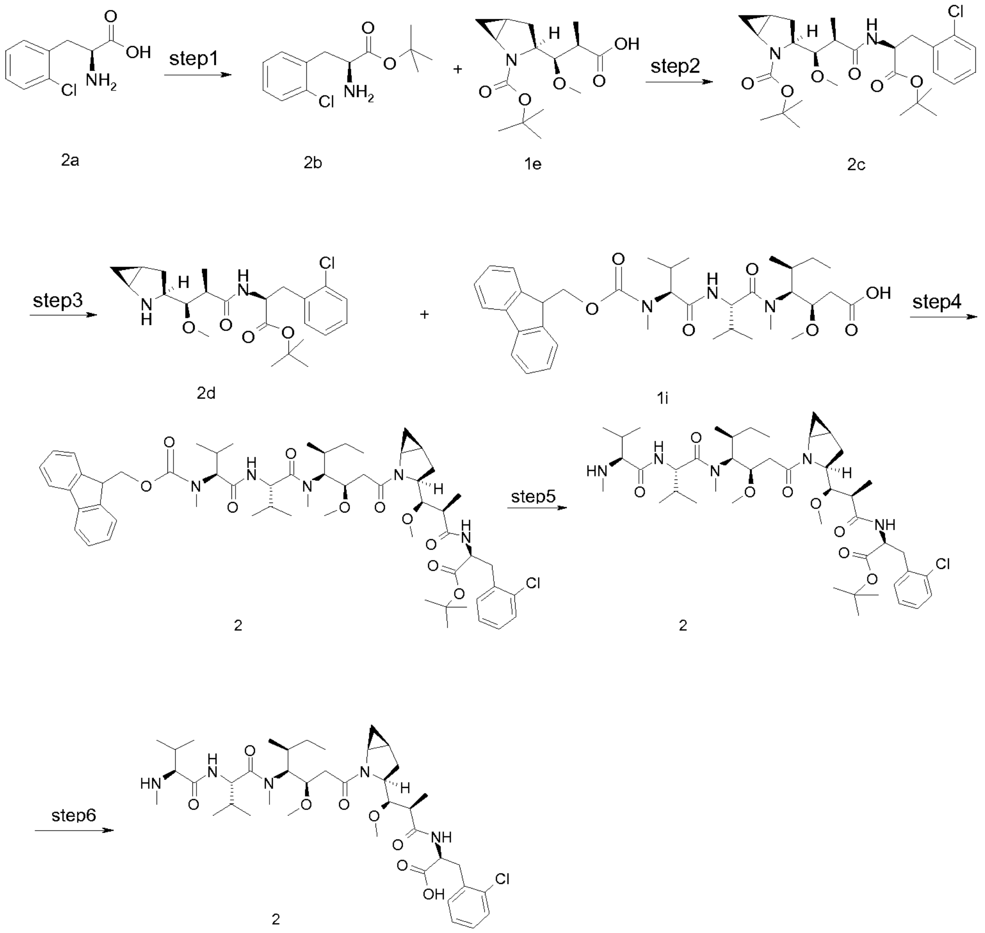


2a 2b 1e 2c

2d 1i

2 2

2

step 1

(*S*)-tert-butyl 2-amino-3-(2-chlorophenyl)propanoate

(S)-2-amino-3-(2-chlorophenyl)propanoic acid **2a** (400 mg, 2.0 mmol, prepared according to the known method "Journal of the American Chemical Society, 1940, 62, 565-8") was dissolved in 10 mL of tert-butyl acetate, and added with perchloric acid (428 mg (70%), 3.00 mmol) and stirred at room temperature for 16 hours. The resulting mixture was added with 6 mL of water and the organic phase was washed with saturated sodium bicarbonate solution (3 mL). The aqueous phase was adjusted to pH = 8 with saturated sodium bicarbonate solution and extracted with dichloromethane (5 mL ×3). The organic phases were combined, washed successively with water (3 mL) and saturated sodium chloride solution (5 mL), dried over anhydrous sodium sulfate, filtered and the filtrate was concentrated under reduced pressure to give the crude title product of (*S*)-tert-butyl 2-amino-3-(2-chlorophenyl)propanoate **2b** (400 mg, white solid). The product was used in the next step without further purification.

Step2

(1*S*,3*S*,5*S*)-tert-butyl 3-((1*R*,2*R*)-3-((*S*)-1-tert-butoxy-3-(2-chlorophenyl)-1-oxopropan-2-ylamino)-1 -methox y-2-methyl-3-oxopropyl)-2-azabicyclo[3.1.0]hexane-2-carboxylate

(2*R*,3*R*)-3-((1*S*,3*S*,5*S*)-2-(tert-butoxycarbonyl)-2-azabicyclo[3.1.0]hexan-3-yl)-3-metho xy-2-methylpropanoic acid 1**e** (110 mg, 0.367 mmol) was dissolved in 6 mL of mixed solvent of dichloromethane and dimethylformamide (V/V = 5: 1), and then added with the crude product of (*S*)-tert-butyl 2-amino-3-(2-chlorophenyl)propanoate **2b** (94 mg, 0.367 mmol), *N,N*-Diisopropylethylamine (0.32 mL, 1.835 mmol) and 2-(7-aza-1H-benzotriazole-1-yl)- *N,N,N',N'*--tetramethyluronium hexafluorophosphate (168 mg, 0.44 mmol). The reaction mixture was stirred under argon atmosphere at room temperature for 1.5 hours and then added with 10 mL of water with stirring. The organic phase was washed with saturated sodium chloride solution (10 mL), dried over anhydrous sodium sulfate, filtered, the filtrate was concentrated under reduced pressure. The residues were purified by silica gel column chromatography using eluent system B to give the title product of (1*S*,3*S*,5*S*)-tert-butyl 3-((1*R*,2*R*)-3-((*S*)-1-tert-butoxy-3-(2-chlorophenyl)-1-oxopropan-2-ylamino)-1-methox y-2-methyl-3-oxopropyl)-2-azabicyclo[3.1.0]hexane-2-carboxylate 2c (112 mg, white foam solid), yield 56.8%.
MS m/z (ESI): 537.3 [M+1]

Step3

(*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-(2-chlorophenyl)propanoate

(1*S*,3*S*,5*S*)-tert-butyl 3-((1*R*,2*R*)-3-((*S*)-1-tert-butoxy-3-(2-chlorophenyl)-1-oxopropan-2-ylamino)-1 -methox y-2-methyl-3-oxopropyl)-2-azabicyclo[3.1.0]hexane-2-carboxylate **2c** (110 mg, 0.205 mmol) was dissolved in 2 mL dioxane and then added with 5.6 M solution of hydrogen chloride in dioxane (0.13 mL, 0.717 mmol). The reaction mixture was stirred under argon atmosphere at room temperature for 1 hour, and then placed in a refrigerator at 0°C for 6 hours. Then, the reaction mixture was added with 5 mL of methylene chloride and 10 mL of saturated sodium bicarbonate solution and stirred for 10 minutes. The aqueous phase was extracted with 5 mL of dichloromethane. The combined methylene chloride phase was washed with saturated sodium chloride solution (20 mL), dried over anhydrous sodium sulfate, filtered, the filtrate was concentrated under reduced pressure to give the crude title product of (*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-(2-chlorophenyl)propanoate **2d** (99 mg, yellow liquid). The product was used in the next step without further purification.
MS m/z (ESI): 437.2 [M+1]

Step4

(*S*)-tert-butyl 2-((2*R*,3*R*)-3-((1*S*,3*S*,5*S*)-2-((5*S*,8*S*,11*S*,12*R*)-11-sec-butyl-1-(9*H*-fluoren-9-yl)-5,8-diiso propyl-12-methoxy-4,10-dimethyl-3,6,9-trioxo-2-oxa-4,7,10-triazatetradecane)-2-azabic yclo[3.1.0]hexan-3-yl)-3-methoxy-2-methylpropanamido)-3-(2-chlorophenyl)propanoat

The crude (*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-(2-chlorophenyl)propanoate **2d** (99 mg, 0.226 mmol), (5*S*,8*S*,11*S*,12*R*)-11-((*S*)-sec-butyl)-1-(9*H*-fluoren-9-yl)-5,8-diisopropyl-12-methoxy-4,1 0-dimethyl-3,6,9-trioxo-2-oxa-4,7,10-triazatetradecan-14-oic acid **1i** (144.4 mg, 0.226 mmol) was dissolved in 6 mL of mixed solvent of dichloromethane and dimethylformamide (V/V = 5: 1), and then added with *N,N*-diisopropylethylamine (0.2 mL, 1.13 mmol) and 2- (7-azobenzotriazole) -*N,N,N',N'*-tetramethyluronium hexafluorophosphate (103.3 mg, 0.271 mmol). The reaction mixture was stirred under argon atmosphere at room temperature for 1 hour and then added with 10 mL of water under stirring. The methylene chloride phase was washed with saturated sodium chloride solution (10 mL), dried over anhydrous sodium sulfate, filtrated, and the filtrate was concentrated under reduced pressure. The residues were purified by silica gel column chromatography eluting with eluent system B to give the title product of (*S*)-tert-butyl 2-((2*R*,3*R*)-3-((1*S*,3*S*,5*S*)-2-((5*S*,8*S*,11*S*,12*R*)-11-sec-butyl-1-(9*H*-fluoren-9-yl)-5,8-diiso propyl-12-methoxy-4,10-dimethyl-3,6,9-trioxo-2-oxa-4,7,10-triazatetradecane)-2-azabic yclo[3.1.0]hexan-3-yl)-3-methoxy-2-methylpropanamido)-3-(2-chlorophenyl)propanoat **2e** (127mg, white foam solid), yield 53.1%.
MS m/z (ESI): 1056.4 [M+1]

Step5

(*S*)-tert-butyl 3-(2-chlorophenyl)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino)butanamido)butanamido)-3 -methoxy-5 -methylheptanoyl)-2-a zabicyclo[3.1.0]hexan-3-yl)-3-methoxy-2-methylpropanamido)propanoate

(*S*)-tert-butyl 2-((2*R*,3*R*)-3-((1*S*,3*S*,5*S*)-2-((5*S*,8*S*,11*S*,12*R*)-11-sec-butyl-1-(9*H*-fluoren-9-yl)-5,8-diiso propyl-12-methoxy-4,10-dimethyl-3,6,9-trioxo-2-oxa-4,7,10-triazatetradecane)-2-azabic yclo[3.1.0]hexan-3-yl)-3-methoxy-2-methylpropanamido)-3-(2-chlorophenyl)propanoat **2e** (127 mg, 0.12 mmol) was dissolved in 2 mL of dichloromethane and then added with 2 mL of diethylamine. The reaction mixture was stirred under argon atmosphere at room temperature for 3 hours. Then the reaction solution was concentrated under reduced pressure to give the crude title product of (*S*)-tert-butyl 3-(2-chlorophenyl)-2-((2*R*,3*R*)-3-((1*S*,3*S*,5*S*)-2-((3*R*,4*S*,5*S*)-4-((*S*)-*N*,3-dimethyl-2-((*S*)-3 -methyl-2-(methylamino)butanamido)butanamido)-3 -methoxy-5 -methylheptanoyl)-2-a zabicyclo[3.1.0]hexan-3-yl)-3-methoxy-2-methylpropanamido)propanoate **2f** (130 mg, yellow sticky material). The product was used in the next step without further purification.
MS m/z (ESI): 834.5 [M+1]

Step 6

(*S*)-3-(2-chlorophenyl)-2-((2*R*,3*R*)-3-((1*S*,3*S*,5*S*)-2-((3*R*,4*S*,5*S*)-4-((*S*)-*N*,3-dimethyl-2-(( *S*)-3-methyl-2-(methylamino)butanamido)butanamido)-3-methoxy-5-methylheptanoyl)-2-azabicyclo[3.1.0]hexan-3-yl)-3-methoxy-2-methylpropanamido)propanoic acid

The crude product of (*S*)-tert-butyl 3-(2-chlorophenyl)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methylamino)butanamido)butanamido)-3-methoxy-5-methylheptanoyl)-2-a zabicyclo[3.1.0]hexan-3-yl)-3-methoxy-2-methylpropanamido)propanoate 2f (100 mg, 0.12 mmol) was dissolved in 1 mL dioxane, and then added with 3 mL of a 5.6 M solution of hydrogen chloride in dioxane . The reaction mixture was stirred at room temperature for 12 hours under an argon atmosphere. Then the reaction solution was concentrated under reduced pressure, and the residues were purified by high performance liquid chromatography to give the title product of (*S*)-3-(2-chlorophenyl)-2-((2*R*,3*R*)-3-((1*S*,3*S*,5*S*)-2-((3*R*,4*S*,5*S*)-4-((*S*)-*N*,3-dimethyl-2-(( *S*)-3-methyl-2-(methylamino)butanamido)butanamido)-3-methoxy-5-methylheptanoyl)-2-azabicyclo[3.1.0]hexan-3-yl)-3-methoxy-2-methylpropanamido)propanoic acid 2 (35 mg, white solid), yield 35.7%.
MS m/z (ESI): 778.7 [M+1]
$^1$H NMR (400 MHz, $CD_3OD$): δ 7.41-7.32(m, 2H), 7.31-7.16(m, 2H), 4.80-4.65(m, 2H), 4.22-4.13 (m, 1H), 4.10-4.03(m, 1H), 3.98-3.91(m, 1H), 3.87-3.82(m, 1H), 3.73-3.63(m, 2H), 3.47-3.12(m, 9H), 3.09-3.01(m, 1H), 2.67-2.57(m, 4H), 2.24-2.11 (m, 3H), 2.09-1.98(m, 1H), 1.89-1.67(m, 3H), 1.51-1.25 (m, 4H), 1.20-0.93(m, 18H), 0.92-0.79(m, 4H), 0.75-0.66(m, 1H).

Example3

(*S*)-2-((2*R*,3*R*)-3-((2*S*,5*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-5-methylpyrrolidin-2-yl)-3-methoxy-2-methylpropanamido)-3-phenylpropanoic acid

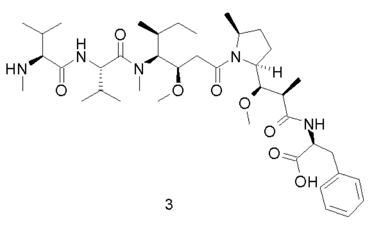

3

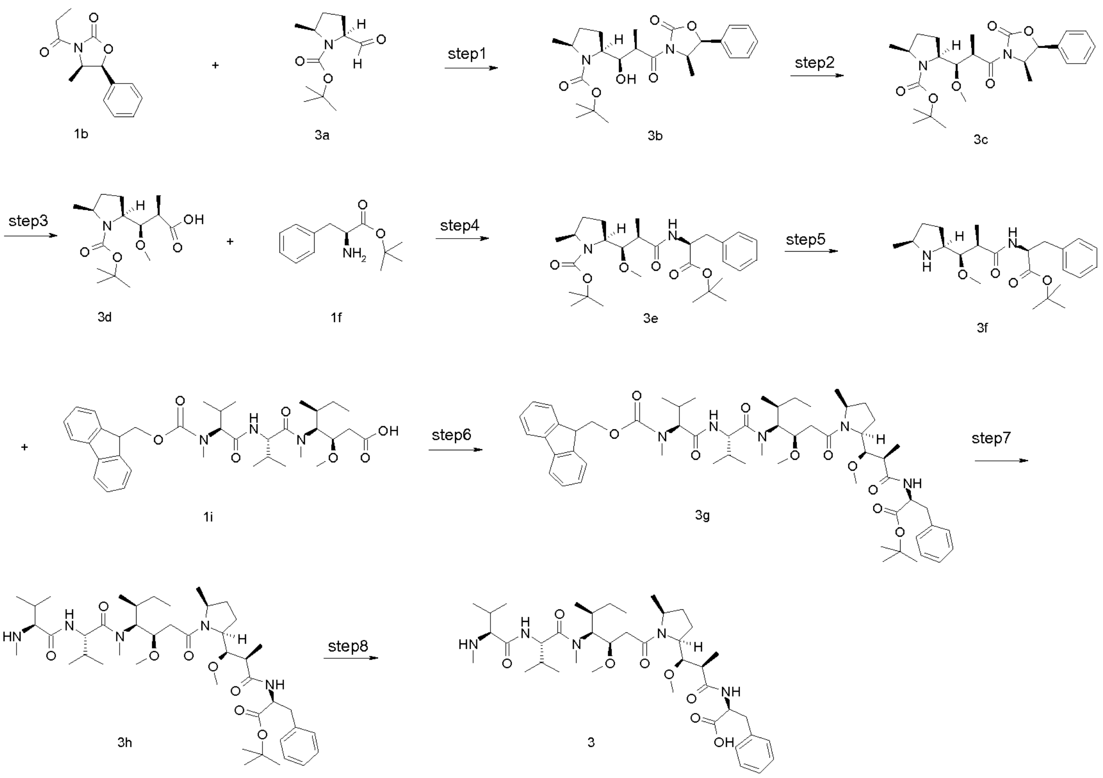


1b 3a 3b 3c 3d 1f 3e 3f 1i 3g 3h 3

step 1

(2*S*,5*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)-5-methylpyrrolidine-1-carboxylate

(4*R*,5*S*)-4-methyl-5-phenyl-3-propionyloxazolidin-2-one **1b** (0.992 g, 4.2 mmol) was dissolved in 20 mL of dichloromethane, and cooled to 0 °C under argon atmosphere. The reaction mixture was added with triethylamine (0.69 mL, 4.96 mmol) and then dibutylboron trifluoromethanesulfonate (4.4 mL, 4.4 mmol) dropwise, and the mixture was stirred at 0°C for 50 minutes. The reaction solution was cooled to -75°C and added with a 5 mL solution of (2*S*,5*S*)-tert-butyl 2-formyl-5-methylpyrrolidine-1-carboxylate 3a (900 mg, 4.2 mmol, prepared according to the known method of " US 20120195857 ") in methylene chloride, and then stirred at -75°C for 1.5 hours, at 0°C for 1.5 hours and at room temperature for 1 hour. Then, the reaction mixture was added with a 36 mL of mixture of phosphate buffer (pH = 7.0) and methanol (V/V = 1: 3) at room temperature. The reaction mixture was cooled to 0°C, and added with a 36 mL of mixture of methanol and hydrogen peroxide (30%) (V/V = 2: 1), and then stirred for 1 hour at room temperature. After completion of the reaction, the organic phase was concentrated under reduced pressure and 15 mL of water was added. The aqueous phase was extracted with ether (30 mL × 3) and the combined ether phases were washed successively with 5% sodium bicarbonate solution, water, saturated sodium chloride solution (150 mL), dried over anhydrous sodium sulfate, filtered, the filtrate was concentrated under reduced pressure. The residues were purified by silica gel column chromatography using eluent system B to give the title product of (2*S*,5*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)-5-methylpyrrolidine-1-carboxylate **3b** (600 mg, white solid), yield 33%.

Step2

(2*S*,5*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)-5-methylpyrrolidine-1-carboxylate

(2*S*,5*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)-5-methylpyrrolidine-1-carboxylate **3b** (600 mg, 1.34 mmol) was dissolved in 15 mL of methylene chloride, and added with 1g of a crushed molecular sieves. The mixture was added with 1,8-bisdimethylaminonaphthalene (740 mg, 3.45 mmol) and trimethyloxonium tetrafluoroborate (500 mg, 3.38 mmol) at 0°C under argon atmosphere. The reaction mixture was wrapped with a tin foil and stirred at room temperature for 38 hours. After completion of the reaction, the

resulting mixture was filtered and the filter cake was washed with dichloromethane. The filtrate was combined and the organic phase was washed with saturated ammonium chloride solution (20 mL × 3) to remove excess 1,8-bis-dimethylaminonaphthalene, then washed with saturated sodium chloride solution and dried over anhydrous sodium sulfate, filtered, and the filtrate was concentrated under reduced pressure. The residues were purified by silica gel column chromatography using eluent system B to give the title product of (2*S*,5*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)-5-methylpyrrolidine-1-carboxylate **3c** (200 mg, white solid), yield 32%.

Step3

(2*R*,3*R*)-3-((2*S*,5*S*)-1-(tert-butoxycarbonyl)-5-methylpyrrolidin-2-yl)-3-methoxy-2-meth ylpropanoic acid

(2*S*,5*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)-5-methylpyrrolidine-1-carboxylate **3c** (200 mg, 0.43 mmol) was dissolved in 22 mL of tetrahydrofuran and cooled to 0°C under argon atmosphere. The mixture was then added dropwise slowly with hydrogen peroxide (186 mg, 1.6 mmol) and lithium hydroxide monohydrate (58 mg, 1.37 mmol). The reaction mixture was strried at 0°C for 10 minutes, and then the ice bath was removed and further strried at room temperature for 44 hours. After completion of the reaction, the reaction solution was added with sodium sulfite solid (220 mg, 1.74 mmol) and stirred at room temperature for 1 hour, and then 15 mL of water was added. The organic phase was concentrated under reduced pressure and the residues were extracted with dichloromethane (20 mL × 2). The aqueous phase was added dropwise with 2N hydrochloric acid until pH of 3 to 4, then extracted with ethyl acetate (20 mL × 3), and the ethyl acetate phase was washed successively with water and saturated sodium chloride solution, dried over anhydrous sodium sulfate, filtered and the filtrate was concentrated under reduced pressure to give the crude title product of (2*R*,3*R*)-3-((2*S*,5*S*)-1-(tert-butoxycarbonyl)-5-methylpyrrolidin-2-yl)-3-methoxy-2-meth ylpropanoic acid **3d** (120 mg, white solid). The product was used in the next step without further purification.

Step4

(2*S*,5*S*)-tert-butyl 2-((1*R*,2*R*)-3-((*S*)-1-tert-butoxy-1-oxo-3-phenylpropan-2-ylamino)-1-methoxy-2-methyl -3-oxopropyl)-5-methylpyrrolidine-1-carboxylate

The crude product of (2*R*,3*R*)-3-((2*S*,5*S*)-1-(tert-butoxycarbonyl)-5-methylpyrrolidin-2-yl)-3-methoxy-2-meth ylpropanoic acid **3d** (106 mg, 0.35 mmol) was dissolved in 4.8 mL of mixed solvent of dichloromethane and dimethylformamide (V/V = 5: 1), and then added with (*S*) -tert-butyl 2-amino-3-phenylpropionic acid **1f** (80 mg, 0.36 mmol). The mixture was added with *N,N*-diisopropylethylamine (0.30 mL, 1.74 mmol) and 2-(7-azobenzotriazole) -*N,N,N',N'*-tetramethyluronium hexafluorophosphate (160 mg, 0.42 mmol) under an argon atmosphere, and then stirred for 2 hours at room temperature. After completion of the reaction, the reaction mixture was added with 10 mL of dichloromethane, and then washed successively with water (5 mL × 2) and saturated sodium chloride solution, dried over anhydrous sodium sulfate, filtered and the filtrate was concentrated under reduced pressure . The residues were purified by silica gel column chromatography using eluent system B to give the title product of (2*S*,5*S*)-tert-butyl 2-((1*R*,2*R*)-3-((*S*)-1-tert-butoxy-1-oxo-3-phenylpropan-2-ylamino)-1-methoxy-2-methyl -3-oxopropyl)-5-methylpyrrolidine-1-carboxylate **3e** (138 mg, colorless viscous liquid), yield 78%.

Step5

(*S*)-tert-butyl 2-((2*R*,3*R*)-3-methoxy-2-methyl-3-((2*S*,5*S*)-5-methylpyrrolidin-2-yl)propanamido)-3-ph enylpropanoate

(2*S*,5*S*)-tert-butyl 2-((1*R*,2*R*)-3-((*S*)-1-tert-butoxy-1-oxo-3-phenylpropan-2-ylamino)-1-methoxy-2-methyl -3-oxopropyl)-5-methylpyrrolidine-1-carboxylate **3e** (150 mg, 0.267 mmol) was dissolved in 2.2 mL of dioxane, and then added with 4 M solution of hydrogen chloride in dioxane (0.160 mL, 0.896 mmol). The reaction system was sealedand stirred for 7 hours at room temperature and then placed in a refrigerator for 16 hours at 4°C. The reaction solution was concentrated under reduced pressure, and the residues were added with 15 mL of dichloromethane, and then cooled to 0°C, and added with saturated sodium bicarbonate solution dropwise to adjust to pH 8. The aqueous phase was extracted with dichloromethane (8 mL × 2) and the organic phases were combined. The organic phase was washed with saturated sodium chloride solution and dried over anhydrous sodium sulfate, filtered and the filtrate was concentrated under reduced pressure to give the title product of (*S*)-tert-butyl 2-((2*R*,3*R*)-3-methoxy-2-methyl-3-((2*S*,5*S*)-5-methylpyrrolidin-2-yl)propanamido)-3-ph enylpropanoate **3f** (80 mg, colorless viscous solid), yield 74%.

Step6

(*S*)-tert-butyl 2-((2*R*,3*R*)-3-((2*S*,5*S*)-1-((5*S*,8*S*,11*S*,12*R*)-11-sec-butyl-1-(9*H*-fluoren-9-yl)-5,8-diisopro pyl-12-methoxy-4,10-dimethyl-3,6,9-trioxo-2-oxa-4,7,10-triazatetradecane)-5-methylpy rrolidin-2-yl)-3-methoxy-2-methylpropanamido)-3-phenylpropanoate

(*S*)-tert-butyl 2-((2*R*,3*R*)-3-methoxy-2-methyl-3-((2*S*,5*S*)-5-methylpyrrolidin-2-yl)propanamido)-3-ph enylpropanoate **3f** (80 mg, 0.198 mmol), (5*S*,8*S*,11*S*,12*R*)-11-((*S*)-sec-butyl)-1-(9*H*-fluoren-9-yl)-5,8-diisopropyl-12-methoxy-4,1 0-dimethyl-3,6,9-trioxo-2-oxa-4,7,10-triazatetradecan-14-oic acid **1i** (136 mg, 0.213 mmol) was dissolved in 4.8 mL of mixed solvent of dichloromethane and dimethylformamide (V/V = 5: 1), and then added with *N,N*-diisopropylethylamine (0.170 mL, 0.98 mmol) and 2- (7-azobenzotriazole) -*N,N,N',N'*-tetramethyluronium hexafluorophosphate (100 mg, 0.263 mmol). The reaction mixture was stirred under argon atmosphere at room temperature for 1 hour. After completion of the reaction, the reaction mixture was added with 15 mL of dichloromethane and washed with water (6 mL × 2). The aqueous phase was extracted with dichloromethane (5 mL), and the combined dichloromethane phases were washed with saturated sodium chloride solution and dried over anhydrous sodium sulfate, filtered and the filtrate was concentrated under reduced pressure. The residues were purified by silica gel column chromatography using eluent system B to give the title product of (*S*)-tert-butyl 2-((2*R*,3*R*)-3-((2*S*,5*S*)-1-((5S,8*S*,11*S*,12*R*)-11-sec-butyl-1-(9*H*-fluoren-9-yl)-5,8-diisopro pyl-12-methoxy-4,10-dimethyl-3,6,9-trioxo-2-oxa-4,7,10-triazatetradecane)-5-methylpy rrolidin-2-yl)-3-methoxy-2-methylpropanamido)-3-phenylpropanoate **3g** (168 mg, white foam solid), yield 81%.

Step7

(*S*)-tert-butyl 2-((2*R*,3*R*)-3-((2*S*,5*S*)-1-((3*R*,4*S*,5*S*)-4-((*S*)-*N*,3-dimethyl-2-((*S*)-3-methyl-2-(methylami no)butanamido)butanamido)-3-methoxy-5-methylheptanoyl)-5-methylpyrrolidin-2-yl)-3 -methoxy-2-methylpropanamido)-3-phenylpropanoate

(*S*)-tert-butyl 2-((2*R*,3*R*)-3-((2*S*,5*S*)-1-((5*S*,8*S*,11*S*,12*R*)-11-sec-butyl-1-(9*H*-fluoren-9-yl)-5,8-diisopro pyl-12-methoxy-4,10-dimethyl-3,6,9-trioxo-2-oxa-4,7,10-triazatetradecane)-5-methylpy rrolidin-2-yl)-3-methoxy-2-methylpropanamido)-3-phenylpropanoate **3g** (167 mg, 0.16 mmol) was dissolved in 2 mL of dichloromethane, and added with 2 mL of diethylamine. The reaction mixture was stirred under argon atmosphere at room temperature for 3 hours. After completion of the reaction, the reaction solution was concentrated under reduced pressure to give the crude title product of (*S*)-tert-butyl 2-((2*R*,3*R*)-3-((2*S*,5*S*)-1-((3*R*,4*S*,5*S*)-4-((*S*)-*N*,3-dimethyl-2-((*S*)-3-methyl-2-(methylami no)butanamido)butanamido)-3-methoxy-5-methylheptanoyl)-5-methylpyrrolidin-2-yl)-3 -methoxy-2-methylpropanamido)-3-phenylpropanoate **3h** (180 mg, yellow liquid), The product was used in the next step without further purification.
MS m/z (ESI): 802.6 [M+1]

Step8

(*S*)-2-((2*R*,3*R*)-3-((2*S*,5*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-5-methylpyrrolidin-2-yl)-3 -methoxy-2-methylpropanamido)-3 -phenylpropanoic acid

The crude product of (*S*)-tert-butyl 2-((2*R*,3*R*)-3-((2*S*,5*S*)-1-((3*R*,4*S*,5*S*)-4-((*S*)-*N*,3-dimethyl-2-((*S*)-3-methyl-2-(methylami no)butanamido)butanamido)-3-methoxy-5-methylheptanoyl)-5-methylpyrrolidin-2-yl)-3 -methoxy-2-methylpropanamido)-3-phenylpropanoate **3h** (130 mg, 0.16 mmol) was dissolved in 1 mL of dioxane, and added with 3 mL of 5.6 M solution of hydrogen chloride in dioxane. The reaction system was sealed and stirred for 12 hours at room temperature. After completion of the reaction, the reaction solution was concentrated under reduced pressure. The residues were purified by high performance liquid chromatography to give the title product of (*S*)-2-((2*R*,3*R*)-3-((2*S*,5*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-5-methylpyrrolidin-2-yl)-3-methoxy-2-methylpropanamido)-3-phenylpropanoic acid **3** (28 mg, white solid), yield 23%.
MS m/z (ESI): 746.7 [M+1]
$^1H$ NMR (400 MHz, $CD_3OD$): δ 7.31-7.14(m, 5H), 4.79-4.67(m, 2H), 4.25-4.13 (m, 1H), 4.10-3.97(m, 2H), 3.77-3.66(m, 1H), 3.60-3.52(m, 1H), 3.51-3.42(m, 1H), 3.41-3.12(m, 7H), 2.97-2.85(m, 1H), 2.67(d, 3H), 2.48-2.40(m, 2H), 2.30-2.02(m, 4H), 1.93-1.73(m, 2H), 1.70-1.55(m, 1H), 1.53-1.28(m, 5H),1.26-1.11(m, 7H), 1.10-0.99(m, 14H), 0.98-0.83(m, 4H).

Example 4

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 *H*-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido)-3-phenylpropanoic acid

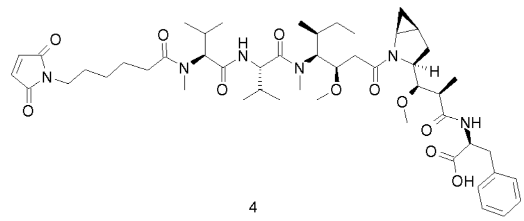

4

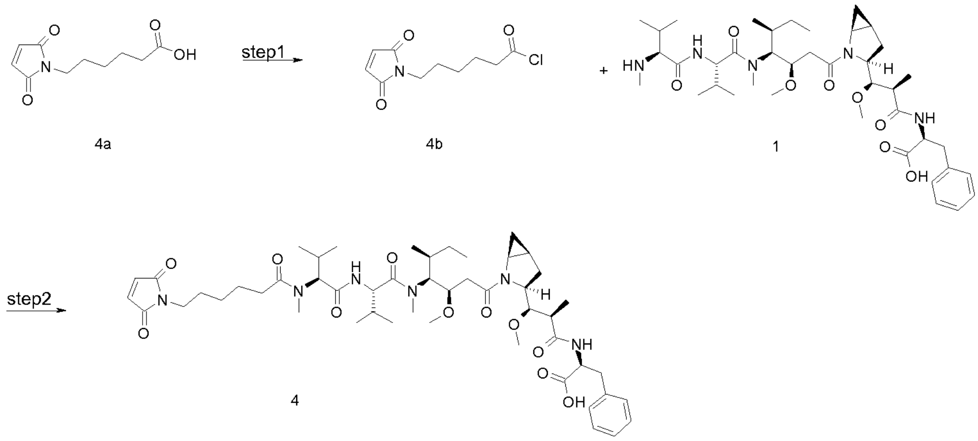


4a 4b 1

4

Step 1

6-(2,5-dioxo-2,5-dihydro-1*H*-pyrrol-1-yl)hexanoyl chloride

6-(2,5-dioxo-2,5-dihydro-1*H*-pyrrol-1-yl)hexanoic acid **4a** (1.5 g, 7.10 mmol, prepared according to the known method of "Journal of Medcinal Chemistry, 2013, 56(24), 9955-9968") was added with a drop of *N, N*-dimethylformamide, and then added with 15 mL of oxalyl chloride dropwise with vigorous stirring under an argon atmosphere after cooling in a dry ice bath. Then the reaction mixture was stirred at room temperature for 1 hour. After completion of the reaction, the reaction mixture was concentrated under reduced pressure, and the residues were dissolved in methylene chloride and concentrated under reduced pressure to give the crude title product of 6-(2,5-dioxo-2,5-dihydro-1*H*-pyrrol-1-yl)hexanoyl chloride **4b.** The product was used in the next step without further purification.

Step2

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 *H*-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido)-3-phenylpropanoic acid

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo [3.1.0]hexan-3-yl)-3-methoxy-2-methylpropanamido)-3-phenylpropanoic acid **1** (17 mg, 0.023 mmol) was dissolved in 1 mL of dichloromethane and added with *N, N*-diisopropylethylamine (20 μL, 0.115 mmol). Then the mixture was added dropwise with a preformed solution of 6- (2,5-dioxo-2,5-dihydro-1*H*-pyrrol-1-yl) hexanoyl chloride **4b** (7.9 mg, 0.034 mmol) in dichloromethane under an argon atmosphere in an ice bath, and then stirred at room temperature for 5 hours. After completion of the reaction, 10 mL of methanol was added and the reaction mixture was concentrated under reduced pressure. The residues were purified by high

performance liquid chromatography to give the title product of (*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 *H*-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido)-3-phenylpropanoic acid **4** (9 mg, white solid), yield 42%

MS m/z (ESI): 937.4 [M+1]

$^1$H NMR (400 MHz, $CD_3OD$): δ 7.37-7.17(m, 5H), 6.84-6.79(m, 2H), 4.80-4.71(m, 2H), 4.69-4.56(m, 2H), 4.26-4.19(m, 1H), 4.14-4.07(m, 1H), 3.92-3.86(m, 1H), 3.84-3.78(m, 1H), 3.77-3.60(m, 1H), 3.55-3.47(m, 2H), 3.42-3.23(m, 5H), 3.18-3.12 (m, 2H), 3.07-3.03(m, 2H), 3.02-2.82(m, 2H), 2.66-2.58(m, 2H), 2.54-2.46(m, 1H), 2.46-2.38(m, 2H), 2.30-2.14(m, 2H), 2.09-1.99(m, 1H), 1.90-1.78(m, 1H), 1.75-1.56 (m, 6H),1.48-1.28(m, 6H), 1.20-0.79(m, 22H), 0.77-0.69(m,1H).

Example5

(*S*)-3-(2-chlorophenyl)-2-((2*R*,3*R*)-3-((1*S*,3*S*,5*S*)-2-((3*R*,4*S*,5*S*)-4-((*S*)-2-((*S*)-2-(6-(2,5-d ioxo-2,5-dihydro-1*H*-pyrrol-1-yl)-*N*-methylhexanamido)-3-methylbutanamido)-*N*,3-dim ethylbutanamido)-3-methoxy-5-methylheptanoyl)-2-azabicyclo[3.1.0]hexan-3-yl)-3-met hoxy-2-methylpropanamido)propanoic acid

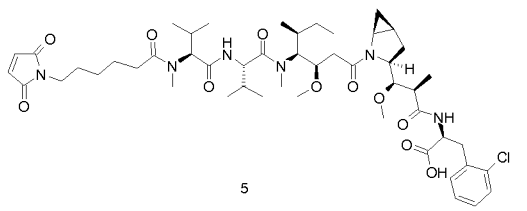

5

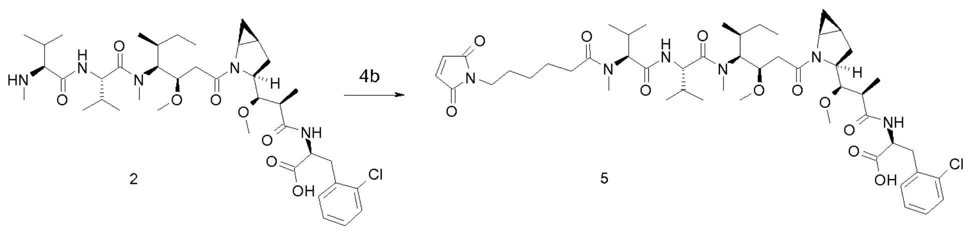


2

5

(*S*)-3-(2-chlorophenyl)-2-((2*R*,3*R*)-3-((1*S*,3*S*,5*S*)-2-((3*R*,4*S*,5*S*)-4-((*S*)-*N*,3-dimethyl-2-(( *S*)-3-methyl-2-(methylamino)butanamido)butanamido)-3-methoxy-5-methylheptanoyl)-2-azabicyclo[3.1.0]hexan-3-yl)-3-methoxy-2-methylpropanamido)propanoic acid **2** (18 mg, 0.023 mmol) was dissolved in 1 mL of dichloromethane and added with *N*,*N*-diisopropylethylamine (0.02 mL, 0.115 mmol) . The mixture was added dropwise with a preformed solution of 6- (2,5-dioxo-2,5-dihydro-1H-pyrrol-1-yl) hexanoyl chloride **4b** (7.9 mg, 0.034 mmol) in dichloromethane under argon atmosphere in an ice bath, and then stirred at room temperature for 4 hours. The reaction was quenched with 5 mL of methanol and then concentrated under reduced pressure. The residues were purified by high performance liquid chromatography to give the title product of (*S*)-3-(2-chlorophenyl)-2-((2*R*,3*R*)-3-((1*S*,3*S*,5*S*)-2-((3*R*,4*S*,5*S*)-4-((*S*)-2-((*S*)-2-(6-(2,5-d ioxo-2,5-dihydro-1*H*pyrrol-1-yl)-*N*-methylhexanamido)-3-methylbutanamido)-*N*,3-dim ethylbutanamido)-3-methoxy-5-methylheptanoyl)-2-azabicyclo[3.1.0]hexan-3-yl)-3-met hoxy-2-methylpropanamido)propanoic acid **5** (6 mg, white solid) , yield 26.6%.

MS m/z (ESI): 971.5 [M+1]

$^1$H NMR (400 MHz, $CD_3OD$): δ 7.42-7.34(m, 2H), 7.33-7.19(m, 2H), 6.83-6.78(m, 2H), 4.83-4.70(m, 2H), 4.68-4.56 (m, 2H), 4.24-4.16(m, 1H), 4.13-4.05(m, 1H), 4.03-3.95(m, 1H), 3.91-3.84(m, 1H), 3.76-3.65(m, 1H), 3.54-3.48(m, 2H), 3.47-3.18(m, 5H), 3.17-2.96(m, 6H), 2.67-2.58(m, 2H), 2.53-2.38(m, 3H), 2.28-2.18(m, 2H), 2.09-2.00(m, 1H), 1.92-1.57(m, 7H), 1.51-1.28(m, 6H), 1.21-0.82(m, 22H), 0.78-0.69(m, 1H).

Example 6

(*S*)-2-((2*R*,3*R*)-3-((2*S*,5*S*)-1-((3*R*,4*S*,5*S*)-4-((*S*)-2-((*S*)-2-(6-(2,5-dioxo-2,5-dihydro-1*H*-p yrrol-1-yl)-*N*-methylhexanamido)-3-methylbutanamido)-*N*,3-dimethylbutanamido)-3-m ethoxy-5-methylheptanoyl)-5-methylpyrrolidin-2-yl)-3-methoxy-2-methylpropanamido) -3-phenylpropanoic acid

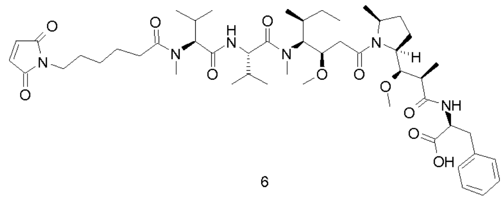

6

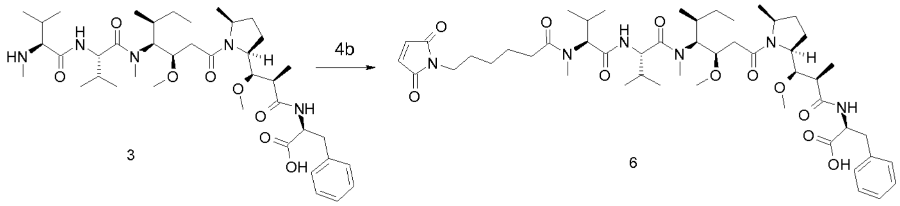


3

6

(*S*)-2-((2*R*,3*R*)-3-((2*S*,5*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-5-methylpyrrolidin-2-yl)-3-methoxy-2-methylpropanamido)-3-phenylpropanoic acid **3** (18 mg, 0.024 mmol) was dissolved in 1 mL of dichloromethane and added with *N*, *N*-diisopropylethylamine (0.02 mL, 0.12 mmol) The mixture was added dropwisely with a preformed solution of 6-(2,5-dioxo-2,5-dihydro-1*H*-pyrrol-1-yl) hexanoyl chloride **4b** (8.3 mg, 0.036 mmol) in dichloromethane under argon atmosphere in an ice bath. The above reaction mixture was stirred at room temperature for 4 hours. The reaction was quenched with 5 mL of methanol and then concentrated under reduced pressure. The residues were purified by high performance liquid chromatography to give the title product of (*S*)-2-((2*R*,3*R*)-3-((2*S*,5*S*)-1-((3*R*,4*S*,5*S*)-4-((*S*)-2-((*S*)-2-(6-(2,5-dioxo-2,5-dihydro-1*H*-p yrrol-1-yl)-*N*-methylhexanamido)-3-methylbutanamido)-*N*,3-dimethylbutanamido)-3-m ethoxy-5-methylheptanoyl)-5-methylpyrrolidin-2-yl)-3-methoxy-2-methylpropanamido) -3-phenylpropanoic acid **6** (7 mg, white solid), yield 30.9%.

MS m/z (ESI): 939.5 [M+1]

$^1$H NMR (400 MHz, $CD_3OD$): δ 7.30-7.15(m, 5H), 6.83-6.78(m, 2H), 4.78-4.69(m, 2H), 4.69-4.56 (m, 2H), 4.24-4.12(m, 1H), 4.10-3.96(m, 2H), 3.60-3.44(m, 3H), 3.41-3.22(m, 4H), 3.16-3.10(m, 2H), 3.07-3.02(m, 2H), 3.01-2.86(m, 2H), 2.52-2.38(m, 4H), 2.31-2.15(m, 3H), 2.09-1.99(m, 1H), 1.91-1.77(m, 2H), 1.71-1.56(m, 5H), 1.52-1.28(m, 9H), 1.26-1.14(m, 6H), 1.11-0.77(m,18H).

Example7

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(2-fluorophenyl)propanoic acid

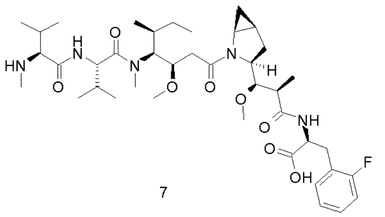

7

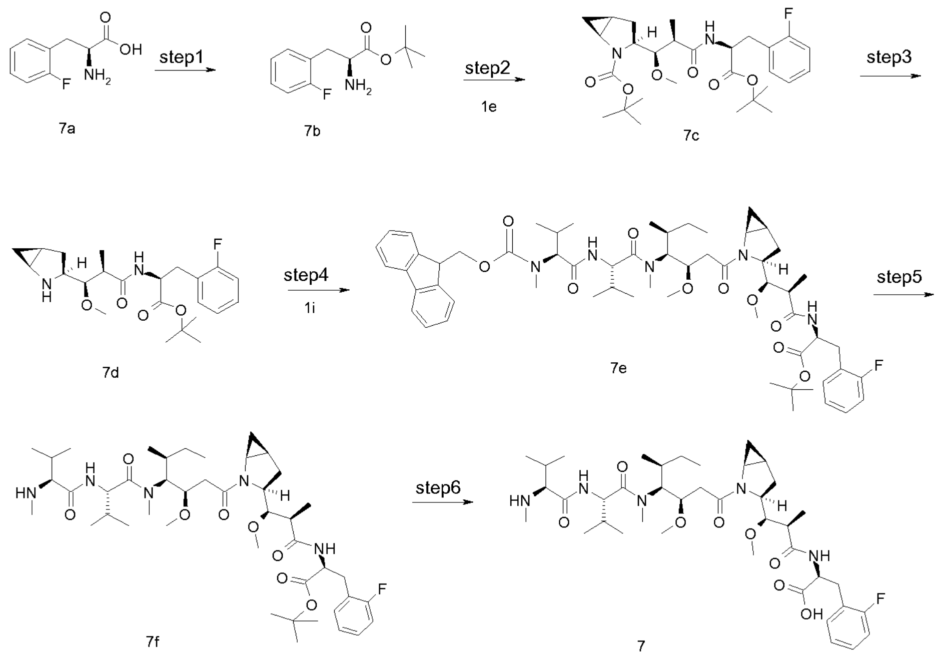


Step 1

(S)-tert-butyl 2-amino-3-(2-fluorophenyl)propanoate

(S)- butyl 2-amino-3-(2-fluorophenyl)propanoate **7a** (400 mg, 2.18 mmol, prepared according to the known method of "Advanced Synthesis & Catalysis, 2012, 354(17), 3327-3332") was dissolved in 10 mL of tert-butyl acetate, and added wutg perchloric acid (300 mg(70%), 3.3 mmol) . The mixture was stirred at room temperature for 16 hours. After the reaction, 6 mL of water was added and the organic phase was washed with saturated sodium bicarbonate solution (3 mL). The aqueous phase was adjusted to pH = 8 with saturated sodium bicarbonate solution and extracted with dichloromethane (5 mL ×3). The organic phases were combined, washed successively with water (3 mL) and saturated sodium chloride solution (5 mL), dried over anhydrous sodium sulfate, filtered and the filtrate was concentrated under reduced pressure to obtain the crude title product of (*S*)-tert-butyl 2-amino-3-(2-fluorophenyl)propanoate **7b** (390 mg, yellow oil). The product was used in the next step without further purification.

Step 2

(1*S*,3*S*,5*S*)-tert-butyl 3-((1*R*,2*R*)-3-(((*S*)-1-(tert-butoxy)-3-(2-fluorophenyl)-1-oxopropan-2-yl)amino)-1-meth oxy-2-methyl-3-oxopropyl)-2-azabicyclo[3.1.0]hexane-2-carboxylate

(2*R*,3*R*)-3-((1*S*,3*S*,5*S*)-2-(tert-butoxycarbonyl)-2-azabicyclo[3.1.0]hexan-3-yl)-3-metho xy-2-methylpropanoic acid **1e** (100 mg, 0.334 mmol) was dissolved in 6 mL of mixed solvent of dichloromethane and dimethylformamide (V/V = 5: 1) , and added with the crude product of (*S*)-tert-butyl 2-amino-3-(2-fluorophenyl)propanoate **7b** (80 mg, 0.334 mmol), *N,N*-diisopropylethylamine (0.29 mL, 1.67 mmol) and 2-(7-azobenzotriazole)-*N*,*N*,*N'*,*N'*-tetramethyluronium hexafluorophosphate (152.3 mg, 0.40 mmol). The reaction mixture was stirred under argon atmosphere at room temperature for 1 hour. After completion of the reaction, the reaction mixture was added with 10 mL of water under stirring, and the dichloromethane phase was washed with saturated sodium chloride solution (10 mL), dried over anhydrous sodium sulfate, filtered and the filtrate was concentrated under reduced pressure . The residues were purified by silica gel column chromatography using eluent system B to give the title product of (1*S*,3*S*,5*S*)-tert-butyl 3-((1*R*,2*R*)-3-(((*S*)-1-(tert-butoxy)-3-(2-fluorophenyl)-1-oxopropan-2-yl)amino)-1-meth oxy-2-methyl-3-oxopropyl)-2-azabicyclo[3.1.0]hexane-2-carboxylate **7c** (173 mg, colorless liquid), yield 99.5%.
MS m/z (ESI): 521.2 [M+1]

Step 3

(*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-(2-fluorophenyl)propanoate

(1*S*,3*S*,5*S*)-tert-butyl 3-((1*R*,2*R*)-3-(((*S*)-1-(tert-butoxy)-3-(2-fluorophenyl)-1-oxopropan-2-yl)amino)-1-meth oxy-2-methyl-3-oxopropyl)-2-azabicyclo[3.1.0]hexane-2-carboxylate **7c** (173 mg, 0.33 mmol) was dissolved in 2 mL of dioxane, and added with 5.6 M solution of hydrogen chloride in dioxane (0.21 mL, 1.16 mmol). The mixture was stirred under argon atmosphere for 1 hour at room temperature and then placed in a refrigerator for 12 hours at 0°C. The reaction solution was then concentrated under reduced pressure, and added with 5 mL of dichloromethane and 10 mL of saturated sodium bicarbonate solution, and stirred for 10 minutes. The aqueous phase was extracted with dichloromethane (5 mL × 3). The combined dichloromethane phase was washed with saturated sodium chloride solution (10 mL), dried over anhydrous sodium sulfate, filtered and the filtrate was concentrated under reduced pressure to give the crude title product of (*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-(2-fluorophenyl)propanoate **7d** (77 mg, yellow liquid). The product was used in the next step without further purification.
MS m/z (ESI): 421.2 [M+1]

Step 4

(*S*)-tert-butyl 2-((2*R*,3*R*)-3-((1*S*,3*S*,5*S*)-2-((5*S*,8*S*,11*S*,12*R*)-11-((*S*)-sec-butyl)-1-(9*H*-fluoren-9-yl)-5,8 -diisopropyl-12-methoxy-4,10-dimethyl-3,6,9-trioxo-2-oxa-4,7,10-triazatetradecan-14-o yl)-2-azabicyclo[3.1.0]hexan-3-yl)-3-methoxy-2-methylpropanamido)-3-(2-fluoropheny l)propanoate

The crude product of (*S*)-tert-butyl 2-((2*R*,3*R*)-3-((1*S*,3*S*,5*S*)-2-azabicyclo[3.1.0]hexan-3-yl)-3-methoxy-2-methylpropanam ido)-3-(2-fluorophenyl)propanoate **7d** (77 mg, 0.183 mmol), (5*S*,8*S*,11*S*,12*R*)-11-((*S*)-sec-butyl)-1-(9*H*-fluoren-9-yl)-5,8-diisopropyl-12-methoxy-4,1 0-dimethyl-3,6,9-trioxo-2-oxa-4,7,10-triazatetradecan-14-oic acid **1i** (116.8 mg, 0.183 mmol) was dissolved in 6 mL of mixed solvent of dichloromethane and dimethylformamide (V/V = 5: 1), and then added with *N*, *N*-diisopropylethylamine (0.16 mL, 0.915 mmol) and 2- (7-azobenzotriazole) *-N, N, N', N'*-tetramethyluronium hexafluorophosphate (84 mg, 0.22 mmol). The reaction mixture was stirred under argon atmosphere at room temperature for 1 hour. The reaction mixture was then added with 6 mL of water and the dichloromethane phase was washed with saturated sodium chloride solution (10 mL) and dried over anhydrous sodium sulfate, filtrated and the filtrate was concentrated under reduced pressure. The residues were purified by silica gel column chromatography using eluent system B to give the title product of (*S*)-tert-butyl 2-((2*R*,3*R*)-3-((1*S*,3*S*,5*S*)-2-((5*S*,8*S*,11*S*,12*R*)-11-((*S*)-sec-butyl)-1-(9*H*-fluoren-9-yl)-5,8 -diisopropyl-12-methoxy-4,10-dimethyl-3,6,9-trioxo-2-oxa-4,7,10-triazatetradecan-14-o yl)-2-azabicyclo[3.1.0]hexan-3-yl)-3-methoxy-2-methylpropanamido)-3-(2-fluoropheny l)propanoate 7e (190.5 mg, yellow sticky material), yield 100%.
MS m/z (ESI): 1040.6 [M+1]

Step 5

(*S*)-tert-butyl 2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyla mino)butanamido)butanamido)-3-methoxy-5-methylheptanoyl)-2-azabicyclo[3.1.0]hexa n-3-yl)-3-methoxy-2-methylpropanamido)-3-(2-fluorophenyl)propanoate (*S*)-tert-butyl

2-((2*R*,3*R*)-3-((1*S*,3*S*,5*S*)-2-((5*S*,8*S*,11*S*,12*R*)-11-((*S*)-sec-butyl)-1-(9*H*-fluoren-9-yl)-5,8 -diisopropyl-12-methoxy-4,10-dimethyl-3,6,9-trioxo-2-oxa-4,7,10-triazatetradecan-14-o yl)-2-azabicyclo[3.1.0]hexan-3-yl)-3-methoxy-2-methylpropanamido)-3-(2-fluoropheny l)propanoate **7e** (190.5 mg, 0.183 mmol) was dissolved in 1.5 mL of dichloromethane and added with 2mL of diethylamine. The reaction mixture was stirred under argon atmosphereatmosphere at room temperature for 3 hours. After completion of the reaction, the reaction solution was concentrated under reduced pressure to give the crude title product of (*S*)-tert-butyl 2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyla mino)butanamido)butanamido)-3-methoxy-5-methylheptanoyl)-2-azabicyclo[3.1.0]hexa n-3-yl)-3-methoxy-2-methylpropanamido)-3-(2-fluorophenyl)propanoate **7f** (150 mg, yellow sticky material). The product was used in the next step without further purification.

MS m/z (ESI): 818.5 [M+1]

Step 6

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(2-fluorophenyl)propanoic acid

The crude product of (*S*)-tert-butyl 2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyla mino)butanamido)butanamido)-3-methoxy-5-methylheptanoyl)-2-azabicyclo[3.1.0]hexa n-3-yl)-3-methoxy-2-methylpropanamido)-3-(2-fluorophenyl)propanoate **7f** (150 mg, 0.183 mmol) was dissolved in 1 mL of dioxane, and added with 3 mL of 5.6 M solution of hydrogen in chloride dioxane. The resulting mixture was stirred under argon atmosphere for 12 hours at room temperature. After completion of the reaction, the reaction solution was concentrated under reduced pressure, and the redidual solvent was spin-coated with ethylether. The residues were purified by high performance liquid chromatography to give the title product of (*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(2-fluorophenyl)propanoic acid 7 (28 mg, white solid), yield 20%.

MS m/z (ESI): 762.7 [M+1]

$^1$H NMR (400 MHz, $CD_3OD$): δ 7.38-7.18(m, 2H), 7.13-7.01(m, 2H), 4.80-4.67(m, 2H), 4.30-4.15(m, 1H), 4.13-4.01(m, 1H), 3.96-3.83(m, 2H), 3.75-3.60(m, 2H), 3.42-3.11(m, 9H), 3.06-2.95(m, 1H), 2.70-2.58(m, 4H), 2.28-2.01 (m, 4H), 1.88-1.70(m, 3H), 1.57-1.25 (m, 4H), 1.22-0.95(m, 18H), 0.92-0.80(m, 4H), 0.78-0.65(m, 1H).

Example 8

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 H-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido)-3-(2-fluorophenyl)propanoic acid

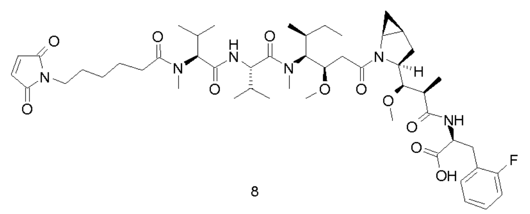

8

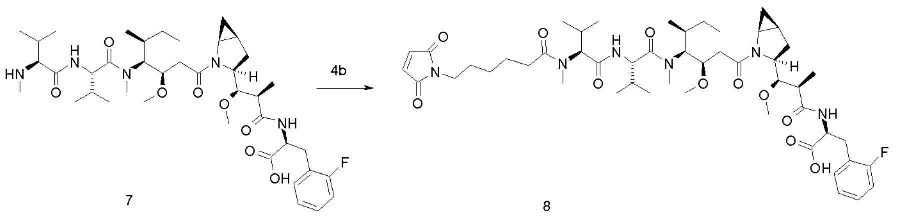


7 8

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(2-fluorophenyl)propanoic acid 7 (25 mg, 0.033 mmol) was dissolved in 3 mL of dichloromethane and added with *N*,*N*-diisopropylethylamine(0.029 mL, 0.164 mmol) The mixture was added dropwise with a preformed solution of 6- (2,5-dioxo-2,5-dihydro-1*H*-pyrrol-1-yl) hexanoyl chloride **4b** (11.3 mg, 0.049 mmol) in dichloromethane under argon atmosphere in ice bath, and then stirred at room temperature for 3 hours. The reaction mixture was then added with 5 mL of water and stirred for 20 minutes. The organic phase was dried over anhydrous sodium sulfate, filtered, and the filtrate was concentrated under reduced pressure. The residues were purified by high performance liquid chromatography to give the title product of (*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 H-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido)-3-(2-fluorophenyl)propanoic acid **8** (7 mg, yellow sticky material), yield 22.4%.

MS m/z (ESI): 955.4 [M+1]

$^1$H NMR (400 MHz, $CD_3OD$): δ 7.36-7.30(m, 1H), 7.29-7.21(m, 1H), 7.17-7.02(m, 2H), 6.83-6.79(m, 2H), 4.81-4.71(m, 2H), 4.69-4.55 (m, 2H), 4.25-4.15(m, 1H), 4.13-4.04(m, 1H), 3.96-3.85(m, 2H), 3.70-3.61(m, 1H), 3.55-3.46(m, 3H), 3.40-3.21 (m, 4H), 3.18-3.10(m, 2H), 3.07-2.96(m, 4H), 2.67-2.56(m, 2H), 2.54-2.34(m, 3H), 2.29-2.17(m, 2H), 2.10-1.99(m, 1H), 1.89-1.57(m, 7H), 1.52-1.28(m, 6H), 1.21-1.11 (m, 4H), 1.07-0.96(m, 6H), 0.95-0.81(m, 12H), 0.80-0.69(m, 1H).

Example9

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-4-methylenepyrrolidin-2-yl )-3-methoxy-2-methylpropanamido)-3-phenylpropanoic acid

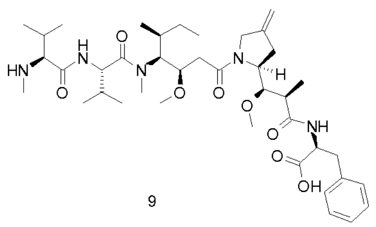

9

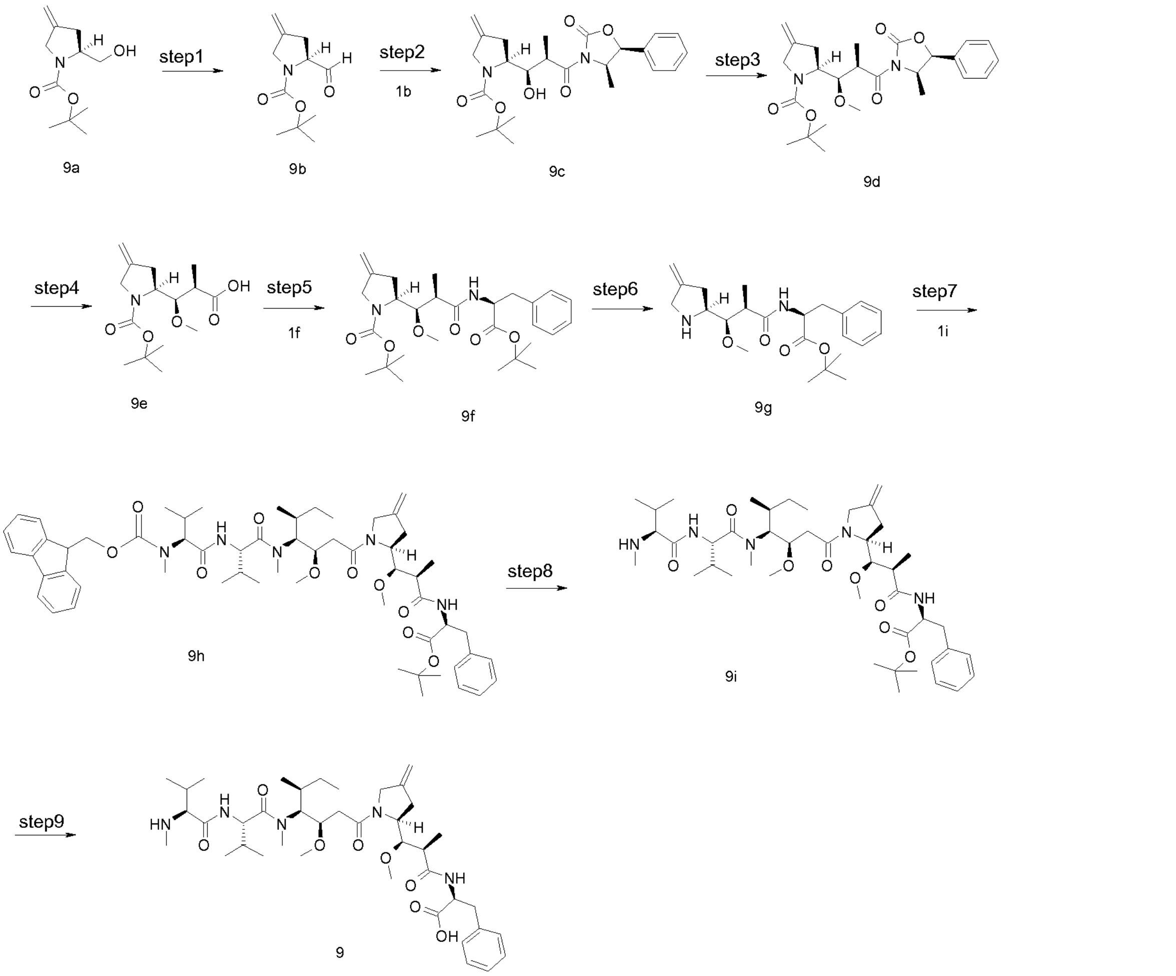


9a 9b 9c 9d 9e 9f 9g 9h 9i 9

Step 1

(*S*)-tert-butyl 2-formyl-4-methylenepyrrolidine-1-carboxylate

(*S*)-tert-butyl 2-(hydroxymethyl)-4-methylenepyrrolidine-1-carboxylate **9a** (1.32 g, 6.19 mmol, prepared according to the known method of "From Journal of Organic Chemistry, 2003, 68(10), 3923-3931") was dissolved in 15 mL of dichloromethane and cooled to 0°C. The mixture was added with *N,N*-diisopropylethylamine(5.38 mL, 30.9 mmol), dimethyl sulfoxide(7.26 g, 92.9 mmol) and sulfur trioxide-pyridine complex (7.26 g, 92.9 mmol) under argon atmosphere, and stirred at 0°C for 3 hours. The reaction was then quenched by phosphate buffer (pH = 7), and washed successively with water and saturated sodium chloride solution, dried over anhydrous sodium sulfate, filtered and the filtrate was concentrated under reduced pressure , . The residues were purified by silica gel column chromatography using eluent system B to give the title product of (*S*)-tert-butyl 2-formyl-4-methylenepyrrolidine-1-carboxylate **9b** (1.1 g, light yellow liquid), yield 84.2%.

Step2

(*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)-4-methylenepyrrolidine-1-carboxylate

(4*R*,5*S*)-4-methyl-5-phenyl-3-propionyloxazolidin-2-one **1b** (1.43 g, 6.15 mmol) was dissolved in 30 mL of dichloromethane, and cooled to 0°C under argon atmosphere. The solution was added dropwise with triethylamine (0.98 mL, 7.08 mmol) and dibutylboron trifluoromethanesulfonate (6.65 mL, 6.65 Mmol), and then stirred at 0 °C for 50 min. The reaction mixture was cooled to -78°C and added with preformed solution of (*S*)-tert-butyl 2-formyl-4-methylenepyrrolidine-1-carboxylate **9b** (1.43 g, 6.15 mmol) in dichloromethane, then the mixture was stirred at -78 °C for 2 hours, at 0 °C for 1 hour, at room temperature for 1 hour. The reaction mixture was quenched by a mixture of 36 mL of phosphate buffer (pH = 7) and methanol (V/V = 3: 1), then added with a mixture of methanol and hydrogen peroxide (V/V = 1: 2) at 0°C, and stirred at room temperature for 1 hour. After completion of the reaction, the methanol and the organic phase were removed. The residual aqueous phase was extracted with methylene chloride, the organic phases were combined, washed successively with water and saturated sodium chloride solution, dried over anhydrous sodium sulfate, filtered and the filtrate was concentrated under reduced pressure . The residues were purified by silica gel column chromatography using eluent system B to give the title product of (*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)-4-methylenepyrrolidine-1-carboxylate **9c** (810 mg, white foam solid), yield 30%.
MS m/z (ESI): 354.2 [M-100+1]

Step3

(*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)-4-methylenepyrrolidine-1-carboxylate

(*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)-4-methylenepyrrolidine-1-carboxylate **9c** (810 mg, 1.82 mmol) was dissolved in 15 mL of dichloromethane, and added with crushed molecular sieves and potassium carbonate (1.25 g, 9.11 mmol), methyl trifluoromethanesulfonate (897.7 mg, 5.47 mmol), then the mixture was stirred at room temperature for 12 hours. After completion of the reaction, the reaction solution was filtered, the filter cake was washed with dichloromethane. The organic phases were combined and concentrated under reduced pressure. The residues were purified by silica gel column chromatography using eluent system B to give the title product of (*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)-4-methylenepyrrolidine-1-carboxylate **9d** (280 mg, white foam solid), yield 33.5%.
MS m/z (ESI): 359.2 [M-100+1]

Step4

(2*R*,3*R*)-3-((*S*)-1-(tert-butoxycarbonyl)-4-methylenepyrrolidin-2-yl)-3-methoxy-2-meth ylpropanoic acid

(*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)-4-methylenepyrrolidine-1-carboxylate **9d** (400 mg, 0.87 mmol) was dissolved in 20 mL of tetrahydrofuran, and added with lithium hydroxide monohydrate (62.2 mg, 1.484 mmol). The reaction mixture was cooled to 0°C under argon atmosphere, and then added with 30% hydrogen peroxide (112.7 mg, 3.31 mmol) dropwise. The mixture was allowed to

react at room temperature for 12 hours. After completion of the reaction, sodium sulfite (416 mg, 3.3 mmol) was added to the reaction solution, and the mixture was stirred at room temperature for 1 hour. The tetrahydrofuran was concentrated under reduced pressure and the residues were dissolved in water and extracted with dichloromethane. The aqueous phase was diluted with dilute hydrochloric acid to adjust the pH to 3 to 4, and then extracted with dichloromethane (30 mL × 3). The combined organic phases were washed successively with water and saturated sodium chloride solution, dried over anhydrous sodium sulfate, filtered and the filtrate was concentrated under reduced pressure to give the crude title product of (2*R*,3*R*)-3-((*S*)-1-(tert-butoxycarbonyl)-4-methylenepyrrolidin-2-yl)-3-methoxy-2-meth ylpropanoic acid **9e** (230 mg, colorless liquid). The product was used in the next step without further purification.
MS m/z (ESI): 298.2 [M-1]

Step5

(*S*)-tert-butyl 2-((1*R*,2*R*)-3-(((*S*)-1-(tert-butoxy)-1-oxo-3-phenylpropan-2-yl)amino)-1-methoxy-2-met hyl-3-oxopropyl)-4-methylenepyrrolidine-1-carboxylate

(2*R*,3*R*)-3-((*S*)-1-(tert-butoxycarbonyl)-4-methylenepyrrolidin-2-yl)-3-methoxy-2-meth ylpropanoic acid **9e** (220 mg, 0.735 mmol) was dissolved in 6 mL of dichloromethane, and added with (*S*)-tert-butyl 2-amino-3-phenylpropanoate **If** (178.8 mg, 0.809 mmol), *N,N*-diisopropylethylamine (0.51 mL, 2.94 mmol) and 2- (7-azobenzotriazole) -*N,N,N ',N'*-tetramethyluronium hexafluorophosphate (363 mg, 0.956 mmol). The reaction mixture was stirred under argon atmosphere at room temperature for 3 hours. After completion of the reaction, the reaction solution was washed successively with water and a saturated sodium chloride solution,dried over anhydrous sodium sulfate, filtered and the filtrate was concentrated under reduced pressure . The residues were purified by silica gel column chromatography using eluent system B to give the title product of (*S*)-tert-butyl 2-((1*R*,2*R*)-3-(((*S*)-1-(tert-butoxy)-1-oxo-3-phenylpropan-2-yl)amino)-1-methoxy-2-met hyl-3-oxopropyl)-4-methylenepyrrolidine-1-carboxylate **9f** (271 mg, white solid), yield 66%.
MS m/z (ESI): 503.3 [M+1]

Step 6

(*S*)-tert-butyl 2-((2*R*,3*R*)-3-methoxy-2-methyl-3-((*S*)-4-methylenepyrrolidin-2-yl)propanamido)-3-phe nylpropanoate

(*S*)-tert-butyl 2-((1*R,2*R)-*3*-(((*S*)-1-(tert-butoxy)-1-oxo-3-phenylpropan-2-yl)amino)-1-methoxy-2-met hyl-3-oxopropyl)-4-methylenepyrrolidine-1-carboxylate **9f** (270 mg, 0.537 mmol) was dissolved in 4 mL of 1,4-dioxane, and added with 4 *M* solution of hydrogen in chloride dioxane (0.335 mL, 1.881 mmol). The mixture was stirred for 1 hour at room temperature and placed in a refrigerator for 12 hours at 0~4°C. The reaction mixture was concentrated under reduced pressure, and the residues were dissolved in methylene chloride and added with saturated sodium bicarbonate solution to adjust the pH to 8 to 9. The aqueous phase was extracted with methylene chloride. The organic phases were combined, washed successively with water and saturated sodium chloride solution, dried over anhydrous sodium sulfate, filtered and the filtrate was concentrated under reduced pressure to give the crude title product of (*S*)-tert-butyl 2-((2R,3R)-3-methoxy-2-methyl-3-((*S*)-4-methylenepyrrolidin-2-yl)propanamido)-3-ph enylpropanoate **9g** (210 mg, lightyellow oil). The product was used in the next step without further purification.
MS m/z (ESI): 403.4 [M+1]

Step7

(*S*)-tert-butyl 2-((2*R*,3*R*)-3-((*S*)-1-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)-4-m ethylenepyrrolidin-2-yl)-3-methoxy-2-methylpropanamido)-3-phenylpropanoate

The crude product of (*S*)-tert-butyl 2-((2*R*,3*R*)-3-methoxy-2-methyl-3-((*S*)-4-methylenepyrrolidin-2-yl)propanamido)-3-phe nylpropanoate **9g** (210 mg, 0.521 mmol), (5*S*,8*S*,11*S*,12*R*)-11-((*S*)-sec-butyl)-1-(9*H* fluoren-9-yl)-5,8-diisopropyl-12-methoxy-4,10-dimethyl-3,6,9-trioxo-2-oxa-4,7,10-triazatetradecan -14-oic acid **1i** (365.9 mg, 0.547 mmol) was dissolved in 6 mL of dichloromethane, and added with *N,N*-diisopropylethylamine (0.4537 mL, 2.609 mmol) and 2- (7-azobenzotriazole) -*N, N, N*', *N'*-tetramethyluronium hexafluorophosphate (257.8 mg, 0.678 mmol). The reaction mixture was stirred under argon atmosphere at room temperature for 2 hours. After completion of the reaction, the reaction solution was washed successively with water and a saturated sodium chloride solution, dried over anhydrous sodium sulfate, filtered and the filtrate was concentrated under reduced pressure. The residues were purified by silica gel column chromatography using eluent system B to give the title product of (*S*)-tert-butyl *2*-((2*R*,3*R*)-3-((*S*)-1-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)-4-m ethylenepyrrolidin-2-yl)-3-methoxy-2-methylpropanamido)-3-phenylpropanoate **9h** (345 mg, white foam solid), yield 64.7%.
MS m/z (ESI): 1022.5 [M+1]

Step8

(*S*)-tert-butyl 2-((2*R*,3*R*)-3-((*S*)-1-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)-4-methylenepyrrolidin-2-yl)-3-methoxy-2-methylpropanamido)-3-phenylpropanoate

(*S*)-tert-butyl 2-((2*R*,3*R*)-3-((*S*)-1-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)-4-m ethylenepyrrolidin-2-yl)-3-methoxy-2-methylpropanamido)-3-phenylpropanoate **9h** (345 mg, 0.337 mmol) was dissolved in 2 mL of dichloromethane, and added wtih 3 mL of diethylamine.The reaction mixture was stirred under argon atmosphere at room temperature for 2 hours. After completion of the reaction, the reaction solution was concentrated under reduced pressure to give the crude title product of (*S*)-tert-butyl 2-((2*R*,3*R*)-3-((*S*)-1-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)-4-methylenepyrrolidin-2-yl)-3-methoxy-2-methylpropanamido)-3-phenylpropanoate **9i** (375 mg, yellow oil). The product was used in the next step without further purification.
MS m/z (ESI): 800.5 [M+1]

Step9

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-4-methylenepyrrolidin-2-yl )-3-methoxy-2-methylpropanamido)-3-phenylpropanoic acid

The crude product of (*S*)-tert-butyl 2-((2*R*,3*R*)-3-((*S*)-1-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)-4-methylenepyrrolidin-2-yl)-3-methoxy-2-methylpropanamido)-3-phenylpropanoate **9i** (370 mg, 0.462mmol) was added with 7 mL of 4M solution of hydrogen chloride in dioxane solution. The reaction system was sealed and stirred at room temperature for 12 hours. After completion of the reaction, the reaction solution was concentrated under reduced pressure. The residues were purified by high performance liquid chromatography to obtain the product of (*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-4-methylenepyrrolidin-2-yl )-3-methoxy-2-methylpropanamido)-3-phenylpropanoic acid **9** (30 mg, white solid), yield 11.9%.
MS m/z (ESI): 744.7 [M+1]
$^1$H NMR (400 MHz, $CDCl_3$) δ 10.57(s, 1H), 10.00(s, 1H), 8.41-8.26(m, 2H), 7.52-6.99(m, 5H), 5.34(s, 1H), 5.02-4.95(m, 4H), 4.38-4.33(m, 2H), 4.16(m, 2H), 3.93-3.75(m, 3H), 3.49-3.07(m, 6H), 2.94-2.32(m, 16H), 2.30-2.01(m, 4H), 1.75-1.65(m, 2H), 1.32-0.83(m, 16H).

Example 10

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-4-methylenepyrrolidin-2-yl)-3-methoxy-2-methylpropanamido)-3-phenylpropanoic acid

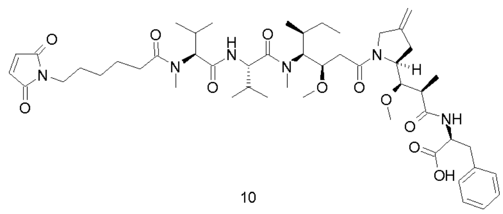

10

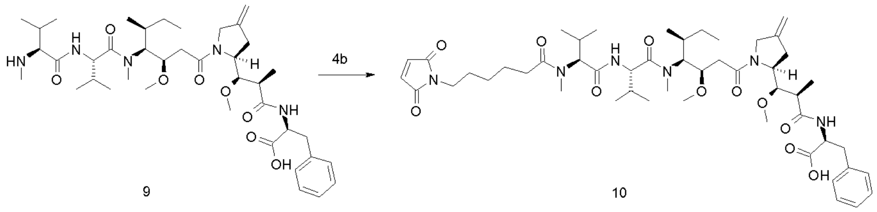


9 10

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-4-methylenepyrrolidin-2-yl )-3-methoxy-2-methylpropanamido)-3-phenylpropanoic acid **9** (22 mg, 0.0295 mmol) was dissolved in 3 mL of dichloromethane, and added with *N*, *N*-diisopropylethylamine (19 mg, 0.1479 mmol). The mixture was cooled to 0°C, and added dropwise with a preformed solution of 6-(2,5-dioxo-2,5-dihydro-1*H*-pyrrol-1-yl) hexanoyl chloride 4b (10.1 mg, 0.0443 mmol) in dichloromethane. The reaction mixture was stirred under argon atmosphere at room temperature for 2.5 hours. The reaction was then quenched with methanol, and then concentrated under reduced pressure. The residues were purified by high performance liquid chromatography to obtain the title product of (*S*)-2-((2*R*,3*R*)-3-((*S*)-1-((3*R*,4*S*,5*S*)-4-((*S*)-2-((*S*)-2-(6-(2,5-dioxo-2,5-dihydro-1H-pyrro l-1-yl)-*N*-methylhexanamido)-3-methylbutanamido)-*N*,3-dimethylbutanamido)-3-metho xy-5-methylheptanoyl)-4-methylenepyrrolidin-2-yl)-3-methoxy-2-methylpropanamido)-3-phenylpropanoic acid **10** (1.3 mg, white sticky material) , yield 4.6%.
MS m/z (ESI): 937.9 [M+1]

Example 11

(*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3 -methoxy-2-methylpropanamido)-3 -(2-fluorophenyl)propanoic acid

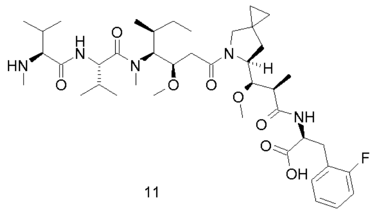

11

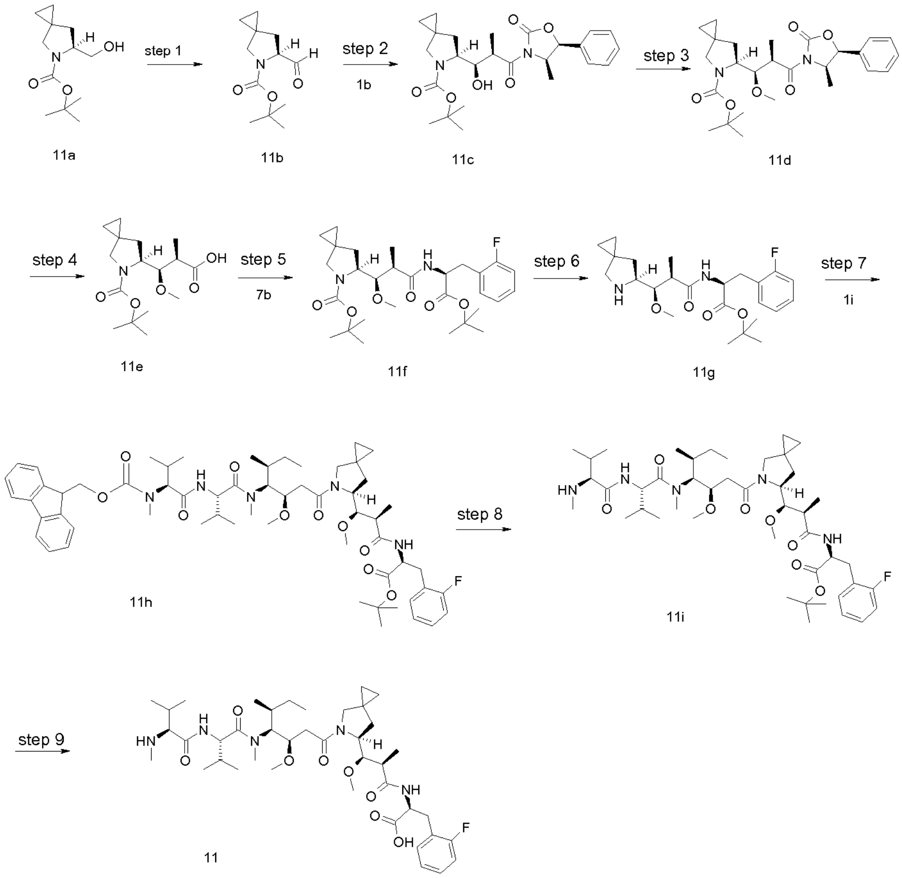


Step 1

(*S*)-tert-butyl 6-formyl-5-azaspiro[2.4]heptane-5-carboxylate

(*S*)-tert-butyl 6-(hydroxymethyl)-5-azaspiro[2.4]heptane-5-carboxylate **11a** (2.37 g, 10.4 mmol, prepared according to the known method of "Bioorganic & Medicinal Chemistry Letters, 2013, 23(9), 2653-2658") was dissolved in 40 mL of dichloromethane, and cooled to 0°C. The solution was added with *N,N*-diisopropylethylamine (10.8 mL, 62.5 mmol), dimethylsulfoxide (12.2 g, 156.4 mmol) and a sulfur trioxide-pyridine complex 6.63 g, 41.7 mmol) under argon atmosphere. The reaction mixture was stirred at 0 °C for 3 hours. After completion of the reaction, the reaction mixture was quenched by addition of phosphate buffer (pH = 7), and washed successively with water and saturated sodium chloride solution, dried over anhydrous sodium sulfate, filtered and the filtrate was concentrated under reduced pressure. The residues were purified by silica gel column chromatography using eluent system B to give the title product of (*S*)-tert-butyl 6-formyl-5-azaspiro[2.4]heptane-5-carboxylate **11b** (2 g, yellow liquid), yield 88%.

Step2

(*S*)-tert-butyl 6-((1*R*,2*R*)-1-hydroxy-2-methyl-3-((4*R*,5*S*)-4-methyl-2-oxo-5-phenyloxazolidin-3-yl)-3-oxopropyl)-5-azaspiro[2.4]heptane-5-carboxylate

(4*R*,5*S*)-4-methyl-5-phenyl-3-propionyloxazolidin-2-one **1b** (2 g, 8.88 mmol) was dissolved in 35 mL of dichloromethane and added with triethylamine (1.42 mL, 10.2 mmol) dropwise under argon atmosphere. The mixture was cooled to 0 °C, and added with dibutylboron trifluoromethanesulfonate (9.59 mL, 9.59 mmol) dropwise, and then stirred at 0 ° C for 50 minutes. A preformed solution of (S) -tert-butyl 6-formyl-5-azaspiro [2.4] heptane-5-carboxylate **11b** (2 g, 8.88 mmol) in dichloromethane was added at 78 ° C and stirred at -78 °C for 2 hours , then stirred at 0 °C for 1 hour and at room temperature for 1 hour. The reaction mixture was quenched by the addition of a mixture of phosphate buffer (pH = 7.0) and methanol (V/V = 3: 1). A mixture of methanol and hydrogen peroxide (V/V = 1: 2) was added at 0 ° C, and the mixture was stirred at room temperature for 1 hour. After concentration under reduced pressure, the residues were dissolved in water and extracted with methylene chloride. The organic phases were combined, washed successively with water and saturated sodium chloride solution, dried over anhydrous sodium sulfate, filtered and the filtrate was

concentrated under reduced pressure . The resulting residues were purified by silica gel column chromatography using eluent system B to give the title product of (*S*)-tert-butyl 6-((1*R*,2*R*)-1-hydroxy-2-methyl-3-((4*R*,5*S*)-4-methyl-2-oxo-5-phenyloxazolidin-3-yl)-3-oxopropyl)-5-azaspiro[2.4]heptane-5-carboxylate **11c** (1.8g, white foam solid), yield 44.2%.
MS m/z (ESI): 459.4 [M+1]

Step3

(*S*)-tert-butyl 6-((1*R*,2*R*)-1-methoxy-2-methyl-3-((4*R*,5*S*)-4-methyl-2-oxo-5-phenyloxazolidin-3-yl)-3 -oxopropyl)-5-azaspiro[2.4]heptane-5-carboxylate

(*S*)-tert-butyl 6-((1*R*,2*R*)-1-hydroxy-2-methyl-3-((4*R*,5*S*)-4-methyl-2-oxo-5-phenyloxazolidin-3-yl)-3-oxopropyl)-5-azaspiro[2.4]heptane-5-carboxylate **11c** (1.8 g, 3.92 mmol) was dissolved in 30 mL of dichloromethane, and added with crushed molecular sieves. Potassium carbonate (3.78 g, 27.48 mmol) and methyl trifluoromethanesulfonate (3.22 g, 19.64 mmol) was added under argon atmosphere and the reaction mixture was stirred at room temperature for 12 hours. After completion of the reaction, the reaction solution was filtered and the filter cake was washed with dichloromethane. The organic phases were combined and the organic phase was concentrated under reduced pressure. The residues were purified by silica gel column chromatography using eluent system A to give the title product of (*S*)-tert-butyl 6-((1*R*,2*R*)-1-methoxy -2-methyl-3-((4*R*,5*S*)-4-methyl-2-oxo-5-phenyloxazolidin-3-yl)-3-oxopropyl)-5-azaspir o[2.4]heptane-5-carboxylate **11d** (930 mg, colorless oil), yield 50.2%.
MS m/z (ESI): 473.4 [M+1]

Step4

(2*R*,3*R*)-3-((*S*)-5-(tert-butoxycarbonyl)-5-azaspiro[2.4]heptan-6-yl)-3-methoxy-2-methy lpropanoic acid

(*S*)-tert-butyl 6-((1*R*,2*R*)-1-methoxy -2-methyl-3-((4*R*,5*S*)-4-methyl-2-oxo-5-phenyloxazolidin-3-yl)-3-oxopropyl)-5-azaspir o[2.4]heptane-5-carboxylate **11d** (1.03 g, 2.8 mmol) was dissolved in 20 mL of tetrahydrofuran, and added with lithium hydroxide monohydrate (155 mg, 3.7 mmol). 30% hydrogen peroxide (939 mg, 8.28 mmol) was added dropwise under argon atmosphere. The reaction mixture was stirred at room temperature for 12 hours. After completion of the reaction, sodium sulfite (1.04 g, 8.28 mmol) was added to the reaction solution, and the mixture was stirred at room temperature for 1 hour. The tetrahydrofuran was concentrated under reduced pressure and the residues were dissolved in water and extracted with dichloromethane. The aqueous phase was diluted with dilute hydrochloric acid to adjust the pH to 3 to 4, and then extracted with dichloromethane (30 mL x 5). The combined organic phases were washed succesively with water and saturated sodium chloride solution, dried over anhydrous sodium sulfate, filtered and the filtrate was concentrated under reduced pressure to give the crude title product of (2*R*,3*R*)-3-((*S*)-5-(tert-butoxycarbonyl)-5-azaspiro[2.4]heptan-6-yl)-3-methoxy-2-methy lpropanoic acid **11e** (700 mg, colorless viscous liquid). The product was used in the next step without purification.
MS m/z (ESI): 314.4 [M+1]

Step5

(*S*)-tert-butyl 6-((1*R*,2*R*)-3-(((*S*)-1-(tert-butoxy)-3-(2-fluorophenyl)-1-oxopropan-2-yl)amino)-1-meth oxy-2-methyl-3-oxopropyl)-5-azaspiro[2.4]heptane-5-carboxylate

The crude product of (2*R*,3*R*)-3-((*S*)-5-(tert-butoxycarbonyl)-5-azaspiro[2.4]heptan-6-yl)-3-methoxy-2-methy lpropanoic acid 11**e** (350 mg, 1.117 mmol) was dissolved in 6 mL of dichloromethane, and added with (*S*)-tert-butyl 2-amino-3-(2-fluorophenyl)propanoate **7b** (267 mg, 1.117 mmol). The mixture was added with *N,N*-diisopropylethylamine (720 mg, 5.587 mmol) and 2- (7-azobenzotriazole) -*N,N,N* ',*N*'-tetramethyluronium hexafluorophosphate (509.8 mg, 1.341 mmol) under argon atmosphere, and then stirred at room temperature for 2 hours. After completion of the reaction, the reaction solution was washed successively with water and saturated sodium chloride solution, dried over anhydrous sodium sulfate, filtered and the filtrate was concentrated under reduced pressure . The residues were purified by silica gel column chromatography using eluent system B to give the title product of (*S*)-tert-butyl 6-((1*R*,2*R*)-3-(((*S*)-1-(tert-butoxy)-3-(2-fluorophenyl)-1-oxopropan-2-yl)amino)-1-meth oxy-2-methyl-3-oxopropyl)-5-azaspiro[2.4]heptane-5-carboxylate **11f** (570 mg, colorless oil), yield 95.3%.
MS m/z (ESI): 535.3 [M+1]

Step 6

(*S*)-tert-butyl 3-(2-fluorophenyl)-2-((2*R*,3*R*)-3-methoxy-2-methyl-3-((*S*)-5-azaspiro[2.4]heptan-6-yl)p ropanamido)propanoate

(*S*)-tert-butyl 6-((1*R*,2*R*)-3-(((*Si*)-1-(tert-butoxy)-3-(2-fluorophenyl)-1-oxopropan-2-yl)amino)-1-meth oxy-2-methyl-3-oxopropyl)-5-azaspiro[2.4]heptane-5-carboxylate **11f** (570 mg, 1.049 mmol) was dissolved in 8 mL of 1,4-dioxane, and added with 4 M soluding of hydrogen chloride in dioxane (0.749 mL, 4.196 mmol). The mixture was stirred for 1 hour at room temperature and placed in a refrigerator for 12 hours at 0-4 °C. The reaction mixture was concentrated under reduced pressure, and the residues were dissolved in methylene chloride. The saturated sodium bicarbonate solution was added dropwise to adjust the pH to 8 to 9, and the aqueous phase was extracted with methylene chloride. The organic phases were combined, washed successively with water and saturated sodium chloride solution, dried over anhydrous sodium sulfate, filtered and the filtrate was concentrated under reduced pressure to give the crude title product of (*S*)-tert-butyl 3-(2-fluorophenyl)-2-((2*R*,3*R*)-3-methoxy-2-methyl-3-((*S*)-5-azaspiro[2.4]heptan-6-yl)p ropanamido)propanoate **11g** (440 mg, lightyellow oil). The product was used in the next step without further purification.
MS m/z (ESI): 435.4 [M+1]

Step 7

(*S*)-tert-butyl 2-((2*R*,3*R*)-3-((*S*)-5-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)-5-az aspiro[2.4]heptan-6-yl)-3-methoxy-2-methylpropanamido)-3-(2-fluorophenyl)propanoat e

The crude product of (*S*)-tert-butyl 3-(2-fluorophenyl)-2-((2*R*,3*R*)-3-methoxy-2-methyl-3-((*S*)-5-azaspiro[2.4]heptan-6-yl)p ropanamido)propanoate **11g** (440 mg, 1.013 mmol), (5*S*,8*S*,11*S*,12*R*)-11-((*S*)-sec-butyl)-1-(9*H*-fluoren-9-yl)-5,8-diisopropyl-12-methoxy-4,1 0-dimethyl-3,6,9-trioxo-2-oxa-4,7,10-triazatetradecan-14-oic acid **1i** (645.8 mg, 1.013 mmol) was dissolved in 10 mL of dichloromethane, and added with *N*, *N*-diisopropylethylamine (0.88 mL, 5.06 mmol) and 2- (7-azobenzotriazole) -*N, N, N*', *N'*-tetramethyluronium hexafluorophosphate (500.5 mg, 1.317 mmol). The reaction mixture was stirred under argon atmosphere at room temperature for 2 hours. After completion of the reaction, the reaction solution was successively washed with water and a saturated sodium chloride solution, dried over anhydrous sodium sulfate, filtered and the filtrate was concentrated under reduced pressure . The residues were purified by silica gel column chromatography using eluent system B to give the title product of (*S*)-tert-butyl 2-((2*R*,3*R*)-3-((*S*)-5-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)-5-az aspiro[2.4]heptan-6-yl)-3-methoxy-2-methylpropanamido)-3-(2-fluorophenyl)propanoat e **11h** (570 mg, white solid), yield 53.4%.
MS m/z (ESI): 1054.9 [M+1]

Step 8

(*S*)-tert-butyl 2-((2*R*,3*R*)-3-((*S*)-5-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)-5-azaspiro[2.4]heptan-6-yl)-3-methoxy-2-methylpropanamido)-3-(2-fluorophenyl)propanoate

(*S*)-tert-butyl 2-((2*R*,3*R*)-3-((*S*)-5-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)-5-az aspiro[2.4]heptan-6-yl)-3-methoxy-2-methylpropanamido)-3-(2-fluorophenyl)propanoat e **11h** (560 mg, 0.531 mmol) was dissolved in 2 mL of dichloromethane, and added with 6 mL of diethylamine. The mixture was stirred at room temperature for 2.5 hours. After completion of the reaction, the reaction solution was concentrated under reduced pressure to give the crude title product of (*S*)-tert-butyl 2-((2*R*,3*R*)-3-((*S*)-5-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)-5-azaspiro[2.4]heptan-6-yl)-3-methoxy-2-methylpropanamido)-3-(2-fluorophenyl)propanoate **11i** (550 mg, white sticky material). The product was used in the next step without further purification.
MS m/z (ESI): 832.5 [M+1]

Step9

(*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3 -methoxy-2-methylpropanamido)-3 -(2-fluorophenyl)propanoic acid

The crude product of (*S*)-tert-butyl 2-((2*R*,3*R*)-3-((*S*)-5-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)-5-azaspiro[2.4]heptan-6-yl)-3-methoxy-2-methylpropanamido)-3-(2-fluorophenyl)propanoate **11i** (450 mg, 0.541 mmol) was added with 7 mL of 4M solutin of hydrogen chloride in dioxane. The reaction system was sealed and stirred at room temperature for 12 hours. After completion of the reaction, the reaction solution was concentrated under reduced pressure. The residues were purified by high performance liquid chromatographyto obtain the title product (*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3-methoxy-2-methylpropanamido)-3-(2-fluorophenyl)propanoic acid 11 (403 mg, white solid), yield 96%.

MS m/z (ESI): 776.7 [M+1]

$^1$H NMR (400 MHz, $CD_3OD$) δ 7.30-7.22(m, 2H), 7.12-7.04(m, 2H), 4.72-4.68(m, 2H), 4.13-4.07(m, 2H), 3.96-3.94(m, 1H), 3.70-3.66(m, 2H), 3.50-3.47(m, 2H), 3.40-3.37(m, 3H), 3.34-3.28(m, 4H), 3.26-3.22(m, 2H), 3.11(s,1H), 3.05-2.91(m, 2H), 2.67-2.65(m, 3H), 2.57-2.43(m, 2H), 2.39-2.28(m, 2H), 2.25-2.16(m, 3H), 1.93-1.88 (m, 2H), 1.55-1.43(m, 2H), 1.23-1.21(d, 2H), 1.16-1.08(m, 3H), 1.08-0.97 (m, 10H), 0.89-0.83(m, 3H), 0.66-0.53(m, 3H), 0.46-0.43(m, 2H).

Example12

(*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-(2-fluorophenyl)propanoic acid

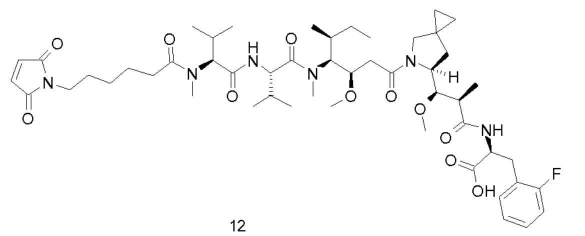

12

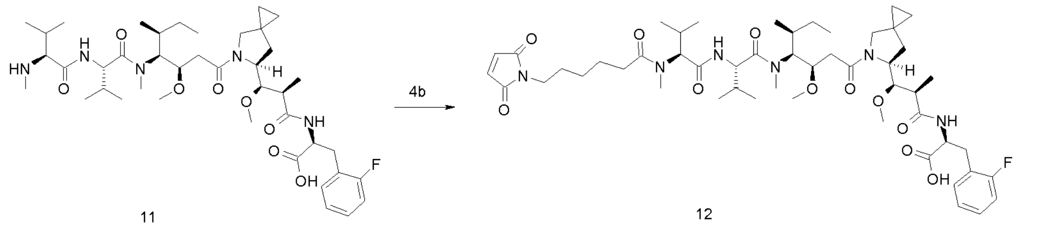


11

12

(*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3-methoxy-2-methylpropanamido)-3-(2-fluorophenyl)propanoic acid **11** (150 mg, 0.193 mmol) was dissolved in 7 mL of dichloromethane, and added with *N,N*-diisopropylethylamine (87.3 mg, 0.677 mmol). The reaction mixture was cooled to 0°C under argon atmosphere, and added dropwise with a preformed solution of 6-(2,5-dioxo-2,5-dihydro-1*H*-pyrrol-1-yl)hexanoyl chloride **4b** (57.6 mg, 0.251 mmol) in dichloromethane, and then stirred at room temperature for 2 hours. The reaction was quenched with methanol and concentrated under reduced pressure. The residues were purified by high performance liquid chromatography to obtain the title product of (*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-(2-fluorophenyl)propanoic acid **12** (14.7 mg, white solid) , yield 7.8%.

MS m/z (ESI): 969.9 [M+1]

$^{1}$H NMR (400 MHz, $CD_3OD$) δ 7.29-7.23(m, 2H), 7.10-7.04(m, 2H), 6.79-6.78(m, 2H), 4.69-4.54(m, 3H), 4.18-4.07(m, 3H), 3.98-3.92(m, 1H), 3.75-3.71(m, 2H), 3.50-3.47(m, 3H), 3.42-3.39(m, 2H), 3.34-3.32(m, 5H), 3.27-3.19(m, 4H), 3.09-2.95(m, 5H), 2.49-2.47(m, 2H), 2.41-2.36(m, 2H), 2.29-2.18(m, 3H), 2.09-2.02(m, 2H), 1.90-1.87(m, 2H), 1.63-1.59(m, 4H), 1.49(s, 2H), 1.32-1.28(m, 3H), 1.21-1.12(m, 3H), 1.00-0.81(m, 12H), 0.62-0.55(m, 3H), 0.46-0.40(m, 2H).

Example 13

(*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3-methoxy-2-methylpropanamido)-3-phenylpropanoic acid

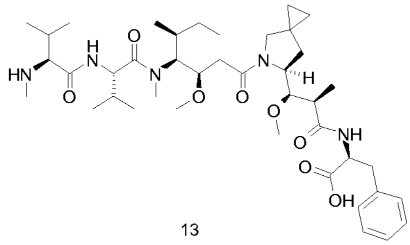

13

The preparation method was similar to Example 11, except that the material of Step 5 was replaced with (2*R*,3*R*)-3-((*S*)-5-(tert-butoxycarbonyl)-5-azaspiro[2.4]heptan-6-yl)-3-methoxy-2-methy lpropanoic acid **11e** and (*S*)-tert-butyl 2-amino-3-phenylpropanoate **1f** to give the title product of (*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3-methoxy-2-methylpropanamido)-3-phenylpropanoic acid **13** (219 mg, white solid).

MS m/z (ESI): 758.7 [M+1]

$^{1}$H NMR (400 MHz, $CD_3OD$) δ 7.31-7.19(m, 5H), 4.70-4.68(m, 2H), 4.14-4.03(m, 2H), 3.95-3.93(m, 1H), 3.69-3.66(m, 2H), 3.48-3.45(m, 2H), 3.43-3.38(m, 3H), 3.34-3.29 (m, 4H), 3.23-3.21(m, 2H), 3.11(s, 1H), 2.97-2.89(m, 2H), 2.67-2.65(m, 3H), 2.51-2.43(m, 2H), 2.39-2.27(m, 2H), 2.21-2.06(m, 3H), 1.88-1.82(m, 2H), 1.41-1.39 (m, 2H), 1.23-1.21(d, 2H), 1.16-1.10(m, 3H), 1.08-0.98(m, 10H), 0.89-0.84(m, 3H), 0.63-0.52(m, 3H), 0.46-0.43(m, 2H).

Example 14

(*S*)-2-((2*R*,3*R*)-3-((*S*)-5-((3*R*,4*S*,5*S*)-4-((*S*)-2-((*S*)-2-(6-(2,5-dioxo-2,5-dihydro-1H-pyrro l-1-yl)-N-methylhexanamido)-3-methylbutanamido)-N,3-dimethylbutanamido)-3-metho xy-5-methylheptanoyl)-5-azaspiro[2.4]heptan-6-yl)-3-methoxy-2-methylpropanamido)-3-phenylpropanoic acid

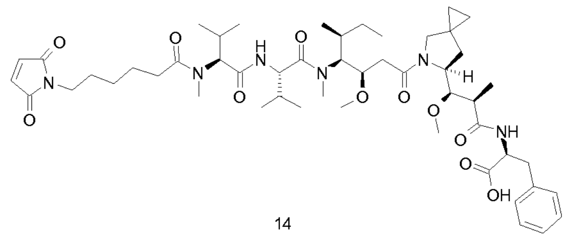

14

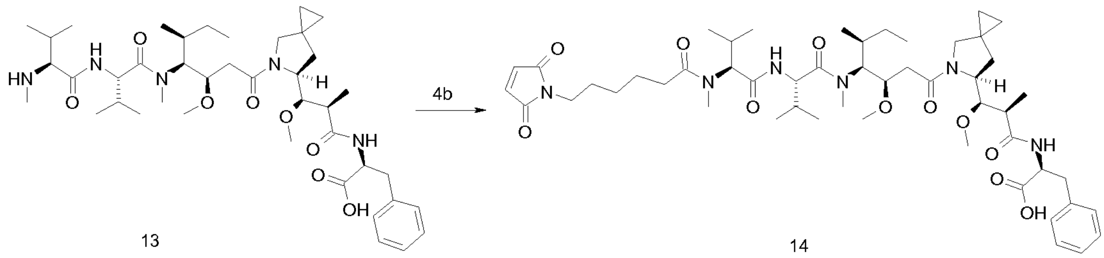


13

14

(*S*)-2-((2*R*,3*R*)-3-((*S*)-5-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)-5-azaspiro[2.4]heptan-6-yl) -3-methoxy-2-methylpropanamido)-3-phenylpropanoic acid **13** (120 mg, 0.158 mmol) was dissolved in 5 mL of dichloromethane, and added with *N*, *N*-diisopropylethylamine (71.5 mg, 0.554 mmol). The mixture was cooled to 0°C under argon atmosphere, and added dropwise with a preformed solution of 6-(2,5-dioxo-2,5-dihydro-1*H*-pyrrol-1-yl)hexanoyl chloride **4b** (47.1 mg, 0.2059 mmol) in dichloromethane, and then stirred at room temperature for 2 hours. After completion of the reaction, 1 mL of methanol was added, and stirred for 10 minutes. The mixture was concentrated under reduced pressure. The residues were purified by high performance liquid chromatography to give the title product of (*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-phenylpropanoic acid **14** (5.1 mg, white solid) , yield3.39%.

MS m/z (ESI): 951.9 [M+1]

$^1$H NMR (400 MHz, $CD_3OD$) $\delta$ 7.29-7.18(m, 5H), 6.80-6.79(m, 2H), 4.75-4.61(m, 3H), 4.21-3.99(m, 3H), 3.94-3.91(m, 1H), 3.74-3.70(m, 2H), 3.51-3.44(m, 3H), 3.42-3.37(m, 2H), 3.34-3.28(m, 5H), 3.23-3.21(m, 4H), 3.10-2.86(m, 5H), 2.49-2.36(m, 4H), 2.32-2.17(m, 3H), 2.12-2.03(m, 2H), 1.88-1.80(m, 2H), 1.67-1.58(m, 4H), 1.49(s, 2H), 1.37-1.26(m, 3H), 1.22-1.13(m, 3H), 1.00-0.83(m, 12H), 0.63-0.51(m, 3H), 0.43-0.40(m, 2H).

Example 15

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(p-tolyl)propanoic acid

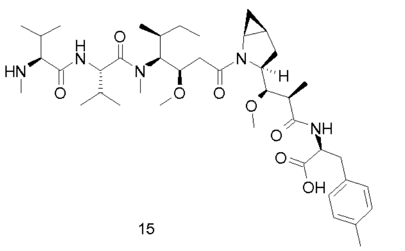

15

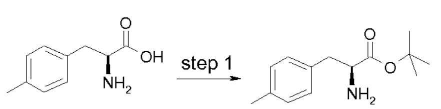


15a 15b

(S)-tert-butyl 2-amino-3-(p-tolyl)propanoate

(S)-2-amino-3-(p-tolyl)propanoic acid **15a** (400 mg, 2.23 mmol, prepared according to the known method of "Organic & Biomolecular Chemistry, 2004, 2(18), 2684-2691") was dissolved in 10 mL of tert-butyl acetate. The mixture was added with perchloric acid (336.3 mg(70%), 3.34 mmol) under argon atmosphere, and stirred at room temperature for 16 hours. After the reaction, 10 mL of water was added. The aqueous phase was adjusted to pH = 8 with saturated sodium bicarbonate solution and extracted with dichloromethane (5 mL $\times$3). The organic phases were combined, washed

with saturated sodium chloride solution (5 mL), dried over anhydrous sodium sulfate, filtered and the filtrate was concentrated under reduced pressure to obtain the title product (*S*)-tert-butyl 2-amino-3-(*p*-tolyl)propanoate **15b** (370 mg, white solid), yield 70%.

The preparation method was similar to Example 1, except that the raw material in step 4 was replaced with (2*R*,3*R*)-3-((1*S*,3*S*,5*S*)-2-(*tert*-butoxycarbonyl)-2-azabicyclo[3.1.0]hexan-3-yl)-3-metho xy-2-methylpropanoic acid **1e** and (S)-tert-butyl 2-amino-3-(*p*-tolyl)propanoate **15b,** to obtain the title product (*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(p-tolyl)propanoic acid 15 (30 mg, white solid).

MS m/z (ESI): 758.8 [M+1]

$^1$H NMR (400 MHz, $CD_3OD$): $\delta$ 7.19-7.07(m, 4H), 4.82-4.68(m, 2H), 4.30-4.18 (m, 1H), 4.15-4.05(m, 1H), 3.89-3.84(m, 1H), 3.83-3.76(m, 1H), 3.74-3.62(m, 2H), 3.47-3.12(m, 9H), 2.89-2.79(m, 1H), 2.70-2.59(m, 4H), 2.34-2.03(m, 7H), 1.91-1.75 (m, 1H), 1.73-1.53(m, 2H), 1.50-1.24(m,4H),1.22-0.92(m, 18H), 0.90-0.79(m, 4H), 0.75-0.64(m,1H).

Example 16

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(thiophen-2-yl)propanoic acid

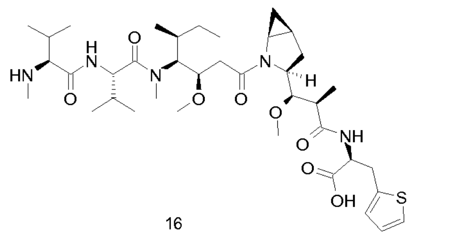

16

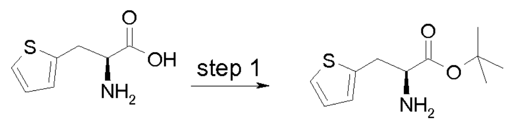


16a 16b

Step 1

(*S*)-tert-butyl 2-amino-3-(thiophen-2-yl)propanoate

(S)-2-amino-3-(thiophen-2-yl)propanoic acid **16a** (400 mg, 2.33 mmol, prepared according to the known method of "European Journal of Organic Chemistry, 2006, (5), 1113-1116") was dissolved in 10 mL of tert-butyl acetate. The mixture was cooled to 0°C under argon atmosphere, and added dropwise with perchloric acid(352 mg(70%), 3.5 mmol). The reaction mixture was stirred at room temperature for 16 hours and then added with water, and adjusted to pH = 8~9 with saturated sodium bicarbonate solution and extracted with dichloromethane. The organic phases were combined, washed successively with water and saturated sodium chloride solution, dried over anhydrous sodium sulfate, filtered and the filtrate was concentrated under reduced pressure to obtain the crude title product of (S)-tert-butyl 2-amino-3-(thiophen-2-yl)propanoate 16b (370 mg, light yellow oil). The product was used in the next step without further purification.

MS m/z (ESI): 228.3 [M+1].

The preparation method was similar to Example 1, except that the raw material in step 4 was replaced with (2*R*,3*R*)-3-((1*S*,3*S*,5*S*)-2-(*tert*-butoxycarbonyl)-2-azabicyclo[3.1.0]hexan-3-yl)-3-metho xy-2-methylpropanoic acid **1e** and (*S*)-tert-butyl 2-amino-3-(thiophen-2-yl)propanoate **16b,** to obtain the title product of (*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3-yl)-3-methoxy-2-methylpropanamido)-3-(thiophen-2-yl)propanoic acid **16** (18 mg, white solid).

MS m/z (ESI): 527.6 [M+1]

$^1$H NMR (400 MHz, $CDCl_3$) δ 11.00(s, 1H), 8.44(s, 1H), 7.17-7.15(d, 1H), 6.94-6.88 (d, 2H), 4.97-4.95(m, 2H), 4.10-4.08(m, 1H), 3.85-3.83(m, 2H), 3.71-3.69(m, 2H), 3.52-3.50(m, 2H), 3.37-3.34(m, 12H), 3.06-3.04(m, 2H), 2.80-2.78(m, 2H), 2.3-2.26 (m, 4H), 1.64-1.59(m, 3H), 1.48-1.46(m, 2H), 1.31-1.259(m, 12H), 1.08-0.98(m, 8H), 0.89-0.82(m, 4H).

Example 17

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3 -yl)-3 -methoxy-2-methylpropanamido)-3 -(3 -fluorophenyl)propanoic acid

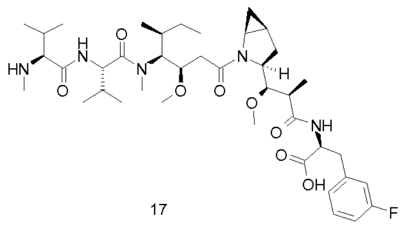

17

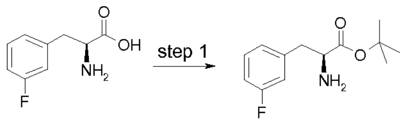


17a

17b

Step 1

(*S*)-tert-butyl 2-amino-3-(3-fluorophenyl)propanoate

(S)-2-amino-3-(3-fluorophenyl)propanoic acid **17a** (549 mg, 3 mmol, prepared according to the known method of "Advanced Synthesis & Catalysis, 2012, 354(17), 3327- 3332") was dissolved in 15 mL of tert-butyl acetate. The mixture was added with perchloric acid (450 mg(70%), 4.5 mmol) under argon atmosphere at 0°C, then stirred at room temperature for 12 hours. After the reaction, 30 mL of water was added, and the organic phase was washed successively with 20 mL of IN hydrochloric acid and saturated sodium bicarbonate solution. The aqueous phase was combined, adjusted to pH = 8~9 with saturated sodium bicarbonate solution and extracted with dichloromethane (100 mL ×2). The organic phases were combined, dried over anhydrous sodium sulfate, filtered and the filtrate was concentrated under reduced pressure to obtain the crude title product of (*S*)-tert-butyl 2-amino-3-(3-fluorophenyl)propanoate **17b** (600 mg, oil). The product was used in the next step without further purification.

The preparation method was similar to Example 1, except that the raw material in step 4 was replaced with (2*R*,3*R*)-3-((1*S*,3*S*,5*S*)-2-(*tert*-butoxycarbonyl)-2-azabicyclo[3.1.0]hexan-3-yl)-3-metho xy-2-methylpropanoic acid **1e** and(*S*)-tert-butyl 2-amino-3-(3-fluorophenyl)propanoate **17b,** to obatine the title product of (*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(met hylamino)butanamido)butanamido)-3-methoxy-5-methylheptanoyl)-2-azabicyclo[3.1.0] hexan-3 -yl)-3 -methoxy-2-methylpropanamido)-3 -(3 -fluorophenyl)propanoic acid **17** (25 mg, white solid).

MS m/z (ESI): 762.8 [M+1]

$^1$H NMR (400 MHz, $CD_3OD$): δ 7.29-7.21(m, 1H), 7.08-6.87(m, 3H), 4.81-4.73(m, 1H), 4.72-4.67(m, 1H), 4.61-4.53(m, 1H), 4.29-4.22(m, 1H), 4.15-4.06(m, 1H), 3.99-3.93(m, 1H), 3.75-3.58(m, 2H), 3.43-3.12(m, 9H), 2.99-2.90(m, 1H), 2.69-2.60 (m, 4H), 2.30-1.97(m, 4H), 1.87-1.77(m, 1H), 1.63-1.53(m, 1H), 1.49-1.36(m, 1H), 1.18-0.92(m, 22H), 0.91-0.82(m, 4H), 0.81-0.71(m,1H).

Example 18

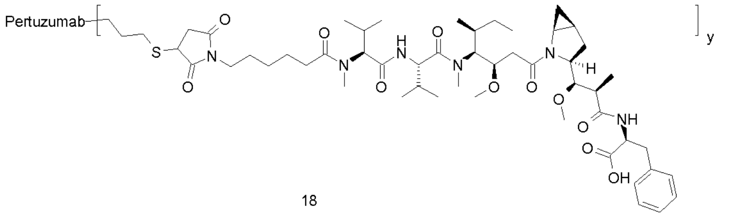

18

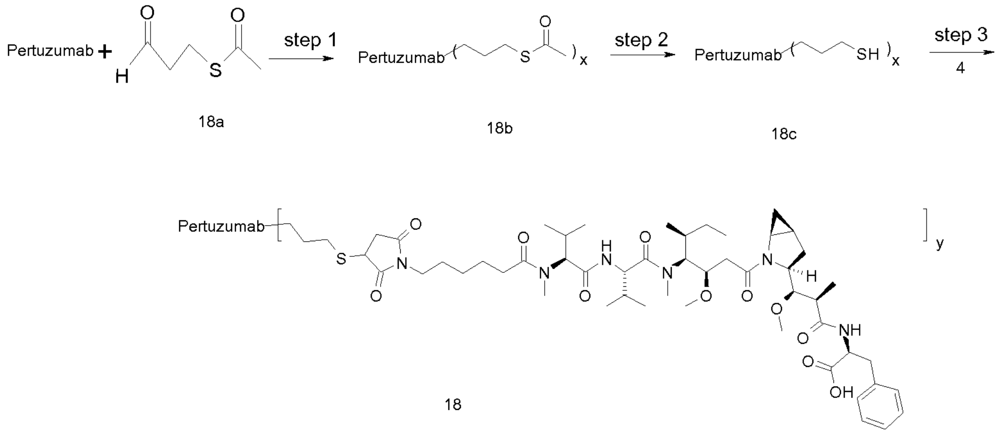


18a

18b

18c

18

Step 1

S-(3-oxopropyl) ethanethioate **18a** (0.35 mg, 2.65 μmol) was dissolved in 0.45 mL of acetonitrile. The Pertuzumab in acetic acid/sodium acetate buffer (10.85 mg/ml, 4.5 mL, 0.488 mmol) with pH 4.5 was added with the solution of S-(3-oxopropyl) ethanethioate **18a** in acetonitrile and then added with 1.0 mL of an aqueous solution of sodium cyanoborohydride (7.06 mg, 112μmol) dropwise. The reaction mixture was stirred at 25 °C for 2 hours. After completion of the reaction, the residues were desalted and purified with a Sephadex G25 gel column (elution phase: 0.05 M PBS solution at pH 6.5) to give the title product **18b** solution which was used directly in the next step.

Step 2

The solution of **18b** (15.0 mL) was added with 0.45 mL of a 2.0 M solution of hydroxylamine hydrochloride and stirred at 25 °C for 30 minutes. The reaction solution was desalted with Sephadex G25 gel column (Elution phase: 0.05 *M* PBS solution at pH 6.5) to give the solution of Pertuzumab-propanethiol **18c** (concentration 1.65 mg/ml, 22.6 mL)

Step 3

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 *H*-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido) **4** (1.09 mg, 1.16 μmol) was dissolved in 1.1 mL of acetonitrile and added with Pertuzumab-propanethiol solution **18c** (1.65 mg/mL, 11.3 mL). After stirred at 25 °C for 4 hours, the reaction mixture was desalted with Sephadex G25 gel column (Elution phase: 0.05*M* PBS solution at pH 6.5), and filtered under sterile conditions through a 0.2 μm filter to give the title product **18** in PBS buffer (0.75 mg/mL, 19.5 mL), which was then stored at 4 °C.
Q-TOF LC/MS: characteristic peaks: 148119.54($M_{Ab}$+0D), 149331.45($M_{Ab}$+1D), 150407.02 ($M_{Ab}$+2D, 151297.79($M_{Ab}$+3D), 152448.85($M_{Ab}$+4D), 153782.23($M_{Ab}$+5D).
average value: y=2.0.

Example 19

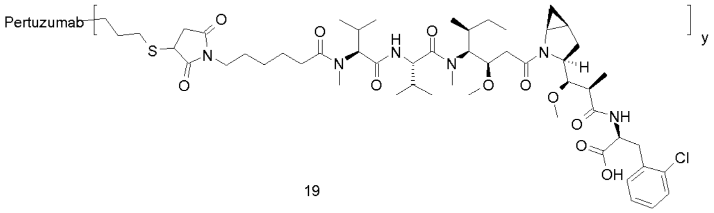

19

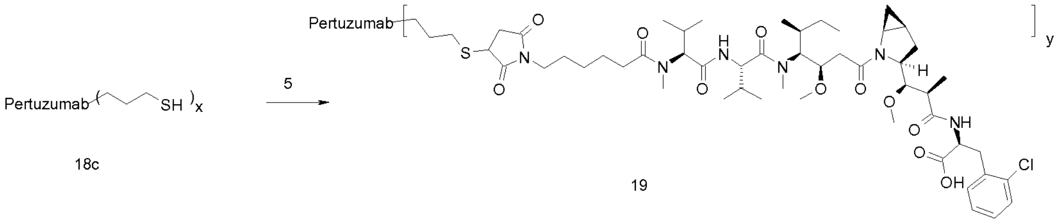


18c

19

(*S*)-3-(2-chlorophenyl)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1*H*pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpropanamido)propanoic acid **5** (1.39 mg, 1.43 μmol) was dissolved in 1.1mL of acetonitrile and added with pertuzumab-propanethiol solution **18c** (1.65 mg/mL, 11.3 mL), and stirred at 25 °C for 4 hours. The reaction mixture was desalted and purified by Sephadex G25 gel column (eluting phase: 0.05 M PBS solution, pH 6.5), filtrated under a sterile condition through a 0.2 μm filter to obtain the title product of **19** in PBS buffer (0.78 mg/mL, 20.0 mL), and then stored at 4 °C.

Q-TOF LC/MS: characteristic peaks: 148119.68($M_{Ab}$+0D), 149308.79($M_{Ab}$+1D), 150194.76 ($M_{Ab}$+ 2D), 151354.52($M_{Ab}$+3D), 152410.57($M_{Ab}$+4D), 153375.31($M_{Ab}$+5D).

average value: y=1.9.

Example 20

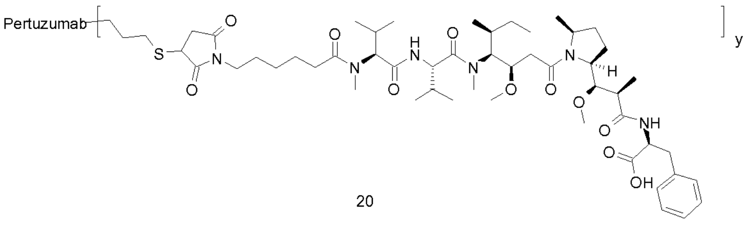

20

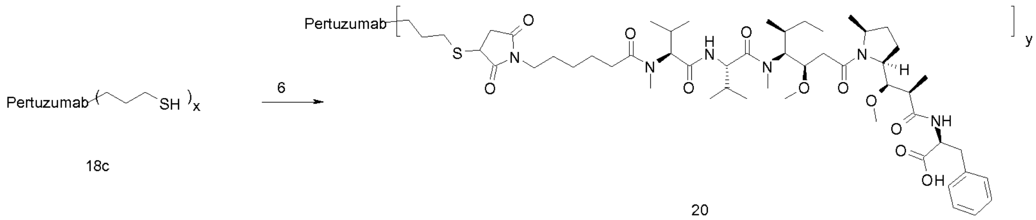


18c

20

(*S*)-2-((2*R*,3*R*)-3-((2*S*,5*S*)-1-((3*R*,4*S*,5*S*)-4-((*S*)-2-((*S*)-2-(6-(2,5-dioxo-2,5-dihydro-1*H*-pyrrol-1-yl)-*N*-methylhexanamido)-3-methylbutanamido)-*N*,3-dimethylbutanamido)-3-methoxy-5-methylheptanoyl)-5-methylpyrrolidin-2-yl)-3-methoxy-2-methylpropanamido)-3-phenylpropanoic acid **6** (1.08 mg, 1.15 μmol) was dissolved in 1.25mL of acetonitrile and added with Pertuzumab-propanethiol solution **18c** (1.50 mg/mL, 12.5 mL), and stirred at 25 °C for 4 hours. The reaction mixture was desalted and purified by Sephadex G25 gel column (eluting phase: 0.05 *M* PBS solution, pH 6.5), filtered under a sterile condition through a 0.2 μm filter to obtain the title product of **20** in PBS buffer (0.74 mg/mL, 19.0 mL), and then stored at 4 °C.

Q-TOF LC/MS: characteristic peaks: Q-TOF LC/MS: 148253.27($M_{Ab}$+0D), 149263.59 ($M_{Ab}$+1D), 150315.25($M_{Ab}$+2D),

151334.45($M_{Ab}$+3D), 152383.92($M_{Ab}$+4D), 153446.37 ($M_{Ab}$+5D).
average value: y=2.2.

Example 21

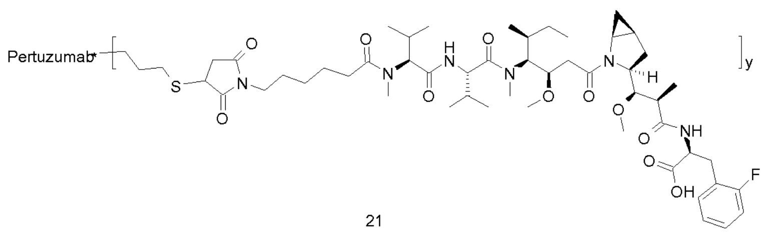

21

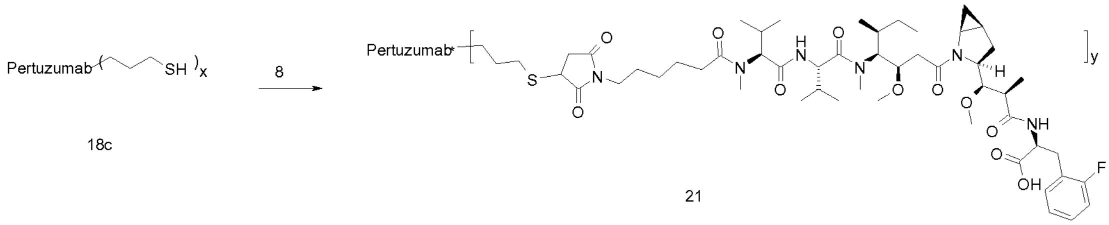


18c

21

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 H-pyrrol-1-yl)-N-methylhexanamido)-3-methylbutanamido)-N,3-dimethylbutanamido)-3 -methoxy-5 -methylheptanoyl)-2-azabicyclo [3.1. 0]hexan-3 -yl)-3 -methoxy-2-methylpro panamido)-3-(2-fluorophenyl)propanoic acid **8** (3.0 mg, 3.0 μmol) was dissolved in 1.0 mL of acetonitrile and added with pertuzumab-propanethiol solution **18c** (2.11 mg/mL, 10.0 mL), stirring at 25 °C for 4 hours.Then the above reaction mixture was desalted and purified by Sephadex G25 gel column (eluting phase: 0.05 *M* PBS solution, pH 6.5), filtrated under a sterile condition through a 0.2 μm filter to obtain the title product of **21** in PBS buffer(1.31 mg/mL, 12.5 mL), and then stored at 4 °C frozen storage.
Q-TOF LC/MS: characteristic peaks: 148312.73($M_{Ab}$+0D), 149515.61($M_{Ab}$+1D), 150459.55 ($M_{Ab}$+ 2D), 151521.47($M_{Ab}$+3D), 152580.02($M_{Ab}$+4D).
average value: y=1.7.

Example 22

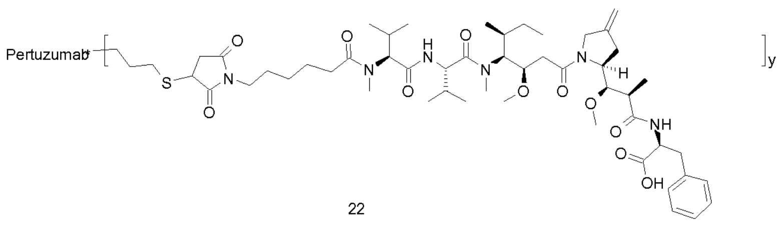

22

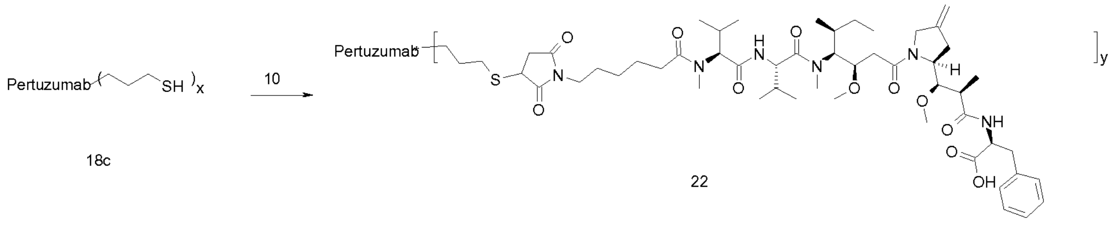


18c

22

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-4-methylenepyrrolidin-2-yl)-3-methoxy-2-methylpropanamido)-3-phenylpropanoic acid **10** (1.50 mg, 1.60 μmol) was dissolved in 1.0mL of acetonitrile and added with pertuzumab-propanethiol solution **18c** (2.11 mg/mL, 10.0 mL), and stirred at 25 °C for 4 hours. The reaction mixture was desalted and purified by Sephadex G25 gel column (eluting phase: 0.05 *M* PBS solution, pH 6.5), filtrated under a sterile condition through a 0.2 μm filter to obtain the title product of **22** in PBS buffer (1.28 mg/mL, 13.0 mL), and then stored at 4 °C.
Q-TOF LC/MS: characteristic peaks: 148411.82($M_{Ab}$+0D), 149412.97($M_{Ab}$+1D), 150468.08 ($M_{Ab}$+ 2D), 151496.41($M_{Ab}$+3D), 152580.37($M_{Ab}$+4D).
average value: y=2.1.

Example 23

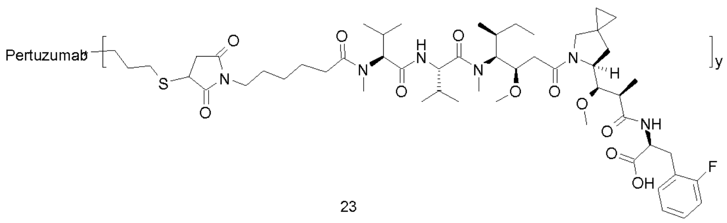

23

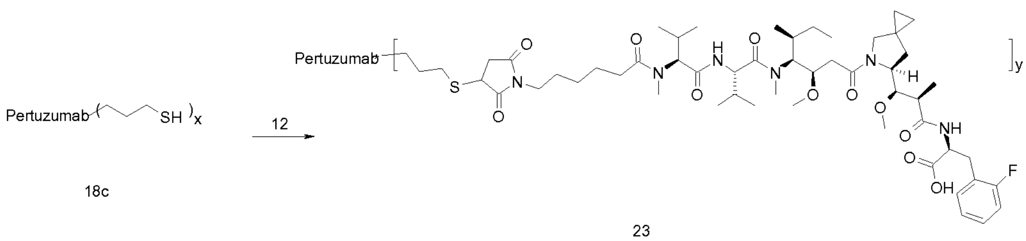


18c

23

(*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-(2-fluorophenyl)propanoic acid **12** (0.86 mg, 0.89 μmol) was dissolved in 0.6mL of acetonitrile and added with Pertuzumab-propanethiol solution **18c** (2.06 mg/mL, 6.0 mL), and stirred at 25 °C for 4 hours. The reaction mixture was desalted and purified by Sephadex G25 gel column (eluting phase: 0.05 *M* PBS solution, pH 6.5), filtrated under a sterile condition through a 0.2 μm filter to obtain the title product of **23** in PBS buffer (0.70 mg/mL, 15 mL), and then stored at 4 °C.
Q-TOF LC/MS: characteristic peaks: 148092.94($M_{Ab}$+0D), 149296.82($M_{Ab}$+1D), 150339.86 ($M_{Ab}$+ 2D), 151416.51($M_{Ab}$+3D), 152516.25($M_{Ab}$+4D), 153422.64($M_{Ab}$+5D).
average value: y=1.7.

Example 24

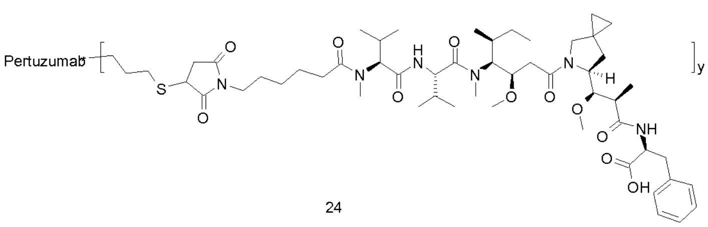

24

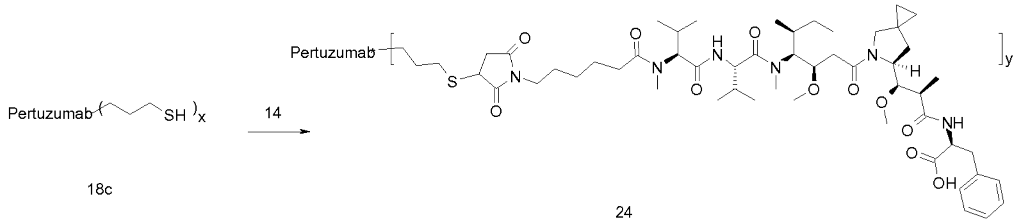


18c

24

(*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-phenylpropanoic acid **14** (0.73 mg, 0.78 μmol) was dissolved in 0.6mL of acetonitrile and added with Pertuzumab-propanethiol solution **18c** (2.06 mg/mL, 6.0 mL), and stirred at 25 °C for 4 hours. The reaction mixture was desalted and purified by Sephadex G25 gel column (eluting phase: 0.05 *M* PBS solution, pH 6.5), filtrated under a sterile condition through a 0.2 μm filter to obtain the title product of **24** in PBS buffer (0.68 mg/mL, 15.5 mL), and then stored at 4 °C.

Q-TOF LC/MS: characteristic peaks: 148094.99($M_{Ab}$+0D), 149277.83($M_{Ab}$+1D), 150343.15 ($M_{Ab}$+ 2D), 151359.29($M_{Ab}$+3D), 152478.14($M_{Ab}$+4D), 153449.92($M_{Ab}$+5D).

average value: y=1.6.

Example 25

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3 -(2-fluorophenyl)propanoic acid

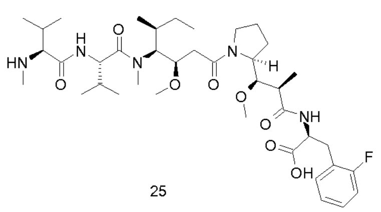

25

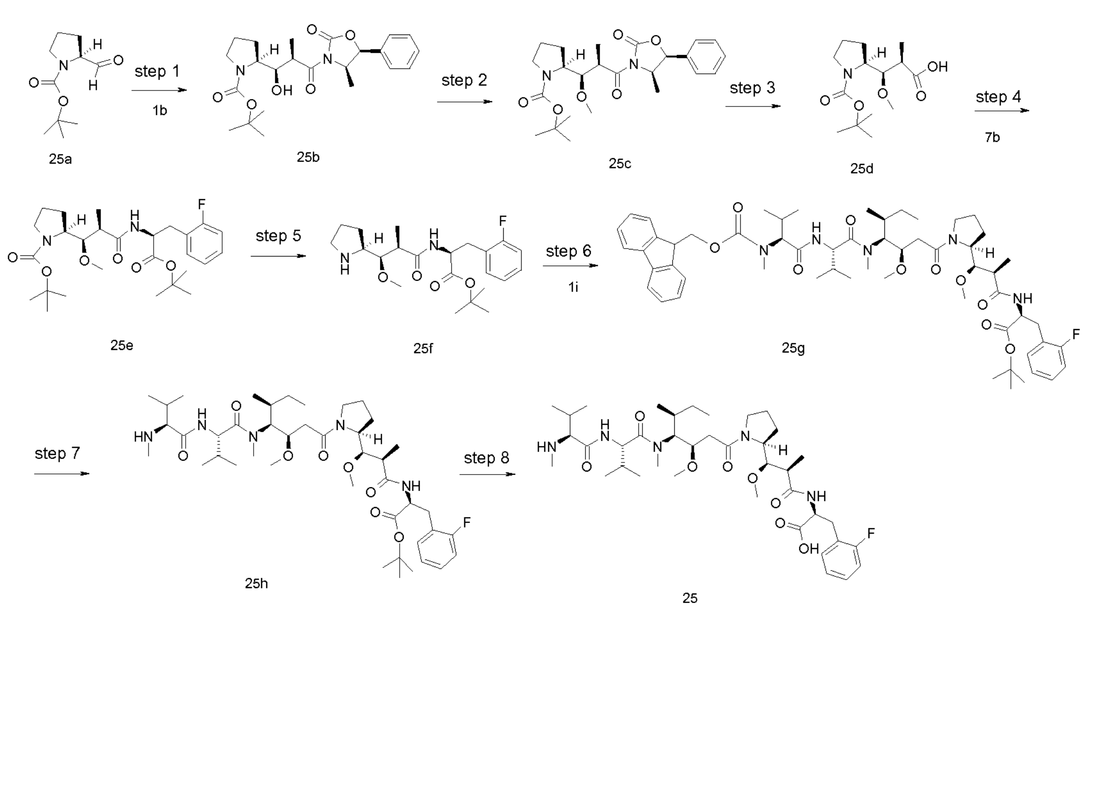


25a 25b 25c 25d 25e 25f 25g 25h 25

Step 1

(*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)pyrrolidine-1-carboxylate

(4*R*,5*S*)-4-methyl-5-phenyl-3-propionyloxazolidin-2-one **1b** (4.6 g, 20.1 mmol) was dissolved in 80 mL of dichloromethane, and cooled to 0°C under argon atomsphere. The reaction solution was added dropwise with triethylamine (3.2 mL, 23.1 mmol) and dibutylboron trifluoromethanesulfonate (20 mL, 20.7 mmol) at 0 °C, and stirred at 0 °C for 50 minutes, then added dropwise with 5 mL preformed solution of (*S*)-tert-butyl 2-formylpyrrolidine-1-carboxylate **25a** (4 g, 20.1 mmol, prepared according to the known method of "Journal of the American Chemical Society, 2011, 133(42), 16901-16910") in dichloromethane at -75 °C, and stirred for 1 hour at -75°C, then 2 hours at 0 ° C and 1 hour at room temprature. A mixture of 60 mL of a phosphate buffer (pH = 7.0) and methanol (V/V = 1: 3) was added to the reaction solution. A mixture of 60 mL of methanol and hydrogen peroxide (30%) (V/V = 2: 1) was added at 0 °C and stirred at room temperature for 1 hour. After completion of the reaction, the organic phase was concentrated under reduced pressure and 15 mL of water was added. The aqueous phase was extracted with ether (30 mL × 3) and the ether phase was combined, washed successively with 5% sodium bicarbonate solution, water, saturated sodium chloride solution, dried over anhydrous sodium sulfate, filtered, the filtrate was concentrated under reduced pressure. The residues were purified by silica gel column chromatography using eluent system B to give the title product of (*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)pyrrolidine-1-carboxylate **25b** (2.26 g, white foam solid), yield 24.9%.
MS m/z (ESI): 333.3 [M-100+1]

Step 2

(*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)pyrrolidine-1-carboxylate

(*S*)-tert-butyl 2-((1*R*,2*R*)-1-hydroxy-2-methyl-3-((4*R*,5*S*)-4-methyl-2-oxo-5-phenyloxazolidin-3-yl)-3-oxopropyl)pyrrolidine-1-carboxylate **25b** (2 g, 4.62 mmol) was dissolved in 20 mL of dichloromethane and added with 2 g of a crushed molecular sieves. The mixture was added with 1,8-bisdimethylaminonaphthalene (2.57 g, 12 mmol), trimethyloxonium tetrafluoroborate (1.71 g, 11.5 mmol) at 0 °C under an argon atmosphere and stirred at room temperature for 17 hours. The resction mixture was filtered and the filter cake was washed with dichloromethane. The filtrate was combined and the organic phase was washed with saturated ammonium chloride solution (20 mL x 3) and saturated sodium chloride solution, dried over anhydrous sodium sulfate, filtered, and the filtrate was concentrated under reduced pressure. The residues were purified by silica gel column chromatography using eluent system B to give the title product of (*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)pyrrolidine-1-carboxylate **25c** (1.3 g, white foam solid), yield 63%.
MS m/z (ESI): 447.3 [M+1]

Step 3

(2*R*,3*R*)-3-((*S*)-1-(tert-butoxycarbonyl)pyrrolidin-2-yl)-3-methoxy-2-methylpropanoic acid

(*S*)-tert-butyl 2-((1*R*,2*R*)-1-methoxy-2-methyl-3-((4*R*,5*S*)-4-methyl-2-oxo-5-phenyloxazolidin-3-yl)-3 -oxopropyl)pyrrolidine-1-carboxylate **25c** (1.3 g, 2.9 mmol) was dissolved in 80 mL of tetrahydrofuran. The solution was cooled to 0 °C under argon atmosphere, and added with 30% hydrogen peroxide (1.25 g, 11 mmol) dropwise and lithium hydroxide monohydrate (207 mg, 4.95 mmol). The reaction mixture was stirred at room temperature for 12 hours. After completion of the reaction, sodium sulfite solid (1.47 g, 11.6 mmol) was added to the reaction solution, and the mixture was stirred at room temperature for 1 hour. A small amount of water was added and the organic phase was concentrated under reduced pressure. A small amount of water was added to dissolve the residues and extracted with dichloromethane (50 mL × 2). The aqueous phase was added with hydrochloric acid until pH= 3 and extracted with dichloromethane (40 mL × 3). The organic phase was washed with water and saturated sodium chloride solution, dried over anhydrous sodium sulfate, filtered and the filtrate was concentrated under reduced pressure to give the crude title product of (2*R*,3*R*)-3-((*S*)-1-(tert-butoxycarbonyl)pyrrolidin-2-yl)-3-methoxy-2-methylpropanoic acid **25d** (870 mg, colorless oil). The product was used in the next step without further purification.

Step4

(*S*)-tert-butyl 2-((1*R*,2*R*)-3-(((*S*)-1-(tert-butoxy)-3-(2-fluorophenyl)-1-oxopropan-2-yl)amino)-1-meth oxy-2-methyl-3-oxopropyl)pyrrolidine-1-carboxylate

(2*R*,3*R*)-3-((*S*)-1-(tert-butoxycarbonyl)pyrrolidin-2-yl)-3-methoxy-2-methylpropanoic acid **25d** (100 mg, 0.368 mmol) was dissolved in 6 mL of dichloromethane and 1.8 mL of dimethylformamide, and added with (*S*)-tert-butyl 2-amino-3-(2-fluorophenyl)propanoate **7b** (97 mg, 0.405mmol). The mixture was added with *N*, *N*-diisopropylethylamine (237.8 mg, 2.844 mmol) and 2- (7-azobenzotriazole) *-N, N, N', N'*-tetramethyluronium hexafluorophosphate (168.2 mg, 0.442 mmol) under argon atmosphere, and stirred at room temperature for 2 hours. The reaction mixture was then added with 10 mL of dichloromethane, and washed succesively with water, saturated sodium chloride solution, dried over anhydrous sodium sulfate, filtered and the filtrate was concentrated under reduced pressure . The residues were purified by silica gel column chromatography using eluent system B to give the title product of (*S*)-tert-butyl 2-((1*R*,2*R*)-3-(((*S*)-1-(tert-butoxy)-3-(2-fluorophenyl)-1-oxopropan-2-yl)amino)-1-meth oxy-2-methyl-3-oxopropyl)pyrrolidine-1-carboxylate **25e** (157 mg, colorless oil), yield 83.9%.
MS m/z (ESI): 509.3 [M+1]

Step 5

(*S*)-tert-butyl 3-(2-fluorophenyl)-2-((2*R*,3*R*)-3-methoxy-2-methyl-3-((*S*)-pyrrolidin-2-yl)propanamido )propanoate

(*S*)-tert-butyl 2-((1*R*,2*R*)-3-(((*S*)-1-(tert-butoxy)-3-(2-fluorophenyl)-1-oxopropan-2-yl)amino)-1-meth oxy-2-methyl-3-oxopropyl)pyrrolidine-1-carboxylate **25e** (157 mg, 0.308 mmol) was dissolved in 2 mL of dioxane, and added with 4 *M* solution of hydrogen chloride in dioxane (0.193 mL, 1.08 mmol). The reaction system was sealed and stirred for 1 hour at room temperature, and then placed in a refrigerator for 12 hours at 4 °C. The reaction solution was concentrated under reduced pressure, and the residues were dissolved in dichloromethane and washed with saturated sodium bicarbonate solution. The aqueous phase was extracted with dichloromethane. The organic phases were combined, washed with saturated sodium chloride solution, dried over anhydrous sodium sulfate, filtered and the filtrate was concentrated under reduced pressure to give the crude title product of (*S*)-tert-butyl 3-(2-fluorophenyl)-2-((2*R*,3*R*)-3-methoxy-2-methyl-3-((*S*)-pyrrolidin-2-yl)propanamido )propanoate **25f** (100 mg, pale yellow oily). The product was used in the next step without further purification.
MS m/z (ESI): 407.2 [M-1]

Step 6

(*S*)-tert-butyl 2-((2*R*,3*R*)-3-((*S*)-1-((5*S*,8*S*,11*S*,12*R*)-11-((*S*)-sec-butyl)-1-(9*H*-fluoren-9-yl)-5,8-diisopropyl-12-methoxy-4,10-dimethyl-3,6,9-trioxo-2-oxa-4,7,10-triazatetradecan-14-oyl)pyrrolidin-2-yl)-3-methoxy-2-methylpropanamido)-3-(2-fluorophenyl)propanoate

(*S*)-tert-butyl 3-(2-fluorophenyl)-2-((2*R*,3*R*)-3-methoxy-2-methyl-3-((*S*)-pyrrolidin-2-yl)propanamido )propanoate **25f** (100 mg, 0.244 mmol), (5*S*,8*S*,11*S*,12*R*)-11-((*S*)-sec-butyl)-1-(9*H*-fluoren-9-yl)-5,8-diisopropyl-12-methoxy-4,1 0-dimethyl-3,6,9-trioxo-2-oxa-4,7,10-triazatetradecan-14-oic acid **1i** (156.1 mg, 0.244 mmol) was dissolved in 7.5 mL of mixed solvent of dichloromethane and dimethylformamide (V/V = 4: 1), and added with *N*, *N*-diisopropylethylamine (158 mg, 1.224 mmol). The mixture was added with 2- (7-azobenzotriazole) *-N, N, N', N'*-tetramethyluronium hexafluorophosphate (121 mg, 0.318 mmol) under argon atmosphere, and stirred at room temperature for 1 hour. The reaction mixture was then diluted with dichloromethane, washed successively with water and saturated sodium chloride solution, dried over anhydrous sodium sulfate, filtered and the filtrate was concentrated under reduced pressure . The residues were purified by silica gel column chromatography using eluent system B to give the title product of (*S*)-tert-butyl 2-((2*R*,3*R*)-3-((*S*)-1-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)pyrr olidin-2-yl)-3-methoxy-2-methylpropanamido)-3-(2-fluorophenyl)propanoate **25g** (242 mg, pale yellow oily), yield96.5%.
MS m/z (ESI): 1028.4 [M+1]

Step 7

(*S*)-tert-butyl 2-((2*R*,3*R*)-3-((*S*)-1-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)pyrrolidin-2-yl)-3-methoxy-2-m ethylpropanamido)-3-(2-fluorophenyl)propanoate

(*S*)-tert-butyl 2-((2*R*,3*R*)-3-((*S*)-1-((5*S*,8*S*,11*S*,12*R*)-11-((*S*)-sec-butyl)-1-(9*H*-fluoren-9-yl)-5,8-diisopr opyl-12-methoxy-4,10-dimethyl-3,6,9-trioxo-2-oxa-4,7,10-triazatetradecan-14-oyl)pyrr olidin-2-yl)-3-methoxy-2-methylpropanamido)-3-(2-fluorophenyl)propanoate **25g** (242 mg, 0.235 mmol) was dissolved in 3 mL of dichloromethane, and added with 3 mL of diethylamine. The mixture was stirred at room temperature for 3 hours and then concentrated under reduced pressure to give the crude title product of (*S*)-tert-butyl 2-((2*R*,3*R*)-3-((*S*)-1-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)pyrrolidin-2-yl)-3-methoxy-2-m ethylpropanamido)-3-(2-fluorophenyl)propanoate **25h** (250 mg, yellow oily). The product was used in the next step without further purification.
MS m/z (ESI): 806.7[M+1]

Step 8

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(2-fluorophenyl)propanoic acid

The crude product of (*S*)-tert-butyl 2-((2*R*,3*R*)-3-((*S*)-1-((3*R*,4*S*,5*S*)-4-((*S*)-*N*,3-dimethyl-2-((*S*)-3-methyl-2-(methylamino)b utanamido)butanamido)-3-methoxy-5-methylheptanoyl)pyrrolidin-2-yl)-3-methoxy-2-m ethylpropanamido)-3-(2-fluorophenyl)propanoate **25h** (262 mg, 0.325mmol) was placed in the reaction flask and added with 7 mL of 4 M solution of hydrogen chloride in dioxane. The reaction system was sealed and stirred at room temperature for 12 hours. The reaction solution was then concentrated under reduced pressure, and the residues were purified by high performance liquid chromatography to give the title product of (*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(2-fluorophenyl)propanoic acid **25** (50 mg, white solid), yield28.4%.
MS m/z (ESI): 750.7 [M+1]
$^1$H NMR (400 MHz, $CD_3OD$) δ 7.35-7.30(m, 1H), 7.20-7.15(m, 1H), 7.09-6.09(m, 2H), 4.81-4.75(m, 1H), 4.70-4.64(m, 1H), 4.15-4.13(m, 1H), 4.05-4.03(m, 1H), 3.84-3.81 (m, 1H), 3.69-3.64(m, 1H), 3.55-3.51(m, 1H), 3.48-3.13(m, 13H), 3.03-2.91(m, 2H), 2.66-2.53(m, 3H), 2.47-2.45(m, 1H), 2.40-2.33(m, 2H), 2.27-2.23 (m, 1H), 2.17-2.11 (m, 2H), 1.96-1.85(m, 3H), 1.63-1.40(m, 4H), 1.21-1.14(m, 3H), 1.09-0.94(m, 12H), 0.87-0.84(m, 3H).

Example 26

(*S*)-2-((2*R*,3*R*)-3-((*S*)-1-((3*R*,4*R*,5*S*)-4-((*S*)-2-((*R*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-fluorop henyl)propanoic acid

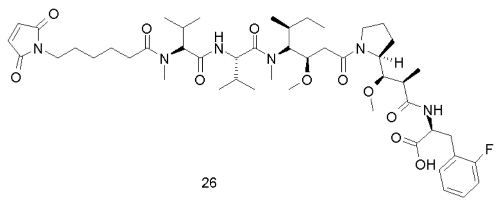

26

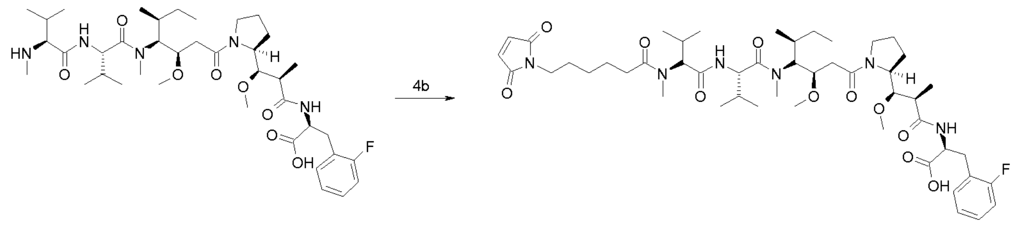


25

26

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(2-fluorophenyl)propanoic acid **25** (20 mg, 0.026 mmol) was dissolved in 3 mL of dichloromethane and then added with *N*, *N*-diisopropylethylamine(13.7 mg, 0.106mmol). The mixture was cooled to 0°C. under argon atmosphere, and then added dropwise with a preformed solution of 6-(2,5-dioxo-2,5-dihydro-1*H*-pyrrol-1-yl) hexanoyl chloride 4b (10.1 mg, 0.0443 mmol) in dichloromethane, and stirred at room temperature for 2 hours. The reaction was quenched with methanol, and then concentrated under reduced pressure. The residues were purified by high performance liquid chromatography to obtain the title product of (*S*)-2-((2*R*,3*R*)-3-((*S*)-1-((3*R*,4*R*,5*S*)-4-((*S*)-2-((*R*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-fluorop henyl)propanoic acid **26** (7 mg, white solid) , yield 28.5%.

MS m/z (ESI): 941.6 [M-1]

$^1$HNMR (400 MHz, $CD_3OD$) δ 7.32-7.21(m, 2H), 7.10-7.00(m, 2H), 6.80-6.78(m, 2H), 4.75-4.55(m, 3H), 4.12-4.06(m, 2H), 3.93-3.89(m, 1H), 3.87-3.78(m, 2H), 3.69-3.63(m, 1H), 3.52-3.47(m, 3H), 3.44-3.28(m, 3H), 3.21-3.10(m, 4H), 3.04-2.96 (m, 4H), 2.53-2.40(m, 4H), 2.35-2.18(m, 2H), 2.13-2.00(m, 2H), 1.94-1.75(m, 4H), 1.68-1.56(m, 5H), 1.37-1.27(m, 4H), 1.20-1.13(m, 3H), 1.05-0.81(m, 18H).

Example 27

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(2-methoxyphenyl)propanoic acid

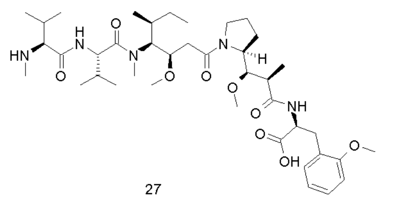

27

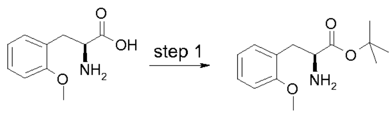


27a

27b

Step 1

(*S*)-tert-butyl-2-amino-3-(2-methoxyphenyl)propanoate

(*S*)-2-amino-3-(2-methoxyphenyl)propanoic acid **27a** (250 mg, 1.28 mmol, prepared according to the known method of "Chemical Communications (Cambridge, United Kingdom), 2013, 49(70), 7744-766") was dissolved in 7 mL of tert-butyl acetate. The solution was added with perchloric acid (270 mg(70%), 1.88 mmol) under argon atmosphere, and stirred at room temperature for 16 hours. The reaction mixture was then added with 10 mL of dichloromethane, and adjusted to pH = 8 with saturated sodium bicarbonate solution. The aqueous phase was separated and extracted with dichloromethane (10 mL ×3). The organic phases were combined, washed with saturated sodium chloride solution (10mL), dried over anhydrous sodium sulfate, filtered and the filtrate was concentrated under reduced pressure to obtain

the title product of (*S*)-tert-butyl 2-amino-3-(2-methoxyphenyl)propanoate **27b** (280 mg, light yellow oil), yield 87%.

The preparation method was similar to Example 1, except that the raw material in step 4 was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(2-methoxyphenyl)propanoate **27b,** to give the title product of (*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(2-methoxyphenyl)propanoic acid **27** (22 mg, off white solid) .
MS m/z (ESI): 760.7[M-1].

Example 28

(*S*)-2-((2*R*,3*R*)-3-((*S*)-1-((3*R*,4*R*,5*S*)-4-((*S*)-2-(*R*)-2-(6-(2,5-dioxo-2,5-dihydro-1*H*-pyrrol -1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-methox yphenyl)propanoic acid

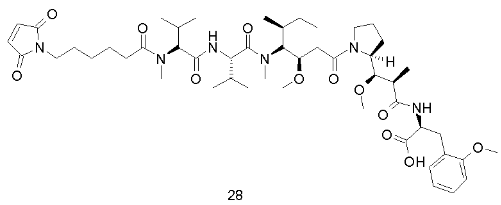

28

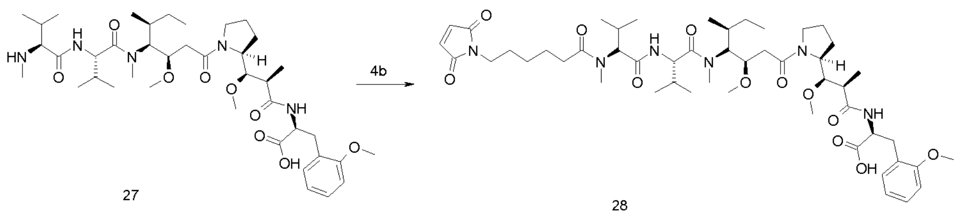


27

28

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(2-methoxyphenyl)propanoic acid **27** (18 mg, 0.023 mmol) was dissolved in 5 mL of dichloromethane. The solution was added with *N*, *N*-diisopropylethylamine(12.19 mg, 0.29 mmol) under argon atmosphere, and then added dropwise with a preformed solution of 6- (2,5-dioxo-2,5-dihydro-1*H*-pyrrol-1-yl) hexanoyl chloride **4b** (6.5 mg, 0.028 mmol) in dichloromethane at 0°C, and stirred at room temperature for 2 hours. The reaction was quenched with methanol, and then concentrated under reduced pressure.The residues were purified by high performance liquid chromatography to give the title product of (*S*)-2-((2*R*,3*R*)-3-((*S*)-1-((3*R*,4*R*,5*S*)-4-((*S*)-2-((*R*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-methox yphenyl)propanoic acid **28** (4 mg, white solid) , yield18.2%.
MS m/z (ESI): 955.5 [M+1]

Example 29

(*S*)-2-((2*R*,3*R*)-3-((*S*)-1-((3*R*,4*R*,5*S*)-4-((*S*)-*N*,3-dimethyl-2-((*S*)-3-methyl-2-(methylami no)butanamido)butanamido)-3-methoxy-5-methylheptanoyl)pyrrolidin-2-yl)-3-methoxy -2-methylpropanamido)-3-(p-tolyl)propanoic acid

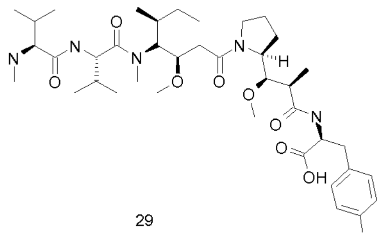

29

The preparation method was similar to Example 25, except that the raw material was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(*p*-tolyl)propanoate **15b,** to give the title product of (*S*)-2-((2*R*,3*R*)-3-((*S*)-1-((3*R*,4*R*,5*S*)-4-((*S*)-*N*,3-dimethyl-2-((*S*)-3-methyl-2-(methylami no)butanamido)butanamido)-3-methoxy-5-methylheptanoyl)pyrrolidin-2-yl)-3-methoxy -2-methylpropanamido)-3-(p-tolyl)propanoic acid **29** (20 mg, white solid) .

MS m/z (ESI): 746.6 [M+1]

$^1$H NMR (400 MHz, $CD_3OD$): δ 7.17-7.05(m, 4H), 4.84-4.67(m, 2H), 4.27-4.15 (m, 1H), 4.12-4.05(m, 1H), 3.89-3.82(m, 1H), 3.78-3.63(m, 2H), 3.57-3.47(m, 1H), 3.43-3.11(m, 7H), 2.92-2.79(m, 1H), 2.67(d, 3H), 2.52-2.44(m, 1H), 2.40-2.15(m, 7H), 2.12-2.01(m, 1H), 1.95-1.69(m, 3H), 1.67-1.49(m, 2H), 1.48-1.27(m,4H),1.23-1.12(m, 5H), 1.11-0.95(m, 14H), 0.94-0.84(m, 3H).

Example 30

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(p-tolyl)pr opanoic acid

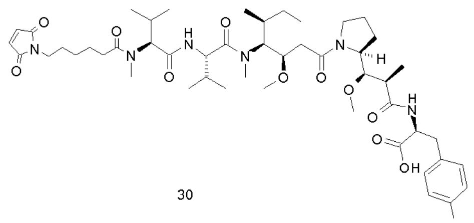

30

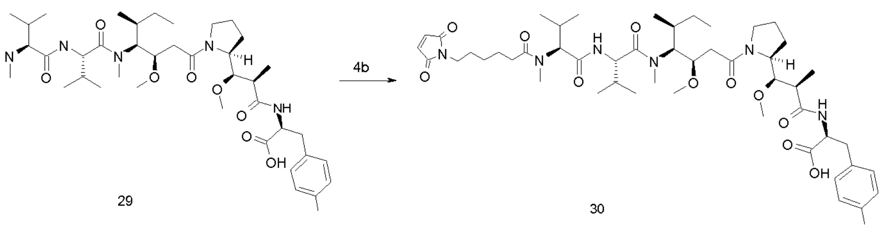


29

30

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(p-tolyl)propanoic acid **29** (12 mg, 0.016 mmol) was dissolved in 1 mL of dichloromethane. The solution was added with *N*, *N*-diisopropylethylamine (0.014 mL, 0.08 mmol) under argon atmosphere, and then added dropwise with a preformed solution of 6- (2,5-dioxo-2,5-dihydro-1*H*-pyrrol-1-yl) hexanoyl chloride **4b** (5.54 mg, 0.024 mmol) in dichloromethane at 0°C, and stirred at room temperature for 4 hours. The reaction was quenched with methano land concentrated under reduced pressure. The residues were purified by high performance liquid chromatography to give the title product of (*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(p-tolyl)pr opanoic acid **30** (4 mg, white solid) , yield 26.5%.

MS m/z (ESI): 939.4 [M+1]

$^1$H NMR (400 MHz, $CD_3OD$): δ 7.22-7.03(m, 4H), 6.85-6.78(m, 2H), 4.83-4.55(m, 3H), 4.27-3.97(m, 3H), 3.90-3.62(m,

3H), 3.59-2.95(m, 14H), 2.94-2.79(m, 1H), 2.54-2.35(m, 3H), 2.34-2.12(m, 5H), 2.08-1.99(m, 1H), 1.93-1.72(m, 3H), 1.71-1.51 (m,5H),1.48-1.24(m, 8H), 1.23-1.12(m, 3H), 1.11-0.78(m, 17H).

Example 31

(*S*)-3-(3-chlorophenyl)-2-((2*R*,3*R*)-3-((*S*)-1-((3*R*,4*R*,5*S*)-4-((*S*)-*N*,3-dimethyl-2-((*S*)-3-m ethyl-2-(methylamino)butanamido)butanamido)-3-methoxy-5-methylheptanoyl)pyrrolid in-2-yl)-3-methoxy-2-methylpropanamido)propanoic acid

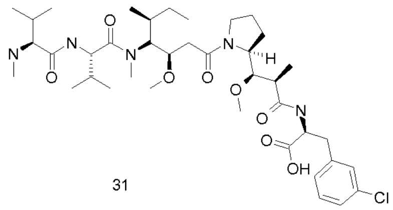

31

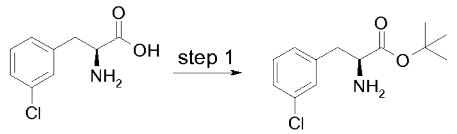


31a 31b

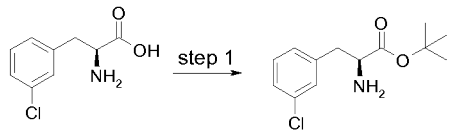


31a 31b

Step 1

(*S*)-tert-butyl 2-amino-3-(3-chlorophenyl)propanoate

(*S*)-2-amino-3-(3-chlorophenyl)propanoic acid **31a** (600 mg, 3 mmol) was dissolved in 15 mL of tert-butyl acetate. The solution was added dropwise with perchloric acid (450 mg(70%), 4.5 mmol) under argon atmosphere at 0°C, and stirred at room temperature for 12 hours. The reaction mixture was then added with 10 mL of water and the organic phase was washed successively with 20 mL of IN hydrochloric acid and saturated sodium bicarbonate solution. The aqueous phase was combined and adjusted to pH = 8~9 by adding dropwise with saturated sodium bicarbonate solution and extracted with dichloromethane (100 mL ×2). The organic phases were combined, dried over anhydrous sodium sulfate, filtered and the filtrate was concentrated under reduced pressure to give the crude title product of (*S*)-tert-butyl 2-amino-3-(3-chlorophenyl)propanoate **31b** (500 mg, oil). The product was used in the next step without further purification.

The preparation method was similar to Example 25, except that the raw material in step 4 was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(3-chlorophenyl)propanoate **31b,** to give the title product of (*S*)-3-(3-chlorophenyl)-2-((2*R*,3*R*)-3-((*S*)-1-((3*R*,4*R*,5*S*)-4-((*S*)-*N*,3-dimethyl-2-((*S*)-3-m ethyl-2-(methylamino)butanamido)butanamido)-3-methoxy-5-methylheptanoyl)pyrrolid in-2-yl)-3-methoxy-2-methylpropanamido)propanoic acid **31** (4 mg, white solid) .

MS m/z (ESI): 766.6 [M+1]

$^1$H NMR (400 MHz, $CD_3OD$): δ 7.31-7.12(m, 4H), 4.73-4.51(m, 2H), 4.22-4.05(m, 1H), 3.92-3.83(m, 1H), 3.76-3.65(m, 1H), 3.62-3.51(m, 1H), 3.50-3.12(m, 9H), 3.09-2.98(m, 1H), 2.59-2.39(m, 4H), 2.38-2.24(m, 1H), 2.23-2.17(m, 1H), 2.16-2.01 (m, 3H), 2.00-1.85(m, 2H), 1.84-1.76(m, 1H), 1.73-1.57(m, 2H), 1.43-1.27(m, 5H), 1.25-1.14(m, 3H), 1.10-0.95(m, 13H), 0.94-0.83(m, 5H).

Example 32

(*S*)-2-((2*R*,3*R*)-3-((*S*)-1-((3*R*,4*R*,5*S*)-4-((*S*)-*N*,3-dimethyl-2-((*S*)-3-methyl-2-(methylami no)butanamido)butanamido)-3-methoxy-5-methylheptanoyl)pyrrolidin-2-yl)-3-methoxy -2-methylpropanamido)-3 -(3 -fluorophenyl)propanoic acid

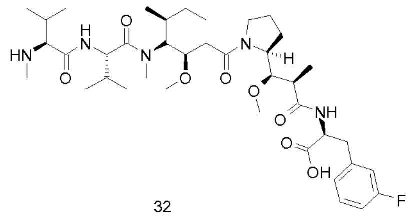

32

The preparation method was similar to Example 25, except that the raw material in step 4 was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(3-fluorophenyl)propanoate **17b,** to give the title product of (*S*)-2-((2*R*,3*R*)-3-((*S*)-1-((3*R*,4*R*,5*S*)-4-((*S*)-*N*,3-dimethyl-2-((*S*)-3-methyl-2-(methylami no)butanamido)butanamido)-3-methoxy-5-methylheptanoyl)pyrrolidin-2-yl)-3-methoxy -2-methylpropanamido)-3-(3-fluorophenyl)propanoic acid **32** (33.5 mg, white solid) .
MS m/z (ESI): 750.7 [M+1]
$^1$H NMR (400 MHz, $CD_3OD$) δ 7.31-7.28(m, 1H), 7.08-6.95(m, 3H),4.77-4.69(m, 2H), 4.09-3.94(m,3H), 3.69-3.66(m,2H),3.48-3.44(m, 2H), 3.38-3.36(m, 3H), 3.34-3.28(m, 6H), 3.26-3.19(m, 2H), 3.13(m,1H), 3.01-2.91(m, 2H), 2.67-2.65(m, 2H),2.54-2.47(m, 2H), 2.34-2.28(m, 2H), 2.18-2.00(m, 2H),1.98-1.77(m, 2H), 1.55-1.42(m, 2H), 1.08-0.98(m, 18H),0.88-0.83 (m, 3H).

Example 33

(*S*)-3-(2,4-dichlorophenyl)-2-((2*R*,3*R*)-3-((*S*)-1-((3*R*,4*R*,5*S*)-4-((*S*)-*N*,3-dimethyl-2-((*S*)-3-methyl-2-(methylamino)butanamido)butanamido)-3-methoxy-5-methylheptanoyl)pyrr olidin-2-yl)-3-methoxy-2-methylpropanamido)propanoic acid

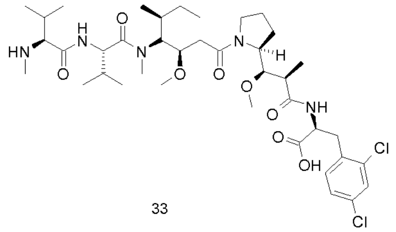

33

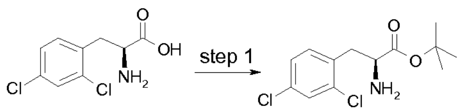


33a 33b

Step 1

(S)-tert-butyl 2-amino-3-(2,4-dichlorophenyl)propanoate

(*S*)-2-amino-3-(2,4-dichlorophenyl)propanoic acid **33a** (1.3 g, 5.57 mmol, prepared according to the known method of "International Journal of Peptide & Protein Research, 1987, 30(1), 13-21") was dissolved in 10 mL of tert-butyl acetate. The solution was added dropwise with perchloric acid (1.2 g(70%), 8.36 mmol) at 0°C under an argon atmosphere, and stirred at room temperature for 12 hours. The reaction mixture was diluted with methylene chloride and added with saturated sodium bicarbonate solution dropwise until pH reached 8 to 9. The aqueous phase was extracted with methylene chloride. The organic phases were combined, washed successively with water and saturated

sodium chloride solution, dried over anhydrous sodium sulfate, filtered and the filtrate was concentrated under reduced pressure to give the crude title product of (*S*)-tert-butyl 2-amino-3-(2,4-dichlorophenyl)propanoate **33b** (3.4 g, light yellow oil). The product was used in the next step without further purification.

The preparation method was similar to Example 25, except that the raw material in step 4 was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(2,4-dichlorophenyl)propanoate **33b,** to give the title product of (*S*)-3-(2,4-dichlorophenyl)-2-((2*R*,3*R*)-3-((*S*)-1-((3*R*,4*R*,5*S*)-4-((*S*)-*N*,3-dimethyl-2-((*S*)-3 -methyl-2-(methylamino)butanamido)butanamido)-3 -methoxy-5 -methylheptanoyl)pyrr olidin-2-yl)-3-methoxy-2-methylpropanamido)propanoic acid **33** (23 mg, white solid) .

MS m/z (ESI): 800.6 [M+1]

$^1$H NMR (400 MHz, $CD_3OD$) δ 7.43-7.42(m, 1H), 7.26-7.23(m,2H), 4.75-4.67(m, 2H), 4.14-4.04(m, 2H), 4.00-3.98(m, 1H), 3.91-3.89(m, 1H), 3.68-3.67(m, 3H), 3.39-3.26(m, 12H), 3.21-3.12(m, 3H), 2.67-2.64(m, 3H), 2.50-2.46(m, 3H), 2.31-2.28 (m, 2H), 2.17-2.15(m, 2H), 2.02-2.00(m, 4H), 1.90-1.88(m, 2H), 1.74-1.72(m, 2H), 1.39-1.37(m, 2H), 1.06-0.98(m, 12H).

Example 34

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(o-tolyl)propanoic acid

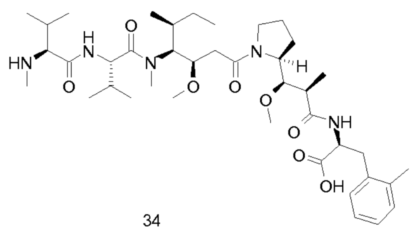

34

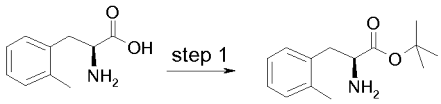


34a 34b

Step 1

(S)-tert-butyl 2-amino-3-(o-tolyl)propanoate

(*S*)-2-amino-3-(o-tolyl)propanoic acid **34a** (100 mg, 0.55 mmol, prepared according to the known method of "International Journal of Peptide & Protein Research, 1987, 30(1), 13-21") was dissolved in 2.5 mL of tert-butyl acetate, and added dropwise with perchloric acid (107 mg(70%), 0.83 mmol) under argon atmosphere. The mixture was stirred at room temperature for 16 hours and then diluted with 5 mL of dichloromethane, and saturated sodium bicarbonate solution was added dropwise to adjust to pH = 8. The aqueous phase was extracted with dichloromethane (5 mL ×3). The organic phases were combined, washed successively with water and saturated sodium chloride solution, dried over anhydrous sodium sulfate, filtered and the filtrate was concentrated under reduced pressure to give the crude title product of (*S*)-tert-butyl 2-amino-3-(o-tolyl)propanoate **34b** (140 mg, colorless oil). The product was used in the next step without further purification.

The preparation method was similar to Example 25, except that the raw material in step 4 was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(o-tolyl)propanoate **34b,** to give the title product of (*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(o-tolyl)propanoic acid **34** (40 mg, white solid) .

MS m/z (ESI): 746.8 [M+1]

$^1$H NMR (400 MHz, $CD_3OD$) δ 8.41-8.39(d, 1H), 8.19-8.17(d, 1H), 7.18-7.05(m, 4H), 4.80-4.78(d, 1H), 4.71-4.69(d, 1H), 3.86-3.84(m, 1H), 3.76-3.72(m, 1H), 3.69-3.63 (m, 2H), 3.48-3.42(d, 2H), 3.38-3.23(m, 5H), 3.20-3.12(m, 4H), 2.98-2.87 (m, 2H), 2.66-2.65(d, 3H), 2.53-2.50(d, 1H), 2.46-2.44(d, 1H), 2.41-2.26(m, 4H), 2.21-2.14(m, 2H), 2.10-2.04(m, 1H),

1.87-1.85(m, 2H), 1.77-1.74(m, 1H), 1.62-1.53 (m, 2H), 1.33-1.28(m, 3H), 1.23-1.21(d, 2H), 1.16-1.14(d, 2H), 1.08-0.96(m, 14H), 0.90-0.84(m, 3H).

Example 35

(*S*)-2-((2*R*,3*R*)-3-((*S*)-1-((3*R*,4*R*,5*S*)-4-((*S*)-*N*,3-dimethyl-2-((*S*)-3-methyl-2-(methylami no)butanamido)butanamido)-3-methoxy-5-methylheptanoyl)pyrrolidin-2-yl)-3-methoxy -2-methylpropanamido)-3-(thiophen-2-yl)propanoic acid

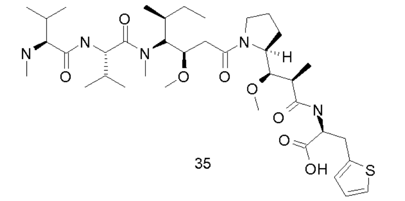

35

The preparation method was similar to Example 25, except that the raw material in step 4 was replaced with (2*R*,3*R*)-3-((*S*)-1-(tert-butoxycarbonyl)pyrrolidin-2-yl)-3-methoxy-2-methylpropanoic acid **25d** and (*S*)-tert-butyl 2-amino-3-(thiophen-2-yl)propanoate **16b,** to give the title product of (*S*)-2-((2*R*,3*R*)-3-((*S*)-1-((3*R*,4*R*,5*S*)-4-((*S*)-*N*,3-dimethyl-2-((*S*)-3-methyl-2-(methylami no)butanamido)butanamido)-3-methoxy-5-methylheptanoyl)pyrrolidin-2-yl)-3-methoxy -2-methylpropanamido)-3-(thiophen-2-yl)propanoic acid **35** (2.6 mg, white solid). $^1$H NMR (400 MHz, $CD_3OD$) δ 7.21-7.19(m, 1H), 6.91-6.90(m, 2H), 4.80-4.65(m, 2H), 4.16-4.08(m, 2H), 3.93-3.90(m, 1H), 3.70-3.66(m, 2H), 3.55-3.52(m, 1H), 3.48-3.45(m, 2H), 3.40-3.26(m, 6H), 3.25-3.13(m, 4H), 2.96-2.82(t, 1H), 2.69-2.65(d, 3H), 2.52-2.47(m, 2H), 2.39-2.29(m, 1H), 2.21-2.14(m, 2H), 1.92-(s, 3H), 1.63-1.61(m, 2H), 1.40-1.29(m, 3H), 1.23-1.14(m, 3H), 1.10-0.98(m, 13H), 0.88-0.85 (t, 3H).

Example 36

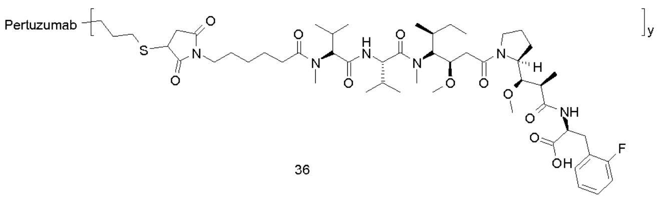

36

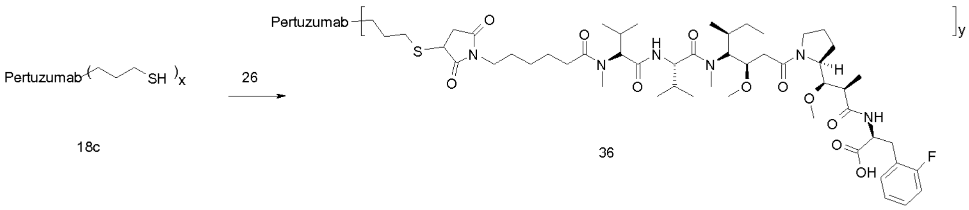

18c

26

36

(*S*)-2-((2*R*,3*R*)-3-((*S*)-1-((3*R*,4*R*,5*S*)-4-((*S*)-2-((*R*)-2-(6-(2,5-dioxo-2,5-dihydro-1H-pyrro l-1-yl)-N-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-fluorop henyl)propanoic acid **26** (5.45 mg, 5.78 μmol) was dissolved in 1.2mL of acetonitrile and added with Pertuzumab-propanethiol solution **18c** (7.56 mg/mL, 12 mL), and stirred at 25 °C for 4 hours.Then the reaction mixture was desalted and purified by Sephadex G25 gel column (eluting phase: 0.05 *M* PBS solution, pH 6.5), filtrated under a sterile condition through a 0.2 μm filter to obtain the title product of **36** in PBS buffer(3.51 mg/mL, 27.5 mL), and then stored at 4 °C.
Q-TOF LC/MS: characteristic peaks: 148094.39($M_{Ab}$+0D), 149111.06($M_{Ab}$+1D), 150167.12 ($M_{Ab}$+ 2D), 151188.93($M_{Ab}$+3D), 152243.46($M_{Ab}$+4D), 153272.96($M_{Ab}$+5D).
average value: y=1.9

Example 37

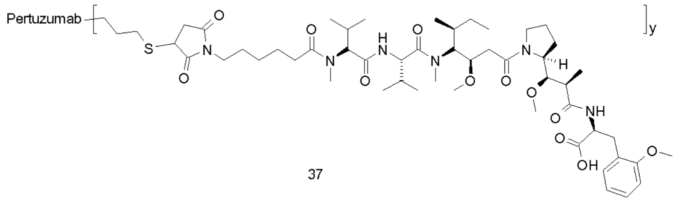

37

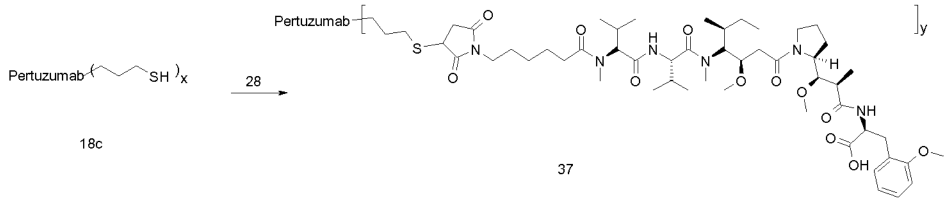


18c

37

(*S*)-2-((2*R*,3*R*)-3-((*S*)-1-((3*R*,4*R*,5*S*)-4-((*S*)-2-((*R*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-methox yphenyl)propanoic acid **28** (1.75 mg, 1.83 μmol) was dissolved in 0.9 mL of acetonitrile and added with Pertuzumab-propanethiol solution **18c** (2.31 mg/mL, 9.0 mL), and stirred at 25 °C for 6 hours.Then the reaction mixture was desalted and purified by Sephadex G25 gel column (eluting phase: 0.05 *M* PBS solution, pH 6.5), filtrated under a sterile condition through a 0.2 μm filter to obtain the title product of **37** in PBS buffer (1.35 mg/mL, 13.0 mL), and then stored at 4 °C.

Q-TOF LC/MS: characteristic peaks: 148093.25($M_{Ab}$+0D), 149281.78($M_{Ab}$+1D), 150474.33 ($M_{Ab}$+ 2D), 151372.72($M_{Ab}$+3D), 152373.24($M_{Ab}$+4D), 153469.40($M_{Ab}$+5D).

average value: y=2.3.

Example 38

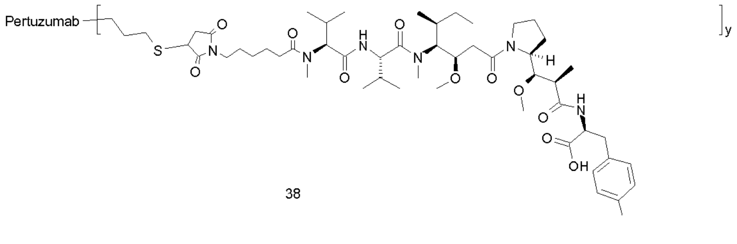

38

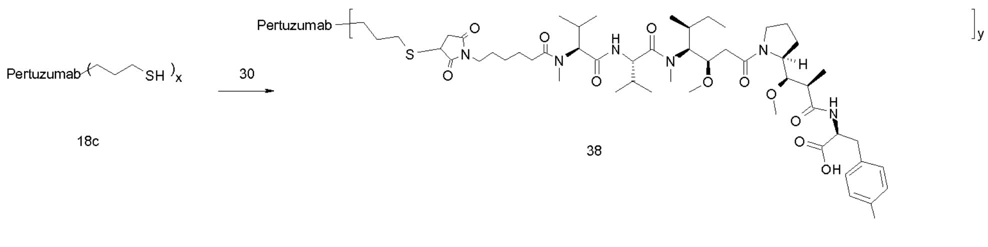


18c

38

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro l-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(p-tolyl)pr opanoic acid **30** (1.07 mg, 1.14 μmol) was dissolved in 1.25mL of acetonitrile and added with Pertuzumab-propanethiol solution **18c**(1.5 mg/mL, 12.5 mL), and stirred at 25 °C for 4.5 hours. The reaction mixture was desalted and purified by Sephadex G25 gel column (eluting phase: 0.05 *M* PBS solution, pH 6.5), filtered

under a sterile condition through a 0.2 μm filter to obtain the title product of **38** in PBS buffer(0.74 mg/mL, 17.5 mL), and then stored at 4 °C.
Q-TOF LC/MS: characteristic peaks: 148247.08($M_{Ab}$+0D), 149265.32($M_{Ab}$+1D), 150276.10 ($M_{Ab}$+ 2D), 151334.50($M_{Ab}$+3D), 152392.21($M_{Ab}$+4D), 153388.72($M_{Ab}$+5D).
average value: y=2.4.

Example 39

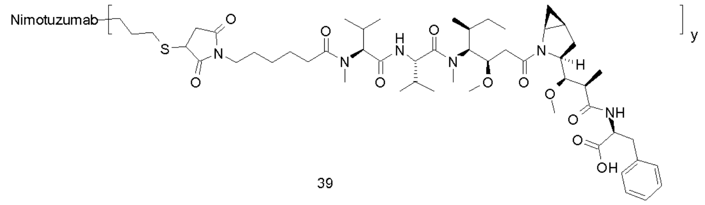

39

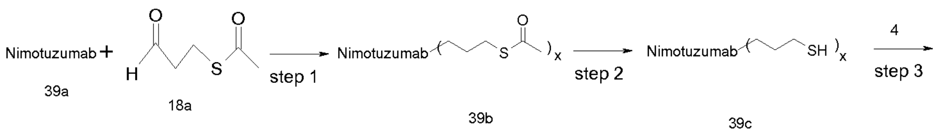


39a 18a 39b 39c

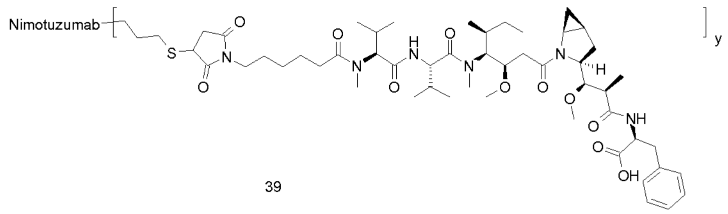

39

Step 1

S-(3-oxopropyl) ethanethioate **18a** (2.44 mg, 18.5 μmol) was dissolved in 3.0 mL of acetonitrile. The Nimotuzumab in acetic acid/sodium acetate buffer (10.22 mg/ml, 30 mL, 0.204 mmol) with pH 4.3 was added with the solution of S-(3-oxopropyl) ethanethioate **18a** in acetonitrile, and then added with 1.2 mL of an aqueous solution of sodium cyanoborohydride (49.86 mg, 793 μmol)dropwise. The reaction mixture was stirred at 25 °C for 2 hours. The reaction solution was firstly purified with PBS buffer (250 mL) containing 10% acetonitrile by 30 KDa ultrafiltration, then purified by a 30 KDa ultrafiltration pack using PBS buffer (200 mL) with pH = 6.5 to remove the unreacted S-(3-oxopropyl) ethanethioate **18a** and sodium cyanoborohydride to give the title product of **39b** in PBS buffer (about75 mL), which was used directly in the next step.

Step 2

**39b** in PBS buffer solution (75.0 mL) was added with 2.0 mL of a 2.0 M solution of hydroxylamine hydrochloride, and then placed on a shaked in a water bath at 25 ° C for 30 minutes. The reaction solution was purified by 30 KDa ultrafiltration with PBS buffer with pH = 6.5 to give the title product of Nimotuzumab-propanethiol **39c** in PBS buffer solution (concentration 5.38 mg/ml, 55 mL).

Step3

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 *H*-pyrrol-1-yl)-*N*-methylhexanamido)-3-methylbutanamido)-*N*,3-dimethylbutanamido)-3-methoxy-5-methylheptanoyl)-2-azabicyclo[3.1.0]hexan-3-yl)-3-methoxy-2-methylpro panamido) **4** (4.48 mg, 4.78 μmol) was dissolved in 1.1 mL of acetonitrile and added with Nimotuzumab-propanethiol in PBS buffer **39c** (5.38 mg/mL, 11 mL). After shaked on a shaker in a water bath at 25 °C for 4 hours, the reaction was stopped.
The resulting mixture was desalted and purified with a Sephadex G25 gel column (Elution phase: 0.05 M PBS solution with pH 6.5) to give the crude title product of **39** in PBS buffer (2.96 mg/mL, 20.5 mL), which was further

concentrated to about 6 mL by centrifugation, and desalted and purified again with a Sephadex G25 gel column (Elution phase: 0.05 M PBS solution at pH 6.5) to give the title product of **39** in PBS buffer (4.25 mg/mL, 11.8 mL), and then stored at 4 °C.
Q-TOF LC/MS: characteristic peaks: 150188.68($M_{Ab}$+0D), 151234.76($M_{Ab}$+1D), 152248.46 ($M_{Ab}$+ 2D), 153419.10($M_{Ab}$+3D), 154312.17($M_{Ab}$+4D), 155358.48 ($M_{Ab}$+5D).
average value: y=2.2.

Example 40

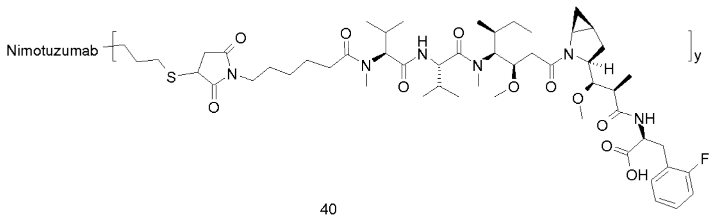

40

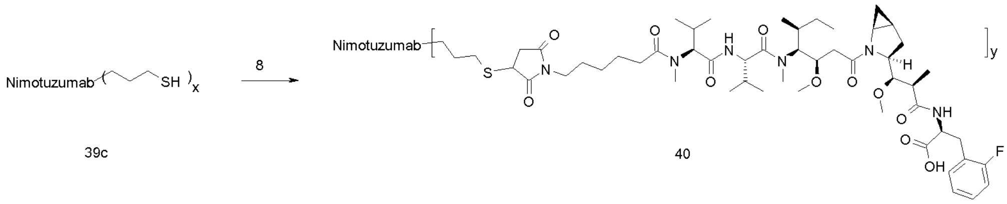


39c

40

(*S*)-2-((2*R*,3*R*)-3-((1*S*,3*S*,5*S*)-2-((3*R*,4*S*,5*S*)-4-((*S*)-2-((*S*)-2-(6-(2,5-dioxo-2,5-dihydro-1 *H*-pyrrol-1-yl)-*N*-methylhexanamido)-3-methylbutanamido)-*N*,3-dimethylbutanamido)-3 -methoxy-5 -methylheptanoyl)-2-azabicyclo [3.1. 0]hexan-3 -yl)-3 -methoxy-2-methylpro panamido)-3-(2-fluorophenyl)propanoic acid **8** (4.32 mg, 4.52 μmol) was dissolved in 1.1 mL of acetonitrile and added with Nimotuzumab-propanethiol **39c** in PBS buffer (5.38 mg/mL, 11 mL). After shaked on a shaker in a water bath at 25 ° C for 4 hours, the reaction was stopped.

The resulting mixture was desalted with Sephadex G25 gel column (Elution phase: 0.05 *M* PBS solution with pH 6.5) to give the crude title product of **40** in PBS buffer(2.92 mg/mL, 20 mL), which was further concentrated to about 5.5 mL, and desalted again with Sephadex G25 gel column (Elution phase: 0.05 *M* PBS solution with pH 6.5) to give the title product of **40** in PBS buffer (4.25 mg/mL, 11.6 mL), then stored at 4 °C. Q-TOF LC/MS:characteristic peaks: 150186.98($M_{Ab}$+0D), 151374.09($M_{Ab}$+1D), 152287.22 ($M_{Ab}$+ 2D), 153353.26($M_{Ab}$+3D), 154501.80($M_{Ab}$+4D), 155575.57($M_{Ab}$+5D).
average value: y=2.2.

Example 41

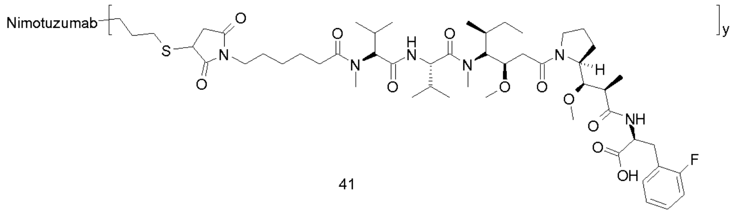

41

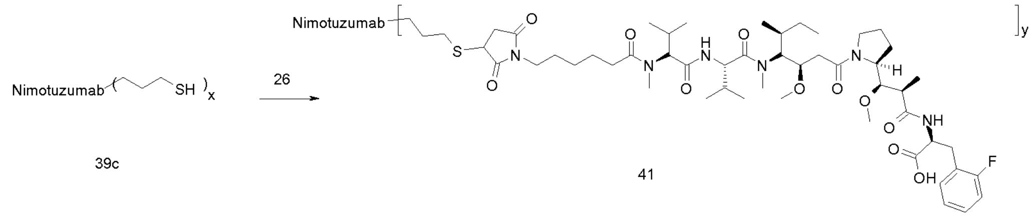


39c

41

(*S*)-2-((2*R*,3*R*)-3-((*S*)-1-((3*R*,4*R*,5*S*)-4-((*S*)-2-((*R*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(2-fluorop henyl)propanoic acid **26** (4.45 mg, 4.72 μmol) was dissolved in 1.1 mL of acetonitrile and added with Nimotuzumab-propanethiol **39c** in PBS buffer (5.38 mg/mL, 11 mL). After shaked on a shaker in a water bath at 25 °C for 4 hours, the reaction was stopped. The resulting mixture was desalted with Sephadex G25 gel column (Elution phase: 0.05 *M* PBS solution at pH 6.5) to give the crude title product of **41** in PBS buffer (2.96 mg/mL, 20 mL), which was further centrifuged and concentrated to about 5.5 mL and desalted again with Sephadex G25 gel column (Elution phase: 0.05 *M* PBS solution with pH 6.5) to give the title product of **41** in PBS buffer(4.33 mg/mL, 11 mL), then stored at 4 °C.

Q-TOF LC/MS: characteristic peaks: 150186.05($M_{Ab}$+0D), 151362.44($M_{Ab}$+1D), 152261.90 ($M_{Ab}$+ 2D), 153438.29($M_{Ab}$+3D), 154339.30($M_{Ab}$+4D), 155511.23($M_{Ab}$+5D).

average value: y=2.3.

Example 42

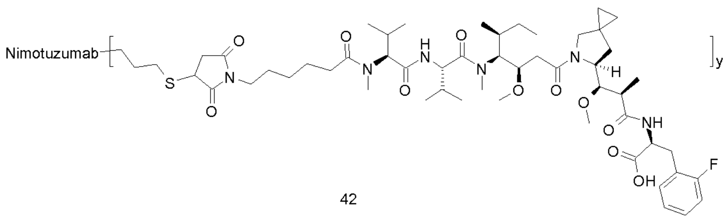

42

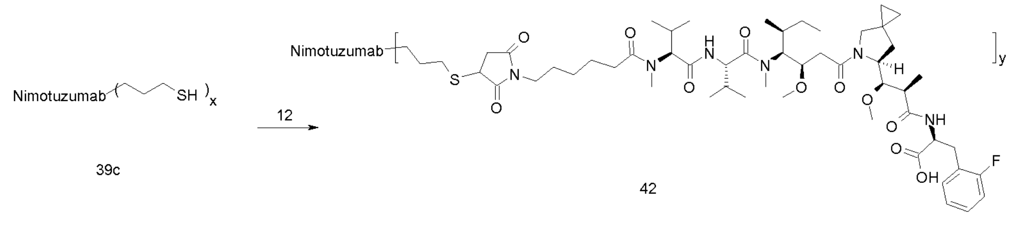


39c

42

(*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-(2-fluorophenyl)propanoic acid **12** (0.56 mg, 0.58 μmol) was dissolved in 0.42 mL of acetonitrile and added with Nimotuzumab-propanethiol solution **39c** (2.06 mg/mL, 4.2 mL). After shaked on a shaker in a water bath at 25 °C for 5 hours, the reaction was stopped.

The reaction mixture was desalted with Sephadex G25 gel column (Elution phase: 0.05 *M* PBS solution at pH 6.5) to give the crude title product of **42** in PBS buffer (0.74 mg/mL, 10 mL), which was further centrifuged to about 5.5 mL and desalted again with Sephadex G25 gel column (Elution phase: 0.05 *M* PBS solution with pH 6.5) to give the title product of **42** in PBS buffer(1.15 mg/mL, 6 mL), then stored at 4 °C.

Q-TOF LC/MS: characteristic peaks: 150188.42($M_{Ab}$+0D), 151387.82($M_{Ab}$+1D), 152472.27 ($M_{Ab}$+ 2D), 153528.33($M_{Ab}$+3D).

average value: y=1.0.

Example 43

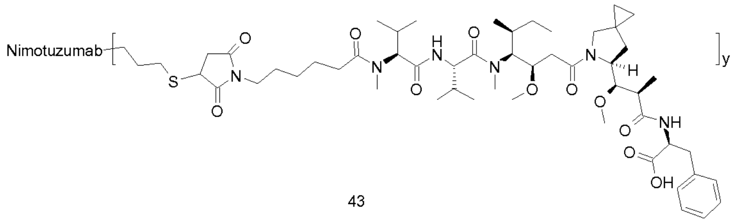

43

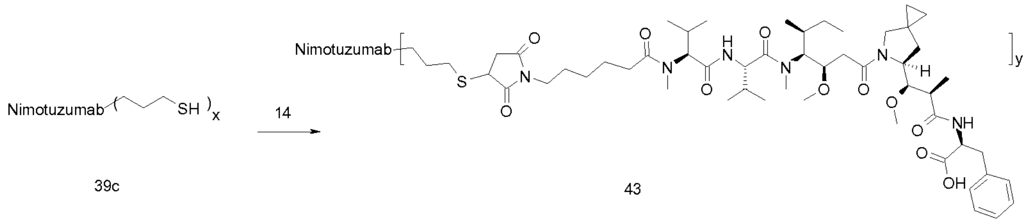


39c

43

(*S*)-2-((2*R*,3*R*)-3-((*S*)-5-((3*R*,4*S*,5*S*)-4-((*S*)-2-((*S*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)-5-azaspiro[2.4]heptan-6-yl)-3-methoxy-2-methylpropanamido)-3-phenylpropanoic acid **14** (0.60 mg, 0.63 μmol) was dissolved in 0.42 mL of acetonitrile and added with Nimotuzumab-propanethiol solution **39c** (2.06 mg/mL, 4.2 mL). After shaked on a shaker in a water bath at 25 °C for 5 hours, the reaction was stopped.

The reaction mixture was desalted with Sephadex G25 gel column (Elution phase: 0.05 *M* PBS solution at pH 6.5) to give the crude title product of **43** in PBS buffer (0.78 mg/mL, 9.5 mL), which was further centrifuged and concentrated to about 5.5 mL and desalted again with Sephadex G25 gel column (Elution phase: 0.05 *M* PBS solution with pH 6.5) to give the title product of **43** in PBS buffer(1.16 mg/mL, 6 mL), then stored at 4 °C.
Q-TOF LC/MS: characteristic peaks: 150188.39($M_{Ab}$+0D), 151251.46($M_{Ab}$+1D), 152442.90 ($M_{Ab}$+ 2D), 153507.73($M_{Ab}$+3D).
average value: y=1.0.

Example 44

(*S*)-2-((2*R*,3*R*)-3-((2*S*,4*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-4-fluoropyrrolidin-2-yl )-3-methoxy-2-methylpropanamido)-3-phenylpropanoic acid

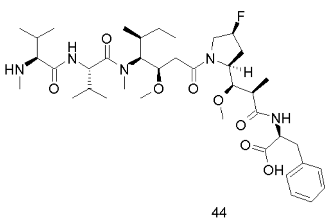

44

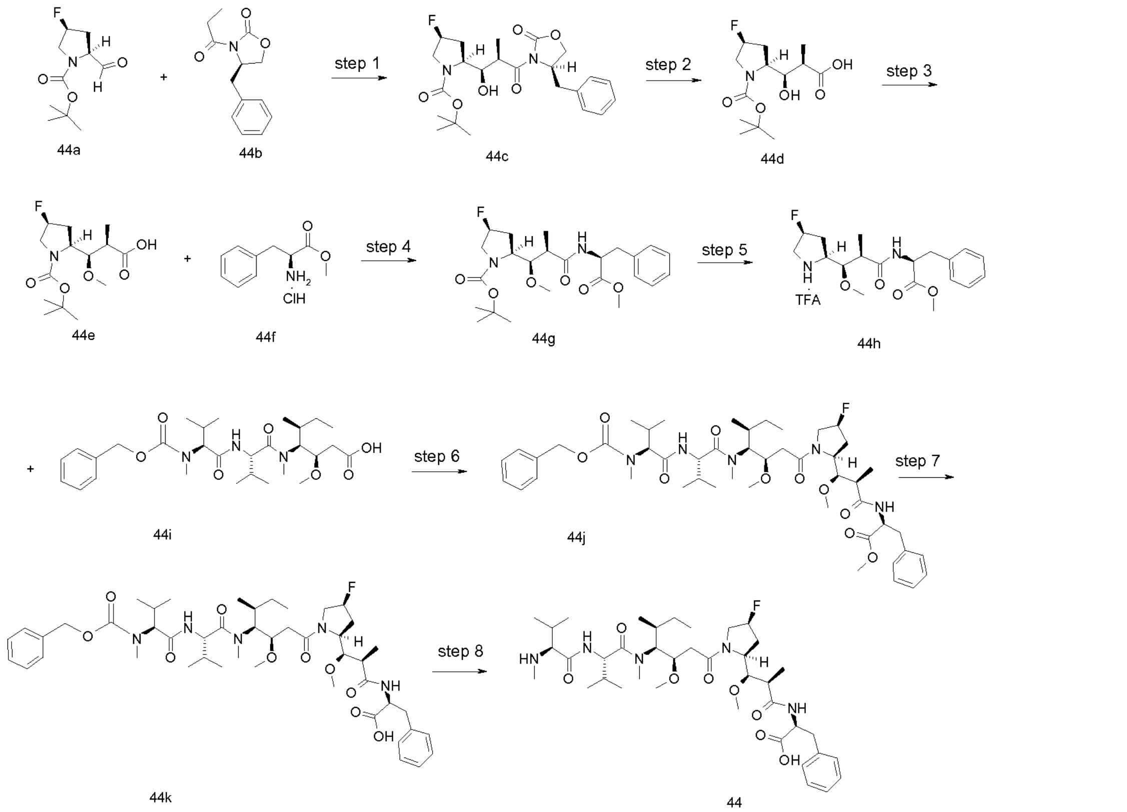


44a 44b 44c 44d 44e 44f 44g 44h 44i 44j 44k 44

Step 1

(2*S*,4*S*)-tert-butyl 2-((1*R*,2*R*)-3-((*R*)-4-benzyl-2-oxooxazolidin-3-yl)-1-hydroxy-2-methyl-3-oxopropyl)-4-fluoropyrrolidine-1 -carboxylate

(*R*)-4-benzyl-3-propionyloxazolidin-2-one **44b** (21 g, 90 mmol, prepared according to the known method of "Tetrahedron Letters, 1999, 40(36), 6545-6547") was dissolved in 300 mL of dichloromethane, and cooled to 0°C under argon atmosphere. The reaction solution was added dropwise with titanium tetrachloride (9.8 mL, 1.1 mmol) at 0 °C, and the solution gradually changed from colorless to yellow to form a yellow solid. *N*,*N*-diisopropylethylamine (40 mL, 225 mmol) was slowly added dropwise with a formation of white smoke, and the solution changed from yellow to reddish brown. The mixture was stirred at 0°C for 1 hour, and then cooled to -78°C and added with 50 mL of (2*S*,4*S*)-tert-butyl 4-fluoro-2-formylpyrrolidine-1-carboxylate **44a** (21.27 g, 98 mmol, prepared according to the known method of "Tetrahedron: Asymmetry, 2014, 25(3), 212-218") in dichloromethane, and stirred for another 1.5 hours at -78°C. The completion of the reaction was monitored by TLC. The reaction solution was added with 200 mL of sodium bicarbonate solution (5%) and the aqueous phase was extracted with dichloromethane (300 mL × 2). The organic phases were combined, washed successively with water (200 mL) and saturated sodium chloride solution (200 mL), dried over anhydrous magnesium sulfate, filtered, and the filtrate was concentrated under reduced pressure. The residues were purified by silica gel column chromatography using eluent system B to give the title product of (2*S*,4*S*)-tert-butyl 2-((1*R*,2*R*)-3-((*R*)-4-benzyl-2-oxooxazolidin-3-yl)-1-hydroxy-2-methyl-3-oxopropyl)-4-fluoropyrrolidine-1-carboxylate **44c** (19 g, light yellow solid), yield 43.2%.
MS m/z (ESI): 351.06 [M-100+1]

Step 2

(2*R*,3*R*)-3-((2*S*,4*S*)-1-(tert-butoxycarbonyl)-4-fluoropyrrolidin-2-yl)-3-hydroxy-2-methy lpropanoic acid

(2*S*,4*S*)-tert-butyl 2-((1*R*,2*R*)-3-((*R*)-4-benzyl-2-oxooxazolidin-3-yl)-1-hydroxy-2-methyl-3-oxopropyl)-4-fluoropyrrolidine-1-carboxylate **44c** (19 g, 42 mmol) was dissolved in 200 mL of tetrahydrofuran and 50 mL of water, and cooled to 0°C under argon atmosphere. 30% hydrogen peroxide (17.2 mL, 147 mmol) was slowly added dropwise, and 80 mL of lithium hydroxide monohydrate (2.86 g, 68 mmol) was added. The reaction mixture was stirred at 0 °C for 5 hours. A solution of 100 mL of sodium sulfite (21.2 g, 168 mmol) was added to the reaction solution and further stirred

at 25 °C for 16 hours. After completion of the reaction, the organic phase was concentrated under reduced pressure and the residues were washed with dichloromethane (200 mL × 3). The aqueous phase was adjusted to pH 3 with 1N hydrochloric acid and extracted with ethyl acetate (200 mL × 4). The organic phases were combined and concentrated under reduced pressure. The residues were dissolved in 350 mL of sodium bicarbonate solution (5%), washed with dichloromethane (200 mL × 2). The aqueous phase was adjusted to pH 3 with 2N hydrochloric acid, extracted with ethyl acetate (200 mL × 5), and the organic phase was combined, dried over anhydrous sodium sulfate, filtered, and the filtrate was concentrated under reduced pressure. to give the crude title product of (2*R*,3*R*)-3-((2*S*,4*S*)-1-(tert-butoxycarbonyl)-4-fluoropyrrolidin-2-yl)-3-hydroxy-2-methy lpropanoic acid **44d** (11.6 g, light yellow viscous liquid). The product was used in the next step without further purification.
MS m/z (ESI): 191.47 [M-100+1]

Step 3

(2*R*,3*R*)-3-((2*S*,4*S*)-1-(tert-butoxycarbonyl)-4-fluoropyrrolidin-2-yl)-3-methoxy-2-meth ylpropanoic acid

(2*R*,3*R*)-3-((2*S*,4*S*)-1-(tert-butoxycarbonyl)-4-fluoropyrrolidin-2-yl)-3-hydroxy-2-methy lpropanoic acid **44d** (11.6 g, 39.8 mmol) was dissolved in 200 mL of tetrahydrofuran and added with methyl iodide (91 g, 640 mmol) under nitrogen atmosphere at 0 °C. Then sodium hydride (7.32 g (60%), 183 mmol) was added in portions, and stirred at 0 °C for 48 hours. The reaction mixture was then quenched by the addition of 500 mL of ice water and then extracted with ethyl acetate (150 mL). The aqueous phase was washed with ethylether (150 mL x 2), adjusted to pH=3 with 2N hydrochloric acid and extracted with ethyl acetate (250 mL x 3). The organic phase was combined, dried over anhydrous sodium sulfate, filtered, and the filtrate was concentrated under reduced pressure. The residues were purified by silica gel column chromatography using eluent system B to give the title product of (2*R*,3*R*)-3-((2*S*,4*S*)-1-(tert-butoxycarbonyl)-4-fluoropyrrolidin-2-yl)-3-methoxy-2-meth ylpropanoic acid **44e** (8.0 g, light yellow viscous liquid), yield 65.6%.
MS m/z (ESI): 205.68 [M-100+1]

Step4

(2*S*,4*S*)-tert-butyl 4-fluoro-2-((1*R*,2*R*)-1-methoxy-3-(((*S*)-1-methoxy-1-oxo-3-phenylpropan-2-yl)amino)-2-methyl-3-oxopropyl)pyrrolidine-1-carboxylate

(2*R*,3*R*)-3-((2*S*,4*S*)-1-(tert-butoxycarbonyl)-4-fluoropyrrolidin-2-yl)-3-methoxy-2-meth ylpropanoic acid **44e** (580 mg, 1.9 mmol) was dissolved in 15 mL of acetonitrile, and added with (*S*)-methyl 2-amino-3-phenylpropanoate hydrochloride **44f** (480 mg, 2.2 mmol, prepared according to the known method of "Journal of Heterocyclic Chemistry, 2013, 50(2), 320-325"), *N, N*-diisopropylethylamine (1.42 mL, 8 mmol) and 2-(7-azobenzotriazole) -*N, N, N'*, *N'*-tetramethyluronium hexafluorophosphate (1.07 g, 2.8 mmol). The reaction mixture was stirred at room temperature for 12 hours. When the reaction was complete by TLC, the reaction mixture was diluted with 40 mL of ethyl acetate, washed succesively with saturated ammonium chloride solution (20 mL) and saturated sodium chloride solution (20 mL), dried over anhydrous sodium sulfate, filtered, and the filtrate was concentrated under reduced pressure. The residues were purified by silica gel column chromatography using eluent system B to give the title product of (2*S*,4*S*)-tert-butyl 4-fluoro-2-((1*R*,2*R*)-1-methoxy-3-(((*S*)-1-methoxy-1-oxo-3-phenylpropan-2-yl)amino)-2-methyl-3-oxopropyl)pyrrolidine-1-carboxylate **44g** (750 mg, white solid), yield83.2%.
MS m/z (ESI): 222.62 [M-100+1]

Step5

(*S*)-methyl 2-((2*R*,3*R*)-3-((2*S*,4*S*)-4-fluoropyrrolidin-2-yl)-3-methoxy-2-methylpropanamido)-3-phe nylpropanoate trifluoroacetate

(2*S*,4*S*)-tert-butyl 4-fluoro-2-((1*R*,2*R*)-1-methoxy-3-(((*S*)-1-methoxy-1-oxo-3-phenylpropan-2-yl)amino)-2-methyl-3-oxopropyl)pyrrolidine-1-carboxylate **44g** (738 mg, 1.58 mmol) was dissolved in 15 mL of dichloromethane, and added dropwise with trifluoroacetic acid (3 mL, 30 mmol), then stirred at room temperature for 12 hours. When the reaction was complete by TLC, the reaction solution was concentrated under reduced pressure to give the crude title product of (*S*)-methyl 2-((2*R*,3*R*)-3-((2*S*,4*S*)-4-fluoropyrrolidin-2-yl)-3-methoxy-2-methylpropanamido)-3-phe nylpropanoate trifluoroacetate **44h** (1.24 g, yellow viscous liquid). The product was used in the next step without further purification.
MS m/z (ESI): 236.39 [M+1]

Step 6

(*S*)-methyl 2-((2*R*,3*R*)-3-((2*S*,4*S*)-1-((5*S*,8*S*,11*S*,12*R*)-11-((*S*)-sec-butyl)-5,8-diisopropyl-12-methox y-4,10-dimethyl-3,6,9-trioxo-1-phenyl-2-oxa-4,7,10-triazatetradecan-14-oyl)-4-fluoropy rrolidin-2-yl)-3-methoxy-2-methylpropanamido)-3-phenylpropanoate

(*S*)-methyl 2-((2*R*,3*R*)-3-((2*S*,4*S*)-4-fluoropyrrolidin-2-yl)-3-methoxy-2-methylpropanamido)-3-phe nylpropanoate trifluoroacetate **44h** (572 mg, 1.56 mmol) and (*5S,*8*S,*11*S,*12*R)*-11-*((S)*-sec-butyl)-5,8-diisopropyl-12-methoxy-4,10-dimethyl-3,6,9-tri oxo-1-phenyl-2-oxa-4,7,10-triazatetradecan-14-oic acid **44i** (868 mg, 1.56 mmol, prepared according to the known method of "WO2007008848") was dissolved in 15 mL of acetonitrile, and added with *N, N*-diisopropylethylamine (2.0 mL, 12 mmol) and 2-(7-azobenzotriazole) -*N, N, N'*, *N'*-tetramethyluronium hexafluorophosphate (890 mg, 2.34 mmol). The reaction mixture was stirred at room temperature for 12 hours. When the reaction was complete by TLC , the reaction mixture was diluted with 50 mL of ethyl acetate, washed successively with saturated ammonium chloride solution (30 mL) and saturated sodium chloride solution (30 mL), dried over anhydrous sodium sulfate, filtered, and the filtrate was concentrated under reduced pressure. The residues were purified by silica gel column chromatography using eluent system B and system A to give the title product of (*S*)-methyl 2-((2*R*,3*R*)-3-((2*S*,4*S*)-1-((5*S*,8*S*,11*S*,12*R*)-11-((*S*)-sec-butyl)-5,8-diisopropyl-12-methox y-4,10-dimethyl-3,6,9-trioxo-1-phenyl-2-oxa-4,7,10-triazatetradecan-14-oyl)-4-fluoropy rrolidin-2-yl)-3-methoxy-2-methylpropanamido)-3-phenylpropanoate **44j** (1.4 g, white foam solid), yield99.9%.
MS m/z (ESI): 898.99 [M+1]

Step 7

(*S*)-2-((2*R*,3*R*)-3-((2*S*,4*S*)-1-((5*S*,8*S*,11*S*,12*R*)-11-((*S*)-sec-butyl)-5,8-diisopropyl-12-met hoxy-4,10-dimethyl-3,6,9-trioxo-1-phenyl-2-oxa-4,7,10-triazatetradecan-14-oyl)-4-fluor opyrrolidin-2-yl)-3-methoxy-2-methylpropanamido)-3-phenylpropanoic acid

(*S*)-methyl 2-((2*R*,3*R*)-3-((2*S*,4*S*)-1-((5*S*,8*S*,11*S*,12*R*)-11-((*S*)-sec-butyl)-5,8-diisopropyl-12-methox y-4,10-dimethyl-3,6,9-trioxo-1-phenyl-2-oxa-4,7,10-triazatetradecan-14-oyl)-4-fluoropy rrolidin-2-yl)-3-methoxy-2-methylpropanamido)-3-phenylpropanoate **44j** (180 mg, 0.2 mmol) was dissolved in 0.5 mL of methanol, 0.5 mL of tetrahydrofuran and 0.5 mL of water, and then cooled to 0 °C, and added with lithium hydroxide monohydrate (34 mg, 0.8 mmol). The mixture was stirred at 23 °C for 2 hours. When the reaction was complete by TLC and MS monitor, the reaction mixture was diluted with 2 mL of water, added with 1N hydrochloric acid to adjust the pH about 4.5, and extracted with ethyl acetate (5 mL × 3). The combined organic phases were dried over anhydrous magnesium sulfate, filtered and the filtrate was concentrated under reduced pressure to give the crude title product of (*S*)-2-((2*R*,3*R*)-3-((2*S*,4*S*)-1-((5*S*,8*S*,11*S*,12*R*)-11-((*S*)-sec-butyl)-5,8-diisopropyl-12-met hoxy-4,10-dimethyl-3,6,9-trioxo-1-phenyl-2-oxa-4,7,10-triazatetradecan-14-oyl)-4-fluor opyrrolidin-2-yl)-3-methoxy-2-methylpropanamido)-3-phenylpropanoic acid **44k** (160 mg, light yellow foamy solid). The product was used in the next step without further purification.
MS m/z (ESI): 883.73 [M-1].

Step8

(*S*)-2-((2*R*,3*R*)-3-((2*S*,4*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-4-fluoropyrrolidin-2-yl )-3-methoxy-2-methylpropanamido)-3-phenylpropanoic acid

The crude product of (*S*)-2-((2*R*,3*R*)-3-((2*S*,4*S*)-1-((5*S*,8*S*,11*S*,12*R*)-11-((*S*)-sec-butyl)-5,8-diisopropyl-12-met hoxy-4,10-dimethyl-3,6,9-trioxo-1-phenyl-2-oxa-4,7,10-triazatetradecan-14-oyl)-4-fluor opyrrolidin-2-yl)-3-methoxy-2-methylpropanamido)-3-phenylpropanoic acid **44k** (160 mg, 0.2 mmol) was dissolved in 10 mL of anhydrous ethanol and added with 30 mg of palladium on carbon (10%). The reaction system was PURGED with hydrogen three times and stirred at 23 °C for 15 hours. When the reaction was complete by TLC, the reaction mixture was filtered through celite to remove palladium on carbon, and the filtrate was concentrated under reduced pressure to give 120 mg of the residues. The residues were purified by high performance liquid chromatography to give the title product of (*S*)-2-((2*R*,3*R*)-3-((2*S*,4*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-4-fluoropyrrolidin-2-yl )-3-methoxy-2-methylpropanamido)-3-phenylpropanoic acid **44** (29 mg, white solid), yield 21.5%.
MS m/z (ESI): 749.94 [M+1].
$^1$H NMR (400 MHz, $CD_3OD$) δ 7.29-7.14(m, 5H), 4.81-4.71(m, 2H), 4.18-4.13(m, 2H), 4.05-4.03(m, 2H), 3.84-3.81(m, 1H), 3.63-3.60(m, 1H), 3.58-3.52(m, 1H), 3.48-3.13(m, 14H), 3.00-2.91(m, 1H), 2.66-1.81(m, 11H), 1.63-1.38(m, 2H),

1.21-1.10(m, 5H), 1.09-0.94(m, 14H), 0.91-0.82(m, 3H).

Example 45

(*S*)-2-((2*R*,3*R*)-3-((2*S*,4*S*)-1-((3*R*,4*S*,5*S*)-4-((*S*)-2-((*S*)-2-(6-(2,5-dioxo-2,5-dihydro-1*H*-p yrrol-1-yl)-*N*-methylhexanamido)-3-methylbutanamido)-*N*,3-dimethylbutanamido)-3-m ethoxy-5-methylheptanoyl)-4-fluoropyrrolidin-2-yl)-3-methoxy-2-methylpropanamido)-3-phenylpropanoic acid

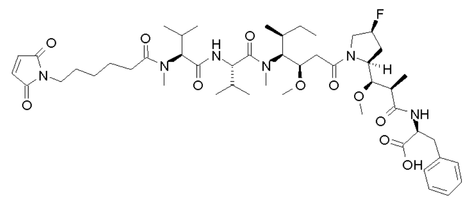

45

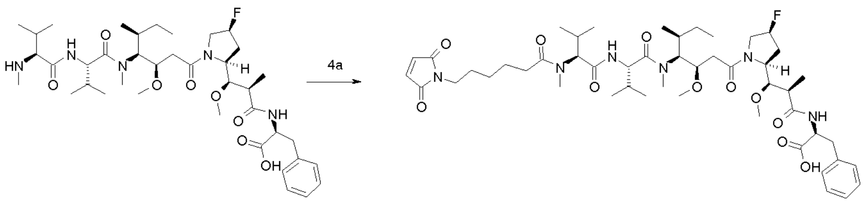


44

45

(*S*)-2-((2*R*,3*R*)-3-((2*S*,4*S*)-1-((3*R*,4*S*,5*S*)-4-((*S*)-*N*,3-dimethyl-2-((*S*)-3-methyl-2-(methyl amino)butanamido)butanamido)-3-methoxy-5-methylheptanoyl)-4-fluoropyrrolidin-2-yl )-3-methoxy-2-methylpropanamido)-3-phenylpropanoic acid **44** (1.0 g, 1.34 mmol) was dissolved in 20 mL of acetonitrile, and added with *N, N*-diisopropylethylamine (0.53 mL, 3.0 mmol) and 2- (7-azobenzotriazole) -*N, N, N* ', *N*'-tetramethyluronium hexafluorophosphate (560 mg, 1.48 mmol). The reaction mixture was stirred at 23 °C for 30 minutes and added with 6-(2,5-dioxo-2,5-dihydro-1*H*-pyrrol-1-yl)hexanoic acid **4a** (300 mg, 1.41 mmol). When the reaction was complete by TLC, the reaction mixture was added with 50 mL of ethyl acetate and concentrated under reduced pressure. The residues were purified by flash column chromatography eluting with eluent systems B and A to give the crude title product of 1.0 g, which was further purified by high performance liquid chromatography to give the title product of (*S*)-2-((2*R*,3*R*)-3-((2*S*,4*S*)-1-((3*R*,4*S*,5*S*)-4-((*S*)-2-((*S*)-2-(6-(2,5-dioxo-2,5-dihydro-1*H*-p yrrol-l-yl)-*N*-methylhexanamido)-3-methylbutanamido)-*N*,3-dimethylbutanamido)-3-m ethoxy-5-methylheptanoyl)-4-fluoropyrrolidin-2-yl)-3-methoxy-2-methylpropanamido)-3-phenylpropanoic acid **45** (140 mg, white solid), yield 11%.
MS m/z (ESI): 942.67 [M+1]
$^1$HNMR (400 MHz, $CD_3OD$) δ 7.34-7.14(m, 5H), 6.83-6.80(m, 2H), 5.20-5.10(m, 1H), 5.01-4.96(m, 1H), 4.78-4.57(m, 2H), 4.18-4.01(m, 2H), 4.00-3.80(m, 1H), 3.56-3.41(m, 5H), 3.35-3.24(m, 10H), 3.19-2.90(m, 4H), 2.60-2.40(m, 4H), 2.39-2.00(m, 4H), 1.93-1.58(m, 6H), 1.50-1.10(m, 7H), 1.09-0.82(m, 21H).

Example 46

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3 -(4-fluorophenyl)propanoic acid

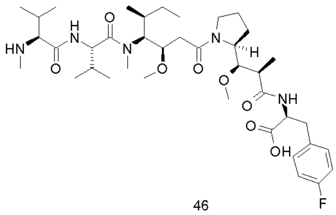

46

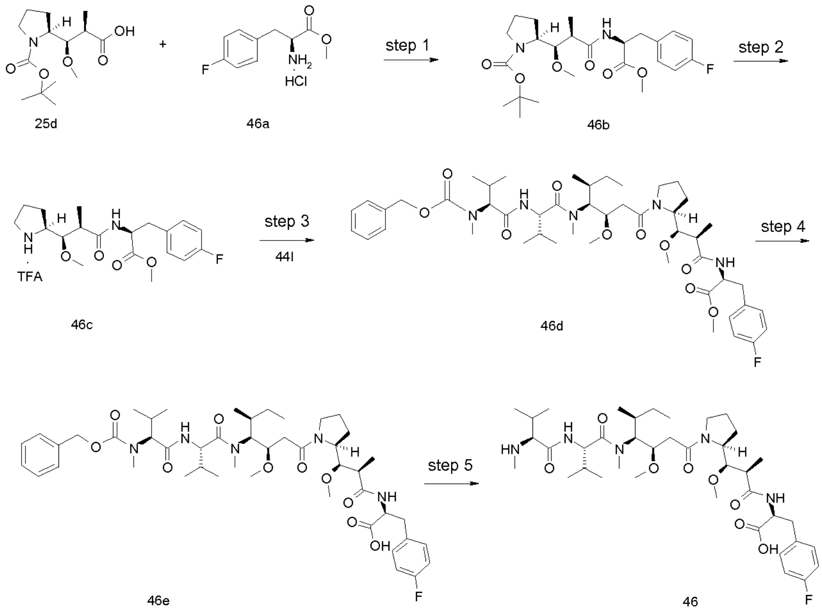


25d 46a 46b

46c 46d

46e 46

Step 1

(*S*)-tert-butyl 2-((1*R*,2*R*)-3-(((*S*)-3-(4-fluorophenyl)-1-methoxy-1-oxopropan-2-yl)amino)-1-methoxy-2-methyl-3-oxopropyl)pyrrolidine-1-carboxylate

(2*R*,3*R*)-3-((*S*)-1-(tert-butoxycarbonyl)pyrrolidin-2-yl)-3-methoxy-2-methylpropanoic acid **25d** (1.0 g, 3.5 mmol) was dissolved in 15 mL of acetonitrile, and added with (*S*)-methyl 2-amino-3-(4-fluorophenyl)propanoate hydrochloride **46a** (820 mg, 4 mmol, prepared according to the known method of "Tetrahedron, 2003, 59(21), 3719-3727"), and *N, N*-diisopropylethylamine (2.7 mL, 15 mmol) and 2- (7-azobenzotriazole) *-N, N, N'*, *N'*-tetramethyluronium hexafluorophosphate (1.9 g, 5.0 mmol). The reaction mixture was stirred at 23 °C for 12 hours. When the reaction was complete by TLC, the reaction mixture was diluted with 50 mL of ethyl acetate and washed successively with saturated ammonium chloride solution (30 mL) and saturated sodium chloride solution (30 mL), dried over anhydrous sodium sulfate, filtered, and the filtrate was concentrated under reduced pressure. The residues were purified by silica gel column chromatography using eluent system B to give the title product of (*S*)-tert-butyl 2-((1*R*,2*R*)-3-(((*S*)-3-(4-fluorophenyl)-1-methoxy-1-oxopropan-2-yl)amino)-1-methoxy-2-methyl-3-oxopropyl)pyrrolidine-1-carboxylate **46b** (1.35 g, white solid), yield 82.6%.
MS m/z (ESI): 366.46 [M-100-1].

Step2

(*S*)-methyl 3-(4-fluorophenyl)-2-((2*R*,3*R*)-3-methoxy-2-methyl-3-((*S*)-pyrrolidin-2-yl)propanamido )propanoate trifluroacetate

(*S*)-tert-butyl 2-((1*R*,2*R*)-3-(((*S*)-3-(4-fluorophenyl)-1-methoxy-1-oxopropan-2-yl)amino)-1-methoxy-2-methyl-3-oxopropyl)pyrrolidine-1-carboxylate **46b** (1.34 g, 2.87 mmol) was dissolved in 20 mL of dichloromethane and added dropwise with trifluoroacetic acid (3 mL, 30 mmol), and stirred at 23 °C for 12 hours. When the reaction was complete by TLC, the reaction solution was concentrated under reduced pressure to give the crude title product of (*S*)-methyl 3-(4-fluorophenyl)-2-((2*R*,3*R*)-3-methoxy-2-methyl-3-((*S*)-pyrrolidin-2-yl)propanamido )propanoate trifluroacetate **46c** (1.92 g, yellow viscous liquid). The product was used in the next step without further purification.
MS m/z (ESI): 366.85 [M+1]

Step3

(*S*)-methyl 2-((2*R*,3*R*)-3-((*S*)-1-((5*S*,8*S*,11*S*,12*R*)-11-((*S*)-sec-butyl)-5,8-diisopropyl-12-methoxy-4,10-dimethyl-3,6,9-trioxo-1-phenyl-2-oxa-4,7,10-triazatetradecan-14-oyl)pyrrolidin-2-yl)-3-methoxy-2-methylpropanamido)-3-(4-fluorophenyl)propanoate

(*S*)-methyl 3-(4-fluorophenyl)-2-((2*R*,3*R*)-3-methoxy-2-methyl-3-((*S*)-pyrrolidin-2-yl)propanamido)propanoate trifluroacetate **46c** (367 mg, 1.0 mmol) and (5*S*,8*S*,11*S*,12*R*)-11-((*S*)-sec-butyl)-5,8-diisopropyl-12-methoxy-4,10-dimethyl-3,6,9-tri oxo-1-phenyl-2-oxa-4,7,10-triazatetradecan-14-oic acid **44i** (549 mg, 1.0 mmol) was dissolved in 10 mL of acetonitrile, and added with *N, N*-diisopropylethylamine (1.24 mL, 7 mmol) and 2- (7-azobenzotriazole) -*N, N, N* ', *N*'-tetramethyluronium hexafluorophosphate (570 mg, 1.5 mmol). The reaction mixture was stirred at 23 °C for 12 hours. When the reaction was complete by TLC, the reaction mixture was diluted with 30 mL of ethyl acetate and washed successively with saturated ammonium chloride solution (20 mL) and saturated sodium chloride solution (20 mL), dried over anhydrous sodium sulfate, filtered, and the filtrate was concentrated under reduced pressure. The residues were purified by silica gel column chromatography eluting with eluent systems B and A to give the title product of (*S*)-methyl 2-((2*R*,3*R*)-3-((*S*)-1-((5*S*,8*S*,11*S*,12*R*)-11-((*S*)-sec-butyl)-5,8-diisopropyl-12-methoxy-4,10-dimethyl-3,6,9-trioxo-1-phenyl-2-oxa-4,7,10-triazatetradecan-14-oyl)pyrrolidin-2-yl )-3-methoxy-2-methylpropanamido)-3-(4-fluorophenyl)propanoate **46d** (760 mg, white foam solid), yield84.6%.
MS m/z (ESI): 898.10 [M+1]

Step 4

(*S*)-2-((2*R*,3*R*)-3-((*S*)-1-((5*S*,8*S*,11*S*,12*R*)-11-((*S*)-sec-butyl)-5,8-diisopropyl-12-methox y-4,10-dimethyl-3,6,9-trioxo-1-phenyl-2-oxa-4,7,10-triazatetradecan-14-oyl)pyrrolidin-2-yl)-3-methoxy-2-methylpropanamido)-3-(4-fluorophenyl)propanoic acid

(*S*)-methyl 2-((2*R*,3*R*)-3-((*S*)-1-((5*S*,8*S*,11*S*,12*R*)-11-((*S*)-sec-butyl)-5,8-diisopropyl-12-methoxy-4, 10-dimethyl-3,6,9-trioxo-1-phenyl-2-oxa-4,7,10-triazatetradecan-14-oyl)pyrrolidin-2-yl )-3-methoxy-2-methylpropanamido)-3-(4-fluorophenyl)propanoate **46d** (180 mg, 0.2 mmol) was dissolved in 0.5 mL of methanol, 0.5 mL of tetrahydrofuran and 0.5 mL of water, and cooled to 0 °C, and then added with lithium hydroxide monohydrate (34 mg, 0.8 mmol) and stirred at 23 °C for 2 hours. When the reaction was complete by TLC and Ms monitor, the reaction mixture was diluted with 2 mL of water, and added with 1*N* hydrochloric acid to adjust the pH to about 4.5, and extracted with ethyl acetate (5 mL × 3). The combined organic phases were dried over anhydrous magnesium sulfate, filtered and the filtrate was concentrated under reduced pressure to give the crude title product of (*S*)-2-((2*R*,3*R*)-3-((*S*)-1-((5*S*,8*S*,11*S*,12*R*)-11-((*S*)-sec-butyl)-5,8-diisopropyl-12-methox y-4,10-dimethyl-3,6,9-trioxo-1-phenyl-2-oxa-4,7,10-triazatetradecan-14-oyl)pyrrolidin-2-yl)-3-methoxy-2-methylpropanamido)-3-(4-fluorophenyl)propanoic acid **46e** (160 mg, white solid). The product was used in the next step without further purification.
MS m/z (ESI): 883.78 [M-1]

Step 5

(*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3 -(4-fluorophenyl)propanoic acid

The crude product of (*S*)-2-((2*R*,3*R*)-3-((*S*)-1-((5*S*,8*S*,11*S*,12*R*)-11-((*S*)-sec-butyl)-5,8-diisopropyl-12-methox y-4,10-dimethyl-3,6,9-trioxo-1-phenyl-2-oxa-4,7,10-triazatetradecan-14-oyl)pyrrolidin-2-yl)-3-methoxy-2-methylpropanamido)-3-(4-fluorophenyl)propanoic acid **46e** (160 mg, 0.2 mmol) was dissolved in 10 mL of anhydrous ethanol and added with 30 mg of palladium on carbon (10%). The reaction mixture was replaced with hydrogen three times and stirred at room temperature for 15 hours. When the reaction was complete by TLC, the reaction mixture was filtered through celite to remove palladium on carbon, and the filtrate was concentrated under reduced pressure to give 130 mg of the residues. The residues were purified by high performance liquid chromatography to give the title product of (*S*)-2-((2*R*,3*R*)-3-((*S*)-1-((3*R*,4*S*,5*S*)-4-((*S*)-*N*,3-dimethyl-2-((*S*)-3-methyl-2-(methylamino)butanamido)butanamido)-3-methoxy-5-methylheptanoyl)pyrrolidin-2-yl)-3-methoxy-2-methylpropanamido)-3-(4-fluorophenyl)propanoic acid **46** (34 mg, white solid), yield 25.2%.
MS m/z (ESI): 749.94 [M+1]
$^1$H NMR (400 MHz, $CD_3OD$) δ 7.31-7.24(m, 2H), 7.02-6.92(m, 2H), 4.48-4.58(m, 2H), 4.22-4.11(m, 1H), 3.85-3.40(m, 4H), 3.39-3.04(m, 14H), 3.00-2.90(m, 1H), 2.66-2.40(m, 5H), 2.39-2.02(m, 4H), 1.99-1.75(m, 3H), 1.73-1.24(m, 3H), 1.20-1.10(m, 4H), 1.09-0.95(m, 16H), 0.94-0.80(m, 3H).

Example 47

(*S*)-2-((2*R*,3*R*)-3-((*S*)-1-((3*R,*4*S*,5*S*)-4-((*S*)-2-((*S*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(4-fluorop henyl)propanoic acid

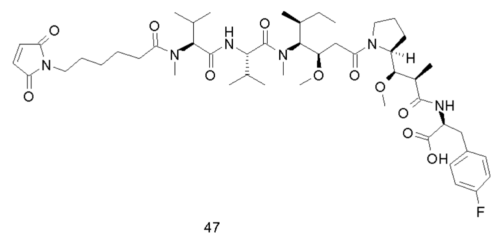

47

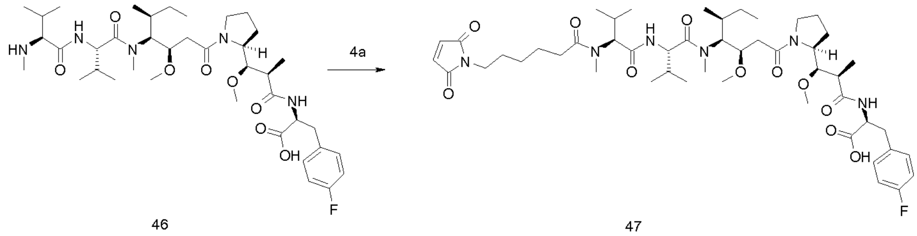


46 47

6-(2,5-dioxo-2,5-dihydro-1*H*-pyrrol-1-yl)hexanoic acid **4a** (434 mg, 2.06 mmol) was dissolved in 20 mL of acetonitrile, and added with *N*,*N*-diisopropylethylamine (844 mL, 6.55 mmol) and 2- (7-azobenzotriazole) -*N, N, N* ', *N*'-tetramethyluronium hexafluorophosphate (781 mg, 1.87 mmol). The reaction mixture was stirred at 23 °C for 30 minutes and added with (*S*)-2-((2*R*,3*R*)-3-((*S*)-1-((3*R*,4*S*,5*S*)-4-((*S*)-*N*,3-dimethyl-2-((*S*)-3-methyl-2-(methylamin o)butanamido)butanamido)-3-methoxy-5-methylheptanoyl)pyrrolidin-2-yl)-3-methoxy-2-methylpropanamido)-3-(4-fluorophenyl)propanoic acid **46** (1.4 g, 1.87 mmol). When the reaction was complete by TLC, the reaction mixture was diluted with 50 mL of ethyl acetate, and concentrated under reduced pressure. The residues were purified by silica gel column chromatography eluting with eluent systems B and A to give the crude title product of 1.2 g, which was further purified by high performance liquid chromatography to give the title product of (*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(4-fluorop henyl)propanoic acid **47** (670 mg, white solid), yield 37.9%.
MS m/z (ESI): 944.54 [M+1]
$^1$H NMR (400 MHz, $CD_3OD$) δ 12.80(br.s, 1H), 8.56-8.52(m, 1H), 8.42-8.20(m, 1H), 8.18-8.10(m, 1H), 7.30-7.18(m, 2H), 7.07-6.75(m, 4H), 4.64-4.34(m, 3H), 4.08-3.94(m, 1H), 3.80-3.50(m, 2H), 3.46-2.70(m, 15H), 2.50-1.80(m, 6H), 1.73-1.38(m,7H), 1.30-1.10(m, 12H), 1.09-0.62(m, 20H).

Example48

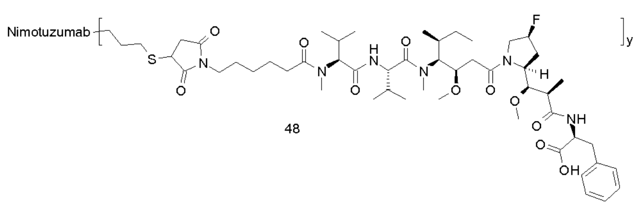


48

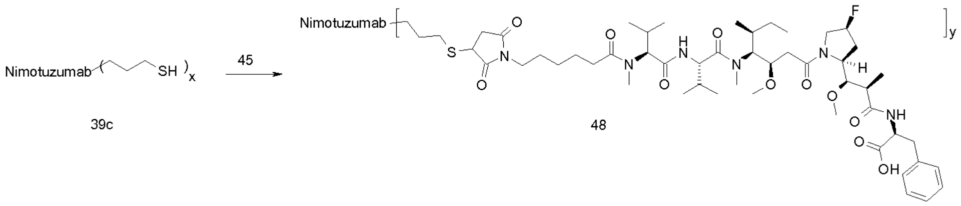


39c 48

(*S*)-2-((2*R*,3*R*)-3-((2*S*,4S)-1-((3*R*,4*S*,5*S*)-4-((*S*)-2-((*S*)-2-(6-(2,5-dioxo-2,5-dihydro-1*H*-pyrrol-1-yl)-*N*-methylhexanamido)-3-methylbutanamido)-*N*,3-dimethylbutanamido)-3-methoxy-5-methylheptanoyl)-4-fluoropyrrolidin-2-yl)-3-methoxy-2-methylpropanamido)-3-phenylpropanoic acid **45** (1.1 mg, 1.16 μmol) was dissolved in 1.1 mL of acetonitrile and added with Nimotuzumab-propanethiol **39c** in PBS buffer (1.63 mg/mL, 11.4 mL). After stirred on a shaker in a water bath at 25 ° C for 4 hours, the reaction mixture was desalted with Sephadex G25 gel column (Elution phase: 0.05 *M* PBS solution at pH 6.5), and filtered under sterile conditions through a 0.2 μm filter to give the title product **48** in PBS buffer (0.75 mg/mL, 19.8 mL), then stored at 4 °C.
Q-TOF LC/MS: characteristic peaks: 150186.5($M_{Ab}$+0D), 151364.1($M_{Ab}$+1D), 152262.3 ($M_{Ab}$+2D), 153435.7($M_{Ab}$+3D), 154499.6($M_{Ab}$+4D), 155427.5($M_{Ab}$+5D).
average value: y=2.0.

Example 49

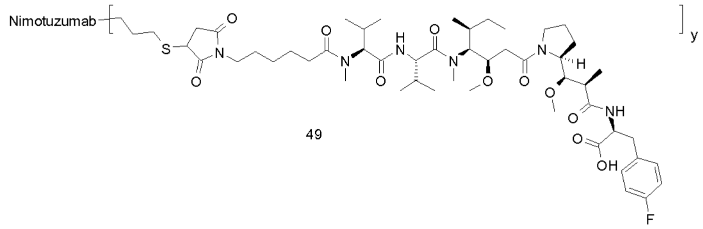

49

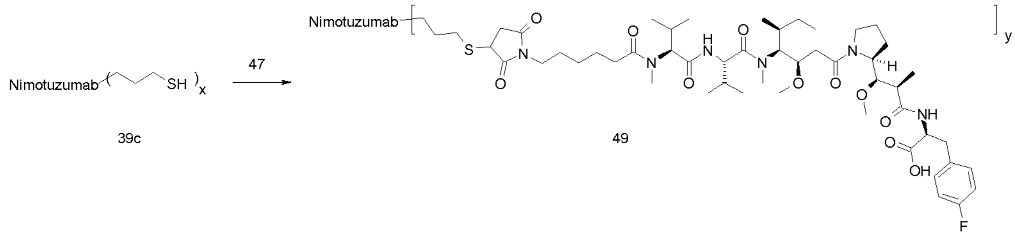


39c 49

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrrol-1-yl)-*N*-methylhexanamido)-3-methylbutanamido)-*N*,3-dimethylbutanamido)-3-methoxy-5-methylheptanoyl)pyrrolidin-2-yl)-3-methoxy-2-methylpropanamido)-3-(4-fluorophenyl)propanoic acid **47** (1.1 mg, 1.16 μmol) was dissolved in 1.1 mL of acetonitrile and added with Nimotuzumab-propanethiol **39c** in PBS buffer (1.63 mg/mL, 11.4 mL). After stirred on a shaker in a water bath at 25 ° C for 4 hours, the reaction mixture was desalted with Sephadex G25 gel column (Elution phase: 0.05 *M* PBS solution at pH 6.5), and filtered under sterile conditions through a 0.2 μm filter to give the title product **49** in PBS buffer (0.75 mg/mL, 19.8 mL), then stored at 4 °C.
Q-TOF LC/MS: characteristic peaks: 150185.5($M_{Ab}$+0D), 151364.7($M_{Ab}$+1D), 152261.2 ($M_{Ab}$+2D), 153436.2($M_{Ab}$+3D), 154499.9($M_{Ab}$+4D), 155428.6($M_{Ab}$+5D)..
average value: y=2.0.

Example 50

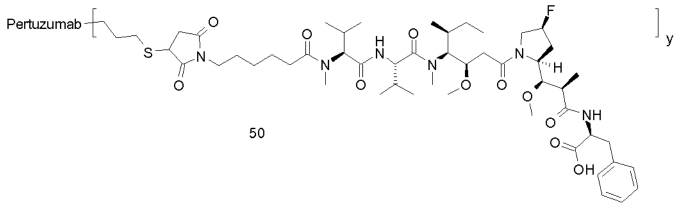

50

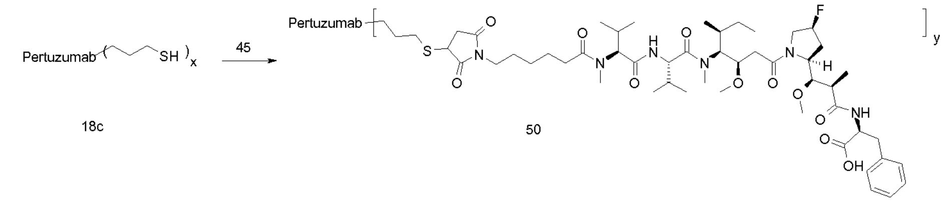


18c

50

(*S*)-2-((2*R*,3*R*)-3-((2*S*,4*S*)-1-((3*R*,4*S*,5*S*)-4-((*S*)-2-((*S*)-2-(6-(2,5-dioxo-2,5-dihydro-1*H*-p yrrol-1-yl)-*N*-methylhexanamido)-3-methylbutanamido)-*N*,3-dimethylbutanamido)-3-m ethoxy-5-methylheptanoyl)-4-fluoropyrrolidin-2-yl)-3-methoxy-2-methylpropanamido)-3-phenylpropanoic acid **45** (1.09 mg, 1.16 μmol) was dissolved in 1.1 mL of acetonitrile and added with Pertuzumab-propanethiol **18c** solution(1.65 mg/mL, 11.3 mL). After stirred on a shaker at 25 ° C for 4 hours, the reaction mixture was desalted with Sephadex G25 gel column (Elution phase: 0.05 *M* PBS solution at pH 6.5), and filtered under sterile conditions through a 0.2 μm filter to give the title product **50** in PBS buffer (0.75 mg/mL, 19.5 mL), then stored at 4 °C.
Q-TOF LC/MS: characteristic peaks: 148095.6($M_{Ab}$+0D), 149111.5($M_{Ab}$+1D), 150165.3 ($M_{Ab}$+2D), 151184.7($M_{Ab}$+3D), 152255.2$M_{Ab}$+4D), 153297.5($M_{Ab}$+5D).
average value: y=2.0.

Example 51

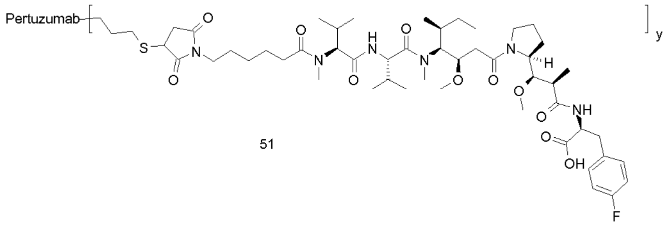

51

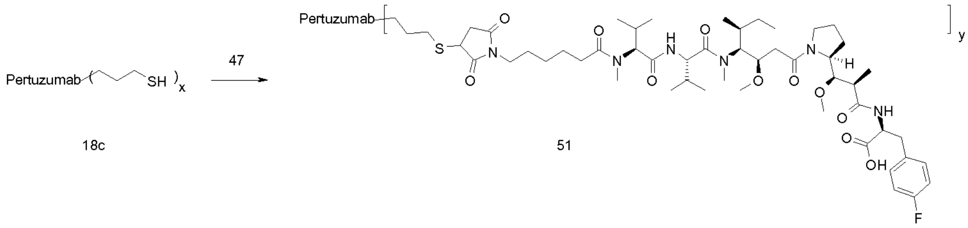


18c

51

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(4-fluorop henyl)propanoic acid **47** (1.09 mg, 1.16 μmol) was dissolved in 1.1 mL of acetonitrile and added with Pertuzumab-propanethiol solution **47c** (1.65 mg/mL, 11.3 mL). After stirred on a shaker at 25 ° C for 4 hours, the reaction mixture was desalted with Sephadex G25 gel column (Elution phase: 0.05 *M* PBS solution at pH 6.5), and filtered under sterile conditions through a 0.2 μm filter to give the title product **51** in PBS buffer (0.75 mg/mL, 19.5 mL), then stored at 4 °C.
Q-TOF LC/MS: characteristic peaks: 148096.2($M_{Ab}$+0D), 149112.2($M_{Ab}$+1D), 150165.4 ($M_{Ab}$+2D), 151184.8($M_{Ab}$+3D), 152255.1($M_{Ab}$+4D), 153297.6($M_{Ab}$+5D).

average value: y=2.0.

Example52

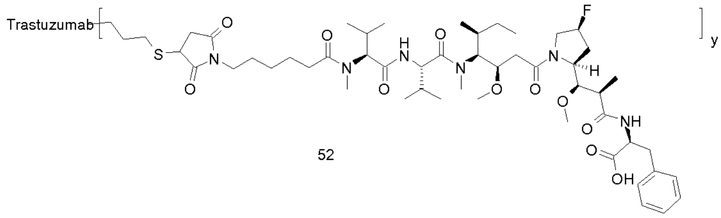

52

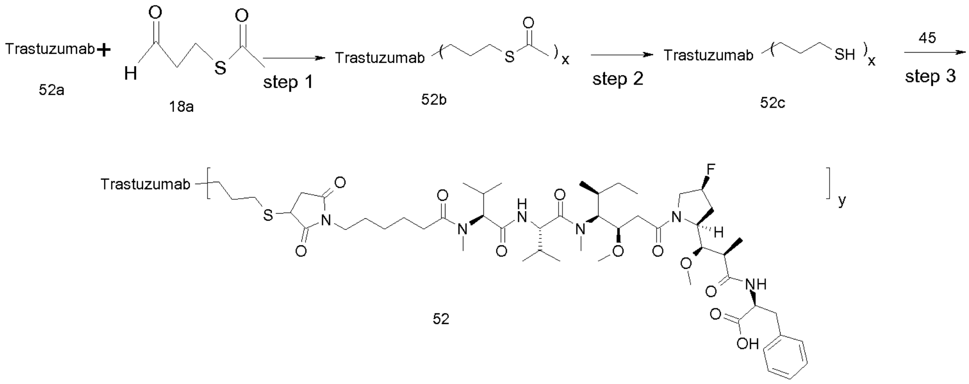


Trastuzumab 52a

18a

52b

52c

52

Step 1

*S*-(3-oxopropyl) ethanethioate **18a** (0.35 mg, 2.65 μmol) was dissolved in 0.45 mL of acetonitrile. The Trastuzumab in acetic acid/sodium acetate buffer (10.0 mg/ml, 4.5 mL, 0.304 μmol) with pH 4.5 was added with the solution of S-(3-oxopropyl) ethanethioate **18a** in acetonitrile solution, and then added with 1.0 mL of an aqueous solution of sodium cyanoborohydride (7.06 mg, 112μmol) dropwise. The reaction mixture was stirred at 25 °C for 2 hours. After completion of the reaction, the reaction mixture was purified by desalting with a Sephadex G25 gel column (elution phase: 0.05 M PBS solution at pH 6.5) to give the title product **52b** solution, which was used directly in the next step.

Step 2

The solution of **52b** (about 15.0 mL) was added with 0.45 mL of a 2.0 *M* solution of hydroxylamine hydrochloride and the reaction mixture was shaked in a shaker at 25 °C for 30 minutes. The reaction solution was desalted with a Sephadex G25 gel column (elution phase: 0.05 M of PBS solution) to give the title product of Trastuzumab-propanethiol **52c** solution (concentration 1.65 mg/ml, 22.6 mL).

Step 3

(*S*)-2-((2*R*,3*R*)-3-((2*S*,4*S*)-1-((3*R*,4*S*,5*S*)-4-((*S*)-2-((*S*)-2-(6-(2,5-dioxo-2,5-dihydro-1*H*-p yrrol-1-yl)-*N*-methylhexanamido)-3-methylbutanamido)-*N*,3-dimethylbutanamido)-3-m ethoxy-5-methylheptanoyl)-4-fluoropyrrolidin-2-yl)-3-methoxy-2-methylpropanamido)-3-phenylpropanoic acid **45** (1.1 mg, 1.2 μmol) was dissolved in 1.1 mL of acetonitrile and added with Trastuzumab-propanethiol solution **52c** (1.65 mg/mL, 11.3 mL). After placed on a shaker at 25 ° C for 4 hours, the reaction mixture was desalted with Sephadex G25 gel column (Elution phase: 0.05 *M* PBS solution at pH 6.5), and filtered under sterile conditions through a 0.2 μm filter to give the title product **52** in PBS buffer (0.72 mg/mL, 20 mL), then stored at 4 °C.
Q-TOF LC/MS: characteristic peaks: 148062.9($M_{Ab}$+0D), 149235.2($M_{Ab}$+1D), 150259.8 ($M_{Ab}$+2D), 151268.2($M_{Ab}$+3D), 152341.9($M_{Ab}$+4D), 153356.4($M_{Ab}$+5D).
average value: y=2.0.

Example 53

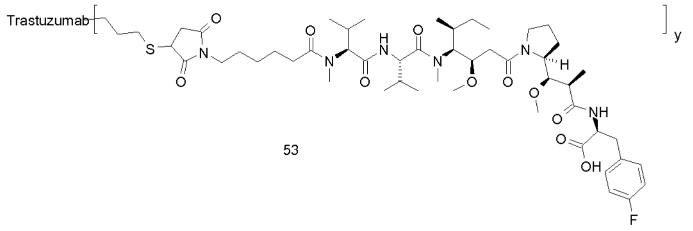

53

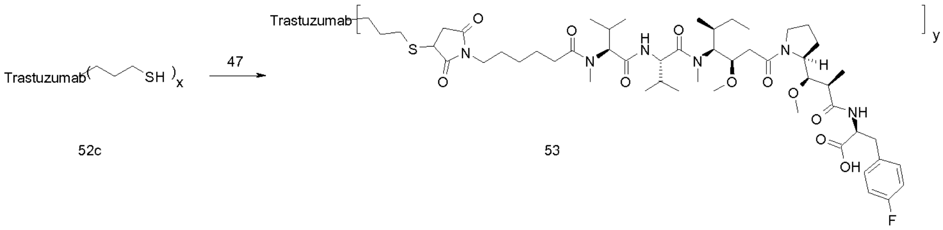


52c

53

(*S*)-2-((2*R*,3*R*)-3-((*S*)-1-((3*R*,4*S*,5*S*)-4-((*S*)-2-((*S*)-2-(6-(2,5-dioxo-2,5-dihydro-1*H*-pyrro 1-1-yl)-*N*-methylhexanamido)-3-methylbutanamido)-*N*,3-dimethylbutanamido)-3-metho xy-5-methylheptanoyl)pyrrolidin-2-yl)-3-methoxy-2-methylpropanamido)-3-(4-fluorop henyl)propanoic acid **47** (1.12 mg, 1.2 μmol) was dissolved in 1.1 mL of acetonitrile and added with Trastuzumab-propanethiol solution **52c** (1.65 mg/mL, 11.3 mL). After placed on a shaker at 25 °C for 4 hours, the reaction mixture was desalted with Sephadex G25 gel column (Elution phase: 0.05 *M* PBS solution at pH 6.5), and filtered under sterile conditions through a 0.2 μm filter to give the title product **53** in PBS buffer (0.70 mg/mL, 20.5 mL), then stored at 4 °C.
Q-TOF LC/MS: characteristic peaks: 148065.2($M_{Ab}$+0D), 149244.6($M_{Ab}$+1D), 150254.9 ($M_{Ab}$+2D), 151280.9($M_{Ab}$+3D), 152301.6($M_{Ab}$+4D), 153457.9($M_{Ab}$+5D).
average value: y=2.0.

**BIOLOGICAL ASSAY**

**Test Example 1: In vitro inhibition test of compound of formula (D) on tumor cell proliferation**

1. Experiment Object

The object of this experiment is to test the in vitro inhibition effect of the formula (D) according to the present invention on the proliferation of HepG2 tumor cells (human hepatocellular carcinoma cells, Chinese Academy of Sciences cell bank, No. #TCHu 72) and A549 tumor cells (human lung adenocarcinoma, Chinese Academy of Sciences cell bank, No. #TCHu150). The cells were treated with different concentrations of the compound in vitro, after 76 hours incubation, the cell proliferation was detected by CCK-8 regent (Cell Counting Kit-8, Dojindo, No. CK04), and the activity of the compound in vitro was evaluated according to the IC50 value.

2. Experimental protocol

The following is an example of a method for in vitro proliferation inhibition test of HepG2 cells for the purpose of exemplifying the test of the compounds according to the present invention for the proliferation inhibitory activity on tumor cells in vitro. This method is also applicable to, but not limited to, the in vitro proliferation inhibition tests on other tumor cells.

2.1 *Cell preparation*

HepG2 cells in logarithmic growth phase were washed with PBS (phosphate buffer, ThermoFisher) once, and added with 2-3ml trypsin (0.25% trypsin-EDTA (1x), Gibico, Life Technologies) to digest for 2-3min. 10-15ml of cell culture medium (DMEM/F12 medium, Invitrogen; 10% (v/v) was added after the cells were completely digested. The

digested HepG2 cells were eluted, and centrifuged at 1000rpm for 3min. The supernatant was discarded, and then 10-20ml cell culture medium was added to resuspend the cells to prepare a single cell suspension.

2.2 *Cell plating*

The HepG2 single cell suspension was mixed and the cell density was adjusted to $6\times10^4$ cells/ml with cell culture medium. The density-adjusted cell suspension was mixed uniform and added to a 96-well cell culture plate at 100μl/well. The plates were incubated in a 5% $CO_2$ incubator at 37 °C for 18-20 hours.

2.3 *Compound preparation*

The compound was dissolved with DMSO (dimethylsulfoxide, Shanghai Titan Technology Co., Ltd.) to prepare a storage solution having an initial concentration of 10 mM.

10μl of 10mM compound was added to the wells of the first column of U-shaped bottom 96-well plate (sample plate 1), 90μl of DMSO was added to each compound sample well of the first column, i.e., the stock solution was diluted 10 times as the starting point. Followed by a 3-fold gradient dilution were performed, with each compound concentration diluting 10 times. The diluent was DMSO solution. 60μl of 100% DMSO was added to $12^{th}$ column and 20μl of 1mM positive drug control was added to $11^{th}$ column.

Take a new U-shaped bottom 96-well plate (sample plate 2) and each sample in the sample plate 1 was diluted 20 times with the complete medium. Then, take a new U-shaped bottom 96-well plate (sample plate 3) and each well sample in the sample plate 2 was subjected to a final 10-fold dilution.

2.4 *Sample loading*

After the cell pre-culture was completed, the 96-well cell culture plate was removed and the supernatant was discarded. Each sample dilution in the 96-well sample plate 3 was successively added to a 96-well cell culture plate at 100μl/well. Each compound sample was test in duplication at each concentration. The loading operation should not exceed 30 min. After completion of the loading operation, the 96-well cell culture plate was incubated at 37 °C for about 76 hours in a 5% $CO_2$ incubator.

2.5 *Coloring operation*

The 96-well cell culture plate was taken and CCK-8 was added to each well at 100μl/well. The cell culture plate was gently pat and mixed for more than 10 times and incubated at 37 °C for 2 hours in a 5% $CO_2$ incubator.

2.6 *Reading plate*

The 96-well cell culture plate was taken and placed in a microplate reader (PerkinElmer, VICTOR 3) and the absorbance at 450nm was measured using a microplate reader.

In accordance with the above procedure, the inhibition activity of the compound according to the present invention on the proliferation of A549 tumor cells in vitro was tested. The cell culture medium was also DMEM/F12.

3. Data analysis

Data was analyzed with Microsoft Excel and Graphpad Prism 5. The results are shown in Table 1.

Table 1

| Compound No. | $IC_{50}$/nM | |
|---|---|---|
| | HepG2 | A549 |
| 1 | 8.60 | 12.75 |
| 2 | 4.12 | 2.33 |
| 3 | 9.33 | 16.17 |
| 7 | 283 | 776.4 |
| 9 | 55 | 114.4 |

(continued)

| Compound No. | $IC_{50}$/nM | |
|---|---|---|
| | HepG2 | A549 |
| 11 | 6.6 | 27.23 |
| 13 | 11.7 | 30.24 |
| 15 | 47.45 | 75.45 |
| 16 | 13.92 | 52.99 |
| 44 | | 179 |
| 25 | 174.7 | 200.5 |
| 29 | 58.64 | 1.8 |
| 31 | 100.5 | 130.1 |
| 32 | 17.40 | 13.51 |
| 33 | 4.66 | 5.00 |
| 34 | 18.33 | 32.32 |
| 35 | 30.56 | 17.98 |
| 46 | | 106 |

**Test Example 2: In vitro tumor cell proliferation inhibition test of antibody-drug conjugate of the present invention against HER2 target**

The object of this experiment is to test the in *vitro* proliferation inhibition effect of the antibody-drug conjugate against HER2 target according to the present invention on SK-BR-3 tumor cells (human breast cancer cells, ATCC, HTB-30). The cells were treated with different concentrations of the compound in vitro, after 76 hours of incubation, the cell proliferation was detected by CCK-8 regent (Cell Counting Kit-8, Dojindo, No.CK04), and the activity of the compound in vitro was evaluated according to the IC50 value.

According to the test method of test example 1, the test cell was SK-BR-3, and the cell culture medium was McCoy's 5A medium (Gibco, NO. 16600-108) containing 10% FBS. The relevant compounds were tested and the results were shown in Table 2.

Table 2

| Compound No. | $IC_{50}$/nM |
|---|---|
| | SK-BR-3 |
| 18 | 0.026 |
| 19 | 0.147 |
| 20 | 0.466 |
| 21 | 0.031 |
| 22 | 0.28 |
| 23 | 0.201 |
| 24 | 0.174 |
| 36 | 0.052 |
| 37 | 0.215 |
| 38 | 0.101 |
| 50 | 0.1 |
| 51 | 0.373 |

Conclusion: The antibody-drug conjugates against HER2 target according to the present invention have significant proliferation inhibition effect on SK-BR-3 tumor cell.

**Test Example 3: In vitro tumor cell proliferation inhibition test of antibody-drug conjugate of the present invention against EGFR target**

The object of this experiment is to test the in *vitro* proliferation inhibition effect of the antibody-drug conjugate against EGFR target according to the present invention on HCC827 tumor cells (non-small cell lung cancer cells, Chinese Academy of Sciences cell bank, No. #TCHu153). The cells were treated with different concentrations of the compound in vitro, after 76 hours of incubation, the cell proliferation was detected by CCK-8 regent (Cell Counting Kit-8, Dojindo, No.CK04), and the activity of the compound in vitro was evaluated according to the IC50 value.
According to the test method of test example 1, the test cell was HCC827, and the cell culture medium was RPMI1640 medium (Invitrogen) containing 10% FBS (v/v). The relevant compounds were tested and the results were shown in Table 3.

Table 3

| Compound No. | $IC_{50}$/nM |
|---|---|
| | HCC827 |
| 39 | 0.353 |
| 40 | 0.255 |
| 41 | 0.451 |
| 48 | 0.993 |

Conclusion: The antibody-drug conjugates against EGFR target according to the present invention have significant proliferation inhibition effect on HCC827 tumor cell.

**Test Example 4: Tumor inhibition rate test of NCI-N87**

1. Experiment Object

To evaluate and compare the effect of antibody-cytotoxin conjugates according to the present invention on transplanted tumors of NCI-N87 cell (HER2 overexpressing human gastric cancer cells, ATCC, CRL-5822) in nude mice.

2. Test Drugs

Sample: Compound 18, Compound 21, Compound 36.
Positive control: Pertuzumab, Trastuzumab.
Preparation method: all prepared with physiological saline.

3. Test animals

BALB/cA-nude mice, 6-7 weeks, female, purchased from Shanghai Slack Experimental Animal Co., Ltd. Certificate number: SCXK (Shanghai) 2012-0002. Feeding environment: SPF level.

4. Test procedures

Nude mice were inoculated subcutaneously with human gastric cancer NCI-N87 cells, and the animals were randomly divided into groups (D0) after tumor growth to 100-200mm$^3$. Administration dosage and regimen are shown in Table 4. Mice were measured for tumor volume 2-3 times a week, and the body weight was measured and the data was recorder.
The calculation formula of tumor volume (V) is as follows:

$$V = 1/2 \times a \times b^2$$

wherein: a and b represented length and width respectively.

$T/C(\%)=(T-T_0)/(C-C_0)\times 100\%$ wherein T and C represent the tumor volume at the end of the experiment; $T_0$ and $C_0$ represent the tumor volume at the beginning of the experiment.

Table 4. Effects of compounds (18, 21, 36) on transplanted tumor of human gastric carcinoma NCI-N87 in nude mouse

| Croups | Administration | Route | Mean tumor volume ($mm^3$) | | Mean tumor volume ($mm^3$) | | %T/C | %tumor inhibition rate | P Value | Number of animals /group |
|---|---|---|---|---|---|---|---|---|---|---|
| | | | D0 | SEM | D21 | SEM | D21 | D21 | D21 | |
| Solvent | D0,7 | IV | 107.8 | ±3.0 | 742.8 | ±92.6 | - | - | - | 12 |
| Pertuzumab 60mg/kg | D0,7 | IV | 115.5 | ±2.9 | 380.1 | ±76.3 | 42 | 58 | 0.018 | 6 |
| Trastuzumab 7.5mg/kg | D0,7 | IV | 109.7 | ±4.4 | 366.5 | ±90.9 | 40 | 60 | 0.019 | 6 |
| 18 (3mg/kg) | D0,7 | IV | 113.1 | ±2.4 | 87.6 | ±6.7 | -22 | 122 | 0 | 6 |
| 21 (3mg/kg) | D0,7 | IV | 122.1 | ±4.0 | 20 | ±20.0 | -84 | 184 | 0 | 6 |
| 36 (3mg/kg) | D0,7 | IV | 119.1 | ±6.1 | 95.8 | ±22.3 | -20 | 120 | 0 | 6 |

D0: The time of the first administration; Pertuzumab and Trastuzumab: First dose doubling; The P value was compared with the solvent control group; Student's t test was adopted. The number of mice at the beginning of the experiment was as follows: Cotrol group n = 12, Treatment group n=6.

5. Result

The compounds of the present invention can significantly inhibit the growth of HER2 overexpressing NCI-N87 nude mice subcutaneous transplanted tumor, and the tumor-bearing mice are better tolerant to the above drugs.

**Test Example 5. The efficacy test of EGFRV3 antibody and its ADC on HCC827 transplanted tumor in nude mice**

1. Experiment Object

In this test, Nude-nude mice were used as the test animals to evaluate the efficacy of the ADC compound 39 and 40 of the present invention on transplanted tumor of human non-small cell lung cancer HCC827 in nude mouse after multi-dose intraperitoneal injection.

The tumor inhibition effect was observed after multi-dose intraperitoneal injection once of ADC compounds 39 and 40 of the present invention, and the experiment was finished on day 38 after administration. The test results show that the tumor inhibition rate of ADC compound 39 (0.05 mg/mouse) is 62.78%, and the difference is statistically significant compared with the control group ($p<0.05$). The tumor inhibition rate of ADC 40 (0.025 mg/mouse) is 49.20%, which is not significantly different from that of blank control group. The inhibition rate of ADC compound 40 (0.05 mg/mouse) is 86.8%, which is significantly different from that of blank control group ($p<0.01$). The tumor inhibition rate of ADC 40 (0.1 mg/mouse) is 93.41%, which is significantly different from that of blank control group ($p <0.05$).

2. Test drugs and materials

2.1 *Test drugs*

The ADC compounds of the present invention: ADC compound 39, ADC compound 40.

2.2 *Formulation method*

All prepared with physiological saline.

2.3 *Test animals*

Nude-nude mice, SPF, 16-20 g, ♀, purchased from Shanghai Xi Puer Bikai Experimental Animal Co., Ltd. Certificate number: SCXK (Shanghai) 2008-0016.

3. Test protocol

Tumor cell transplantation was performed after the nude mice were adapted to laboratory environment for three days. HCC827 cells were inoculated subcutaneously in the right rib of nude mice ($4\times10^6$ + 50% matrigel/mouse). On day 21 after inoculation, the drug was administered when the tumor grew to 209.41 + 25.93 $mm^3$ (d1). The specific administration dose and method were shown in Table 5.

Mice were measured for tumor volume twice a week, the body weight of the nude mice was weighed and the data were recorded.

Excel statistical software: mean value is calculated as avg; SD is calculated as STDEV; SEM is calculated as STDEV/SQRT; P value between different groups is calculated as TTEST.

Tumor volume (V) is calculated as:

$$\text{Tumor volume (V) is calculated as: } V=1/2\times L_{length}\times L_{short}^2$$

$$\text{Relative volume (RTV)} = V_T/V_0$$

$$\text{Tumor Inhibition Rate (\%)} = (C_{RTV}-T_{RTV})/C_{RTV}\ (\%)$$

wherein $V_0$ and $V_T$ represent the tumor volume at the beginning of the experiment and at the end of the experiment,

respectively. $C_{RTV}$ and $T_{RTV}$ represent the relative tumor volume of blank control group (Blank) and test group at the end of the experiment, respectively.

Table 5. Effect of test antibody on HCC827 xenografts in nude mice

| Groups | Administration | Route | Mean tumor volume ($mm^3$) D0 | SEM | Mean tumor volum ($mm^3$) D38 | SEM | %tumor inbihition rate D38 | PValue (vs blank) | Numbe r of animal /group |
|---|---|---|---|---|---|---|---|---|---|
| Solvent | d1-14/一次 | ip | 210.6 | 23.1 | 1710.5 | 281.7 | - | - | 5 |
| 39 (0.05 mg/kg) | d1-14/一次 | ip | 208.6 | 26.5 | 598.4 | 115.1 | 62.78 | 0.02026 6 | 5 |
| 40 (0.025mg/kg) | d1-14/一次 | ip | 209.6 | 25.5 | 868.8 | 167.4 | 49.20 | 0.05831 3 | 5 |
| 40 (0.05mg/kg) | d1-14/一次 | ip | 209.6 | 28.1 | 273.6 | 129.6 | 86.8 | 0.00307 3 | 5 |
| 40 (0.1mg/kg) | d1-14/一次 | ip | 210.5 | 30.1 | 130.4 | 53.5 | 93.41 | 0.00166 2 | 5 |

4. Result

The experimental results show that the tumor inhibition rate of ADC compound 39(0.5 mg/mouse) is 62.78% at day 38, and the difference was statistically significant (p <0.05) compared with the blank control group. The tumor inhibition rate of ADC compound 40 (0.025 mg/mouse) is 49.20%, which is not significantly different from that of blank control group (P>0.05). The tumor inhibition rate of ADC compound 40 (0.05mg/mouse) and ADC compound 40 (0.1mg/mouse) are 86.8% and 93.41%, respectively, which is significantly different from the blank control group (P<0.01). In addition, the anti-tumor effect of ADC compound 40 in three different dose groups is dose dependent.

ÐòÁÐ±í

<110> ½–ËÕºãÈðÒ½Ò©¹É•ÝÓÐÏÞ¹«Ë¾¡¢ÉÏº£ºãÈðÒ½Ò©ÓÐÏÞ¹«Ë¾

<120> ÅäÌå–Ï¸°û¶¾ÐÔÒ©ÎïÅ¼ÁªÎï¡¢ÆäÖÆ±¸•½•¨¼°ÆäÓ¦ÓÃ

<130> 350015CG

<160> 6

<170> PatentIn version 3.3

<210> 1
<211> 214
<212> PRT
<213> ÈË¹¤ÐòÁÐ

<220>
<223> ÅÁÍ×Öéµ¥¿¹£¨Pertuzumab£©ÇáÁ´

<400> 1

Asp Ile Gln Met Thr Gln Ser Pro Ser Ser Leu Ser Ala Ser Val Gly
1 5 10 15

Asp Arg Val Thr Ile Thr Cys Lys Ala Ser Gln Asp Val Ser Ile Gly
20 25 30

Val Ala Trp Tyr Gln Gln Lys Pro Gly Lys Ala Pro Lys Leu Leu Ile
35 40 45

Tyr Ser Ala Ser Tyr Arg Tyr Thr Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Tyr Ile Tyr Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser

```
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  2
<211>  449
<212>  PRT
<213>  ÈË¹¤ÐòÁÐ

<220>
<223>  ÅÁÍ×Öéµ¥¿¹£¨Pertuzumab£©ÖØÁ´

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160
```

```
Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
```

405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 3
<211> 219
<212> PRT
<213> ÈË¹¤ÐòÁÐ

<220>
<223> ÄáÍ×Öéµ¥¿¹£¨Nimotuzumab£©ÇáÁ´

<400> 3

Asp Ile Gln Met Thr Gln Ser Pro Ser Ser Leu Ser Ala Ser Val Gly
1 5 10 15

Asp Arg Val Thr Ile Thr Cys Arg Ser Ser Gln Asn Ile Val His Ser
20 25 30

Asn Gly Asn Thr Tyr Leu Asp Trp Tyr Gln Gln Thr Pro Gly Lys Ala
35 40 45

Pro Lys Leu Leu Ile Tyr Lys Val Ser Asn Arg Phe Ser Gly Val Pro
50 55 60

Ser Arg Phe Ser Gly Ser Gly Ser Gly Thr Asp Phe Thr Phe Thr Ile
65 70 75 80

Ser Ser Leu Gln Pro Glu Asp Ile Ala Thr Tyr Tyr Cys Phe Gln Tyr
85 90 95

Ser His Val Pro Trp Thr Phe Gly Gln Gly Thr Lys Leu Gln Ile Thr
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 4
<211> 453
<212> PRT
<213> ÈË¹¤ÐòÁÐ

<220>
<223> ÄáÍ×Öéµ¥¿¹£¨Nimotuzumab£©ÖØÁ´

<400> 4

Gln Val Gln Leu Gln Gln Ser Gly Ala Glu Val Lys Lys Pro Gly Ser
1 5 10 15

Ser Val Lys Val Ser Cys Lys Ala Ser Gly Tyr Thr Phe Thr Asn Tyr
20 25 30

Tyr Ile Tyr Trp Val Arg Gln Ala Pro Gly Gln Gly Leu Glu Trp Ile
35 40 45

Gly Gly Ile Asn Pro Thr Ser Gly Gly Ser Asn Phe Asn Glu Lys Phe
50 55 60

Lys Thr Arg Val Thr Ile Thr Ala Asp Glu Ser Ser Thr Thr Ala Tyr
65 70 75 80

Met Glu Leu Ser Ser Leu Arg Ser Glu Asp Thr Ala Phe Tyr Phe Cys
85 90 95

Thr Arg Gln Gly Leu Trp Phe Asp Ser Asp Gly Arg Gly Phe Asp Phe
100 105 110

Trp Gly Gln Gly Thr Thr Val Thr Val Ser Ser Ala Ser Thr Lys Gly
115 120 125

Pro Ser Val Phe Pro Leu Ala Pro Ser Ser Lys Ser Thr Ser Gly Gly
130 135 140

```
Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400
```

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro Gly Lys
450

<210> 5
<211> 214
<212> PRT
<213> ÈË¹¤ÐòÁÐ

<220>
<223> ÇúÍ×Öéµ¥¿¹£¨Trastuzumab£©ÇáÁ´

<400> 5

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

```
Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  6
<211>  450
<212>  PRT
<213>  ÈË¹¤ÐòÁÐ

<220>
<223>  ÇúÍ×Öéµ¥¿¹£¨Trastuzumab£©ÖØÁ´

<400>  6

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125
```

```
Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380
```

```
Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

## Claims

1. A compound of formula (PC-L-Dr) or a pharmaceutically acceptable salt or solvate thereof:

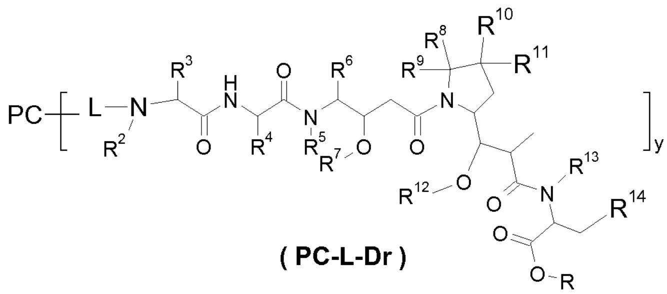

( PC-L-Dr )

wherein:

R, $R^2$-$R^7$ are each selected from the group consisting of hydrogen, halogen, hydroxyl, cyano, alkyl, alkoxy and cycloalkyl;
at least one of $R^8$-$R^{11}$ is selected from the group consisting of halogen, alkenyl, alkyl and cycloalkyl, and the rest of $R^8$-$R^{11}$ are each hydrogen;
or any two of $R^8$-$R^{11}$ are attached to form a cycloalkyl, the rest two are each selected from the group consisting of hydrogen, alkyl and cycloalkyl;
$R^{12}$-$R^{13}$ are each selected from the group consisting of hydrogen, alkyl and halogen;
$R^{14}$ is selected from aryl and heteroaryl, wherein the aryl or heteroaryl are optionally substituted with one or more groups selected from the group consisting of hydrogen, halogen, hydroxy, alkyl, alkoxy and cycloalkyl;
y is 1-8, preferably2-5;
PC is ligand; L is a linker.

2. The compound of (PC-L-Dr) or a pharmaceutically acceptable salt or solvate thereof according to claim 1, which is a compound of (PC-L-D) or a pharmaceutically acceptable salt or solvate thereof:

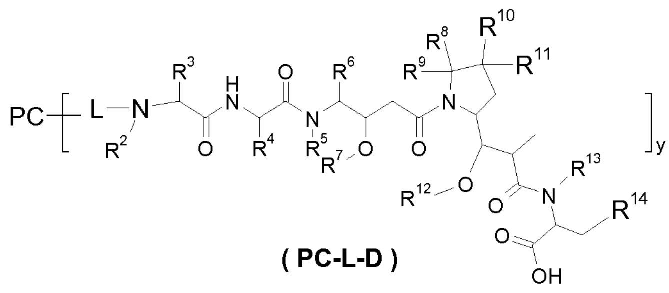

( PC-L-D )

wherein, $R^2$-$R^{14}$ are as defined in claim 1.

**3.** The compound of (PC-L-Dr) or a pharmaceutically acceptable salt or solvate thereof according to claim 1, which is a compound of (PC-L'-Dr) or a pharmaceutically acceptable salt or solvate thereof:

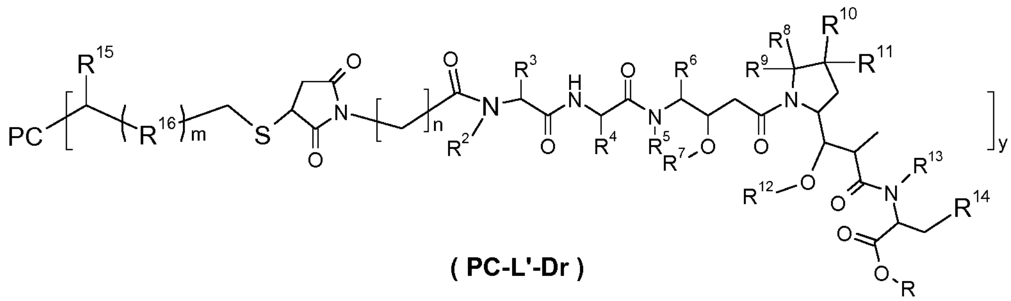

( PC-L'-Dr )

wherein:

$R^{15}$ is selected from the group consisting of hydrogen, halogen, hydroxyl, cyano, alkyl, alkoxy and cycloalkyl;
$R^{16}$ is selected from the group consisting of alkyl, cycloalkyl, alkoxy and heterocyclyl;
n is 2-6, preferably 2-5;
m is 0-5, preferably 1-3;
PC, y, n, R, $R^2$-$R^{14}$ are as defined in claim1.

**4.** The compound of (PC-L-Dr) or a pharmaceutically acceptable salt or solvate thereof according to claim 1, which is a compound of (PC-L'-D) or a pharmaceutically acceptable salt or solvate thereof:

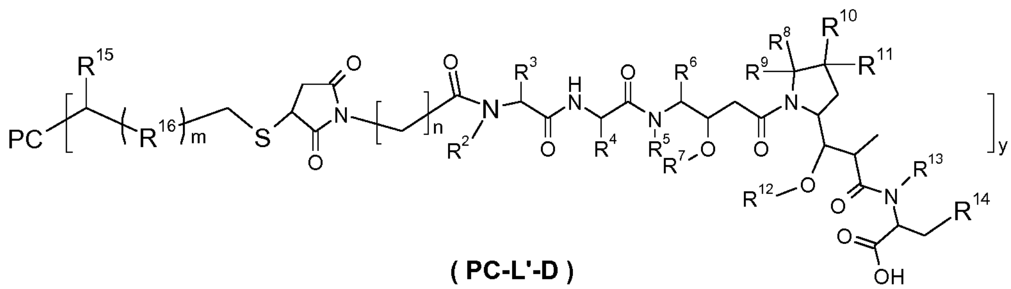

( PC-L'-D )

wherein:

$R^{15}$, $R^{16}$, m are as defined in claim3;
PC, y, n, $R^2$-$R^{14}$ are as defined in claim1.

**5.** The compound of (PC-L'-D) or a pharmaceutically acceptable salt or solvate thereof according to claim 2, which is a compound of (PC-L'-D1) or a pharmaceutically acceptable salt or solvate thereof:

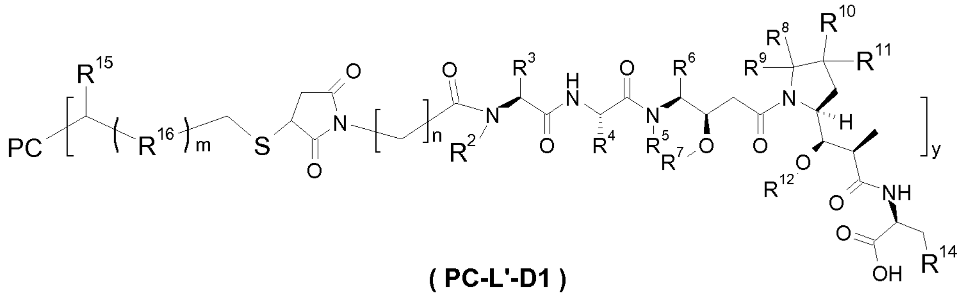

( PC-L'-D1 ) ;

wherein PC, y, n, m, $R^2$-$R^{16}$ are as defined in claim2.

**6.** The compound of (PC-L-Dr) or a pharmaceutically acceptable salt or solvate thereof according to any one of claims 1 to 5, wherein the compound is selected from the group consisting of:

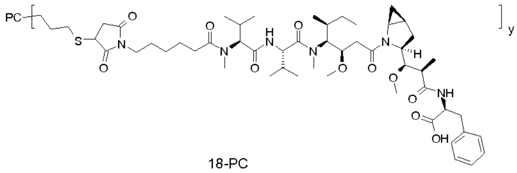

18-PC

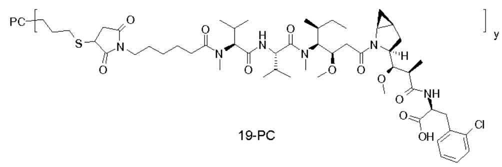

19-PC

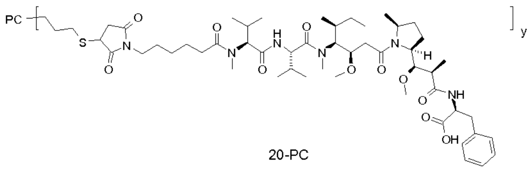

20-PC

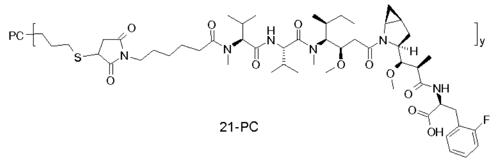

21-PC

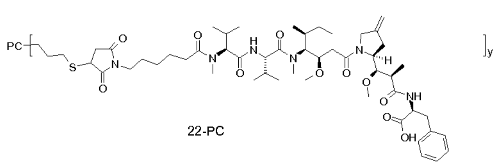

22-PC

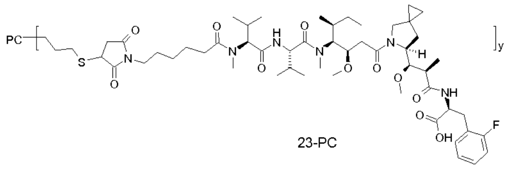

23-PC

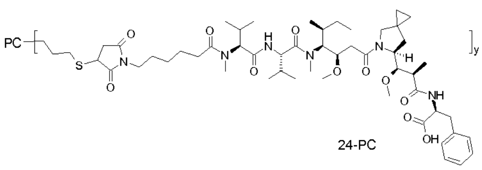

24-PC

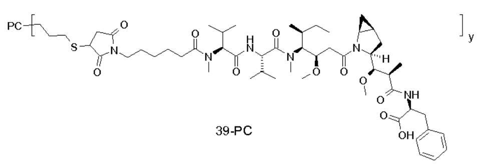

39-PC

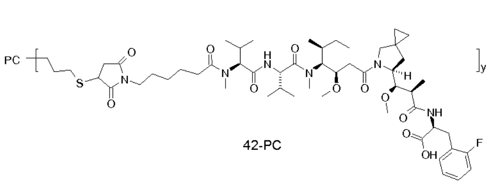

42-PC

and

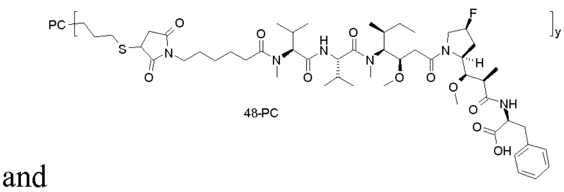

48-PC

;

wherein PC, y are as defined in claim1.

**7.** A ligand-cytotoxic drug conjugate or the pharmaceutically acceptable salt or solvate thereof, which is selected from the group consisting of:

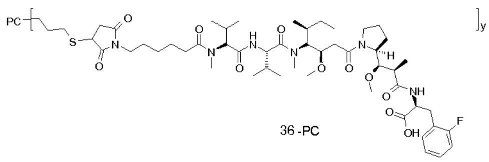

36-PC

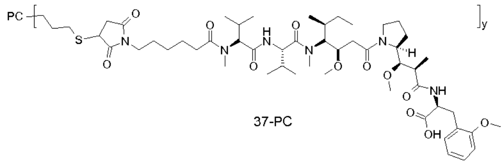

37-PC

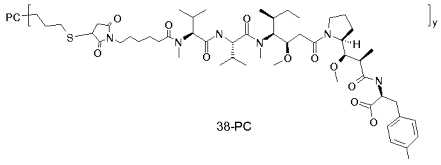

38-PC

and

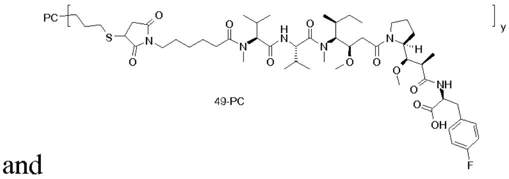

49-PC

;

wherein PC, y are as defined in claim1.

**8.** The compound of (PC-L-Dr) or a pharmaceutically acceptable salt or solvate thereof according to any one of claims 1 to 7, wherein PC is antibody, preferably Pertuzumab, Nimotuzumab or Trastuzumab.

**9.** The compound of (PC-L-Dr) or a pharmaceutically acceptable salt or solvate thereof according to any one of claims 1 to 8, wherein the compound is selected from the group consisting of:

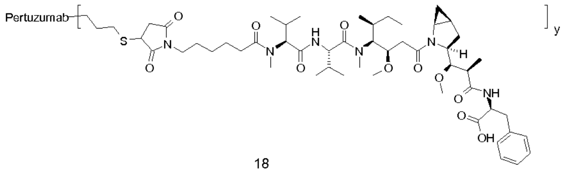

18

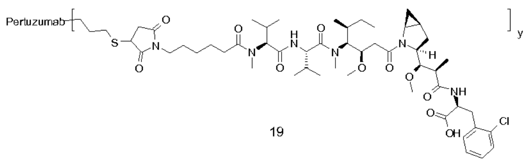

19

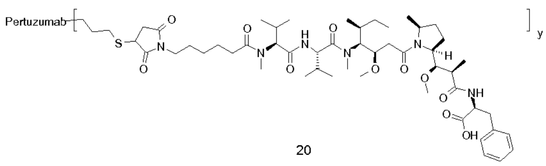

20

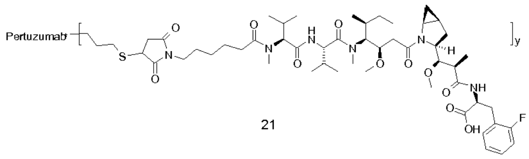

21

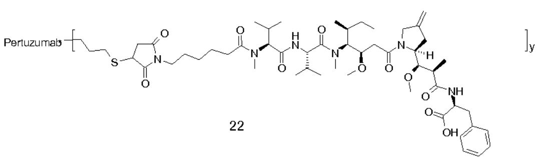

22

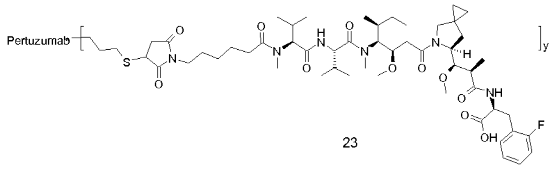

23

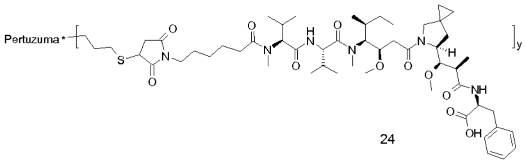

24

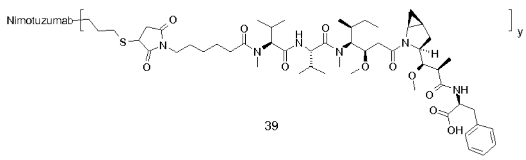

39

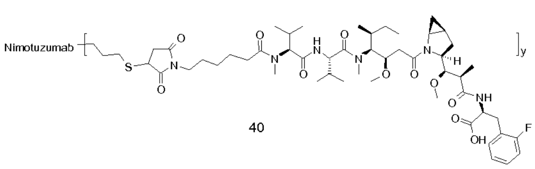

40

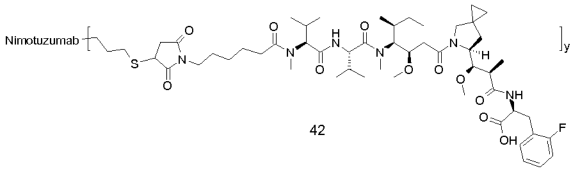

42

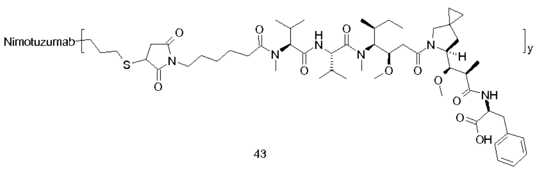

43

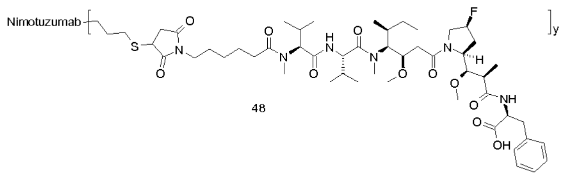

48

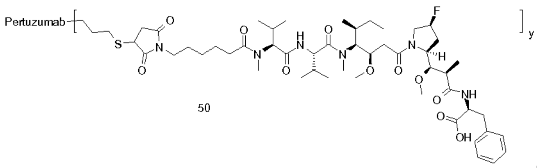

50

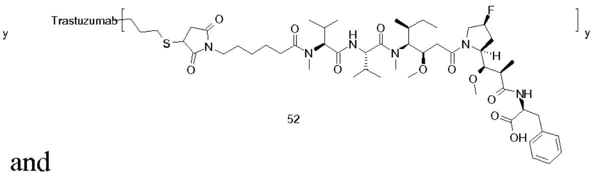

52

and

wherein y is as defined in claim1.

**10.** A ligand-cytotoxic drug conjugate or the pharmaceutically acceptable salt or solvate thereof, which is selected from the group consisting of:

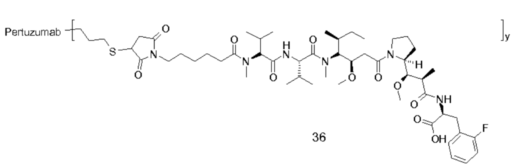

36

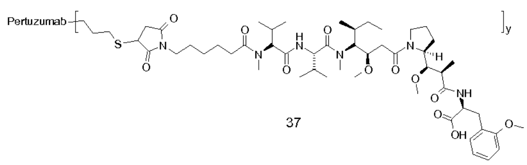

37

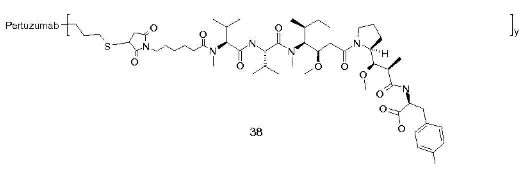

38

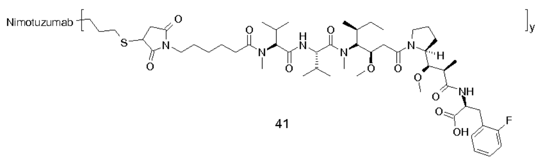

41

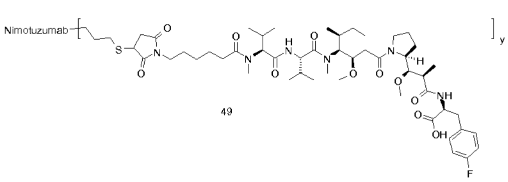

49

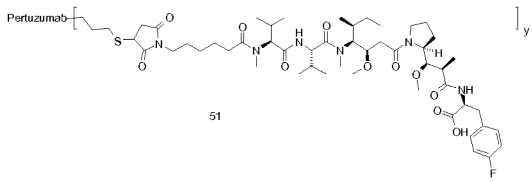

51

and

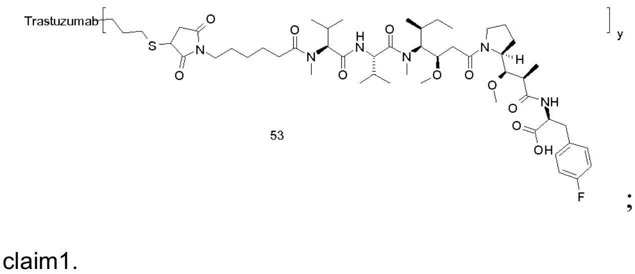

53

;

wherein y is as defined in claim1.

**11.** A compound of formula (Dr):

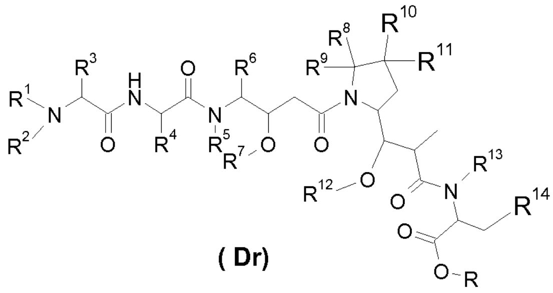

**( Dr)**

or a tautomer, mesomer, racemate, enantiomer, diastereomer, or mixtures thereof, or pharmaceutically acceptable salts thereof,
wherein:

R, $R^1$-$R^7$ are each selected from the group consisting of hydrogen, halogen, hydroxyl, cyano, alkyl, alkoxy and cycloalkyl;
at least one of $R^8$-$R^{11}$ is selected from the group consisting of halogen, alkenyl, alkyl and cycloalkyl, and the rest of $R^8$-$R^{11}$ are hydrogen;
or any two of $R^8$-$R^{11}$ are attached to form a cycloalkyl, the rest two are each selected from the group consisting of hydrogen, alkyl and cycloalkyl;
$R^{14}$ is selected from aryl and heteroaryl, wherein the aryl or heteroaryl are optionally substituted with one or more groups selected from the group consisting of hydrogen, halogen, hydroxy, alkyl, alkoxy and cycloalkyl;
$R^{12}$-$R^{13}$ are each selected from the group consisting of hydrogen, alkyl and halogen.

**12.** The compound of formula (Dr) according to claim 11, which is a compound of formula (D):

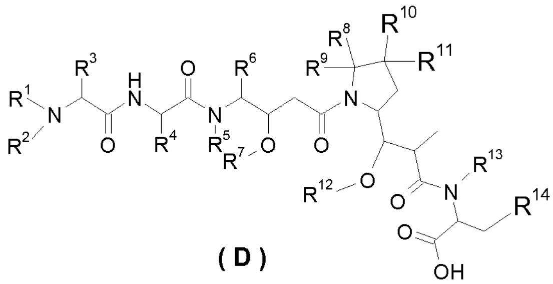

**( D )**

or a tautomer, mesomer, racemate, enantiomer, diastereomer, or mixtures thereof, or pharmaceutically acceptable salts thereof, wherein $R^1$-$R^{14}$ are as defined in claim 11.

**13.** The compound of formula (Dr) according to claim 11, which is a compound of formula (D1):

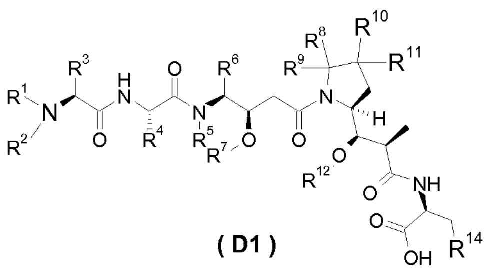

**( D1 )**

or a tautomer, mesomer, racemate, enantiomer, diastereomer, or mixtures thereof, or pharmaceutically acceptable salts thereof, wherein $R^1$-$R^{14}$ are as defined in claim 11.

**14.** The compound of formula (Dr) according to any one of claims 11 to 13, wherein the compound is selected from the group consisting of:

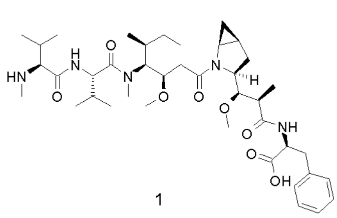
1

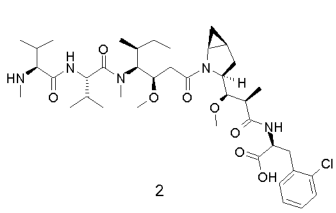
2

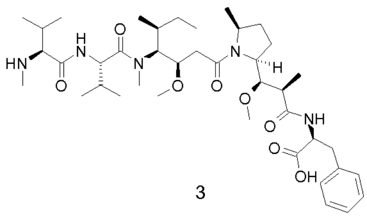
3

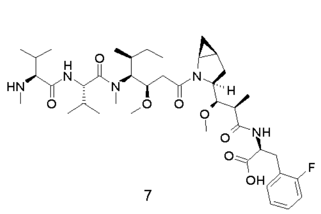
7

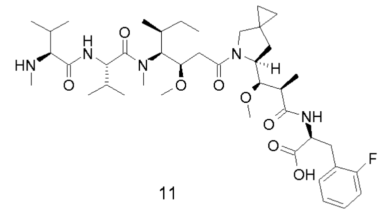
11

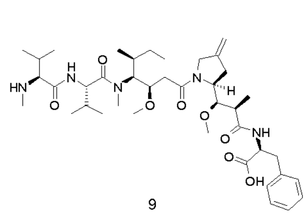
9

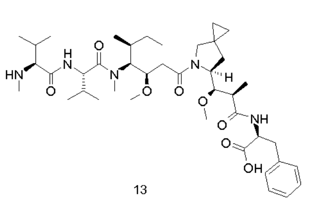
13

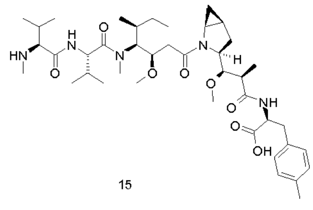
15

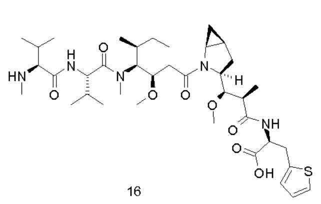
16

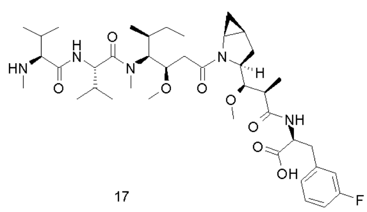
17

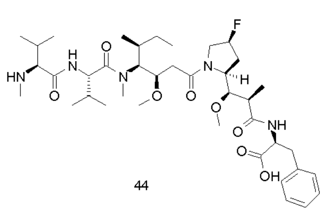
44

.

**15.** A compound which is selected from the group consisting of:

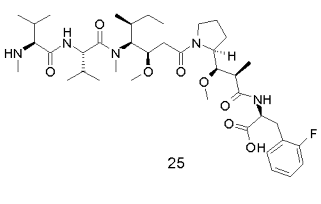
25

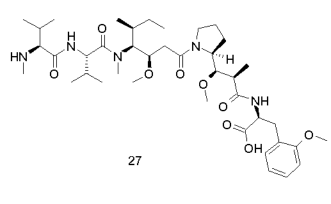
27

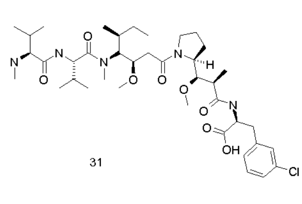
31

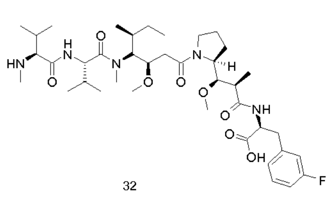
32

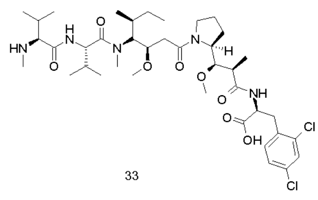
33

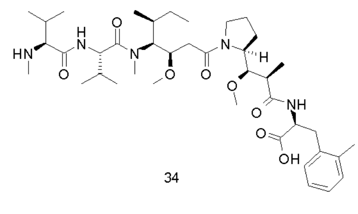
34

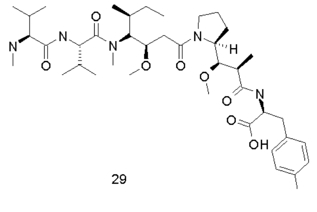
29

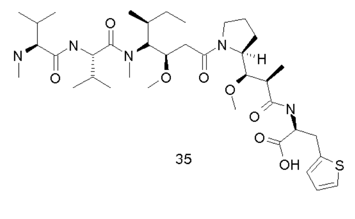
35

and

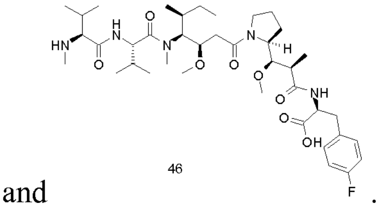
46

.

**16.** A compound of formula ($L_1$-Dr):

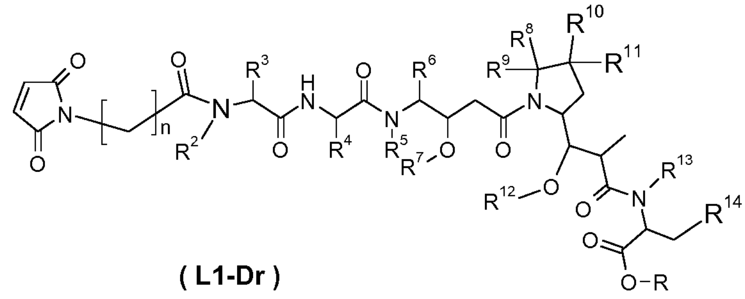

**( L1-Dr )**

wherein:

n is 2-6, preferably 2-5;
R, $R^1$-$R^7$ are each selected from the group consisting of hydrogen, halogen, hydroxyl,cyano, alkyl, alkoxy and cycloalkyl;
at least one of $R^8$-$R^{11}$ is selected from the group consisting of halogen, alkenyl, alkyl and cycloalkyl, and the rest of $R^8$-$R^{11}$ are hydrogen;
or any two of $R^8$-$R^{11}$ are attached to form a cycloalkyl, the rest two are each selected from the group consisting of hydrogen, alkyl and cycloalkyl;
$R^{12}$-$R^{13}$ are each selected from the group consisting of hydrogen, alkyl and halogen,
$R^{14}$ is selected from aryl and heteroaryl, wherein the aryl or heteroaryl are optionally substituted with one or more groups selected from the group consisting of hydrogen, halogen, hydroxy, alkyl, alkoxy and cycloalkyl.

**17.** The compound of (L1-Dr) according to claim 16, which is a compound of (L1-D):

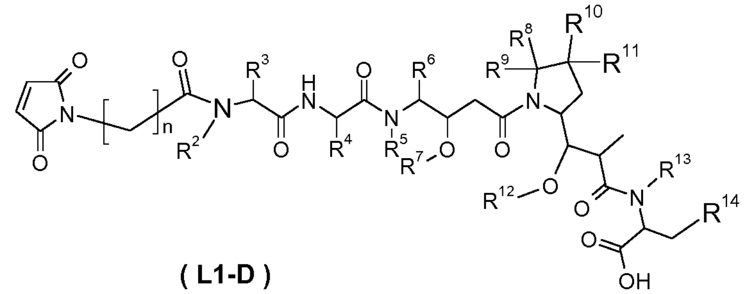

**( L1-D )**

wherein n, $R^2$-$R^{14}$ are as defined in claim 16.

**18.** The compound of formula ($L_1$-D) according to claims 16 or 17, wherein the compound is selected from the group consisting of:

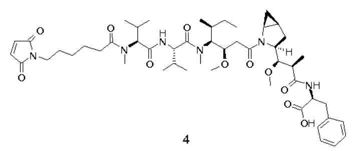

4

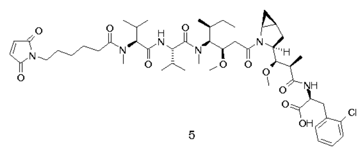

5

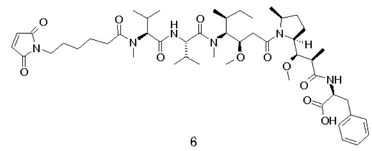

6

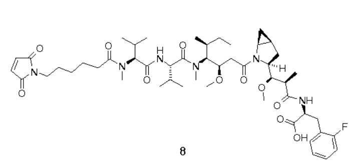

8

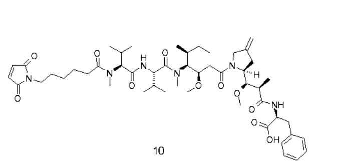

10

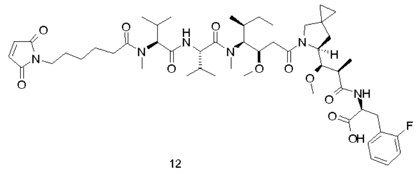

12

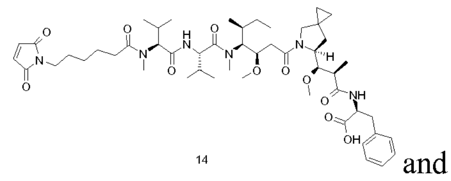

14

and

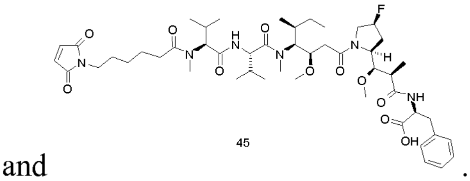

45

.

**19.** An intermediate for the preparation of ligand-cytotoxic drug conjugate, which is selected from the group consisting of:

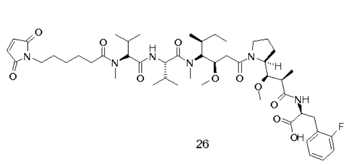

26

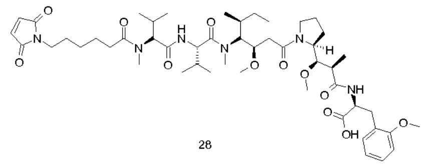

28

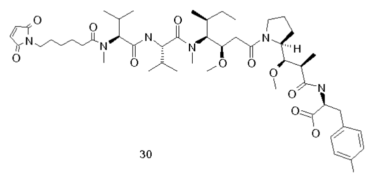

30

and

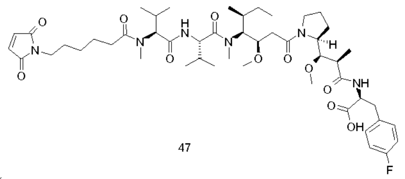

47

.

**20.** A compound of formula (PC-$L_2$):

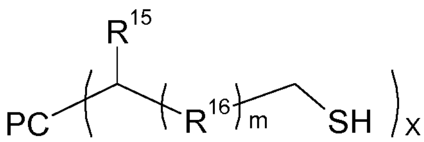

**(PC-L2)**

wherein:

PC is ligand, preferably is antibody, more preferably is Pertuzumab, Nimotuzumab or Trastuzumab;
$R^{15}$ is selected from the group consisting of hydrogen, halogen, hydroxyl, cyano, alkyl, alkoxy and cycloalkyl;
$R^{16}$ is selected from the group consisting of alkyl, cycloalkyl and heterocyclyl;
m is 0-5, preferably 1-3;
X is 0-5, preferably 1-3.

**21.** The compound of formula (PC-$L_2$) according to claim 20, wherein the compound is selected from the group consisting of:

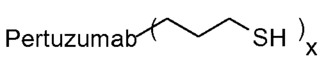

18c

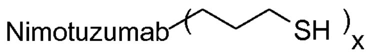

39c

and

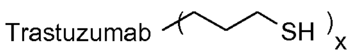

52c

.

**22.** A process for preparing a compound of formula (PC-$L_2$), which comprises the steps of:

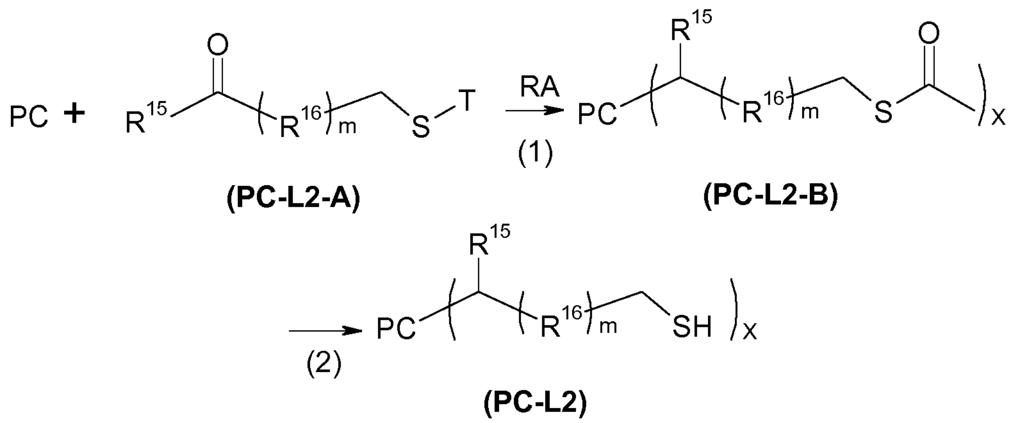


(PC-L2-A)

(PC-L2-B)

(PC-L2)

1) PC and a compound of formula (PC-L2-A) are reacted under a reducing agent RA to give a compound of formula (PC-L2-B); wherein RA is preferably sodium cyanoborohydride or sodium triacetoxyborohydride;
2) a compound of formula (PC-L2-B) is added with a deprotecting agent to remove the protecting group T of the thiol group to give a compound of formula (PC-L2),

wherein T is selected from the group consisting of H, tert-butyl, acetyl, n-propionyl, isopropionyl, triphenylmethyl, methoxymethyl and 2-(trimethylsilyl) ethoxymethyl, preferably H or acetyl;
wherein PC, $R^{15}$, $R^{16}$, m and x are as defined in claim 20.

**23.** A process for preparing a compound of formula (PC-L'-D) according to claim 4, which comprises the steps of:

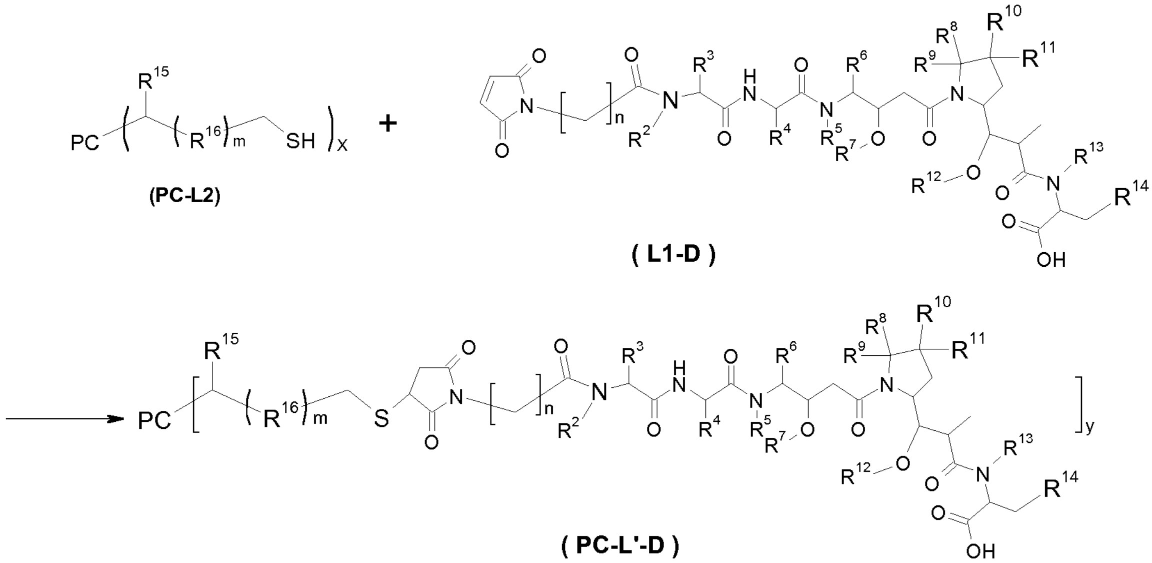

(PC-L2)

( L1-D )

( PC-L'-D )

a compound of formula (PC-L2) is reacted with a compound of formula (L1-D1) to give a compound of formula (PC-L'-D);
wherein PC, m, n, y, $R^2$~$R^{16}$ are as defined in claim 3.

**24.** A compound of formula (D-Aa):

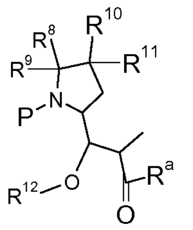
**( D-Aa )**

or a tautomer, mesomer, racemate, enantiomer, diastereomer, or mixtures thereof, or pharmaceutically acceptable salts thereof,
wherein:

any two of $R^8$-$R^{11}$ are attached to form a cycloalkyl, the rest two are optionally selected from the group consisting of hydrogen, alkyl and cycloalkyl;
$R^{12}$ is selected from the group consisting of hydrogen, alkyl and halogen;
P is a hydrogen atom or a protecting group, and the protecting group is preferably Boc, Bn, or Cbz, most preferably Boc;
$R^a$ is selected from the group consisting of hydroxy, amino, alkoxy, cycloalkoxy or alkylamino.

**25.** The compound of formula(D-Aa) according to claim 24, which is a compound of (D-A):

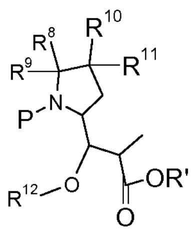
**( D-A )**

or a tautomer, mesomer, racemate, enantiomer, diastereomer, or mixtures thereof, or pharmaceutically acceptable salts thereof, wherein:

P , $R^8$-$R^{12}$ are as defined in claim 24;
R' is selected from the group consisting of hydrogen, alkyl and cycloalkyl.

**26.** The compound of formula (D-A) according to claim 24, wherein the compound is selected from the group consisting of:

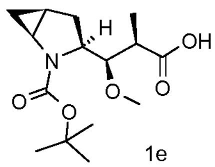
1e

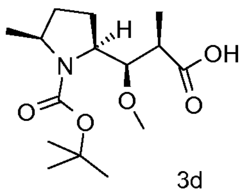
3d

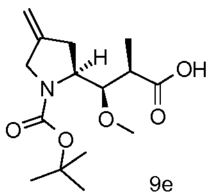
9e

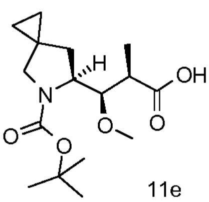
11e

and

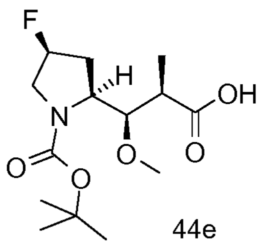
44e

.

**27.** A pharmaceutical composition comprising a therapeutically effective amount of the ligand-cytotoxic drug conjugate

according to any one of claims 1-10, or a drug according to any one of claims 11-19 or a pharmaceutically acceptable salt or solvate thereof, and one or more pharmaceutically acceptable carriers, diluents or excipients.

**28.** The use of the ligand-cytotoxic drug conjugate according to any one of claims 1-10, or a drug according to any one of claims 11-19 or a pharmaceutically acceptable salt or solvate thereof, or a pharmaceutical composition according to claim 27, in the preparation of a medicament for the treatment of cancer, wherein the cancer is associated with HER2, HER3 or EGFR expression.

**29.** The ligand-cytotoxic drug conjugate according to any one of claims 1-10, or a drug according to any one of claims 11-19 or a pharmaceutically acceptable salt or solvate thereof, or a pharmaceutical composition according to claim 27, for use in the preparation of a medicament for the treatment of cancer, wherein the mammal is human, the cancer is selected from the group consisting of breast cancer, ovarian cancer, stomach cancer, endometrial cancer, salivary gland cancer, lung cancer, colon cancer, renal cancer, colorectal cancer, thyroid cancer, pancreatic cancer, prostate cancer, bladder cancer, acute lymphocytic leukemia, acute myeloid leukemia, acute promyelocytic leukemia, chronic myelogenous leukemia, chronic lymphocytic leukemia, Hodgkin's lymphoma, non-Hodgkin's lymphoma and relapsed anaplastic large cell lymphoma, preferably breast cancer, Hodgkin's lymphoma or relapsed anaplastic large cell lymphoma; more preferably breast cancer.

# INTERNATIONAL SEARCH REPORT

International application No.
PCT/CN2016/072129

**A. CLASSIFICATION OF SUBJECT MATTER**

A61K 47/48 (2006.01) i; C07K 16/30 (2006.01) i; A61P 35/00 (2006.01) i

According to International Patent Classification (IPC) or to both national classification and IPC

B. FIELDS SEARCHED

Minimum documentation searched (classification system followed by classification symbols)

A61K, C07K, A61P

Documentation searched other than minimum documentation to the extent that such documents are included in the fields searched

Electronic data base consulted during the international search (name of data base and, where practicable, search terms used)

CNABS; CNKI; DWPI; ISI Web of knowledge and Keywords: 3.1.0, azabicyclo, pyrrolidine, cytotoxic, auristatine, etc.

C. DOCUMENTS CONSIDERED TO BE RELEVANT

| Category* | Citation of document, with indication, where appropriate, of the relevant passages | Relevant to claim No. |
|---|---|---|
| X | CN 104254342 A (SEATTLE GENETICS INC.) 31 December 2014 (31.12.2014) see the entire document, especially claims 1-41 and description, paragraphs [0031] and [0138] | 1-15, 23, 27-29 |
| X | CN 103826661 A (SEATTLE GENETICS INC.) 28 May 2014 (28.05.2014) see the entire document, especially claims 1-52 and description, paragraphs [0015] and [0112] | 1-15, 23, 27-29 |
| A | CN 104245675 A (MERCK PATENT GMBH) 24 December 2014 (24.12.2014) see the entire document | 1-15, 23, 27-29 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 10 April 2014 | 19 April 2014 |

Name and mailing address of the ISA
State Intellectual Property Office of the P. R. China
No. 6, Xitucheng Road, Jimenqiao
Haidian District, Beijing 100088, China
Facsimile No. (86-10) 62019451

Authorized officer
JIA, Tao
Telephone No. (86-10) 62411993

Form PCT/ISA /210 (second sheet) (July 2009)

## INTERNATIONAL SEARCH REPORT

International application No.
PCT/CN2016/072129

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☒ As only some of the required additional search fees were timely paid by the applicant, this international search report covers only those claims for which fees were paid, specifically claims Nos.:
Claims 1-15 (entirely), 23 (entirely), 27-29 (partially)

4. ☐ No required additional search fees were timely paid by the applicant. Consequently, this international search report is restricted to the invention first mentioned in the claims; it is covered by claims Nos.:

**Remark on protest**

☒ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

## INTERNATIONAL SEARCH REPORT

International application No.
PCT/CN2016/072129

**Continuation of: Box No. III**

This International Searching Authority found multiple inventions in this international application, as follows:

Invention 1, claims 1-10 (entirely), 23 (entirely) and 27-29 (partially), directed to compound presented by Formula (PC-L-Dr) or pharmaceutically acceptable salt or solvate thereof, and preparation method, pharmaceutical composition and medicament preparation use thereof;

Invention 2, claims 11-15 (entirely) and 27-29 (partially), directed to compound presented by Formula (Dr) and pharmaceutical composition and medicament preparation use thereof;

Invention 3, claims 16-18 (entirely) and 27-29 (partially), directed to compound presented by Formula (L1-Dr) and pharmaceutical composition and medicament preparation use thereof;

Invention 4, claims 19 (entirely) and 27-29 (partially), directed to preparation of intermediate for the ligand pharmaceutical conjugate of claim 10 and pharmaceutical composition and medicament preparation use thereof;

Invention 5, claims 20-22 (entirely), directed to compound presented by Formula (PC-L2) and preparation method thereof; and

Invention 6, claims 24-26 (entirely), directed to compound presented by Formula (D-Aa).

The compounds of claims 2-6 are different intermediates relating to different part of the compound of claim 1, which is the final product. Those intermediates do not meet the requirements of unity. Furthermore, with respect to the compound of claim 1, the basic structure (parent nucleus) of the formula has been disclosed by prior art (e.g., CN 104254342 A, publication date: 31 December 2014, see claims 1, 7 and 9-12). Therefore, among invention 1 and the intermediate inventions 2-6, there are no special technical feature that make a contribution over the prior art. The inventions can not be considered to form a single general inventive concept, hence does not meet the requirements of unity of invention as defined in Rules 13.1 PCT.

Form PCT/ISA /210 (extra sheet) (July 2009)

**INTERNATIONAL SEARCH REPORT**
Information on patent family members

International application No.
PCT/CN2016/072129

| Patent Documents referred in the Report | Publication Date | Patent Family | Publication Date |
|---|---|---|---|
| CN 104254342 A | 31 December 2014 | AU 2012351685 A1 | 03 July 2014 |
| | | HK 1200714 A1 | 14 August 2015 |
| | | CA 2859255 A1 | 20 June 2013 |
| | | KR 20140114826 A | 29 September 2014 |
| | | IL 233050 D0 | 31 July 2014 |
| | | JP 2015505850 A | 26 February 2015 |
| | | RU 2014128467 A | 10 February 2016 |
| | | EP 2790731 A2 | 22 October 2014 |
| | | AR 089252 A1 | 06 August 2014 |
| | | MX 2014007121 A | 04 September 2014 |
| | | WO 2013087716 A3 | 22 August 2013 |
| | | US 2015023989 A1 | 22 January 2015 |
| | | WO 2013087716 A2 | 20 June 2013 |
| | | SG 11201403085 PA | 30 October 2014 |
| CN 103826661 A | 28 May 2014 | WO 2012143495 A2 | 26 October 2012 |
| | | CA 2833477 A1 | 26 October 2012 |
| | | KR 20140122167 A | 17 October 2014 |
| | | WO 2012143495 A3 | 13 December 2012 |
| | | WO 2012143496 A2 | 26 October 2012 |
| | | MX 2013012253 A | 17 February 2014 |
| | | RU 2013151600 A | 27 May 2015 |
| | | KR 20140122649 A | 20 October 2014 |
| | | US 2013122024 A1 | 16 May 2013 |
| | | WO 2012143497 A2 | 26 October 2012 |
| | | WO 2012143496 A3 | 21 March 2013 |
| | | WO 2012143497 A3 | 21 March 2013 |
| | | CA 2833690 A1 | 26 October 2012 |
| | | JP 2014512375 A | 22 May 2014 |

Form PCT/ISA /210 (patent family annex) (July 2009)

**INTERNATIONAL SEARCH REPORT**
Information on patent family members

International application No.
PCT/CN2016/072129

| Patent Documents referred in the Report | Publication Date | Patent Family | Publication Date |
|---|---|---|---|
| | | WO 2012143499 A2 | 26 October 2012 |
| | | IL 228841 D0 | 31 December 2013 |
| | | AR 086364 A1 | 11 December 2013 |
| | | WO 2012143499 A3 | 13 December 2012 |
| | | AR 086363 A1 | 11 December 2013 |
| | | SG 194567 A1 | 30 December 2013 |
| | | RU 2013151599 A | 27 May 2015 |
| | | JP 2014515753 A | 03 July 2014 |
| | | CN 103764170 A | 30 April 2014 |
| | | AU 2012244673 A1 | 28 November 2013 |
| | | US 2015030618 A1 | 29 January 2015 |
| | | US 2015246136 A1 | 03 September 2015 |
| | | US 8992932 B2 | 31 March 2015 |
| | | US 2013095123 A1 | 18 April 2013 |
| | | AU 2012244675 A1 | 28 November 2013 |
| | | EP 2699268 A2 | 26 February 2014 |
| | | US 2013066055 A1 | 14 March 2013 |
| | | US 2014127240 A1 | 08 May 2014 |
| CN 104245675 A | 24 December 2014 | WO 2013149704 A1 | 10 October 2013 |
| | | EP 2834221 A1 | 11 February 2015 |
| | | US 2015031670 A1 | 29 January 2015 |
| | | PH 12014501964 A1 | 17 November 2014 |
| | | DE 102012006884 A1 | 10 October 2013 |
| | | CA 2869337 A1 | 10 October 2013 |
| | | SG 11201406059 PA | 27 November 2014 |
| | | CL 2014002640 A1 | 16 January 2015 |
| | | JP 2015512426 A | 27 April 2015 |
| | | KR 20150004820 A | 13 January 2015 |

Form PCT/ISA /210 (patent family annex) (July 2009)

## INTERNATIONAL SEARCH REPORT
Information on patent family members

International application No.
PCT/CN2016/072129

| Patent Documents referred in the Report | Publication Date | Patent Family | Publication Date |
|---|---|---|---|
| | | EA 201401081 A1 | 27 February 2015 |
| | | MX 2014011906 A | 12 November 2014 |
| | | AR 090602 A1 | 26 November 2014 |
| | | PE 24522014 A1 | 28 January 2015 |
| | | AU 2013243020 A1 | 20 November 2014 |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007008603 A
- WO 2013173393 A
- WO 2005081711 A
- WO 2013173391 A
- WO 2013173392 A
- WO 2012010287 A
- US 20100249190 A
- WO 2013072813 A
- US 20120195857 A
- WO 2007008848 A


### Non-patent literature cited in the description


- *Journal of the American Chemical Society,* 2003, vol. 125 (50), 15512-15520
- *Tetrahedron: Asymmetry,* 2006, vol. 17 (4), 603-606
- *Journal of the American Chemical Society,* 1940, vol. 62, 565-8
- *Journal of Medcinal Chemistry,* 2013, vol. 56 (24), 9955-9968
- *Advanced Synthesis & Catalysis,* 2012, vol. 354 (17), 3327-3332
- *From Journal of Organic Chemistry,* 2003, vol. 68 (10), 3923-3931
- *Bioorganic & Medicinal Chemistry Letters,* 2013, vol. 23 (9), 2653-2658
- *Organic & Biomolecular Chemistry,* 2004, vol. 2 (18), 2684-2691
- *European Journal of Organic Chemistry,* 2006, 1113-1116
- *Advanced Synthesis & Catalysis,* 2012, vol. 354 (17), 3327-3332
- *Journal of the American Chemical Society,* 2011, vol. 133 (42), 16901-16910
- *Chemical Communications,* 2013, vol. 49 (70), 7744-766
- *International Journal of Peptide & Protein Research,* 1987, vol. 30 (1), 13-21
- *Tetrahedron Letters,* 1999, vol. 40 (36), 6545-6547
- *Tetrahedron: Asymmetry,* 2014, vol. 25 (3), 212-218
- *Journal of Heterocyclic Chemistry,* 2013, vol. 50 (2), 320-325
- *Tetrahedron,* 2003, vol. 59 (21), 3719-3727